# EUROPEAN PATENT APPLICATION

(11) **EP 4 458 977 A1**
(43) Date of publication of application: **06.11.2024**
(21) Application number: 23171438.7
(22) Date of filing: 03.05.2023
(51) Int. Cl.: C12N 15/87, A61K 31/00, B82Y 5/00, C07K 7/00

(54) **SELF-ASSEMBLING PEPTIDE AMPHIPHILES AS RETROVIRAL TRANSDUCTION ENHANCERS**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to self-assembling peptide amphiphiles consisting of a non-self-assembling peptide moiety having a positive net charge under physiological pH, and a hydrophobic moiety covalently coupled to the peptide moiety, wherein the peptide moiety is composed of a positively charged peptide part and a β-sheet forming peptide part, wherein the hydrophobic moiety is covalently coupled to the β-sheet forming peptide part, wherein the peptide amphiphiles self-assemble into fibrils and form µm-sized aggregates in aqueous medium, and wherein the fibrils are efficiently degradable in cells. The peptide amphiphiles of the present invention are useful as viral transduction enhancers for retroviral gene delivery into cells. Thus, the present invention further relates to the use of peptide amphiphiles as retroviral transduction enhancers in retroviral gene delivery into cells, and to methods for retroviral gene delivery into cells in presence of peptide amphiphiles.

## Description

The present invention relates to self-assembling peptide amphiphiles consisting of a non-self-assembling peptide moiety having a positive net charge under physiological pH, and a hydrophobic moiety covalently coupled to the peptide moiety, wherein the peptide moiety is composed of a positively charged peptide part and a β-sheet forming peptide part, wherein the hydrophobic moiety is covalently coupled to the β-sheet forming peptide part, wherein the peptide amphiphiles self-assemble into fibrils and form µm-sized aggregates in aqueous medium, and wherein the fibrils are efficiently degradable in cells. The peptide amphiphiles of the present invention are useful as viral transduction enhancers for retroviral gene delivery into cells. Thus, the present invention further relates to the use of peptide amphiphiles as retroviral transduction enhancers in retroviral gene delivery into cells, and to methods for retroviral gene delivery into cells in presence of peptide amphiphiles.

### Background of the invention

Non-pathogenic viral particles offer promising perspectives as vectors for the delivery of genetic material into cells in the context of gene therapy and vaccines. Lentiviruses and γ-retroviruses are the vectors of choice in fundamental research as well as most clinical trials that are currently underway. As non-replicant vectors with very low inflammatory response, these viruses allow for safe, stable and long-lasting gene expression, which makes them superior compared to other viral vectors. However, inefficient virion-cell attachment and low concentrations of the viral vectors required to prevent cytotoxicity and immunogenic reactions result in inefficient transduction, which remains an unsolved challenge in retroviral gene delivery. To overcome these limitations transduction-enhancing additives from a broad range of material classes, including polymers, lipids, peptides and others, have been applied so far.

Adjuvants made from peptides provide various advantages over other types of materials, such as their biocompatibility, biodegradability and chemical accessibility providing a platform for customized functionalization. Self-assembling peptides enable the construction of functional materials at the nano- and micrometer scale. So far, mostly α-helical and amyloid fibrils have been reported as promising peptide-based adjuvants for retroviral gene delivery. Several studies have confirmed that a positive net charge of the peptide assemblies represents a prerequisite for promoting gene delivery, as it enables binding to the negatively charged nucleic acid or viral particle. Subsequent attachment of the peptide-virus complex to the cell membrane leads to efficient colocalization and eventually to the uptake of the genetic material.

Peptide amphiphiles (PAs) constitute a class of chimeric molecules that consist of a hydrophobic tail and a charged, hydrophilic peptide domain. The hydrophobic domain triggers self-assembly and structure formation in water, which is then stabilized by the charged amino acid domains. In addition, certain amino acid sequences can give rise to ordered β-sheet structures, which results in fibrillar nanostructures with high aspect ratios. In recent decades, the variety of peptide sequences employed in PAs has been greatly expanded, as well as their applications ranging from neural tissue regeneration to drug delivery, antimicrobial systems, peptide vaccines, immunetherapeutic applications and non-viral gene delivery.

Self-assembling peptides which form amyloid fibrils with a positive net charge and a high β-sheet secondary structure have been identified previously as efficient transduction enhancers. However, due to their intrinsic mechanical strength the in vitro degradation and removal of amyloid fibrils is challenging. Ideally, adjuvants for clinical application should increase transduction efficiency and should be easily removed or degraded after successful transduction.

Therefore, it is the objective of the present invention to provide peptide amphiphiles, which self-assemble into fibrils to (I) increase viral transduction efficiency and (II) show high in vitro degradability. It is a further objective of the present invention to provide methods for retroviral gene delivery into cells with the use of peptide amphiphiles that self-assemble into fibrils to (I) increase viral transduction efficiency and (II) exhibit high in vitro degradability.

The objective of the present invention is solved by the teaching of the independent claims. Further advantageous features, aspects and details of the invention are evident from the dependent claims, the description, the figures, and the examples of the present application.

### Brief description of the invention

The present invention relates to peptide amphiphiles consisting of (i) a non-self-assembling peptide moiety having a positive net charge under physiological pH and having an amino acid sequence that gives rise to ordered β-sheet structures, and (ii) a hydrophobic moiety covalently coupled to the peptide moiety, wherein the peptide amphiphiles self-assemble into fibrils (fibrillar nanostructures) in aqueous medium, and wherein the non-self-assembling peptide moiety without the hydrophobic moiety does not form stable fibrillar aggregates in aqueous medium. The peptide amphiphiles of the present invention particularly exhibit increased viral transduction efficiency and high in vitro degradability.

Preferably, the hydrophobic moiety is a fatty acid, so that after degradation of the fatty acid, the resulting peptide moiety readily disintegrates due to the non-self-assembly properties of the amino acid sequence of the non-self-assembling peptide moiety. Thus, the peptide amphiphiles of the present invention have a positive charge and a β-sheet secondary structure and are more efficiently internalized and degraded by cells due to their fatty acid residue.

The present invention relates to an ex-vivo method for retroviral gene delivery into cells comprising the following steps:
a) providing an aqueous medium containing fibrils of a self-assembled peptide amphiphile, wherein the fibrils have a diameter between 5 - 20 nm and a length greater than 100 nm, and wherein the fibrils form µm-sized fibrillar aggregates in the area range between 10 µm² to 1000 µm² in said aqueous medium, wherein the fibrils of the self-assembled peptide forming the µm-sized fibrillar aggregates amphiphile have a zeta-potential of > 5 mV at pH 7.4, and at 298K; and
   the self-assembling peptide amphiphile consisting of:
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
   and
b) subjecting the aqueous medium containing the fibrils of the self-assembled peptide amphiphile to cells in the presence of a non-pathogenic viral particle.

The present invention further relates to a self-assembling peptide amphiphile consisting
(i) a non-self-assembling peptide moiety having a positive net charge under physiological pH, wherein the peptide moiety consist of at least 5 amino acids, wherein less than 50 % of the at least 5 amino acids are lysine, arginine, and histidine;
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid, and
wherein the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm² in said aqueous medium.

### Description of the invention

Surprisingly, it has been found that peptide amphiphiles consisting of (i) a non-self-assembling peptide moiety having a positive net charge under physiological pH and having an amino acid sequence that gives rise to ordered β-sheet structures, and (ii) a hydrophobic moiety covalently coupled to the peptide moiety, wherein the peptide amphiphiles self-assemble into fibrils (fibrillar nanostructures) and form µm-sized aggregates in aqueous medium, and wherein the non-self-assembling peptide moiety without the hydrophobic moiety does not form stable fibrillar aggregates in aqueous medium, solve the above objective. The peptide amphiphiles of the present invention exhibit increased viral transduction efficiency and high in vitro degradability.

The inventors of the present invention have surprisingly found that the introduction of fatty acids to non-active peptides provide advantageous peptide amphiphiles for enhancing retroviral transduction on par with established materials like RetroNectin, Vectofusin-1 and EF-C. It has been found that fibrillar assemblies of peptide amphiphiles with a positive zeta potential serve as highly potent transduction enhancers, while the corresponding peptides without the hydrophobic moiety are inactive, i.e. does not form stable fibrillar aggregates in aqueous medium. Consequently, even non-active peptide sequences may be rendered active by introduction of fatty acid residues, if the resulting assemblies display a fibrillar β-sheet morphology that form µm-sized aggregates with a positive zeta-potential. Surprisingly, it has been further found that the peptide amphiphiles of the present invention are efficiently degradable in the presence of cells, whereas non-inventive peptide amphiphiles having a peptide moiety which also forms stable fibrillar aggregates without the hydrophobic moiety attached remained stable in the presence of cells. Hence, after degradation of the fatty acid, the resulting peptide backbone of non-inventive peptide amphiphiles remained aggregated while assemblies of non-active peptides of the peptide amphiphiles according to the present invention readily disintegrated. Thus, it has been surprisingly found that fibrous peptide amphiphiles (PAs) can enhance infectivity of viruses and be almost completely degraded by cells within 3 days, if the peptide sequence itself does not form fibrils or high aspect nanostructures without any hydrophobic moiety.

The peptide amphiphiles of the present invention are particularly advantageous for use as transduction enhancers in clinical applications, where the persistence of the peptide additive is problematic, particularly if it remains in an aggregated form.

The inventors of the present invention have found that the fibrils formed by peptide amphiphiles exhibit a tendency to form µm-sized aggregates in aqueous medium which has been surprisingly found to further increase the viral transduction efficacy. However, the formation of µm-sized aggregates is in contradiction to the requirements for clinical applications, where it is necessary to avoid that aggregated peptides are transferred to a patient, e.g. in CAR T cell therapy. Thus, in order to benefit from the positive effect of µm-sized aggregates of fibrils on the viral transduction efficacy, it was required to develop advantageous peptide amphiphiles that self-assemble into fibrils and form µm-sized fibrillar aggregates in aqueous medium, but that also can be efficiently degraded by cells. With this approach, a strong transduction enhancement is achieved and it is ensured that no peptide aggregates will be transferred to a patient.

Thus, the peptide amphiphiles of the present invention represent a potent class of transduction enhancers that share the same physicochemical characteristics with previously reported amyloid-like self-assembling peptides, such as the ability to form fibrous aggregates, having a positive zeta-potential and a high degree of intermolecular β-sheets within the assembled structure, but in addition advantageously exhibit high biocompatibility and in particular efficient degradability in the presence of cells. Therefore, the peptide amphiphiles of the present invention are particularly advantageous for ex *vivo* clinical applications to prevent or completely avoid possible pathogenic causalities associated with fibrillar assemblies.

### Definitions

The term **"amino acid",** as used herein, refers to the standard twenty genetically-encoded L-amino acids, and their corresponding stereoisomers in the "D" form, omega-amino acids, other naturally-occurring amino acids, unconventional amino acids, and chemically derivatized amino acids. Herein, the naturally occurring L-amino acids are particularly preferred. The well-known three letter code or single letter code is also used herein to represent the 20 naturally occurring amino acids (table 1).

**Table 1: Notation of the standard twenty genetically-encoded amino acid**

| Amino acid | 3-Letter | 1-Letter | Amino acid | 3-Letter | 1-Letter |
|---|---|---|---|---|---|
| Alanine | Ala | A | Leucine | Leu | L |
| Arginine | Arg | R | Lysine | Lys | K |
| Asparagine | Asn | N | Methionine | Met | M |
| Aspartic acid | Asp | D | Phenylalanine | Phe | F |
| Cysteine | Cys | C | Proline | Pro | P |
| Glutamic acid | Glu | E | Serine | Ser | S |
| Glutamine | Gln | Q | Threonine | Thr | T |
| Glycine | Gly | G | Tryptophan | Trp | W |
| Histidine | His | H | Tyrosine | Tyr | Y |
| Isoleucine | Ile | I | Valine | Val | V |

The term "**charged amino acid**", as used herein, refers to an amino acid having a charged side chain at neutral pH. Charged amino acids include aspartic acid (Asp, D), glutamic acid (Glu, E), arginine (Arg, R), lysine (Lys, K), and histidine (His, H). The term "**negatively charged amino acid**", as used herein, refers to an amino acid having a negative charge at neutral pH. Negatively charged amino acid include aspartic acid (Asp, D), and glutamic acid (Glu, E). The term "**positively charged amino acid**", as used herein, refers to an amino acid having a positive charge at neutral pH. Positively charged amino acid include arginine (Arg, R), lysine (Lys, K), and histidine (His, H). Herein, the positively charged amino acid lysine (Lys, K) is particularly preferred.

The term "**polar amino acid**", as used herein, refers to an amino acid having a polar, uncharged side chain. Polar, uncharged amino acids include serine (Ser, S), threonine (Thr, T), asparagine (Asn, **N**), glutamine (Gln, Q), and tyrosine (Tyr, Y). The term "**nonpolar amino acids**", as used herein, refers to an amino acid having a nonpolar, uncharged side chain. Nonpolar, uncharged amino acids include alanine (Ala, A), isoleucine (Ile, I), leucine (Leu, L), methionine (Met, M), phenylalanine (Phe, F), proline (Pro, P), tryptophan (Trp, W), valine (Val, V), and glycine (Gly, G). The term "**hydrophobic amino acid**", as used herein, refers to an amino acid having a hydrophobic side chain. Hydrophobic amino acids include alanine (Ala, A), cysteine (Cys, C), proline (Pro, P), isoleucine (Ile, I), leucine (Leu, L), methionine (Met, M), phenylalanine (Phe, F), tryptophan (Trp, W), valine (Val, V). The term "**aliphatic amino acid**", as used herein, refers to a nonpolar amino acid having an aliphatic side chain. Aliphatic amino acids include alanine (Ala, A), isoleucine (Ile, I), leucine (Leu, L), methionine (Met, M), and valine (Val, V). The term "**aromatic amino acid**", as used herein, refers to a nonpolar amino acid having an aromatic hydrophobic side chain. Aliphatic hydrophobic amino acids include phenylalanine (Phe, F), tryptophan (Trp, W), and tyrosine (Tyr, Y). The term "**hydrophobic nonpolar amino acid**", as used herein, refers to an amino acid having a hydrophobic and nonpolar side chain. Hydrophobic, nonpolar amino acids include alanine (Ala, A), phenylalanine (Phe, F), isoleucine (Ile, I), leucine (Leu, L), methionine (Met, M), proline (Pro, P), tryptophan (Trp, W), and valine (Val, V). The term "**small amino acid**", as used herein, refers to the amino acids alanine (Ala, A), cysteine (Cys, C), glycine (Gly, G), and serine (Ser, S) (van der Waals volume Å³; 1 Å = 100 pm = 0.1 nm = 10⁻¹⁰ m).

The term "**peptide**", as used herein, refers to a molecule composed of a single chain of D- or L-amino acids or a mixture of D- and L-amino acids covalently coupled through peptide bonds (amide linkages). Herein, peptides composed of L-amino acids are particularly preferred. The term "**peptide sequence**" or "**amino acid sequence**", as used herein, refers to the specific order of amino acids in a peptide. By convention, the "peptide sequence" or "amino acid sequence" is indicated starting from the amino-terminal end (N-terminus) to the carboxyl-terminal end (C-terminus).

The term "**secondary structure**", as used herein, refers to the highly regular local sub-structures on the peptide backbone chain. The two main types of secondary structure are the α-helix and the β-strand or β-sheets. Thus, the term "**β-sheet**", or "**β-sheet secondary structure**", as used herein, refers to the common motif of the regular protein secondary structure well known in the art. Amino acids differ in their ability to form the various secondary structural elements. Amino acids that preferentially adopt helical conformations in proteins include methionine (Met, M), alanine (Ala, A), leucine (Leu, L), glutamic acid (Glu, E), and lysine (Lys, K). The aromatic amino acids phenylalanine (Phe, F), tryptophan (Trp, W), and tyrosine (Tyr, Y), and the branched aliphatic amino acids isoleucine (Ile, I), and valine (Val, V) are known to preferentially adopt β-strand conformations. Therefore, for the provision of an amino acid sequence that gives rise to ordered β-sheet structures, amino acids having non-polar, uncharged side chains are herein preferably selected on the basis of their propensity to form a beta-sheet secondary structures, e.g. may be selected from methionine (Met, M), valine (Val, V), isoleucine (Ile, I), cysteine (Cys, C), tyrosine (Tyr, Y), phenylalanine (Phe, F), glutamine (Gln, Q), leucine (Leu, L), threonine (Thr, T), alanine (Ala, A), glycine (Gly, G). Thereby, the amino acids valine (Val, V) and isoleucine (Ile, I) represent the preferred amino acids for beta-sheet formation, and alanine (Ala, A) and glycine (Gly, G) represent the preferred amino acids having lowest propensity for beta-sheet formation. Glycine (Gly, G), represents the amino acid having the smallest van der Waals volume, and provides more flexibility in a peptide. Alanine (Ala, A) is less flexible than glycine, but small enough to pose only small steric limits. Isoleucine (Ile, I), leucine (Leu, L), and valine (Val, V) have larger aliphatic hydrophobic side chains. These amino acids are more rigid than glycine (Gly, G) and alanine (Ala, A).

The term "**peptide amphiphile**" or "**PA**", as used herein, generally refers to a peptide-based molecule that self-assembles into supramolecular nanostructures including spherical micelles, twisted ribbons, and high-aspect-ratio nanofibers. Peptide amphiphiles are composed of at least two moieties: a hydrophobic moiety and a peptide moiety, wherein the peptide moiety may include a peptide part of beta-sheet-forming amino acids, and a charged peptide part designed to allow solubility in aqueous media. The hydrophobic moiety is a non-peptide molecule, e.g. a hydrophobic alkyl chain which is covalently linked to the peptide moiety of the peptide amphiphile. A peptide amphiphile may have a net charge at physiological pH, either a positive net charge or negative net charge, or may be zwitterionic.

The term "**amyloid peptide**", as used herein, relates to a peptide that is characterized by alternating amphiphilic pattern composed of hydrophilic (e.g. lysine) and aliphatic (e.g. isoleucine or valine) or aromatic (e.g. phenylalanine, tyrosine, tryptophan) amino acids forming cross β-sheet rich amyloid fibrils (nanofibrils), i.e. can also self-assemble into amyloid-like fibrils without an attached hydrophobic moiety. Thus, amyloid peptides have an alternating amphiphilic sequence that leads to self-assembly into rigid, stable cross-β sheet-rich fibrils and have the disadvantage that they are usually degraded slowly by cells.

The terms "**amorphous aggregate**", or "**non-fibrillar aggregate**" as used herein, refer to a solid non-regular morphology with short-range intermolecular ordered interactions but without long-range crystalline-like order. The short-range intermolecular interactions arise from peptide moieties prone for β-sheet formation. An amorphous aggregate has high variability in diameter, which can range from nm to several hundred of nm size range.

The term "**fibril**", as used herein, refers to a solid threadlike morphology with short-range intermolecular and long-range crystalline-like ordered structure caused by the self-assembly of peptide amphiphile molecules in aqueous medium at 298 K, e.g. in water at 298 K. A single fibril has a minimum diameter of about 6 ± 1 nm and preferably a length of at least 100 nm. Thus, a single fibril has a diameter between 5 - 20 nm and length greater than 100 nm. Fibrils may associate with other fibrils to form bundles of fibrils (high-aspect ratio nanostructures).

The term "**high aspect ratio nanostructure**", as used herein, refers to an elongated or threadlike filament having a diameter of less than 100 nanometers. The term "**high aspect ratio**", as used herein, refers to a ratio of length-to-diameter in the range from 5:1 to 20:1 or greater, e.g. a ratio of length-to-diameter of 10:1.

The term "**fibrillar aggregate**", as used herein, refers to fibrils that associate with other fibrils to form bundles of fibrils (high-aspect ratio nanostructures). The term "**µm-sized fibrillar aggregate**", refers to fibrils that associate with other fibrils to form particles with an area in the range 10 µm² - 1000 µm². Preferably, the "µm-sized fibrillar aggregate" are particles with an area of < 50 µm². Thus,peptide amphiphiles of the present invention self-assembles into fibrils having a diameter between 5 - 20 nm and a length greater than 100 nm in said aqueous medium, and wherein the fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have preferablya zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

The term "**physiological conditions**", as used herein, refers to conditions of the external or internal milieu that may occur in nature for that organism or cell system, in contrast to artificial laboratory conditions. For example, a physiological condition can be water with a pH 7.4 and approx. 150 mM sodium chloride between 20-40°C. The term "**physiological pH**", as used herein, refers to the pH range between 7.35 to 7.45, with the average at 7.40.

The terms "**self-assembly**" and "**self-assemble**", as used herein, refer to the process by which a disordered system of preexisting molecules forms an organized structure or pattern as a consequence of specific, local interactions among the molecules themselves, without external guidance. Thus, the terms "self-assemble" and "self-assembly" refer to formation of a discrete, nonrandom, aggregate structure from molecules, wherein assembly occurs spontaneously through random movements of the molecules due only to the inherent chemical or structural properties of those molecules. Amphiphilic molecules (amphiphiles) composed of both hydrophilic and hydrophobic moieties minimize unfavorable interactions in aqueous media via aggregation, which allows the hydrophilic moieties to be exposed to the aqueous environment, and the hydrophobic moieties to be protected from interactions with water. A variety of assemblies can be formed depending on parameters such as concentration, pH, temperature and geometry of the amphiphiles. The assemblies that can be formed by amphiphiles range from micelles to bilayer structures, such as vesicles, as well as fibrils and gels.

The term "**peptide moiety**", as used herein, refers to a peptide component of a peptide-based molecule. An example of a peptide-based molecule is a peptide amphiphile which includes a non-peptide component, e.g. fatty acid residue that is covalently coupled to a peptide. The peptide of the peptide amphiphile is the peptide component of the peptide-based molecule and is herein referred to as the peptide moiety. The term "peptide moiety" is herein also used for the isolated peptide of a peptide amphiphile, thus to a peptide moiety without the attached non-peptide component.

The terms "**self-assembling peptide moiety**" or "**self-assembling peptide**", as used herein, refer to a peptide that consists of both hydrophobic and hydrophilic amino acids and is able to self-assemble without an attached hydrophobic non-peptide moiety in a similar manner as a peptide amphiphile. For example, a "self-assembling peptide moiety" or "self-assembling peptide" can aggregate into high-aspect-ratio structures such as amyloid fibrils by itself without any attached hydrophobic non-peptide moiety. Examples of "self-assembling peptides" include surfactant-like peptides consisting of a hydrophobic tail composed of several consecutive hydrophobic amino acids and a hydrophilic head composed of one or two hydrophilic amino acids, wherein the hydrophilic amino acids may be also charged amino acids. Similar to self-assembling peptide amphiphiles, these "self-assembling peptides" may also form high-aspect to ratio structures such as ribbons, nanotubes, nanofibres and nanorods. A further example of "self-assembling peptides" are amyloid peptides having an alternating amphiphilic sequence that leads to self-assembly into rigid, stable cross-β sheet-rich fibril

The terms "**non-self-assembling peptide moiety**" or "**non-self-assembling peptide**", as used herein, refer to a peptide that consists of both hydrophobic and hydrophilic amino acids, but is not able to self-assemble into fibrils or high aspect nanostructures by itself. However, non-self-assembling peptide moieties may still form disordered aggregates to a certain degree, but according to the present invention, the non-self-assembling peptide moieties do not form stable nondegradable large aggregates in the presence of cells. However, a peptide amphiphile composed of a "non-self-assembling peptide moiety" and a hydrophobic non-peptide moiety can self-assemble into supramolecular nanostructures including spherical micelles, twisted ribbons, and high-aspect-ratio nanofibers due to the covalently attached hydrophobic non-peptide moiety. Thereby, cleavage of the hydrophobic non-peptide moiety, releases the "non-self-assembling peptide moiety" which results in destruction or the dissolution of the previous supramolecular nanostructure. The free "non-self-assembling peptide moiety" or "non-self-assembling peptide" is herein also referred to as "**non-active peptide**".

The term "**hydrophobic moiety**", as used herein, refers to a hydrophobic non-peptide moiety covalently attached to a peptide moiety in a peptide amphiphile, e.g. a hydrocarbon chain, a fatty acid chain, etc. In general, a "hydrophobic moiety" should be long enough to ensure the amphiphilic behavior and fibril formation of the resulting peptide amphiphile in aqueous medium and under physiological conditions. The term "hydrophobic moiety" generally refers to a synthetic or naturally occurring non-peptide molecule that exhibits hydrophobicity. Commonly known hydrophobic moieties include, but are not limited to lipids such as fatty acyls, glycerolipids, glycerophospholipids, sphingolipids, sterol lipids, prenol lipids, saccharolipids, polyketides, fatty acids such as palmitic acid, caprylic acid, myristic acid, stearic acid, arachidic acid, behenic acid, saturated fatty acids, unsaturated fatty acids, monounsaturated fatty acids, or apolyunsaturated fatty acids, straight-chain fatty acids, branched-chain fatty acids, or functionalized fatty acids. Further examples of hydrophobic moieties are aromatic molecules including, but not limited to fluorenylmethyloxycarbonyl (Fmoc) or 2-naphthylmethyl (NAP). Herein, fatty acids are particularly preferred as hydrophobic moiety.

The term "**fatty acid**", as used herein, generally relates to a saturated or an unsaturated monocarboxylic acid. Fatty acids differ in the length of the carbon chain and unsaturated fatty acids further differ in the number, position and configuration of C=C double bonds.

"**Saturated fatty acids**" have the general formula CH₃(CH₂)ₙCOOH, wherein n is a positive integer. A distinction can be made between medium-chain and long-chain saturated fatty acids. Saturated fatty acids with an average length of 6 to 12 carbon atoms are called medium-chain fatty acids, and long-chain fatty acids are fatty acids with a length of 13 to 24 carbon atoms. Examples of medium-length saturated fatty acids are caproic acid (hexanoic acid, C₅H₁₁COOH), enanthic acid (heptanoic acid, C₆H₁₃COOH), caprylic acid (octanoic acid, C₇H₁₅COOH), perlargonic acid (nonanoic acid, C₈H₁₇COOH), capric acid (decanoic acid, C₉H₁₉COOH), undecanoic acid (C₁₀H₂₁COOH) and lauric acid (dodecanoic acid, C₁₁H₂₃COOH). Examples of longlength fatty acids are tridecanoic acid (C₁₂H₂₅COOH), myristic acid (tetradecanoic acid, C₁₃H₂₇COOH), pentadecanoic acid (C₁₄H₂₉COOH), palmitic acid (hexadecanoic acid, C₁₅H₃₁COOH), margaric acid (heptadecanoic acid, C₁₆H₃₃COOH), stearic acid (octadecanoic acid, C₁₇H₃₅COOH), nonadecanoic acid (C₁₈H₃₇COOH), arachidic acid (eicosanoic acid, C₁₉H₃₉COOH), heneicosanoic acid (C₂₀H₄₁COOH), behenic acid (docosanoic acid, C₂₁H₄₃COOH), tricosanoic acid (C₂₂H₄₅COOH), and lignoceric acid (tetracosanoic acid, C₂₃H₄₇COOH).

Herein preferred C₁₄-C₂₄₋saturated fatty acids include, but are not limited to myristic acid (tetradecanoic acid, C₁₃H₂₇COOH), pentadecanoic acid (C₁₄H₂₉COOH), palmitic acid (hexadecanoic acid, C₁₅H₃₁COOH), margaric acid (heptadecanoic acid, C₁₆H₃₃COOH), stearic acid (octadecanoic acid, C₁₇H₃₅COOH), nonadecanoic acid (C₁₈H₃₇COOH), arachidic acid (eicosanoic acid, C₁₉H₃₉COOH), heneicosanoic acid (C₂₀H₄₁COOH), behenic acid (docosanoic acid, C₂₁H₄₃COOH), tricosanoic acid (C₂₂H₄₅COOH), and lignoceric acid (tetracosanoic acid, C₂₃H₄₇COOH).

Particularly preferred are C₁₄-C₂₀₋saturated fatty acids including, but not limited to myristic acid (tetradecanoic acid, C₁₃H₂₇COOH), palmitic acid (hexadecanoic acid, C₁₅H₃₁COOH), stearic acid (octadecanoic acid, C₁₇H₃₅COOH), and arachidic acid (eicosanoic acid, C₁₉H₃₉COOH), even more preferred are palmitic acid (hexadecanoic acid, C₁₅H₃₁COOH), and stearic acid (octadecanoic acid, C₁₇H₃₅COOH).

Palmitic acid (hexadecanoic acid in IUPAC nomenclature) is a fatty acid with a 16-carbon chain. It is the most common saturated fatty acid found in animals, plants and microorganisms. Its chemical formula is CH₃(CH₂)₁₄COOH, and its C:D (the total number of carbon atoms to the number of carbon-carbon double bonds) is 16:0. Palmitic acid has the following structural formula:

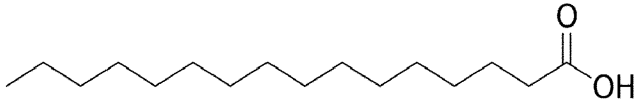

Stearic acid is a saturated fatty acid with an 18-carbon chain. The IUPAC name is octadecanoic acid. It is a waxy solid with the formula CH₃(CH₂)₁₆CO₂H. Stearic acid is one of the most common saturated fatty acids found in nature following palmitic acid. Stearic acid has the following structural formula:

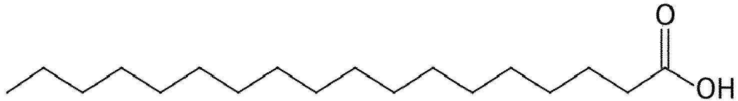

"**Unsaturated fatty acids**" have one or more C=C double bonds in the carbon chain. A double bond may be present in *cis* or *trans* configuration. In cis configuration the two hydrogen atoms adjacent to the double bond are on the same side of the chain, whereas in *trans* configuration the adjacent two hydrogen atoms are on opposite sides of the carbon chain. A *cis*-double bond in a carbon chain causes a bend of the carbon chain and reduces flexibility. As a general rule, the more double bonds the carbon chain has in cis configuration, the more the flexibility is reduced.

"**Mono-unsaturated fatty acids**"" are fatty acids that contain only one C=C double bond in the carbon chain. "**Polyunsaturated fatty acids**" are fatty acids that contain more than one C=C double bond in the carbon chain. In general, fatty acids have a carbon chain with a carboxyl group (-COOH) at one end, and a methyl group (-CH₃) at the other end. The terms "**ω-n**" or "**omega-n**" relates to the well-known nomenclature that classifies unsaturated fatty acids, in particular polyunsaturated fatty acids, by location of a double bond on the n^{th} C-C bond, counting from the methyl group. For example, α-Linolenic acid is classified as omega-3 fatty acid. "**Omega-3 fatty acids**" have a double bond three carbons away from the methyl group, "**omega-6 fatty acids**" have a double bond six carbons away from the methyl group, and "**omega-9 fatty acids**" have a double bond nine carbons away from the methyl group. Polyunsaturated fatty acids with an average length of 16 to 18 carbon atoms are herein called medium-chain polyunsaturated fatty acids, and long-chain polyunsaturated fatty acids are polyunsaturated fatty acids with a length of 20 or more carbon atoms. Fatty acids having two or more cis double bonds that are separated from each other by a single methylene bridge (-CH₂-) are also referred to methylene-interrupted fatty acids. Fatty acids having two or more cis double bonds that are not separated from each other by a single methylene bridge are Conjugated fatty acids. Preferred herein are all-cis-omega-3 and -6 methylene-interrupted fatty acids.

**Table 2: Examples of mono-unsaturated fatty acids**

| **Systematic name** | **Trivial name** | **Short form** | **ω-n** | **trans or cis** |
|---|---|---|---|---|
| cis-9-tetradecenoic acid | myristoleic acid | 14:1(n-5) | ω-5 | *cis* |
| cis-9-hexadecenoic acid | palmitoleic acid | 16:1(n-7) | ω-7 | *cis* |
| cis-6-octadecenoic acid | petroselinic acid | 18:1(n-12) | ω-12 | *cis* |
| cis-9-octadecenoic acid | oleic acid | 18:1(n-9) | ω-9 | *cis* |
| trans-11 -octadecenoic acid | trans-vaccenic acid | 18:1 trans-11 | ω-7 | *trans* |
| cis-11 -octadecenoic acid | cis-vaccenic acid | 18:1(n-7) | ω-7 | *cis* |
| cis-13-eicosenoic acid | paullinic acid | 20:1 (*n*-7) | ω-7 | *cis* |
| cis-9-eicosenoic acid | gadoleic acid | 20:1(n-11) | ω-11 | *cis* |
| cis-11-eicosenoic acid | gondoic acid | 20:1(n-9) | ω-9 | *cis* |
| cis-13-docosenoic acid | erucinic acid | 22:1(n-9) | ω-9 | *cis* |
| cis-15-tetracosenoic acid | nervonic acid | 24:1(n-9) | ω-9 | *cis* |
| trans-9-octadecenoic acid | elaidic acid | 18:1 trans-9 | ω-9 | *trans* |
| t3-hexadecenoic acid | - | trans-16:1(n-13) | ω-13 | *trans* |

Herein preferred monounsaturated fatty acids are C₁₆-C₂₄-cis-omega-n fatty acids including, but not limited to cis-9-octadecenoic acid, cis-9-hexadecenoic acid, cis-11-octadecenoic acid, cis-13-eicosenoic acid, cis-13-eicosenoic acid, cis-11-eicosenoic acid, cis-13-docosenoic acid, and cis-15-tetracosenoic acid.

Particularly preferred are monounsaturated C₁₈-C₂₂-cis-omega-n fatty acids including, but not limited to cis-9-octadecenoic acid, cis-11-octadecenoic acid, cis-13-eicosenoic acid, cis-13-eicosenoic acid, cis-11-eicosenoic acid, and cis-13-docosenoic acid.

**Table 3: Examples of poly-unsaturated fatty acids**

| **Systematic name** | **Trivial name** | **Short form** | **ω-n** | **trans or cis** |
|---|---|---|---|---|
| 9,12-octadecadienoic acid | linoleic acid (LA) | 18:2(n-6) | ω-6 | all-*cis* |
| 6,9,12-octadecatrienoic acid | γ-linolenic acid (GLA) | 18:3(n-6) | ω-6 | all-*cis* |
| octadeca-5,9,12-trienoic acid | pinolenic acid | 18:3 (n-6) | ω-6 | all-*cis* |
| 8E,10E,12E-octadecatrienoic acid | β-calendic acid | 18:3(n-6) | ω-6 | all-*trans* |
| 8Z,10E,12Z-octadecatrienoic acid | jacaric acid | 18:3 (n-6) | ω-6 | 2 *cis*, 1 *trans* |
| 8E,10E,12Z-octadecatrienoic acid | α-calendic acid | 18:3(n-6) | ω-6 | 1 *cis*, 2*trans* |
| 11,14-eicosadienoic acid | - | 20:2(n-6) | ω-6 | all-*cis* |
| 8,11,14-eicosatrienoic acid | dihomo-γ-linolenic acid | 20:3(n-6) | ω-6 | all-*cis* |
| eicosa-5,11,14-trienoic acid | sciadonic acid | 20:3 (n-6) | ω-6 | all-*cis* |
| 5,8,11,14-eicosatetraenoic acid | arachidonic acid (ARA) | 20:4(n-6) | ω-6 | all-*cis* |
| 5Z,8Z,10E,12E,14Z-eicosapentaenoic acid | bosseopentaenoic acid (BPA) | 20:5 (n-6) | ω-6 | 3 *cis,* 2 *trans* |
| 13,16-docosadienoic acid | - | 22:2(n-6) | ω-6 | all-*cis* |
| 7,10,13,16-docosatetraenoic acid | adrenic acid | 22:4(n-6) | ω-6 | all-*cis* |
| 4,7,10,13,16-docosapentaenoic acid | n-6 DPA (osbond acid) | 22:5(n-6) | ω-6 | all-*cis* |
| 9,12,15,18-tetracosatetraenoic acid | - | 24:4(n-6) | ω-6 | all-*cis* |
| 6,9,12,15,18-tetracosapentaenoic acid | - | 24:5(n-6) | ω-6 | all-*cis* |
| 7,10,13-hexadecatrienoic acid | HTA | 16:3(n-3) | ω-3 | all-cis |
| 9,12,15-octadecatrienoic acid | α-linolenic acid (ALA) | 18:3(n-3) | ω-3 | all-*cis* |
| 6,9,12,15-octadecatetraenoic acid | stearidonic acid (SDA) | 18:4(n-3) | ω-3 | all-*cis* |
| 8,11,14,17-eicosatetraenoic acid | ETA | 20:4(n-3) | ω-3 | all-*cis* |
| 5,8,11,14,17-eicosapentaenoic acid | EPA (timnodonic acid) | 20:5(n-3) | ω-3 | all-*cis* |
| 6,9,12,15,18-heneicosapentaenoic acid | HPA | 21:5 (n-3) | ω-3 | all-*cis* |
| 7,10,13,16,19-docosapentaenoic acid | n-3 DPA (clupanodonic acid) | 22:5(n-3) | ω-3 | all-*cis* |
| 4,7,10,13,16,19-docosahexaenoic acid | DHA | 22:6(n-3) | ω-3 | all-*cis* |
| 9,12,15,18,21-tetracosapentaenoic acid | - | 24:5 (*n*-3) | ω-3 | all-*cis* |
| 6,9,12,15,18,21-tetracosahexaenoic acid | nisinic acid | 24:6 (*n*-3) | ω-3 | all-*cis* |
| (9*Z*,11*E*)-octadeca-9,11 -dienoic acid | rumenic acid or bovinic acid | 18:2 (*n*-7) | ω-7 | 1 *cis*, 1 *trans* |
| 5,8,11-eicosatrienoic acid | mead acid | 20:3(n-9) | ω-9 | all-cis |
| 9Z,11E,13E-octadeca-9,11,13-trienoic acid | α-eleostearic acid | 18:3 (n-5) | ω-5 | 1 *cis*, 2 *trans* |
| 9E,11E,13E-octadeca-9,11,13-trienoic acid | β-eleostearic acid | 18:3 (n-5) | ω-5 | all*trans* |
| 9Z,11E,13Z-octadeca-9,11,13-trienoic acid | punicic acid | 18:3 (n-5) | ω-5 | 2 *cis*, 1 *trans* |

Herein preferred polyunsaturated fatty acids are C₁₈-C₂₄-all-cis-omega-n methylene-interrupted fatty acids including, but not limited to *all-cis-*5,8,11,14,17-eicosapentaenoic acid (EPA), *all-cis*-4,7,10,13,16,19-docosahexaenoic acid (DHA), *all-cis*-7,10,13,16,19-docosapentaenoic acid (n-3 DPA), *all-cis-*8,11,14,17-eicosatetraenoic acid (ETA), and *all-cis*-6,9,12,15,18-heneicosapentaenoic acid (HPA), all-cis-9,12,15-octadecatrienoic acid (ALA), *all-cis*-6,9,12,15,-octadecatetraenoic acid (SDA), *all-cis*-9,12,15,18,21-tetracosapentaenoic acid, *all-*cis-6,9,12,15,18,21-tetracosahexaenoic acid, a//-cis-9,12-octadecadienoic acid (LA), a//-cis-6,9,12-octadecatrienoic acid (GLA), a//-cis-11,14-eicosadienoic acid, *all-cis-*8,11,14-eicosatrienoic acid, *all-cis*-5,8,11,14-eicosatetraenoic acid, *all-cis-13,16-*docosadienoic acid, a//-cis-7,10,13,16-docosatetraenoic acid, a//-cis-4,7,10,13,16-docosapentaenoic acid (n-6 DPA), a//-c/s-9,12,15,18-tetracosatetraenoic acid, *all-cis-*6,9,12,15,18-tetracosapentaenoic acid, *all-cis-*5,8,11-eicosatrienoic acid.

Preferred C₁₈-C₂₄-all-cis-omega-3 methylene-interrupted fatty acids include, but are not limited to *all-cis-5,8,11,14,17*-eicosapentaenoic acid (EPA), *all-cis-*4,7,10,13,16,19-docosahexaenoic acid (DHA), *all-cis*-7,10,13,16,19-docosapentaenoic acid (n-3 DPA), *all-cis*-8,11,14,17-eicosatetraenoic acid (ETA), and *all-cis*-6,9,12,15,18-heneicosapentaenoic acid (HPA), *all-cis*-9,12,15-octadecatrienoic acid (ALA), *all-cis*-6,9,12,15,-octadecatetraenoic acid (SDA), *all-cis-*9,12,15,18,21-tetracosapentaenoic acid, *all-cis*-6,9,12,15,18,21-tetracosahexaenoic acid.

Particularly preferred are C₂₀-C₂₂-all-cis-omega-3 methylene-interrupted fatty acids including, but not limited to *all*-*cis*-5,8,11,14,17-eicosapentaenoic acid (EPA), *all-cis-*4,7,10,13,16,19-docosahexaenoic acid (DHA), *all*-*cis*-7,10,13,16,19-docosapentaenoic acid (n-3 DPA), *all*-*cis*-8,11,14,17-eicosatetraenoic acid (ETA), and *all*-*cis*-6,9,12,15,18-heneicosapentaenoic acid (HPA).

Preferred C₁₈-C₂₄-all-cis-omega-6 methylene-interrupted fatty acids include, *all-cis-*9,12-octadecadienoic acid (LA), *all*-*cis*-6,9,12-octadecatrienoic acid (GLA), *all-cis-*11,14-eicosadienoic acid, *all*-*cis*-8,11,14-eicosatrienoic acid, *all*-*cis*-5,8,11,14-eicosatetraenoic acid, *all*-*cis*-13,16-docosadienoic acid, *all*-*cis*-7,10,13,16-docosatetraenoic acid, *all*-*cis*-4,7,10,13,16-docosapentaenoic acid (n-6 DPA), *all-cis-*9,12,15,18-tetracosatetraenoic acid, *all-cis-6,9,12,15,18-tetracosapentaenoic* acid.

Herein preferred are C₂₀-C₂₂-all-cis-omega-6 methylene-interrupted fatty acids including, but not limited to *all*-*cis*-4,7,10,13,16-docosapentaenoic acid (n-6 DPA), *all-cis*-7,10,13,16-docosatetraenoic acid, *all-cis*-5,8,11,14-eicosatetraenoic acid, and *all-*cis-8,11,14-eicosatrienoic acid.

Particularly preferred are *all-cis-5,8,11,14,17-eicosapentaenoic* acid (EPA) and *all-cis*-4,7,10,13,16,19-docosahexaenoic acid (DHA), even more preferred is *all-cis-*5,8,11,14,17-eicosapentaenoic acid (EPA).

Eicosapentaenoic acid (EPA; *all-cis-5,8,11,14,17-eicosapentaenoic* acid) is an omega-3 fatty acid. In physiological literature, it is given the name 20:5(n-3). It also has the trivial name timnodonic acid. In chemical structure, EPA is a carboxylic acid with a 20-carbon chain and five cis double bonds; the first double bond is located at the third carbon from the omega end. Eicosapentaenoic acid has the following structural formula:

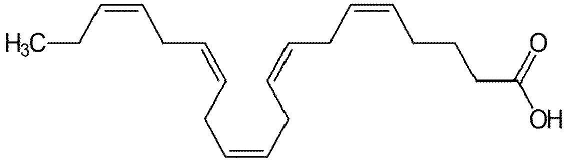

Docosahexaenoic acid (DHA; *all*-*cis*-4,7,10,13,16,19-docosahexaenoic acid) is an omega-3 fatty acid. In physiological literature, it is given the name 22:6(n-3). Docosahexaenoic acid has the following structural formula:

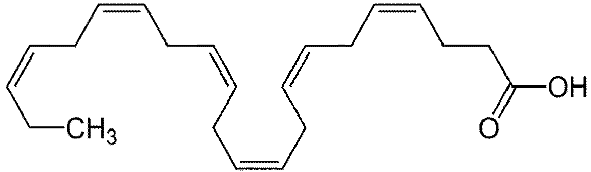

The term "**transfection**", as used herein, relates to the process of deliberately introducing naked or purified nucleic acids into eukaryotic cells. The term transfection is often used to describe nucleic acid-mediated gene transfer into eukaryotic cells.

The terms "**transduction**" or "**viral transduction**", as used herein, relate to the process of introducing foreign genetic material into a cell with a virus or viral vector. Viral transduction is commonly used in the art to enable stable introduction of a gene into the genome of a target cell or cells. The term transduction is often used to describe virus-mediated gene transfer into eukaryotic cells.

The term "**retroviral transduction**", as used herein, refers to the process by which foreign DNA is introduced into a cell by a retrovirus or retroviral vector. Retroviruses have the ability to transform their single-stranded RNA genome into a doublestranded DNA molecule that stably integrates into the genome of dividing target cells. Retroviral transduction has been widely used for cancer and stem cell research.

The term "**gene delivery**", as used herein, relates to the introduction of genetic information into cells to create modified cell lines for biotechnological applications. Thus, gene delivery is the process of introducing foreign genetic material, such as DNA or RNA, into host cells. Gene delivery must reach the genome of the host cell to induce gene expression.

The term "**retroviral gene delivery**", as used herein, relates to the introduction of genetic information into cells to create modified cell lines for biotechnological applications. Retroviral vectors have commonly been used to modify cells for gene therapy. They were derived from natural retroviruses that have evolved gene transfer mechanisms over millions of years. Retroviruses have been classified in various ways but the nomenclature has been standardized in the last decade (see ICTVdB-The Universal Virus Database, v4 at https://ictv.global/). The retroviral vector is derived from the *Retroviridae* family of viruses and can comprise a member of the *Orthoretrovirinae* sub-family, or more typically comprises a retrovirus from a genus selected from *Gammaretrovirus* (e.g MLV), *Lentivirus* (e.g. HIV 1), *Betaretrovirus* (e.g. MMTV), *Alpharetrovirus* (e.g. ALV), *Deltaretrovirus* (e.g. BLV and HTLV-1) and *Epsilonretrovirus* (e.g. wall eye dermal sarcoma virus). For example, vector systems based upon MoMLV (gammaretroviral vectors), and HIV (lentiviral vectors) are often used due to their capacities for efficient gene transfer and their property to integrate into the cell genome, thus allowing a stable genetic modification of the target cell. Successful gene therapy with retroviral vectors requires robust production of appropriately high levels of infectious retroviral particles (referred to as retroviral vector titer), efficient entry of retroviral particles into target cells (proper envelope glycoproteins used to pseudotype retroviral vector particles), and achievement of transgene expression levels that are high enough to elicit a therapeutic effect without undesired toxicity (e.g., due to transgene or retroviral vector insertion into the host cell genome).

The term "**host cell**", as used herein, refers to any cell capable of replicating and/or transcribing and/or translating a heterologous gene. Thus, a "host cell" refers to any eukaryotic or prokaryotic cell (e.g., bacterial cells such as E. coli, yeast cells, mammalian cells, avian cells, amphibian cells, plant cells, fish cells, and insect cells), whether located in vitro or in vivo.

The term "**vector**", as used herein, refers to nucleic acid molecules that transfer DNA from one cell to another. A "**retrovirus vector**" is an infectious virus used to introduce a non-viral gene into mitotic cells in vivo or in vitro.

The terms "**virus**" or "**virus particle**", as used herein, refer to a submicroscopic infectious agent that replicates only inside the living cells of an organism. A non-pathogenic virus or non-pathogenic viral particle relate to a virus or viral particle that does not cause clinical symptoms or diseases. The term "**virion**", as used herein, relates to a single virus particle that is located outside a cell.

The term "**viral transduction enhancer**", as used herein, relates to small molecules used to enhance the viral transduction process and increase target gene expression. Viral transduction is the process of introducing foreign genetic material into a cell with a virus or viral vector and is commonly used by researchers to enable stable introduction of a gene into the genome of a target cell or cells.

The term "**zeta potential**", as used herein, relates to the electrical potential at the slipping plane of colloidal particles measurable by dynamic light scattering. Thus, the zeta-potential characterizes the electrokinetic potential at the slipping plane of colloidal particles and can be measured for self-assembling peptides if they form fibrils (fibrillar aggregates) or amorphous aggregates. The value of "zeta potential", is dependent on the ambient conditions (such as pH value), i.e. it is not a fixed quantity for a substance or a colloid/aggregate. Herein, viral transduction enhancing peptide amphiphiles have a zeta-potential of at least > 5 mV at pH 7.4, 298K, preferably a zeta-potential of at least > 10 mV at pH 7.4, 298K, and particularly preferably a zeta-potential of at least > 15 mV at pH 7.4, 298K.

### Peptide Amphiphiles

In clinical applications, it is of utmost importance to prevent exposure of possibly harmful substances to the patient. Therefore, any adjuvants must be removed or degraded after successful transduction. It has been found that peptide amphiphiles (PAs) are internalized by a variety of cell-lines without affecting cell-viability. The morphology of peptide amphiphile (PA) nanostructures can be destructed via enzymatic cleavage of the fatty acid and the peptide, which enables the creation of degradable transient fibrillar assemblies. Further, PA fibrils can enhance the infectivity by charge driven attachment to and subsequent colocalizations of virions and cells. Thus, peptide amphiphiles (PAs) are highly useful for ex vivo transduction applications, where micrometer sized aggregate formation is still an issue. Up to this point, peptide amphiphiles (PAs) have not been applied as an additive in the context of retroviral transduction in the prior art.

The inventors of the present invention have investigated self-assembling peptide amphiphiles (PAs) for use as retroviral transduction enhancers. Peptide amphiphiles (PAs) with different charges and morphologies were characterized and studied with regards to their ability to enhance the transduction efficiency of retroviral particles. The inventors of the present invention have investigated the impact of the peptide sequence on transduction efficiency and in vitro degradability by attaching palmitic acid to self-assembling and non-self-assembling peptides. The positively charged PA1 (SEQ ID NO. 4) consisting of a non-self-assembling sequence VVVAAAKKK-NH₂ (SEQ ID NO. 1) and attached palmitic acid as hydrophobic residue was identified as a highly efficient adjuvant with less aggregation, improved degradability and an activity comparable to that of the gold standard RetroNectin, Vectofusin-1 and the enhancing factor C (EF-C), that are applied as transduction enhancers in the prior art..

Self-assembling peptides which form amyloid fibrils with a positive net charge and a high β-sheet secondary structure have been identified by the inventors as efficient transduction enhancers. However, due to their intrinsic mechanical strength the in vitro degradation and removal of amyloid fibrils is challenging. Ideally, adjuvants for clinical application should increase transduction efficiency and should be easily removed or degraded after successful transduction.

The inventors of the present invention have selected the non-self-assembling peptides VVVAAAKKK-NH₂ (P1) (SEQ ID NO. 1) and VVVAAAEEE-NH₂ (P3) (SEQ ID NO. 2) and self-assembling peptide backbone FQFKFKC-NH₂ (P2) (SEQ ID NO. 3) as starting material for the attachment of fatty acid residues. The fatty acid conjugates C₁₆-VVVAAAKKK-NH₂ (PA1) (SEQ ID NO. 4) and C₁₆-VVVAAAEEE-NH₂ (PA3) (SEQ ID NO. 5) have been prepared. PA1 (SEQ ID NO. 4) and PA3 (SEQ ID NO. 5) provide β-sheet secondary structures and positively and negatively charged residues at physiologic pH, respectively. Accordingly, the sequences PA1 (SEQ ID NO. 4) and PA3 (SEQ ID NO. 5) have been used to study the effects of charged groups on virions and cells. Further, it has been investigated if the potency of bioactive peptides could be increased by introducing fatty acids. Therefore, peptide amphiphiles (PAs) consisting of a fatty acid as well as a β-sheet forming peptide inspired from the known sequence CKFKFQF (PNF18) (SEQ ID NO. 6) have been designed and studied. CKFKFQF (SEQ ID NO. 6) represents a positively charged self-assembling peptide which acts as a highly efficient transduction enhancer. The conjugation of the palmitic acid to the more hydrophilic N-terminus of the CKFKFQF (SEQ ID NO. 6) peptide yielded C₁₆-CKFKFQF-NH₂ (SEQ ID NO. 7). However, this conjugate had very low solubility in water thus limiting purification. The low solubility is probably a result of attaching the palmitic acid to the more hydrophilic N-terminus of the CKFKFQF (SEQ ID NO. 6) peptide, which interferes with the presentation of hydrophilic groups towards the water phase. Instead, C₁₆-FQFKFKC-NH₂ (PA2) (SEQ ID NO. 8) with a reversed peptide sequence is readily dissolved in water and forms fibrils. The respective peptides without fatty acids (P1-P3) (SEQ ID NO. 1 - 3) were included the investigation to study the impact of the fatty acid tail on transduction efficiency.

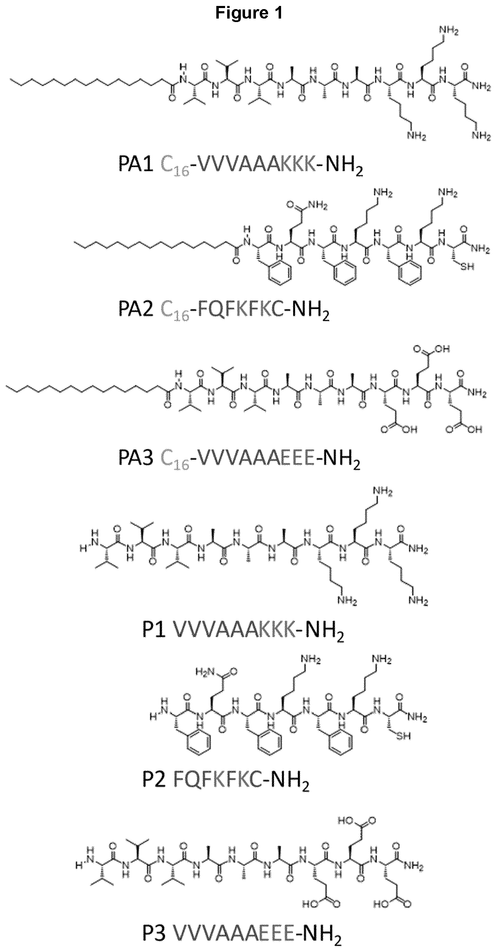

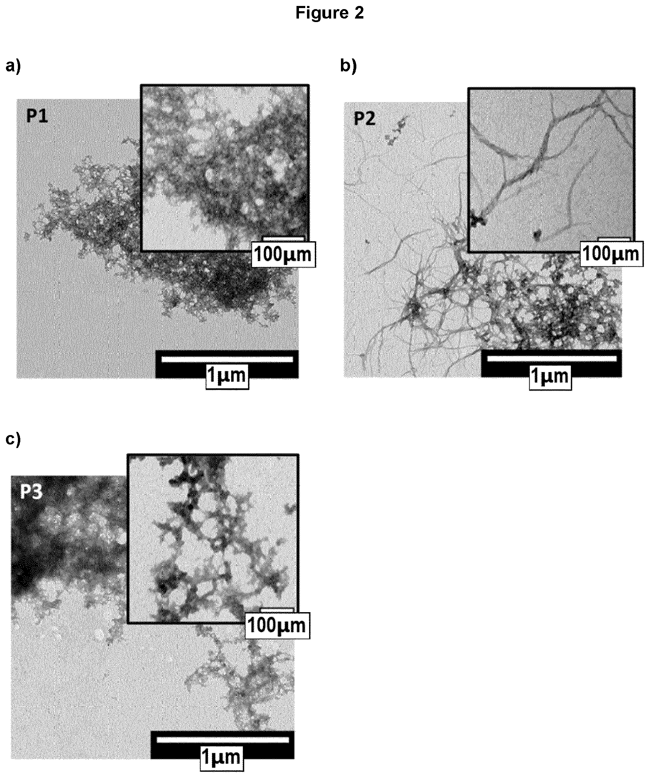

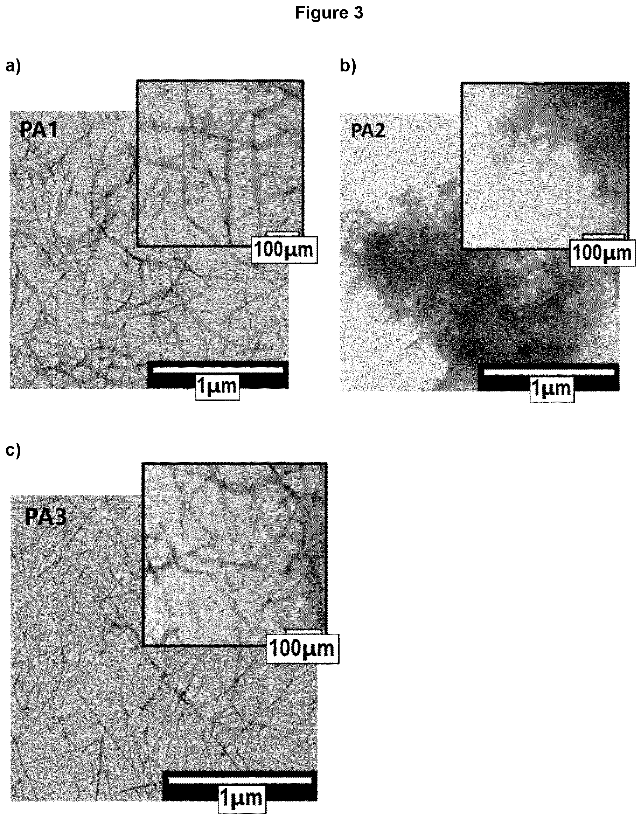

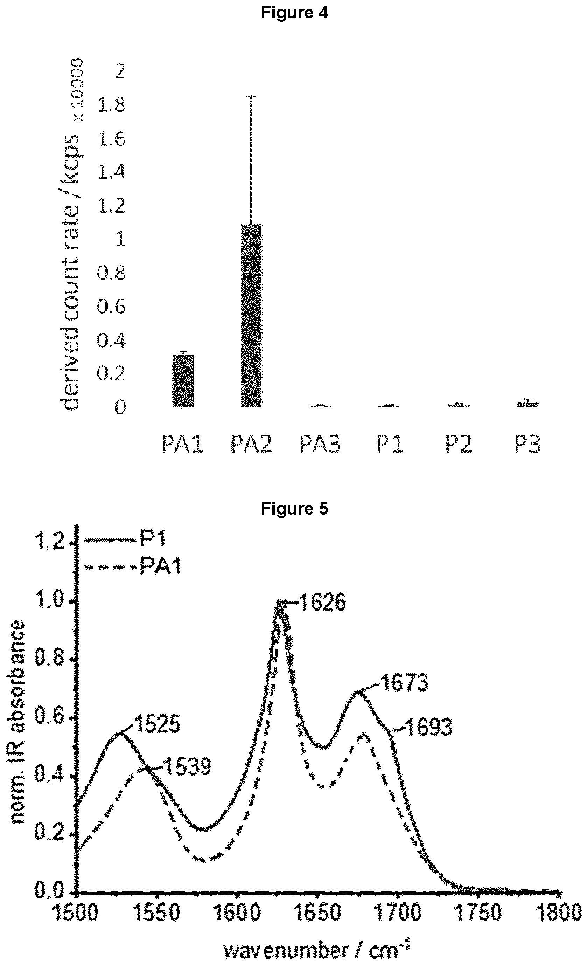

The peptides P1-P3 (SEQ ID NO. 1 - 3) and PA1-PA3 (SEQ ID NO. 4, SEQ ID NO. 5, SEQ ID NO. 8) (Figure 1) were synthesized and then analysed by various methods to assess aggregation and fibril formation. The block-type amphiphilic peptides P1 (SEQ ID NO. 1) and P3 (SEQ ID NO. 2) without the palmitic tail, formed non-fibrillar amorphous aggregates (Figure 2a, 2c), whereas the alternating amphiphilic sequence P2 (SEQ ID NO. 3) assembled into twisted fibrils (Figure 2b) upon dilution from dimethyl sulfoxide (DMSO) stock solution in phosphate-buffered saline (PBS), according to TEM images. All three fatty acid conjugates PA1-PA3 (SEQ ID NO. 4, SEQ ID NO. 5, SEQ ID NO. 8) formed fibrils (Figure 3a-c).The fatty acid conjugates PA1 (SEQ ID NO. 4) and PA2 (SEQ ID NO. 8) assembled into distinct fibrils in water (Figure 3a, 3b), whereas in phosphate-buffered saline (PBS) microscopic clusters of fibrils were formed (Figure 4). Multivalent ions such as phosphate ions are known to interact with basic amino acid residues like lysine causing aggregation, probably through salt bridge formation. Substituting lysine residues with glutamic acid in PA3 (SEQ ID NO. 5) results in distinct fibrils (Figure 3c), which did not bundle or adopt µm-sized aggregates in PBS (Figure 4).

Beside morphologic properties, positive zeta-potential is an essential requirement for highly active transduction enhancers. The zeta-potential characterizes the electrokinetic potential at the slipping plane of colloidal particles and can be measured for self-assembling peptides if they form fibrils or amorphous aggregates. The peptide sequences of PA1 (SEQ ID NO. 4) and PA2 (SEQ ID NO. 8) contain lysine residues that are positively charged at pH 7.4 (physiological pH). PA3 (SEQ ID NO. 5) however, provides negatively charged glutamic acids. As expected, PA1 (SEQ ID NO. 4) and PA2 (SEQ ID NO. 8) displayed a positive zeta-potential while PA3 (SEQ ID NO. 5) showed a negative zeta-potential (table 4). Compared to PA1-PA3(SEQ ID NO. 4, SEQ ID NO. 5, SEQ ID NO. 8), the parent peptides P1-P3 (SEQ ID NO. 1 - 3) displayed a less positive zeta-potential despite having the same peptide sequence. This observation underlines that the zeta-potential is not only dependent on the charged groups within the peptide sequence, but may also be influenced by the size of the assembled structure as well as internal order as previously shown for other peptide amphiphiles PAs.

**Table 4: Tabular summary of physicochemical features of PA1-PA3 and P1-P3 (1 mg/mL, PBS) including zeta-potential, ThT fluorescence (+ = ThT active, - = no ThT fluorescence observed). The β-sheet contents were determined via a quantitative analysis by peak integration of parallel β-sheet at 1630 cm⁻¹ in the amide I region of the 2^{nd} derivative of ATR-IR spectra.**

| Code | Sequence | SEQ ID NO. | Zeta Potential / mV | ThT | β-sheet content / % |
|---|---|---|---|---|---|
| PA1 | C₁₆-VVVAAAKKK-NH₂ | 4 | +22.2 ± 0.7 | + | 57 |
| PA2 | C₁₆-FQFKFKC-NH₂ | 8 | +19.2 ± 3.4 | + | 59 |
| PA3 | C₁₆-VVVAAAEEE-NH₂ | 5 | -29.8 ± 2.7 | + | 61 |
| P1 | VVVAAAKKK-NH₂ | 1 | -2.3 ± 0.4 | - | 48 |
| P2 | FQFKFKC-NH₂ | 3 | +10.1 ± 3.0 | - | 46 |
| P3 | VVVAAAEEE-NH₂ | 2 | -21.2 ± 1.5 | - | 46 |

To gain information on the secondary structure of the assembled peptides, ATR-IR measurements were conducted with a focus on the amide I (1600-1700 cm⁻¹) and amide II (1500-1600 cm⁻¹) regions. The ATR-IR spectrum for P1 (SEQ ID NO. 1) and PA1 (SEQ ID NO. 4) is shown in Figure 5 for illustration purpose, the ATR-IR spectra for P2 (SEQ ID NO. 3), P3 (SEQ ID NO. 2), PA2 (SEQ ID NO. 8), PA3 (SEQ ID NO. 5) are in accordance with the ATR-IR spectrum of Figure 5 and are not shown, but described in the following. Interestingly, β-sheet structural elements were identified for all peptides P1-P3 (SEQ ID NO. 1-3) and PA1-PA3 (SEQ ID NO. 4, 5, 8) with a main peak around 1630 cm-1 which is assigned to parallel β-sheets. Another intense, broad peak in the amide I region was found at approx. 1660-80 cm⁻¹ indicating β-turn structural elements. A small peak at 1694 cm⁻¹ can be assigned to antiparallel β-sheets and was found only for P1-P3 (SEQ ID NO. 1-3), but not in PA1-PA3 (SEQ ID NO. 4, 5, 8). The amide II peak maximum for P1-P3 (SEQ ID NO. 1-3) is located at approx. 1520 cm⁻¹, whereas for PA1-PA3 (SEQ ID NO. 4, 5, 8), it occurs at approx. 1540 cm⁻¹. The changes in the amide I and amide II region indicate the transformation of amorphous aggregates (P1 (SEQ ID NO. 1), P3 (SEQ ID NO. 1-2) to fibrillar assemblies (PA1 (SEQ ID NO. 4), PA3 (SEQ ID NO. 5)) in accordance with TEM measurements. The decrease of the peak at 1694 cm⁻¹ and the shift of the amide II band to higher wavenumbers for PA1-PA3 (SEQ ID NO. 4, 5, 8) can be assigned to increase of parallel β-sheet structures and decrease of antiparallel β-sheet. Similar peak shifts are reported for observations regarding the transition of amorphous aggregates to fibrillar assemblies of Aβ(1-40) and lysozyme for time dependent assembly processes. Further, amorphous aggregates show less β-sheet content compared to fibrillar assemblies. The relative amount of β-sheet secondary structural elements was quantified by integration of the IR absorption bands in the amide I region. Since amino acid side chains can also contribute to the IR absorption in the amide I region, each peptide was only compared to its fatty acid conjugate. The palmitic acid residue does not contribute to the IR absorbance in the amide I region (not shown). A quantitative analysis of the amide I region was executed by integration of the 2^{nd} derivative peak area of the β-sheet (1630 cm⁻¹) and non- ordered or other structural elements (1640-1690 cm⁻¹). According to this, the PA1-PA3 (SEQ ID NO. 4, 5, 8) show a 9-15 % higher β-sheet content compared to their respective parent peptides P1-P3 (SEQ ID NO. 1-3) (not shown).

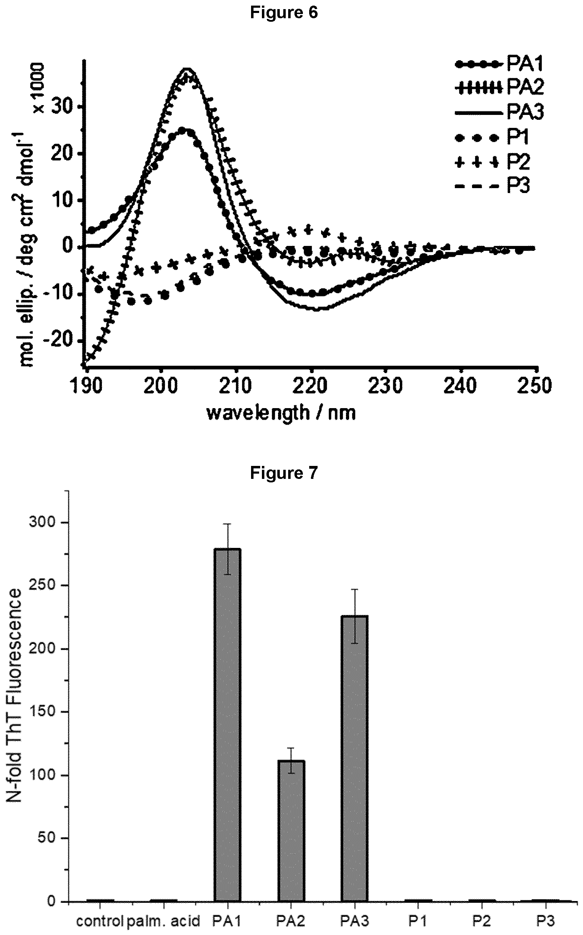

The secondary structure is further confirmed in solution via CD spectroscopy (Figure 6) and ThT fluorescence (Table 2; Figure 7). P1 (SEQ ID NO. 1) and P3 (SEQ ID NO. 2) show CD spectra with characteristics of a random coil structure, whereas the CD spectrum for P2 (SEQ ID NO. 3) could not be assigned to one structural category. By introducing palmitic acid in PA1-PA3 (SEQ ID NO. 4, 5, 8), a predominant β-sheet structure is observed (Figure 6). The CD spectra of PA1 (SEQ ID NO. 4) and PA3 (SEQ ID NO. 4) are in good agreement with literature reports. The fluorescence of ThT upon excitation at 488 nm can only be detected if the molecular rotation of the dye is hindered, that is upon binding to highly ordered structures such as amyloid or β-sheet containing fibrils. As expected, ThT fluorescence could be observed for β-sheet fibril forming PA1-PA3 (SEQ ID NO. 4, 5, 8), but not for P1-P3 (SEQ ID NO. 1-3) (Table 2; Figure 7). Therefore, only PA1-PA3 (SEQ ID NO. 4, 5, 8) form fibrils with high β-sheet content in PBS, which was not observed for the parent peptides P1-P3 (SEQ ID NO. 1-3) without the palmitic acid moiety. Thus, the introduction of the fatty acid residue drives the assembly of the peptides into ordered nanostructures.

The inventors of the present invention have identified PA1 (SEQ ID NO. 4) as potent candidate to enhance viral transduction with improved degradability compared to structural more stable amyloid like peptides, because the (I) all important physicochemical prerequisites for retroviral transduction enhancement are fulfilled and (II) upon degradation of the fatty acid residue the parent peptides P1 (SEQ ID NO. 1) cannot assemble into fibrils.

After the physicochemical characterization, P1-P3 (SEQ ID NO. 1-3) and PA1-PA3 (SEQ ID NO. 4, 5, 8) have been tested as transduction enhancers for HIV-1 on TZM-bl cells, which are well-established and frequently applied model cells for quantifying HIV-1 infectivity. EF-C (QCKIKQIINMWQ) (SEQ ID NO. 9) and PNF18 (CKFKFQF) (SEQ ID NO. 6) served as references. EF-C is a highly potent enhancer similar to other commercially available transduction enhancers such as RetroNectin and Vectofusin-1 serving as gold standards for peptide transduction enhancers. PNF18 is a thoroughly analyzed sequence-optimized version of EF-C showing comparable physicochemical properties and bioactivity.

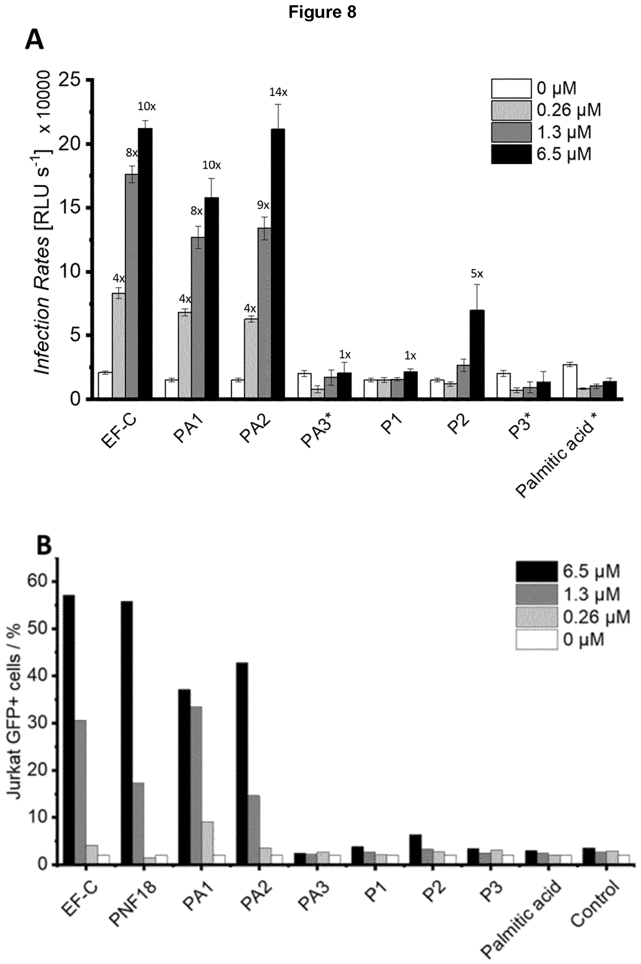

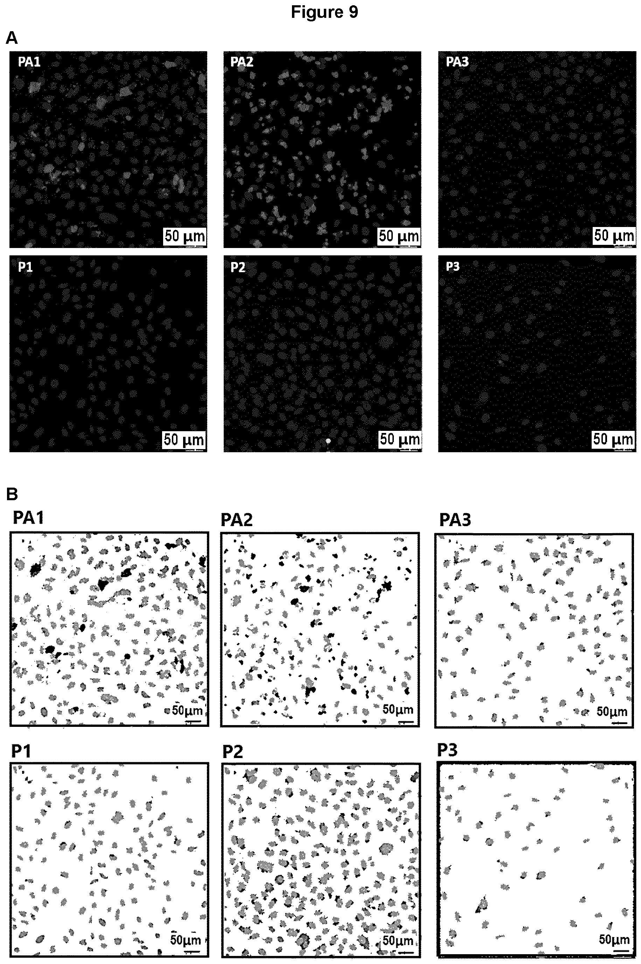

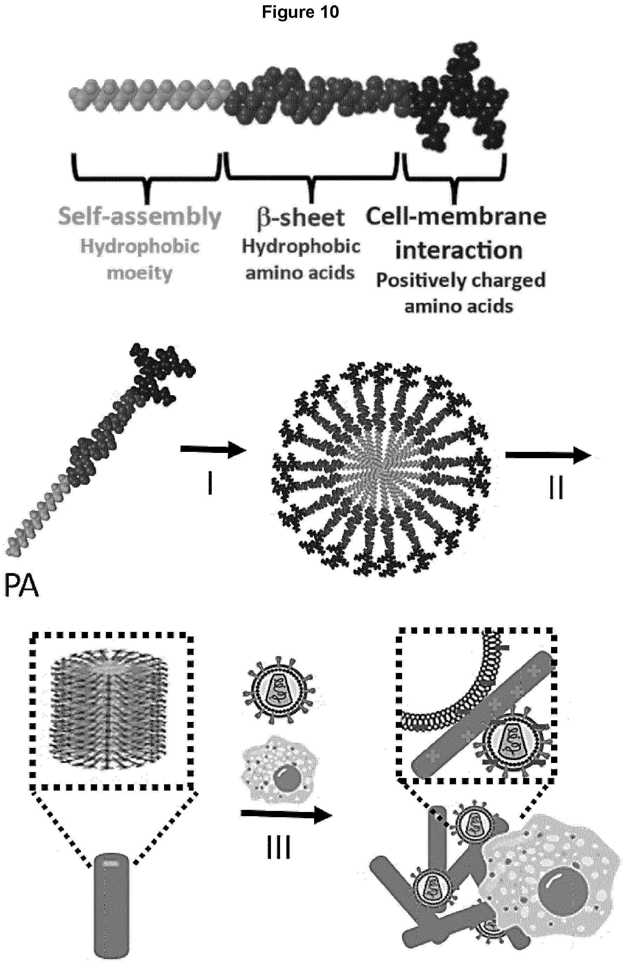

PA1 (SEQ ID NO. 4) and PA2 (SEQ ID NO. 8) show a strong infectivity enhancement of HIV-1 in the range of EF-C and PNF18 (Figure 8a) on TZM-bl cells. The transduction enhancement of PA1 (SEQ ID NO. 4) and PA2 (SEQ ID NO. 8) seems to follow a mechanism analogous to EF-C and PNF18: The positively charged fibrillar aggregates of PA1 (SEQ ID NO. 4) and PA2 (SEQ ID NO. 8) enable efficient interactions with negatively charged viruses and cellular membranes (Figure 10). This is further supported by confocal fluorescence microscopy measurements which visualize the interaction of PA1 (SEQ ID NO. 4) and PA2 (SEQ ID NO. 8) with HeLa cells (Figure 9a,b) and virus-like particles (not shown). Importantly, the parent peptides P1-P3 without fatty-acid residues are inactive (Figure 8a) and did not interact with the cellular membrane (Figure 9a,b). These findings demonstrate that even inactive peptides can be rendered active when a fatty acid residue is introduced driving the self-assembly into fibrils with high β-sheet content. The importance of surface charges as a critical factor for transduction enhancement is demonstrated for PA3 (SEQ ID NO. 5). PA3 (SEQ ID NO. 5) formed β-sheet rich fibrils with a negative zeta-potential and displayed no transduction enhancement (Figure 8a). The negative surface charge prevents efficient cell binding (Figure 9a,b) thereby hindering the colocalization of viral particles with cellular membranes (not shown) in agreement with previous findings for amyloidal peptides. In conclusion, the addition of a fatty acid moiety to peptides can promote the formation of β-sheet rich fibrils which in turn increases their transduction enhancing activity, given that the overall charge is positive.

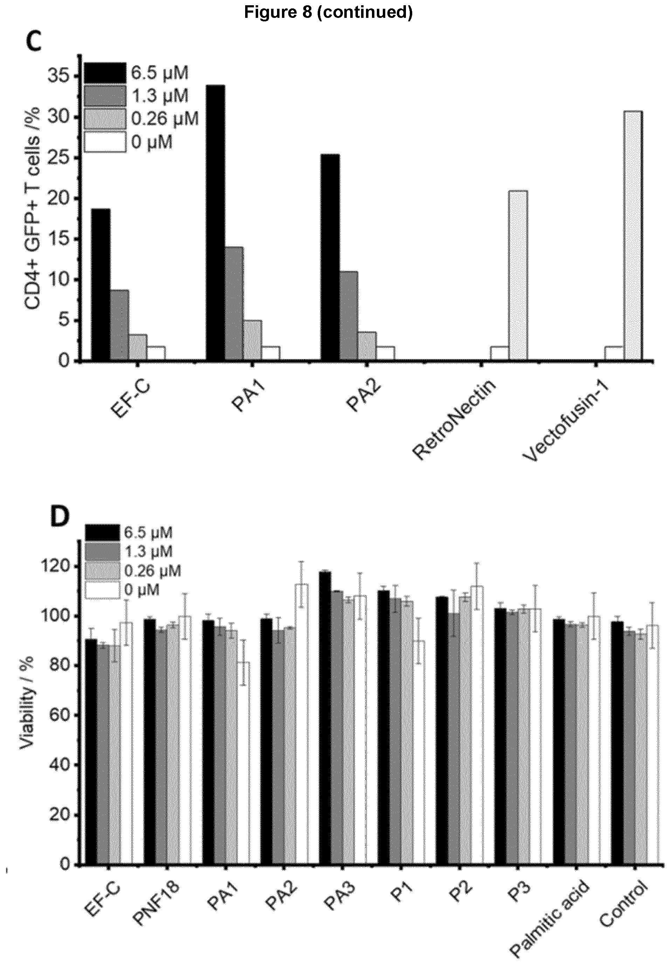

Retroviral vectors have commonly been used for the stable genetic modification of target cells for gene therapy. For instance, CAR T cell therapy is one of the most promising applications of gene therapy and was recently approved for patient use. An indispensable but challenging step in the engineering process of the T-cells is the efficient introduction of therapeutic genes via retroviral transduction. Therefore, we evaluated the effect of P1-P3 (SEQ ID NO. 1-3) and PA1-PA3 (SEQ ID NO. 4, 5, 8) on the transduction of Jurkat cells, a T-cell line frequently applied for studying leukemia, with a γ-retroviral vector pseudotyped with the glycoprotein derived from gibbon ape leukemia virus (*abbr. GaLV* = *G*ibbon *a*pe *L*eukemia *V*irus) to investigate their potential use for gene therapy applications. As expected for this cell-type, without a PA additive almost no transduced GFP+ cells were detected (Figure 8b). However, with an increasing concentration of PA1 (SEQ ID NO. 4) and PA2 (SEQ ID NO. 8) strong transduction enhancement similar to EF-C was achieved. Strikingly, PA1 (SEQ ID NO. 4) and PA2 (SEQ ID NO. 8) showed activity in the range of commercially available gold-standard enhancers RetroNectin and Vectofusin-1 (Figure 8c) also on hard-to-transduce clinically relevant CD4+ T cells, demonstrating that PAs are powerful transduction enhancers.

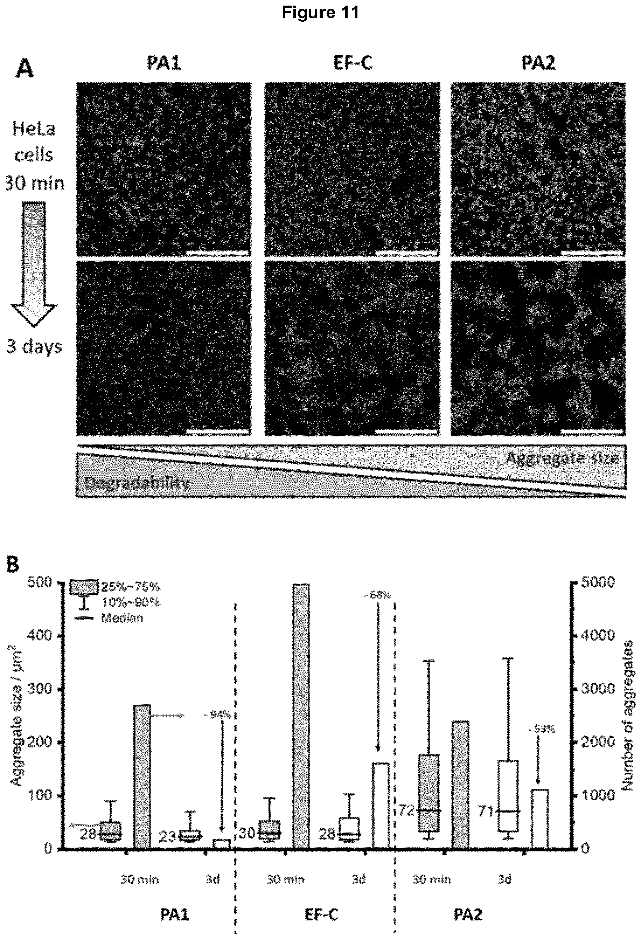

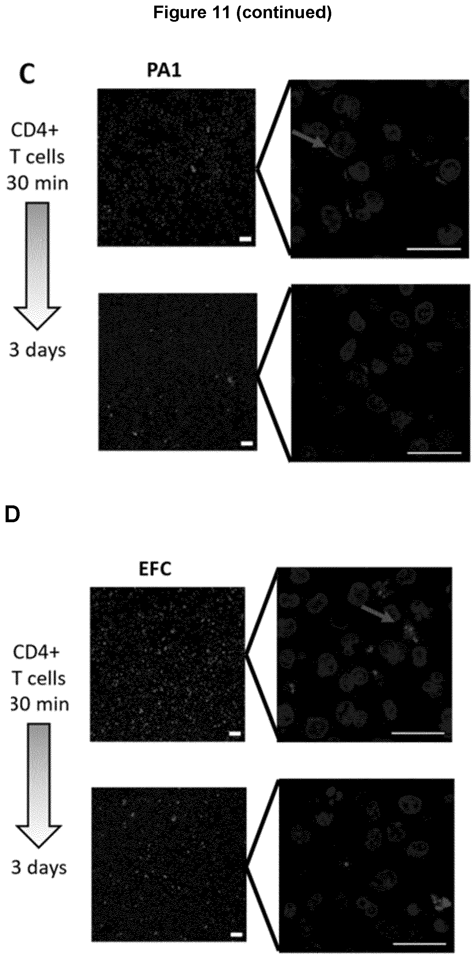

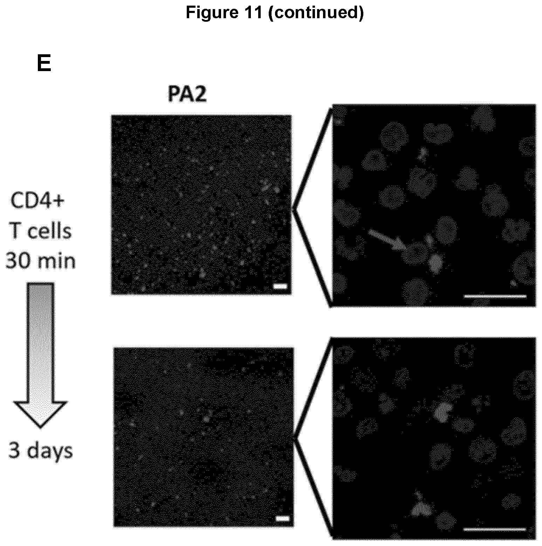

The peptide nanostructures EF-C, PNF18, PA1 (SEQ ID NO. 4) and PA2 (SEQ ID NO. 8) investigated by the inventors as well as other commercially available peptides for transduction efficiency enhancement such as Vectofusin-1 form µm-sized aggregates (Figure 9a,b). While aggregate formation can be an obstacle in clinical applications it does not appear to affect cell viability (Figure 8d). To avoid possible complications upon re-administration of e.g. transduced T-cells the reduction of aggregates (> 10 µm²) is highly desired for clinical applications. Interestingly, PA1 (SEQ ID NO. 4) forms less and smaller aggregates compared to EF-C and PA2 (SEQ ID NO. 8), which could be well degraded (94%) over the course of 3 days in the presence of HeLa cells (Figure 11a, 11b) as well as CD4+ T cells (Figure 11c-e). In contrast, PA2 (SEQ ID NO. 8) forms larger µm-sized aggregates, which remain nearly unchanged in size after 3 days incubation and are not degraded as efficiently (53%) in the presence of HeLa cells (Figure 11b) and CD4+ T cells (Figure 11c-e) In general, amyloid fibrils with high β-sheet content are usually not degraded well by cells. However, PAs can be internalized by cells which may be facilitated by interactions of the fatty acids with cellular membranes. As assemblies of PA1 (SEQ ID NO. 4) and PA2 (SEQ ID NO. 8) are likely degraded by cleavage of the fatty acid residue in cells, the difference in degradability can be traced back to the different self-assembling properties of the parent peptides P1 (SEQ ID NO. 1) and P2 (SEQ ID NO. 3), respectively. The efficient cellular degradation for PA1 (SEQ ID NO. 4) is further emphasized in control experiments without cells where PA1 (SEQ ID NO. 4), PA2 (SEQ ID NO. 8) and EF-C are not significantly changing in size and number over the course of 3 days (not shown). In contrast to P1 (SEQ ID NO. 1), P2 (SEQ ID NO. 3) forms fibrils (Figure 2a). Thus, after cleavage of the fatty acid, fibril formation and microscopic aggregation of P2 (SEQ ID NO. 3) could hinder degradation as observed here. PA1 (SEQ ID NO. 4) forms less aggregates and is degraded more rapidly *in vitro,* which distinguishes it from amyloid-like peptide fibrils, e.g. EF-C or a PA consisting of amyloid-peptide sequence such as PA2 (SEQ ID NO. 8), which show in general higher *in vitro* stability (Figure 11).

Thus, the inventors of the present invention have investigated the effect of the secondary order, morphology, and charge of peptide amphiphiles as additives for promoting retroviral transduction efficiency. The introduction of fatty acids to non-active peptides represents an advantageous approach to provide beneficial PAs for enhancing retroviral transduction on par with established materials like RetroNectin, Vectofusin-1 and EF-C. Thus, the inventors of the present invention could demonstrate that fibrillar assemblies of the peptide amphiphiles of PA1 (SEQ ID NO. 4) and PA2 (SEQ ID NO. 8) with a positive zeta potential serve as highly potent transduction enhancers, while the corresponding peptides without palmitic acid P1 (SEQ ID NO. 1) and P2 (SEQ ID NO. 3) are inactive. Consequently, even non-active sequences may be rendered active by introduction of fatty acid residues, if the resulting assemblies display a fibrillar β-sheet morphology, forms µm-sized aggregates with a positive zeta-potential. Surprisingly, PA1 (SEQ ID NO. 4) could be degraded in the presence of cells efficiently, whereas those of PA2 (SEQ ID NO. 8) remained stable. This can be explained by considering that the unfunctionalized peptide P2 (SEQ ID NO. 3) forms stable fibrillar aggregates and P1 (SEQ ID NO. 1) does not. Hence, after degradation of the fatty acid, the resulting peptide backbone P2 (SEQ ID NO. 8) remains aggregated while assemblies of P1 (SEQ ID NO. 1) readily disintegrate. Thus, PA1 (SEQ ID NO. 4) is a highly interesting candidate as a transduction enhancer in clinical applications, where the persistence of the peptide additive in the form of aggregates is problematic.

In summary, PAs that have block-type sequence design with hydrophobic and cationic charged amino acids, i.e. in PA1 (SEQ ID NO. 4) form moderately sized aggregates that are attaching to cellular membranes, deliver viruses and are biodegradable. Replacing cationic amino acids with anionic amino acids, i.e. in PA3 (SEQ ID NO. 5) prevent cellular attachment and are not suitable for retroviral gene delivery. Changing the sequence design from block-type to an alternating amphiphilic sequence design with aromatic, hydrophobic amino acids results in PAs that form large aggregates (> 50 µm², *i.e*. PA2 (SEQ ID NO. 8)), which can increase virus uptake but that cannot be fast and efficiently degraded by the cells. It has been surprisingly found that a certain degree of aggregation is required to efficiently interact with cells and viruses, the size of the aggregates is therefore an important factor to facilitate biodegradability.

Thus, the present invention relates to peptide amphiphiles consisting of (i) a non-self-assembling peptide moiety having a positive net charge under physiological pH and having an amino acid sequence that gives rise to ordered β-sheet structures, and (ii) a hydrophobic moiety covalently coupled to the peptide moiety, wherein the peptide amphiphiles self-assemble into fibrils having a diameter between 5 - 20 nm and a length greater than 100 nm and form µm-sized fibrillar aggregates in the area range between 10 µm² to 1000 µm² in aqueous medium, and wherein the non-self-assembling peptide moiety without the hydrophobic moiety does not form fibrils and/or µm-sized fibrillar aggregates in aqueous medium.

Thus, the following requirements are preferably fulfilled by the peptide moiety of the peptide amphiphiles of the present invention:
- the peptide moiety itself does not self-assemble into fibrils / µm-sized fibrillar aggregates e.g. at an applied concentrations of <6.5 µM;
- the peptide moiety has a positive net charge positive, thus contains positively charged amino acids (e.g. H, K, R), preferably one or more lysines;
- the peptide moiety preferably contains amino acids known to be prone to form beta-sheet structure (e.g. V, A) and
- the peptide moiety is not cytotoxic;

However, peptide moieties / peptides that can be induced to form fibrillar assemblies at high concentrations (e.g. alternating amphiphilic sequence) are also possible to provide suitable peptide amphiphiles as retroviral transduction enhancers, with the proviso that these peptide moieties / peptides do not form such fibrillar assemblies at lower concentration, e.g. at an applied concentrations of <6.5 µM). Furthermore, peptide moieties / peptides having a sequence that corresponds to cell penetrating peptides and other endogenous peptides, which do not self-assemble into fibrils are also possible herein, e.g. in case these peptide moieties / peptides do not form fibrillar assemblies at lower concentrations, e.g. at an applied concentrations of <6.5 µM).

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid.

Preferably, the terminal amino acid of the first peptide part is selected from lysine or lysine amide.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine, wherein at least two of the at least 5 amino acids are lysine, arginine, and/or histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amid;
   (ib) a β-sheet forming second peptide part consisting of at least 3 amino acids, wherein a first end of the β-sheet forming second peptide part is covalently coupled to the first amino acid of the first peptide part, and
(ii) a hydrophobic moiety covalently coupled to the second end of the β-sheet forming second peptide part, wherein the hydrophobic moiety is selected from the group comprising or consisting of a C₁₄-C₂₄-fatty acid.

Preferably, the terminal amino acid of the first peptide part is selected from lysine or lysine amide.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine, wherein at least two of the at least 5 amino acids are lysine, arginine, and/or histidine, and at least one amino acid is selected from lysine or lysine amid; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine;
   (ib) a β-sheet forming second peptide part consisting of at least 3 amino acids, wherein a first end of the β-sheet forming second peptide part is covalently coupled to the first amino acid of the first peptide part, and
(ii) a hydrophobic moiety covalently coupled to the second end of the β-sheet forming second peptide part, wherein the hydrophobic moiety is selected from the group comprising or consisting of a C₁₄-C₂₄-fatty acid.

Preferably, the terminal amino acid of the first peptide part is selected from lysine or lysine amide.

Thus, the present invention preferably relates to a self-assembling peptide amphiphile consisting of:
(i) a non-self-assembling peptide moiety having a positive net charge under physiological pH, wherein the peptide moiety consist of at least 5 amino acids;
   wherein less than 50 % of the at least 5 amino acids are lysine, arginine, and histidine; and
   wherein the non-self-assembling peptide moiety further comprises:
      (ia) a first peptide part consisting of 1 to 3 amino acids, wherein the amino acid at the N-terminus of the first peptide part is selected from lysine, arginine, and histidine, and wherein at least one of the 1 to 3 amino acids is selected from lysine or lysine amide;
      (ib) a β-sheet forming second peptide part consisting of at least 3 amino acids, wherein the C-terminus of the second peptide part is covalently coupled to the N-terminus of the first peptide part, and
(ii) a hydrophobic moiety covalently coupled to the N-terminus of the second peptide part, wherein the hydrophobic moiety is selected from the group comprising or consisting of a C₁₄-C₂₄-fatty acid.

Preferably, the amino acid at the C-terminus of the first peptide part is selected from lysine or lysine amide, more preferably lysine amide.

Thus, the present invention preferably relates to a self-assembling peptide amphiphile consisting of:
(i) a non-self-assembling peptide moiety having a positive net charge under physiological pH, wherein the peptide moiety consist of at least 5 amino acids; wherein less than 50 % of the at least 5 amino acids are lysine, arginine, and histidine; and wherein at least one amino acid is selected from lysine or lysine amid; and
   wherein the non-self-assembling peptide moiety further comprises:
   (ia) a first peptide part consisting of 1 to 3 amino acids, wherein the amino acid at the N-terminus of the first peptide part is selected from lysine, arginine, and histidine,
   (ib) a β-sheet forming second peptide part consisting of at least 3 amino acids, wherein the C-terminus of the second peptide part is covalently coupled to the N-terminus of the first peptide part, and
(ii) a hydrophobic moiety covalently coupled to the N-terminus of the second peptide part, wherein the hydrophobic moiety is selected from the group comprising or consisting of a C₁₄-C₂₄-fatty acid.

Preferably, the amino acid at the C-terminus of the first peptide part is selected from lysine or lysine amide, more preferably lysine amide.

The non-self-assembling peptide moiety of the peptide amphiphiles of the present invention preferably consists of at least 5 amino acids, preferably wherein at least two of the at least 5 amino acids are selected from lysine, arginine, and histidine, more preferably, wherein at least two of the at least 5 amino acids are lysine, and wherein less than 50 % of the at least 5 amino acids are selected from lysine, arginine, and histidine.

Thus, the present invention preferably relates to a self-assembling peptide amphiphile consisting of:
(i) a non-self-assembling peptide moiety having a positive net charge under physiological pH, wherein the peptide moiety consist of at least 5 amino acids; wherein less than 50 % of the at least 5 amino acids are lysine, arginine, and histidine, wherein at least two of the at least 5 amino acids are lysine, arginine, and histidine; and
   wherein the non-self-assembling peptide moiety further comprises:
   (ia) a first peptide part consisting of 1 to 3 amino acids, wherein the amino acid at the N-terminus of the first peptide part is selected from lysine, arginine, and histidine, and wherein at least one of the 1 to 3 amino acids is selected from lysine or lysine amide;
   (ib) a β-sheet forming second peptide part consisting of at least 3 amino acids, wherein the C-terminus of the second peptide part is covalently coupled to the N-terminus of the first peptide part, and
(ii) a hydrophobic moiety covalently coupled to the N-terminus of the second peptide part, wherein the hydrophobic moiety is selected from the group comprising or consisting of a C₁₄-C₂₄-fatty acid.

Preferably, the amino acid at the C-terminus of the first peptide part is selected from lysine or lysine amide, more preferably lysine amide.

Thus, the present invention preferably relates to a self-assembling peptide amphiphile consisting of:
(i) a non-self-assembling peptide moiety having a positive net charge under physiological pH, wherein the peptide moiety consist of at least 5 amino acids; wherein less than 50 % of the at least 5 amino acids are lysine, arginine, and histidine, wherein at least 2 of the at least 5 amino acids are lysine, arginine, and histidine, and wherein at least one amino acid is selected from lysine or lysine amid; and
   wherein the non-self-assembling peptide moiety further comprises:
   (ia) a first peptide part consisting of 1 to 3 amino acids, wherein the amino acid at the N-terminus of the first peptide part is selected from lysine, arginine, and histidine;
   (ib) a β-sheet forming second peptide part consisting of at least 3 amino acids, wherein the C-terminus of the second peptide part is covalently coupled to the N-terminus of the first peptide part, and
(ii) a hydrophobic moiety covalently coupled to the N-terminus of the second peptide part, wherein the hydrophobic moiety is selected from the group comprising or consisting of a C₁₄-C₂₄-fatty acid.

Preferably, the amino acid at the C-terminus of the first peptide part is selected from lysine or lysine amide, more preferably lysine amide.

Thus, the present invention preferably relates to a self-assembling peptide amphiphile consisting of:
(i) a non-self-assembling peptide moiety having a positive net charge under physiological pH, wherein the peptide moiety consist of at least 5 amino acids; wherein less than 50 % of the at least 5 amino acids are lysine, arginine, and histidine, preferably wherein at least two of the at least 5 amino acids are selected from lysine, arginine, and histidine; and
   wherein the non-self-assembling peptide moiety further comprises:
   (ia) a first peptide part consisting of 1 to 3 amino acids, wherein the amino acid at the N-terminus of the first peptide part is selected from lysine, arginine, and histidine, and wherein at least one of the 1 to 3 amino acids is selected from lysine or lysine amide;
   (ib) a β-sheet forming second peptide part consisting of at least 3 amino acids, wherein the C-terminus of the second peptide part is covalently coupled to the N-terminus of the first peptide part, and
(ii) a hydrophobic moiety covalently coupled to the N-terminus of the second peptide part, wherein the hydrophobic moiety is selected from the group comprising or consisting of a C₁₄-C₂₄-fatty acid.

Preferably, the amino acid at the C-terminus of the first peptide part is selected from lysine or lysine amide, more preferably lysine amide.

Thus, the present invention preferably relates to a self-assembling peptide amphiphile consisting of:
(i) a non-self-assembling peptide moiety having a positive net charge under physiological pH, wherein the peptide moiety consist of at least 5 amino acids;
   wherein less than 50 % of the at least 5 amino acids are selected from lysine, arginine, and histidine, wherein at least one of amino acids is selected from lysine or lysine amide; and preferably wherein at least two of the at least 5 amino acids are selected from lysine, arginine, and histidine, and
   wherein the non-self-assembling peptide moiety further comprises:
      (ia) a first peptide part consisting of 1 to 3 amino acids, wherein the amino acid at the N-terminus of the first peptide part is selected from lysine, arginine, and histidine;
      (ib) a β-sheet forming second peptide part consisting of at least 3 amino acids, wherein the C-terminus of the second peptide part is covalently coupled to the N-terminus of the first peptide part, and
(ii) a hydrophobic moiety covalently coupled to the N-terminus of the second peptide part, wherein the hydrophobic moiety is selected from the group comprising or consisting of a C₁₄-C₂₄-fatty acid.

Preferably, the amino acid at the C-terminus of the first peptide part is selected from lysine or lysine amide, more preferably lysine amide.

Thus, the present invention preferably relates to a self-assembling peptide amphiphile consisting of:
(i) a non-self-assembling peptide moiety having a positive net charge under physiological pH, wherein the peptide moiety consist of at least 5 amino acids; wherein less than 50 % of the at least 5 amino acids are lysine, arginine, and histidine; and
   wherein the non-self-assembling peptide moiety further comprises:
   (ia) a first peptide part consisting of 1 to 3 amino acids, wherein the amino acid at the C-terminus of the first peptide part is selected from lysine, arginine, and histidine, and wherein at least one of the 1 to 3 amino acids is selected from lysine or lysine amid;
   (ib) a β-sheet forming second peptide part consisting of at least 3 amino acids, wherein the N-terminus of the second peptide part is covalently coupled to the C-terminus of the first peptide part, and
(ii) a hydrophobic moiety covalently coupled to the C-terminus of the second peptide part, wherein the hydrophobic moiety is selected from the group comprising or consisting of a C₁₄-C₂₄-fatty acid.

Preferably, the amino acid at the N-terminus of the first peptide part is lysine.

Thus, the present invention preferably relates to a self-assembling peptide amphiphile consisting of:
(i) a non-self-assembling peptide moiety having a positive net charge under physiological pH, wherein the peptide moiety consist of at least 5 amino acids; wherein less than 50 % of the at least 5 amino acids are lysine, arginine, and histidine; and wherein at least one of amino acids is selected from lysine; and wherein the non-self-assembling peptide moiety further comprises:
   (ia) a first peptide part consisting of 1 to 3 amino acids, wherein the amino acid at the C-terminus of the first peptide part is selected from lysine, arginine, and histidine;
   (ib) a β-sheet forming second peptide part consisting of at least 3 amino acids, wherein the N-terminus of the second peptide part is covalently coupled to the C-terminus of the first peptide part, and
(ii) a hydrophobic moiety covalently coupled to the C-terminus of the second peptide part, wherein the hydrophobic moiety is selected from the group comprising or consisting of a C₁₄-C₂₄-fatty acid.

Preferably, the amino acid at the N-terminus of the first peptide part is lysine.

Thus, the present invention preferably relates to a self-assembling peptide amphiphile consisting of:
(i) a non-self-assembling peptide moiety having a positive net charge under physiological pH, wherein the peptide moiety consist of at least 5 amino acids; wherein less than 50 % of the at least 5 amino acids are lysine, arginine, and histidine, wherein at least two of the at least 5 amino acids are lysine, arginine, and/or histidine; and
   wherein the non-self-assembling peptide moiety further comprises:
   (ia) a first peptide part consisting of 1 to 3 amino acids, wherein the amino acid at the C-terminus of the first peptide part is selected from lysine, arginine, and histidine, and wherein at least one of the 1 to 3 amino acids is selected from lysine;
   (ib) a β-sheet forming second peptide part consisting of at least 3 amino acids, wherein the N-terminus of the second peptide part is covalently coupled to the C-terminus of the first peptide part, and
(ii) a hydrophobic moiety covalently coupled to the C-terminus of the second peptide part, wherein the hydrophobic moiety is selected from the group comprising or consisting of a C₁₄-C₂₄-fatty acid.

Preferably, the amino acid at the N-terminus of the first peptide part is lysine.

Thus, the present invention preferably relates to a self-assembling peptide amphiphile consisting of:
(i) a non-self-assembling peptide moiety having a positive net charge under physiological pH, wherein the peptide moiety consist of at least 5 amino acids; wherein less than 50 % of the at least 5 amino acids are lysine, arginine, and histidine, wherein at least two of the at least 5 amino acids are lysine, arginine, and/or histidine, and wherein at least one amino acid is selected from lysine; and wherein the non-self-assembling peptide moiety further comprises:
   (ia) a first peptide part consisting of 1 to 3 amino acids, wherein the amino acid at the C-terminus of the first peptide part is selected from lysine, arginine, and histidine, and wherein at least one of the 1 to 3 amino acids is selected from lysine;
   (ib) a β-sheet forming second peptide part consisting of at least 3 amino acids, wherein the N-terminus of the second peptide part is covalently coupled to the C-terminus of the first peptide part, and
(ii) a hydrophobic moiety covalently coupled to the C-terminus of the second peptide part, wherein the hydrophobic moiety is selected from the group comprising or consisting of a C₁₄-C₂₄-fatty acid.

Preferably, the amino acid at the N-terminus of the first peptide part is lysine.

The following thresholds for interpretation of properties for peptide amphiphiles have been applied for identification of peptide amphiphiles that can be used as retroviral transduction enhancers:
- a peptide amphiphile is considered infectivity enhancing if the infectivity enhancement at 1.3 µM concentration for the compound is at least 2 times larger compared to the infectivity of the virus without the compound (virus only);
- a peptide amphiphile is considered ThT active if a ThT-fluorescence larger than 2 relative to a control samples (PBS) is observed. A virus transduction enhancing peptide amphiphile is preferably ThT active;
- a peptide amphiphile is forming fibrils if fibril morphology is observed in TEM. An infectivity enhancing peptide amphiphile preferably shows no other morphologies such as amorphous aggregates;
- a peptide amphiphile is forming β-sheet secondary structure if a peak around 1625-1635 cm⁻¹ in FT-IR spectroscopy is observed. An active (infectivity enhancing) peptide amphiphile must show β-sheet secondary structure, preferably with a high amount ( >40%) of β-sheets;
- a peptide amphiphile is forming µm-sized aggregates if visible particles (> 10 µm²) are observed in widefield microscopy (brightfield or ThT-staining filter, objective 10 x) or confocal microscopy (stained with proteostat, objective 20 x) at concentrations tested for viral infectivity measurements (0.26 µM, 1.3 µM, 6.5 µM). An infectivity enhancing peptide amphiphiles must form high-aspect ratio nanostructures and particularly preferably forms µm-sized aggregates;
- a peptide amphiphile is defined to be degradable if the number and size of aggregates are decreasing after 3 days in vitro compared to the initial size and number after 30 min incubation in vitro. More specifically, a compound is good degradable if the final number of aggregates in an area of approx. 1000 µm × 1000 µm is less than 50 and the median size is smaller than 50 µm².

### First peptide part

The first peptide part having a first amino acid and a terminal amino acid of the non-self-assembling peptide moiety comprises 1 to 3 positively charged amino acids selected from lysine, arginine, and histidine, wherein the terminal amino acid of the first peptide part is preferably selected from lysine and lysine amide.

Thus, the first peptide part may consist of one amino acid, wherein said amino acid is preferably lysine or lysine amide, or the first peptide part may consist of two amino acids, wherein the first amino acid may be selected from lysine, arginine, and histidine, preferably lysine, and the terminal amino acid is preferably selected from lysine and lysine amide, or the first peptide part may consist of three amino acids, wherein the first amino acid of the first peptide part may be selected from lysine, arginine, and histidine, preferably lysine, and the terminal amino acid is preferably selected from lysine and lysine amide, and wherein the second amino acid between the first amino acid and the terminal amino acid, may be selected from lysine, arginine, and histidine, preferably lysine, or may be also another amino acid, e.g. an amino acid including, but not limited to alanine, glycine, valine, leucine, and isoleucine. However, particularly preferably the second amino acid is not a negatively charged amino acid such as aspartic acid or glutamic acid. Furthermore, it is also particularly preferred that the second amino acid is not proline. In further preferred embodiments the second amino acid is not an aromatic amino acid such as phenylalanine, tryptophan, or tyrosine. Therefore, the first peptide part may include maximum two, but particularly preferably maximum one amino acid that is not a positively charged amino acid such as lysine, arginine, and histidine. Thus, the first peptide part may include maximum two, but particularly preferably maximum one amino acid selected from alanine, glycine, valine, leucine, and isoleucine, more preferably alanine, valine, leucine, and isoleucine.

In preferred embodiments, the first amino acid is the amino acid at the N-terminus of the first peptide part, and the terminal amino acid is the amino acid at the C-terminus of the first peptide part, and the first end is the C-terminal end of the β-sheet forming second peptide part, and the second end is the N-terminal end of the β-sheet forming second peptide part. In such embodiments, the first peptide part of the non-self-assembling peptide moiety comprises 1 to 3 positively charged amino acids selected from lysine, arginine, and histidine, wherein the amino acid at the C-terminus of the first peptide part is preferably lysine or lysine amide. Thus, the first peptide part may consist of one amino acid, wherein said amino acid is preferably lysine or lysine amide, or the first peptide part may consist of two amino acids, wherein the amino acid at N-terminus is selected from lysine, arginine, and histidine, preferably lysine, and the amino acid at the C-terminus of the first peptide part is preferably lysine or lysine amide, or the first peptide part may consist of three amino acids, wherein the amino acid at the N-terminus of the first peptide part is selected from lysine, arginine, and histidine, preferably lysine and the amino acid at the C-terminus of the first peptide part is preferably lysine or lysine amide, and wherein the second amino acid between the amino acid at the N-terminus and the amino acid at the C-terminus, may be also selected from lysine, arginine, and histidine, preferably lysine, but may be also another amino acid, e.g. an amino acid including, but not limited to alanine, glycine, valine, leucine, and isoleucine. However, particularly preferably the second amino acid is not a negatively charged amino acid such as aspartic acid or glutamic acid. Furthermore, it is also particularly preferred that the second amino acid is not proline. In further preferred embodiments the second amino acid is not an aromatic amino acid such as phenylalanine, tryptophan, or tyrosine. Therefore, the first peptide part may include maximum two, but particularly preferably maximum one amino acid that is not a positively charged amino acid such as lysine, arginine, and histidine. Thus, the first peptide part may include maximum two, but particularly preferably maximum one amino acid selected from alanine, glycine, valine, leucine, and isoleucine, more preferably alanine, valine, leucine, and isoleucine.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the first peptide part consists of one amino acid, wherein said amino acid is lysine or lysine amide; or
wherein the first peptide part consists of two amino acids, wherein the first amino acid is selected from lysine, arginine, and histidine, preferably lysine, and the terminal amino acid is lysine or lysine amide.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the first peptide part consists of three amino acids, wherein the first amino acid at of the first peptide part is selected from lysine, arginine, and histidine, preferably lysine; and the terminal amino acid is selected from lysine and lysine amide; and
wherein the second amino acid between the first amino acid and the terminal amino acid of the first peptide part is selected from the group comprising or consisting of lysine, arginine, histidine, alanine, glycine, valine, leucine, and isoleucine.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

In preferred embodiments, the second amino acid between the first amino acid and the terminal amino acid of the first peptide part is selected from the group comprising or consisting of alanine, glycine, valine, leucine, and isoleucine, preferably selected from the group comprising or consisting of alanine, valine, leucine, and isoleucine, more preferably selected from the group comprising or consisting of alanine, valine, and isoleucine.

In further preferred embodiments, the second amino acid between the first amino acid and the terminal amino acid of the first peptide part is selected from the group comprising or consisting of lysine, arginine, and histidine, preferably lysine.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,
wherein the first peptide part is K, KK, KKK, HK, RK, HHK, RRK, HRK, RHK, HKK, RKK, KRK, KHK, KAK, KGK, KIK, KLK, KVK, HAK, HGK, HIK, HLK, HVK, RAK, RGK, RIK, RLK, RVK, KH, KR, KKH, KKR, HKH, HKR, RKH, RKR, KRR, KRH, KHH, KHR, KAH, KGH, KIH, KLH, KVH, KAR, KGR, KIR, KLR, or KVR.

In preferred embodiments, the first peptide part is K, KK, KKK, HK, RK, HHK, RRK, HRK, RHK, HKK, RKK, KRK, KHK, KAK, KGK, KIK, KLK, KVK, HAK, HGK, HIK, HLK, HVK, RAK, RGK, RIK, RLK, or RVK.

In further preferred embodiments, the first peptide part is K, KK, KKK, HK, HHK, HRK, HKK, KHK, KAK, KGK, KIK, KLK, KVK, HAK, HGK, HIK, HLK, or HVK.

In further preferred embodiments, the first peptide part is K, KK, KKK, HK, RK, RRK, RHK, RKK, KRK, KAK, KGK, KIK, KLK, KVK, RAK, RGK, RIK, RLK, or RVK.

In further preferred embodiments, the first peptide part is K, KK, KKK, HK, RK, HHK, RRK, HRK, RHK, HKK, RKK, KRK, or KHK.

In further preferred embodiments, the first peptide part is KAK, KGK, KIK, KLK, KVK, HAK, HGK, HIK, HLK, HVK, RAK, RGK, RIK, RLK, or RVK.

In further preferred embodiments, the first peptide part is K, KK, KKK, KAK, KGK, KIK, KLK, KVK, or HAK.

In particularly preferred embodiments, the first peptide part is K, KK, or KKK.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

The present invention further relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the first amino acid is the amino acid at the N-terminus of the first peptide part, and the terminal amino acid is the amino acid at the C-terminus of the first peptide part, and the first end is the C-terminal end of the β-sheet forming second peptide part, and the second end is the N-terminal end of the β-sheet forming second peptide part,
wherein the first peptide part is K, KK, KKK, HK, RK, HHK, RRK, HRK, RHK, HKK, RKK, KRK, KHK, KAK, KGK, KIK, KLK, KVK, HAK, HGK, HIK, HLK, HVK, RAK, RGK, RIK, RLK, RVK, KH, KR, KKH, KKR, HKH, HKR, RKH, RKR, KRR, KRH, KHH, KHR, KAH, KGH, KIH, KLH, KVH, KAR, KGR, KIR, KLR, KVR, KAA, KGA, KIA, KLA, KVA, KAG, KGG, KIG, KLG, KVG, KAI, KGI, KII, KLI, KVI, KAV, KGV, KIV, KLV, KVV, KAL, KGL, KIL, KLL, or KVL.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids, wherein the amino acid at the N-terminus of the first peptide part is selected from lysine, arginine, and histidine, and wherein at least one of the 1 to 3 amino acids is selected from lysine or lysine amide;
   (ib) a β-sheet forming second peptide part consisting of at least 3 amino acids, wherein the C-terminus of the second peptide part is covalently coupled to the N-terminus of the first peptide part, and
   and
(ii) a hydrophobic moiety covalently coupled to the second end of the β-sheet forming second peptide part, wherein the hydrophobic moiety is selected from the group comprising or consisting of a C₁₄-C₂₄-fatty acid,
wherein the first peptide part is K, KK, KKK, HK, RK, HHK, RRK, HRK, RHK, HKK, RKK, KRK, KHK, KAK, KGK, KIK, KLK, KVK, HAK, HGK, HIK, HLK, HVK, RAK, RGK, RIK, RLK, RVK, KH, KR, KKH, KKR, HKH, HKR, RKH, RKR, KRR, KRH, KHH, KHR, KAH, KGH, KIH, KLH, KVH, KAR, KGR, KIR, KLR, KVR, KAA, KGA, KIA, KLA, KVA, KAG, KGG, KIG, KLG, KVG, KAI, KGI, KII, KLI, KVI, KAV, KGV, KIV, KLV, KVV, KAL, KGL, KIL, KLL, or KVL.

In preferred embodiments, the first peptide part is K, KK, KKK, HK, RK, HHK, RRK, HRK, RHK, HKK, RKK, KRK, KHK, KAK, KGK, KIK, KLK, KVK, HAK, HGK, HIK, HLK, HVK, RAK, RGK, RIK, RLK, RVK, KH, KR, KKH, KKR, HKH, HKR, RKH, RKR, KRR, KRH, KHH, KHR, KAH, KGH, KIH, KLH, KVH, KAR, KGR, KIR, KLR, or KVR.

In preferred embodiments, the first peptide part is K, KK, KKK, HK, RK, HHK, RRK, HRK, RHK, HKK, RKK, KRK, KHK, KAK, KGK, KIK, KLK, KVK, HAK, HGK, HIK, HLK, HVK, RAK, RGK, RIK, RLK, or RVK.

In further preferred embodiments, the first peptide part is K, KK, KKK, HK, HHK, HRK, HKK, KHK, KAK, KGK, KIK, KLK, KVK, HAK, HGK, HIK, HLK, or HVK.

In further preferred embodiments, the first peptide part is K, KK, KKK, HK, RK, RRK, RHK, RKK, KRK, KAK, KGK, KIK, KLK, KVK, RAK, RGK, RIK, RLK, or RVK.

In further preferred embodiments, the first peptide part is K, KK, KKK, HK, RK, HHK, RRK, HRK, RHK, HKK, RKK, KRK, or KHK.

In further preferred embodiments, the first peptide part is KAK, KGK, KIK, KLK, KVK, HAK, HGK, HIK, HLK, HVK, RAK, RGK, RIK, RLK, or RVK.

In further preferred embodiments, the first peptide part is K, KK, KKK, KAK, KGK, KIK, KLK, KVK, or HAK.

In particularly preferred embodiments, the first peptide part is K, KK, or KKK.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

### Second peptide part

The β-sheet forming second peptide part consists of at least 2 amino acids, particularly preferably the β-sheet forming second peptide part consists of at least 3 amino acids, and particularly comprises amino acids having a higher propensity for β-sheet formation Thus, the β-sheet forming second peptide part preferably consists of at least 3 amino acids and comprises amino acids having a higher propensity for β-sheet formation.

The β-sheet forming second peptide part preferably consists of 2 - 12 amino acids, more preferably 3-10 amino acids, more preferably 4-9 amino acids, even more preferably 5 - 7 amino acids.

The β-sheet forming second peptide part preferably comprises at least 40 % amino acids selected from alanine, valine, leucine, and isoleucine, more preferably at least 55 % amino acids selected from alanine, valine, leucine, isoleucine, and glycine, and preferably further comprises maximum 45 % glycine. Particularly preferably, the second peptide part does not include a negatively charged amino acid such as aspartic acid or glutamic acid. Preferably, the second peptide part does not include proline. In preferred embodiments, the second peptide part further comprises an amino acid selected from asparagine, glutamine, arginine, histidine, and lysine, more preferably selected from asparagine, glutamine, and lysine.

In further preferred embodiments, the second peptide part preferably comprises at least 55 % amino acids selected from alanine, valine, leucine, isoleucine, and glycine, and further comprises maximum 45 % glycine, and further comprises at least one of the following:
(i) at least two amino acids selected from leucine and/or isoleucine, and/or
(ii) at least three different amino acids selected from the group comprising or consisting of alanine, glycine, valine, leucine, and isoleucine, and/or
(iii) the amino acid at the second end of the second peptide part is alanine, leucine, or isoleucine.

The inventors of the present invention have further investigated the effects of peptide moiety exchange in peptide amphiphiles on viral transduction efficacy and degradability. The hydrophobic moiety palmitic acid of PA1 has been used to provide a comparison between PA1 and peptide amphiphiles with different peptide moieties. In order to evaluate how attaching palmitic acid to non-assembling peptides is affecting assembly into fibrils and infectivity enhancement, a selection of peptides were tested. Thus, palmitic acid (C₁₆) has been covalently coupled to different peptide moieties in order to investigate if the resulting peptide amphiphiles can maintain infectivity enhancement, if they are also able to form fibrils, aggregate into µm-sized particles and show a positive zeta-potential. Moreover, it has been further investigated if the resulting peptide amphiphiles can be efficiently degraded by cells. A total number of 56 compounds (35 PAs + 22 peptides) were physiochemically characterized and evaluated for increasing infection efficiency.

First it was investigated (Example 4) if the peptide amphiphiles with different peptide moieties are also able to form fibrils, aggregate into µm-sized particles and show a positive zeta-potential. A peptide amphiphile is forming fibrils if fibril morphology is observed in TEM. An infectivity enhancing PA preferably shows no other morphologies such as amorphous aggregates. A peptide amphiphile is forming β-sheet secondary structure if a peak around 1625-1635 cm⁻¹ in FT-IR spectroscopy is observed. An active peptide amphiphile must show β-sheet secondary structure, preferably with a high amount ( >40%) of β-sheets. A peptide amphiphile is forming µm-sized aggregates if visible particles (> 10 µm²) were observed in widefield microscopy (brightfield or ThT-staining filter, objective 10 x) or confocal microscopy (stained with proteostat, objective 20 x) at concentrations tested for viral infectivity measurements (0.26 µM, 1.3 µM, 6.5 µM). An infectivity enhancing PA particularly preferably forms high-aspect ratio nanostructures and particularly preferably forms µm-sized aggregates.

Attaching palmitic acid (C₁₆-Amid) to N-terminus has been identified as a reliable way to create peptide amphiphiles which form fibrils. It has been found that peptides that include a negatively charged amino acid E or a proline P are not preferred. Moreover, the peptides with higher lengths could unexpectedly forming fibrils by themselves without palmitic acid and for these peptides the respective palmitic acid modified PAs are not degradable.

Strikingly, almost all PAs (showed a positive Zeta-potential, while their corresponding peptide-only sequences showed negative or almost neutral (<5mV) zeta-potential. Attaching palmitic acid to net positive charged peptide sequences yields positive zeta-potential likely because the sequences form fibrils with C-terminal positive charged residues facing outwards from the fiber.

Many of the peptide amphiphiles with different sequences show infectivity enhancement (n fold > 2 at 1.3 µM) with comparable or less activity compared to PA1. The peptides which are not enhancing infectivity are either not forming µm-sized aggregates or too large aggregates. If fibrils do not form µm-sized aggregation or too few µm-sized aggregates, no infectivity enhancement can be observed. In contrast very large aggregates also reduce infectivity enhancement. One consideration might be that to many residues with high flexibility close to the hydrophobic residue (G, A) can increase dynamicity of PAs and at the same time a high number of charged residues at the C-terminus may increase solvability and result in less aggregating fibers. Another consideration might be that charged residues, bulky hydrophobic residues, or kinked residues (proline) close to hydrophobic residue hinder efficient assembly, therefore increase dynamicity of the fibers and therefore less aggregation occurs. As already observed for the infectivity data, peptides might not be active because they show either no aggregation or too large aggregates. Such PAs are not forming µm-sized aggregates or too few aggregates as observed via light scattering and confocal microscopy. On the other hand large aggregates reduce infectivity enhancement and degradability. Such PAs are forming too large aggregates as observed via aggregate size analysis by confocal microscopy.

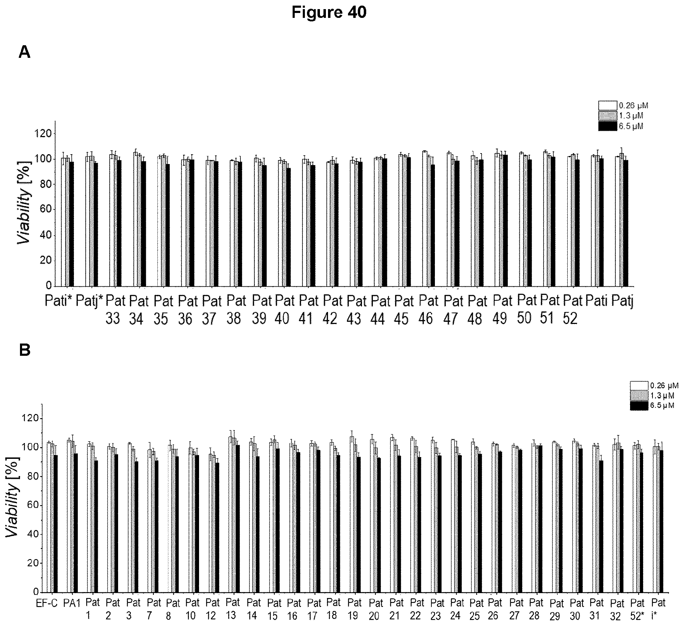

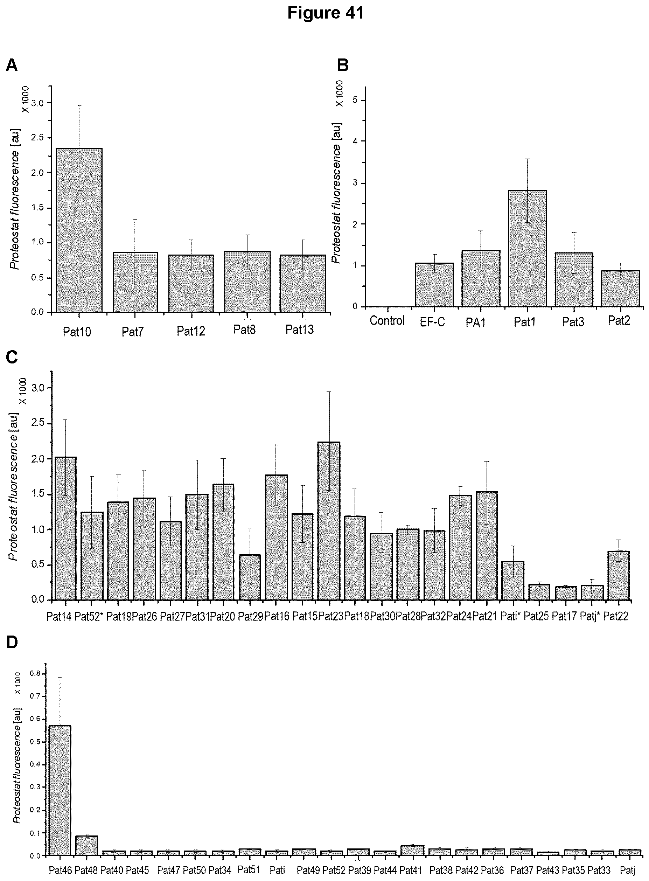

All of the tested peptides (Examples 1 - 4) were biocompatible and not cytotoxic at the tested concentrations on TZMbl cells (Figure 40). For the degradability only the PAs which could be stained with Proteostat (Figure 41) and were visible in microscopy were studied in detail. A summary of all these relevant properties for all peptides is shown in Example 4. In general, the procedure for determine whether a PA is degraded or not, is conducted by confocal laser scanning microscopy of Proteostat stained PAs which are added to Hela cells (2 µg peptide added to 20.000 cells). Microscopy is conducted at day 0 (d0, after 30 min of incubation) and at day 3 (d3, after 72 h of incubation). The number of aggregates >10 µm² is counted via 3D object counter in imaged in an area of 1008 µm × 1008 µm.

A PA is considered to be degradable if the number and the size of aggregates are reduced after 3 days. The efficiency is determined by the percentual degradation (aggregate number at d3 / aggregate number at d0). For the number of aggregation three technical replicates were evaluated for each sample. A large deviation represents a nonhomogeneous spatial distribution of aggregates.

The inventors have obserebed I three different aggregation types in the PA library: fibers that do not aggregate or attach to cells, fibers that form small µm-sized aggregates that attach to cells (*e.g*. PA1), and fibers that form large µm-sized aggregates that attach to cells (*e.g*. PA2).

However, findings from the PA library suggest that fibrils that do not aggregate share a common feature: they consist of a high number of less hydrophobic, flexible amino acids (A, G) and polar amino acids (R, K, E) close to the fatty acid. This could increase dynamicity and solvation and of the self-assembling PAs, which result in less aggregating fibers. PAs with a block-type arrangement of hydrophobic (V, I) and hydrophilic, charged (K, R) amino acids, as found in PA1, form small-sized aggregates. In contrast, strongly aggregating fibrils consist of high amounts of hydrophobic aromatic amino acids and an alternating amphiphilic sequence order as found in PA2. Peptides with an alternating amphiphilic sequence order and high content of hydrophobic amino acids are commonly found in amyloidogenic peptides. The inventors suggest that these properties can also promote intermolecular and fiber-fiber interactions of PAs, leading to increased colloidal aggregation.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 4 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,
wherein the second peptide part preferably comprises at least 40 % amino acids selected from alanine, valine, leucine, and isoleucine.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

Preferably, the second peptide part does not comprise a negatively charged amino acid, i.e. does not comprise aspartic acid or glutamic acid. Preferably, the second peptide part does not comprise proline. Preferably, the second peptide part comprises maximum one amino acid selected from the group comprising or consisting of cysteine, serine, threonine, and lysine.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,
wherein the second peptide part preferably comprises at least 55 % amino acids selected from alanine, valine, leucine, isoleucine, and glycine, and preferably further comprises maximum 45 % glycine.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

Preferably, the second peptide part does not comprise a negatively charged amino acid, i.e. does not comprise aspartic acid or glutamic acid. Preferably, the second peptide part does not comprise proline. Preferably, the second peptide part comprises maximum one amino acid selected from the group comprising or consisting of cysteine, serine, threonine, and lysine.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least two amino acids selected from leucine and/or isoleucine, and/or
wherein the second peptide part comprises maximum three amino acids selected from leucine and/or isoleucine.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the second peptide part comprises at least two amino acids selected from leucine and/or isoleucine, and preferably
wherein the second peptide part comprises maximum three amino acids selected from leucine and/or isoleucine.

Preferably, the second peptide part does not comprise a negatively charged amino acid, i.e. does not comprise aspartic acid or glutamic acid. Preferably, the second peptide part does not comprise proline. Preferably, the second peptide part comprises maximum one amino acid selected from the group comprising or consisting of cysteine, serine, threonine, and lysine.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,
wherein the second peptide part comprises at least three different amino acids selected from the group comprising or consisting of alanine, glycine, valine, leucine, and isoleucine.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the second peptide part comprises at least three different amino acids selected from the group comprising or consisting of alanine, glycine, valine, leucine, and isoleucine.

Preferably the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline. Preferably, the second peptide part comprises maximum one amino acid selected from the group comprising or consisting of cysteine, serine, threonine, and lysine.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,
wherein the amino acid at second end of the second peptide part is alanine, leucine, or isoleucine.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part. In such embodiment, it is preferred that the amino acid at the N-terminus of the second peptide part is alanine, leucine, or isoleucine.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the amino acid at second end of the second peptide part is alanine, leucine, or isoleucine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

Preferably, the second peptide part comprises maximum one amino acid selected from the group comprising or consisting of cysteine, serine, threonine, and lysine. Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part. In such embodiment, it is preferred that the amino acid at the N-terminus of the second peptide part is alanine, leucine, or isoleucine.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,
wherein the second peptide part comprises an amino acid selected from asparagine, glutamine, arginine, histidine, and lysine, preferably selected from asparagine, glutamine, and lysine.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the second peptide part comprises an amino acid selected from asparagine, glutamine, arginine, histidine, and lysine, preferably selected from asparagine, glutamine, and lysine.

Preferably, the amino acid at second end of the second peptide part is alanine, leucine, or isoleucine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

Preferably, the second peptide part comprises maximum one amino acid selected from the group comprising or consisting of cysteine, serine, threonine, and lysine. Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part. In such embodiment, it is preferred that the amino acid at the N-terminus of the second peptide part is alanine, leucine, or isoleucine.

### Linker Exchange

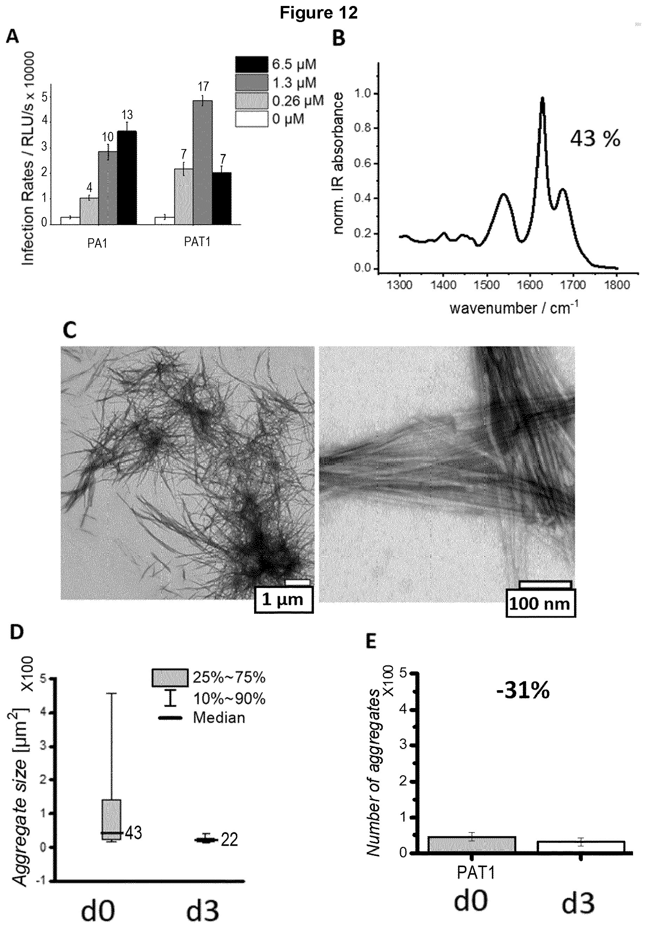

Different PAs inspired by PA1 and PA2 have been prepared and investigated by varying the linkage chemistry (Figures 12, 13, 38). The peptides were tested for cellviability and infectivity enhancement by a HIV-1 infection assay on TZM-bl cells. The peptides were characterized for fibril formation, β-sheet content, µm-sized aggregation, surface charge and amyloid character via TEM, FT-IR, widefield and confocal microscopy, zeta-potential measurements, and ThT-assay, respectively. The number and degradability of µm-sized aggregates were evaluated via confocal microscopy in presence of Hela cells after 30 min and 3 d of incubation at 37°C.

Three different peptide amphiphiles have been analyzed intensively (Example 2):
1) C₁₆-*ester*-SVVVAAAKKK-NH₂ (SEQ. ID NO. 10) (Pat1)
2) C₁₆-*thioester*-CVVVAAAKKK-NH₂ (SEQ. ID NO. 12) (Pat3)
3) C₁₆-*disulfide*-SVVVAAAKKK-NH₂ (SEQ. ID NO. 11) (Pat2)

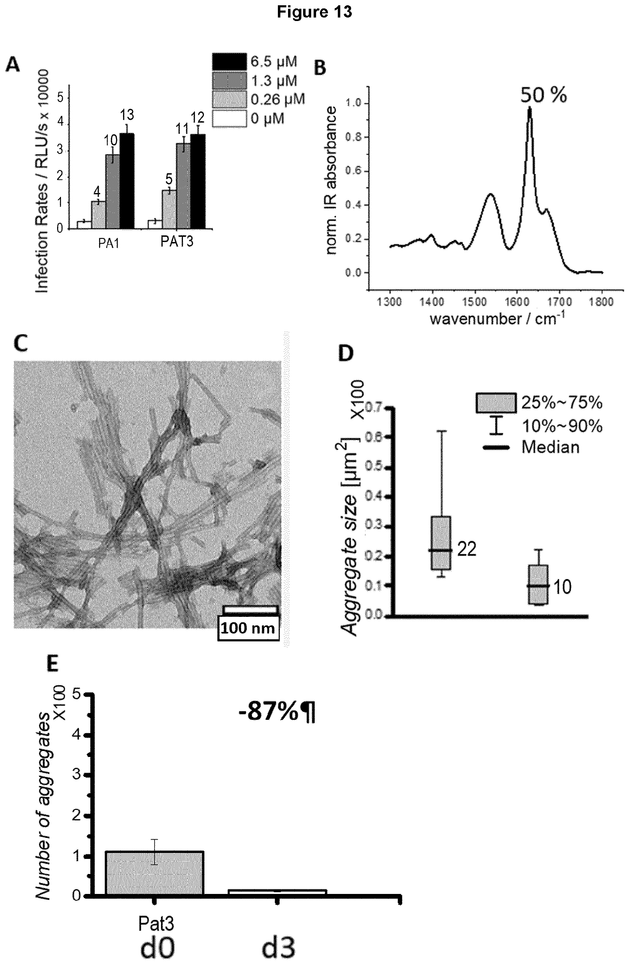

In each case the palmitic acid functionalized and non-functionalized peptides were tested to investigate the influence of the fatty acid. An increase of the infectivity enhancement is observed upon exchange of the linker from amide to ester or thioester. Exchange of the linker from amide to ester linkage enhances infectivity at 1.3 µM concentration 17 times more efficient compared to virus only infection. However, changing from amide to disulfide is reducing infectivity enhancement significantly (n-fold at 1.3 µM 10 → 4) but the peptide is still increasing infectivity. All of these peptides with exchanged linker (N-terminal cysteine or N-terminal serine) show comparable physicochemical properties, but the disulfide exchange results in less aggregates, which is likely the reason why less infectivity enhancement is observed. While no decrease of the infectivity enhancement is observed upon exchange of the linker, a great variability in aggregation behavior and degradation of these aggregates is observed. The peptides can all be stained with the imaging agent Proteostat, but C₁₆-disulfide-CVVVAAAKKK-NH₂ (SEQ. ID NO. 11) cannot be observed via microscopy, because it is not forming large (>10 µm²) aggregates. Aggregate size of PA1 (SEQ. ID NO. 4) used as reference decreases from 28 to 23 µm² and aggregate number decreases 87%. Aggregate size of Pat1 (SEQ. ID NO. 10) decreases from about 43 to 22 µm² and aggregate number decreases by 30% over 3 days (Figure 12D,E). Thus, Pat1 (SEQ. ID NO. 10) forms larger µm-sized fibrillar aggregates in the preferred area range of < 50 µm² than PA1 (SEQ. ID NO. 4). The formation of µm-sized fibrillar aggregates has been found to be an important aspect for the infectivity enhancement. Therefore, the formation of µm-sized fibrillar aggregates of about 43 µm² is likely the reason why more efficient infectivity enhancement is observed in comparison to PA1 (SEQ. ID NO. 4). Although the aggregate number decreases by 30%, it should be noted that the average size of the aggregates is reduced to about 22 µm² in case of Pat1 (SEQ. ID NO. 10) which is comparable with the average size of aggregates of PA1 (SEQ. ID NO. 4) of about 23 µm² after 3 days. (Figure 12D,E) Aggregate size of Pat3 (SEQ. ID NO. 12) decreases from 22 to 10 µm² and aggregate number decreases by 87%, characterizing Pat3 (SEQ. ID NO. 12) as a degradable PA. Thus, Pat3 (SEQ. ID NO. 12) forms µm-sized fibrillar aggregates comparable to the larger µm-sized fibrillar aggregates of PA1 (SEQ. ID NO. 4). The aggregate number of Pat3 (SEQ. ID NO. 12) aggregates decreases by 87%, a little less reduction in comparison to PA1 (SEQ. ID NO. 4). However, the average size of aggregates of Pat3 (SEQ. ID NO. 12) strongly decreases which is especially beneficial for clinical applications. (Figure 13D,E).

Thus, all investigated linker-modified PAs are degradable. The best performing linker exchange is observed for the thioester modification (Pat3), which shows a degradation of 87% and small, well distributed aggregates. The ester modified PA (Pat1) is also forming small aggregates and degraded well, but not as efficient as PA1 or Pat3. The disulfide-modified linker is reducing aggregates size drastically. No aggregates above 10 µm² microscope can be observed. However, initial reduced aggregation also means that infectivity enhancement is significantly (n-fold at 1.3 µM 10 → 4) reduced.

The above results already indicate that the coupling of the hydrophobic moiety to the amino acid side chain of a terminal amino acid of the peptide moiety is beneficial for providing peptide amphiphiles with improved properties for use as retroviral transductions enhancers. Suitable amino acids which allow coupling of fatty acids include cysteine, serine, threonine, and lysine.

Thus, in preferred embodiments, the amino acid at the second end of the β-sheet forming second peptide part may be selected from the group comprising or consisting of cysteine, serine, threonine, and lysine, wherein the hydrophobic moiety is covalently coupled to the amino acid side chain of the respective amino acid, e.g. may be covalently coupled to serine or threonine through an ester linkage or to cysteine through an thioester linkage of disulfide linkage or to the amino group in the side chain of lysine through an amide linkage.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the amino acid at the second end of the second peptide part is selected from the group comprising or consisting of cysteine, serine, threonine, and lysine preferably cysteine or serine, more preferably cysteine, and
wherein the hydrophobic moiety is covalently coupled to the side chain of the amino acid at the second end of the second peptide part.

In preferred embodiments, β-sheet forming second peptide part comprises maximum one amino acid selected from the group comprising or consisting of cysteine, serine, threonine, and lysine.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, the first amino acid is the amino acid at the N-terminus of the first peptide part, and the terminal amino acid is the amino acid at the C-terminus of the first peptide part, and the first end is the C-terminal end of the β-sheet forming second peptide part, and the second end is the N-terminal end of the β-sheet forming second peptide part. In such embodiments, it is preferred that the amino acid at the N-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the N-terminus of the second peptide part.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the amino acid at the second end of the second peptide part is selected from the group comprising or consisting of cysteine, serine, threonine, and lysine preferably cysteine or serine, more preferably cysteine, and
wherein the hydrophobic moiety is covalently coupled to the side chain of the amino acid at the second end of the second peptide part through an ester linkage, thioester linkage, disulfide linkage or amide linkage.

In preferred embodiments, β-sheet forming second peptide part comprises maximum one amino acid selected from the group comprising or consisting of cysteine, serine, threonine, and lysine.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

More preferably, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the amino acid at the second end of the second peptide part is selected from the group comprising or consisting of cysteine, serine, threonine, and lysine preferably cysteine or serine, more preferably cysteine, and
wherein the hydrophobic moiety is covalently coupled to the side chain of the amino acid at the second end of the second peptide part through an ester linkage, thioester linkage, or amide linkage.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, β-sheet forming second peptide part comprises maximum one amino acid selected from the group comprising or consisting of cysteine, serine, threonine, and lysine.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

Even more preferably, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the amino acid at the second end of the second peptide part is selected from the group comprising or consisting of cysteine, serine, and threonine, preferably cysteine or serine, more preferably cysteine, and
wherein the hydrophobic moiety is covalently coupled to the side chain of the amino acid at the second end of the second peptide part through an ester linkage or thioester linkage.

In preferred embodiments, β-sheet forming second peptide part comprises maximum one amino acid selected from the group comprising or consisting of cysteine, serine, threonine, and lysine.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the amino acid at the second end of the second peptide part is selected from the group comprising or consisting of cysteine, serine, threonine, and lysine preferably cysteine or serine, more preferably cysteine, and
wherein the hydrophobic moiety is covalently coupled to the side chain of the amino acid at the second end of the second peptide part, and
wherein the hydrophobic moiety is covalently coupled to the side chain of the amino acid at the second end of the second peptide part through an ester linkage or thioester linkage or amide linkage.

In preferred embodiments, the first amino acid is the amino acid at the N-terminus of the first peptide part, and the terminal amino acid is the amino acid at the C-terminus of the first peptide part, and the first end is the C-terminal end of the β-sheet forming second peptide part, and the second end is the N-terminal end of the β-sheet forming second peptide part. In such embodiments, it is preferred that the amino acid at the N-terminus of the second peptide part is selected from the group comprising or consisting of cysteine, serine, threonine, and lysine preferably cysteine or serine, more preferably cysteine, and 0wherein the hydrophobic moiety is covalently coupled to the side chain of the amino acid at the N-terminus of the second peptide part. Thus, the hydrophobic moiety is covalently coupled to the N-terminus of the second peptide part through an ester linkage, thioester linkage or amide linkage.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the amino acid at the second end of the second peptide part is cysteine, and the hydrophobic moiety is covalently coupled to the side chain of the amino acid at the second end of the second peptide part through a thioester linkage; or
wherein the amino acid at the second end of the second peptide part is serine or threonine, preferably serine, and the hydrophobic moiety is covalently coupled to the side chain of the amino acid at the second end of the second peptide part through an ester linkage; or
wherein the the amino acid at the second end of the second peptide part is lysine, and the hydrophobic moiety is covalently coupled to the side chain of the amino acid at the second end of the second peptide part through an amide linkage.

In preferred embodiments, β-sheet forming second peptide part comprises maximum one amino acid selected from the group comprising or consisting of cysteine, serine, threonine, and lysine.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part. In such embodiments, it is preferred that #the amino acid at the N-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the N-terminus of the second peptide part. Thus, the hydrophobic moiety is covalently coupled to the N-terminus of the second peptide part through an ester linkage, thioester linkage or amide linkage. In preferred embodiments, the amino acid at the N-terminus of the second peptide part is cysteine, wherein the hydrophobic moiety is covalently coupled to the N-terminus of the second peptide part through a thioester linkage. In further preferred embodiments the amino acid at the N-terminus of the second peptide part is serine, or threonine, preferably serine, wherein the hydrophobic moiety is covalently coupled to the N-terminus of the second peptide part through an ester linkage. In further preferred embodiments the amino acid at the N-terminus of the second peptide part is lysine, wherein the hydrophobic moiety is covalently coupled to N-terminus of the second peptide part through the amino group in the side chain of lysine through an amide linkage.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the amino acid at the second end of the second peptide part is selected from the group comprising or consisting of cysteine, serine, threonine, and lysine preferably cysteine or serine, more preferably cysteine, and
wherein the hydrophobic moiety is covalently coupled to the side chain of the amino acid at the second end of the second peptide part, and
wherein the hydrophobic moiety is covalently coupled to the side chain of the amino acid at the second end of the second peptide part through an ester linkage or thioester linkage or amide linkage.

In preferred embodiments, β-sheet forming second peptide part comprises maximum one amino acid selected from the group comprising or consisting of cysteine, serine, threonine, and lysine.

Preferably, the second peptide part comprises an amino acid selected from asparagine, glutamine, arginine, histidine, and lysine, preferably selected from asparagine, glutamine, and lysine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, the first amino acid is the amino acid at the N-terminus of the first peptide part, and the terminal amino acid is the amino acid at the C-terminus of the first peptide part, and the first end is the C-terminal end of the β-sheet forming second peptide part, and the second end is the N-terminal end of the β-sheet forming second peptide part. In such embodiments, it is preferred that #the amino acid at the N-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the N-terminus of the second peptide part. Thus, the hydrophobic moiety is covalently coupled to the N-terminus of the second peptide part through an ester linkage, thioester linkage or amide linkage. In preferred embodiments, the amino acid at the N-terminus of the second peptide part is cysteine, wherein the hydrophobic moiety is covalently coupled to the N-terminus of the second peptide part through a thioester linkage. In further preferred embodiments the amino acid at the N-terminus of the second peptide part is serine, or threonine, preferably serine, wherein the hydrophobic moiety is covalently coupled to the N-terminus of the second peptide part through an ester linkage. In further preferred embodiments the amino acid at the N-terminus of the second peptide part is lysine, wherein the hydrophobic moiety is covalently coupled to N-terminus of the second peptide part through the amino group in the side chain of lysine through an amide linkage.

In further preferred embodiments, the first amino acid is the amino acid at the C-terminus of the first peptide part, and the terminal amino acid is the amino acid at the N-terminus of the first peptide part, and first end is the N-terminal end of the β-sheet forming second peptide part, and the second end is the C-terminal end of the β-sheet forming second peptide part. In such embodiments, it is preferred that the amino acid at the C-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the C-terminus of the second peptide part. Thus, the hydrophobic moiety is covalently coupled to the C-terminus of the second peptide part through an ester linkage, thioester linkage or amide linkage. In preferred embodiments, the amino acid at the C-terminus of the second peptide part is cysteine, wherein the hydrophobic moiety is covalently coupled to the C-terminus of the second peptide part through a thioester linkage. In further preferred embodiments the amino acid at the C-terminus of the second peptide part is serine, or threonine, preferably serine, wherein the hydrophobic moiety is covalently coupled to the C-terminus of the second peptide part through an ester linkage. In further preferred embodiments the amino acid at the C-terminus of the second peptide part is lysine, wherein the hydrophobic moiety is covalently coupled to C-terminus of the second peptide part through the amino group in the side chain of lysine through an amide linkage.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the amino acid at the second end of the second peptide part is cysteine, and the hydrophobic moiety is covalently coupled to the side chain of the amino acid at the second end of the second peptide part through a thioester linkage; or
wherein the amino acid at the second end of the second peptide part is serine or threonine, preferably serine, and the hydrophobic moiety is covalently coupled to the side chain of the amino acid at the second end of the second peptide part through an ester linkage; or
wherein the amino acid at the second end of the second peptide part is lysine, and the hydrophobic moiety is covalently coupled to the side chain of the amino acid at the second end of the second peptide part through an amide linkage.

Preferably, the second peptide part comprises an amino acid selected from asparagine, glutamine, arginine, histidine, and lysine, preferably selected from asparagine, glutamine, and lysine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

Preferably, the second peptide part comprises maximum one amino acid selected from the group comprising or consisting of cysteine, serine, threonine, and lysine. Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, the first amino acid is the amino acid at the N-terminus of the first peptide part, and the terminal amino acid is the amino acid at the C-terminus of the first peptide part, and the first end is the C-terminal end of the β-sheet forming second peptide part, and the second end is the N-terminal end of the β-sheet forming second peptide part. In such embodiments, it is preferred that #the amino acid at the N-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the N-terminus of the second peptide part. Thus, the hydrophobic moiety is covalently coupled to the N-terminus of the second peptide part through an ester linkage, thioester linkage or amide linkage. In preferred embodiments, the amino acid at the N-terminus of the second peptide part is cysteine, wherein the hydrophobic moiety is covalently coupled to the N-terminus of the second peptide part through a thioester linkage. In further preferred embodiments the amino acid at the N-terminus of the second peptide part is serine, or threonine, preferably serine, wherein the hydrophobic moiety is covalently coupled to the N-terminus of the second peptide part through an ester linkage. In further preferred embodiments the amino acid at the N-terminus of the second peptide part is lysine, wherein the hydrophobic moiety is covalently coupled to N-terminus of the second peptide part through the amino group in the side chain of lysine through an amide linkage.

In further preferred embodiments, the first amino acid is the amino acid at the C-terminus of the first peptide part, and the terminal amino acid is the amino acid at the N-terminus of the first peptide part, and first end is the N-terminal end of the β-sheet forming second peptide part, and the second end is the C-terminal end of the β-sheet forming second peptide part. In such embodiments, it is preferred that the amino acid at the C-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the C-terminus of the second peptide part. Thus, the hydrophobic moiety is covalently coupled to the C-terminus of the second peptide part through an ester linkage, thioester linkage or amide linkage. In preferred embodiments, the amino acid at the C-terminus of the second peptide part is cysteine, wherein the hydrophobic moiety is covalently coupled to the C-terminus of the second peptide part through a thioester linkage. In further preferred embodiments the amino acid at the C-terminus of the second peptide part is serine, or threonine, preferably serine, wherein the hydrophobic moiety is covalently coupled to the C-terminus of the second peptide part through an ester linkage. In further preferred embodiments the amino acid at the C-terminus of the second peptide part is lysine, wherein the hydrophobic moiety is covalently coupled to C-terminus of the second peptide part through the amino group in the side chain of lysine through an amide linkage.

### Hydrophobic Moiety Exchange

The inventors of the present invention have investigated the effects of hydrophobic moiety exchange in peptide amphphiles on viral transduction efficacy (Example 3). It has been found that fatty acids are particularly suitable hydrophobic moieties as the resulting peptide amphiphiles are more efficiently internalized and degraded by cells. Thus, after degradation of the fatty acid, the resulting non-self-assembling peptide moiety readily disintegrates due to the non-self-assembly properties of the amino acid sequence of the peptide moiety.

The non-self-assembling peptides VVVAAAKKK-NH₂ (P1) (SEQ ID NO. 1) has been covalently coupled to different hydrophobic moieties in order to investigate if the resulting peptide amphiphiles can maintain infectivity enhancement, if they are also able to form fibrils, aggregate into µm-sized particles and show a positive zeta-potential. Moreover, it has been further investigated if the resulting peptide amphiphiles can be efficiently degraded by cells.

The following hydrophobic moieties have been tested: the two aromatic hydrophobic moieties fluorenylmethyloxycarbonyl (Fmoc) or 2-naphthylmethyl (NAP), the saturated fatty acids C₉ (C₈H₁₇COOH), and C₁₂ (lauric acid; C₁₁H₂₃COOH), the polyunsaturated fatty acids α-linolenic acid (ALA) and eicosapentaenoic acid (EPA).

It has been found that the peptide amphiphiles with naphthalene (Nap, Nap-VVVAAAKKK-NH₂, Pat13) or α-linolenic acid (ALA, α-linolenic acid-VWAAAKKK-NH₂, Pat9) as hydrophobic moiety show almost neutral zeta-potential. The peptide amphiphile with α-linolenic acid (ALA) as hydrophobic moiety also failed to form fibrils in aqueous medium. The peptide amphiphiles with naphthalene (Nap) or perlargonic acid (C₉, C₈H₁₇COOH, C₉-VVVAAAKKK-NH₂, Pat8) as hydrophobic moiety formed fibrils in aqueous medium, but failed to form µm-sized aggregates in aqueous medium. A positive zeta-potential, fibril formation and formation of µm-sized aggregates of these fibrils have been previously identified to be important factor infectivity enhancing. In contrary, the peptide amphiphiles with lauric acid (C₁₂, C₁₂-VVVAAAKKK-NH₂, Pat7), fluorenylmethyloxycarbonyl (Fmoc Fmoc-VWAAAKKK-NH₂, Pat12), and eicosapentaenoic acid (EPA, eicosapentaenoic acid-WVAAAKKK-NH₂, Pat10) have a positive zeta-potential, are able to form fibrils and also form µm-sized aggregates. Also the peptide amphiphiles with fluorenylmethyloxycarbonyl (Fmoc, Pat12), and eicosapentaenoic acid (EPA Pat10) showed infectivity enhancement.

Surprisingly, it has been found that if the length and amount of double bonds ("unsaturatedness") of the fatty acid residue is increased (α-linolenic acid (ALA) → Eicosapentaenoic acid (EPA)) infectivity enhancement is increased strongly. The peptide amphiphile with eicosapentaenoic acid (EPA) as hydrophobic moiety showed even stronger infectivity enhancement than the reference compounds EF-C or PA1. This peptide amphiphile with eicosapentaenoic acid (EPA) as hydrophobic moiety is forming spherical µm-sized aggregates with composed of β-sheet fibrillar structures, resembling of phase separated peptides, and show a high number of equally distributed small aggregates. The peptide amphiphile with Fmoc as hydrophobic moiety is forming strongly aggregated fibrils, which cluster to very large µm-sized aggregates, which might be challenging to degrade by cells.

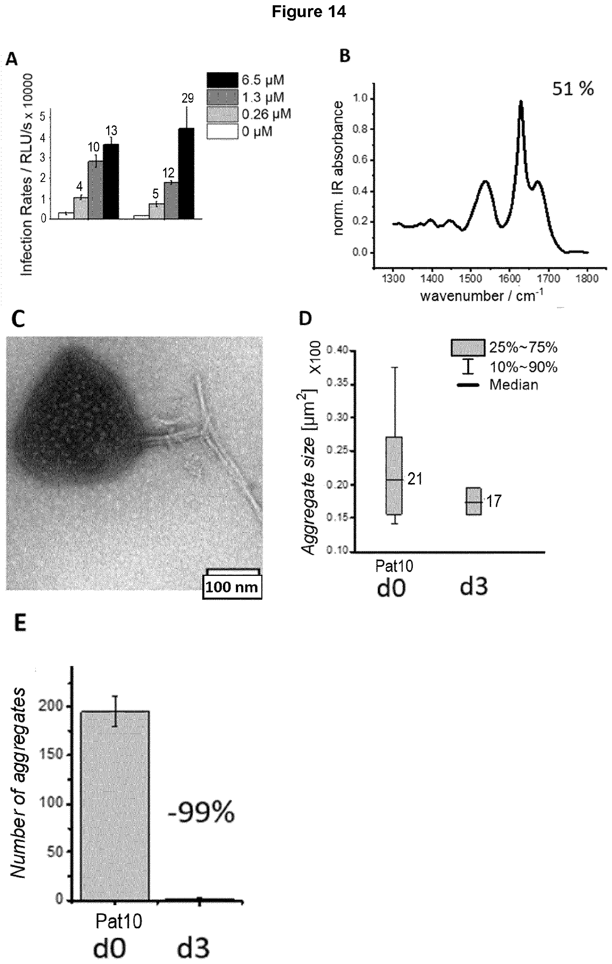

Exchange of the hydrophobic moiety results in almost all of the investigated cases to a reduced activity and degradability. Thus, the peptide amphiphile with eicosapentaenoic acid (EPA) as hydrophobic moiety is performing extraordinary good in terms of activity (outperforming EF-C and PA1) and also has a surprising good degradability (99%). The peptide amphiphile with eicosapentaenoic acid (EPA) forms very small (average size of 6 µm²) and equally distributed aggregates (Figure 14). In that means, it is in the sweet spot of forming aggregates, which is required to transport viral particles to cells but not too large aggregates to avoid degradability problems.

Thus, peptide amphiphiles are herein particularly preferred, wherein the hydrophobic moiety is a fatty acid, so that after degradation of the fatty acid, the resulting peptide moiety readily disintegrates due to the non-self-assembly properties of the amino acid sequence of the non-self-assembling peptide moiety. Thus, the peptide amphiphiles of the present invention have a positive charge and a β-sheet secondary structure and are more efficiently internalized and degraded by cells due to their fatty acid residue.

In several preferred embodiments, the hydrophobic moiety may be a C₁₄-C₂₄₋saturated fatty acid selected from the group comprising or consisting of myristic acid (tetradecanoic acid, C₁₃H₂₇COOH), pentadecanoic acid (C₁₄H₂₉COOH), palmitic acid (hexadecanoic acid, C₁₅H₃₁COOH), margaric acid (heptadecanoic acid, C₁₆H₃₃COOH), stearic acid (octadecanoic acid, C₁₇H₃₅COOH), nonadecanoic acid (C₁₈H₃₇COOH), arachidic acid (eicosanoic acid, C₁₉H₃₉COOH), heneicosanoic acid (C₂₀H₄₁COOH), behenic acid (docosanoic acid, C₂₁H₄₃COOH), tricosanoic acid (C₂₂H₄₅COOH), and lignoceric acid (tetracosanoic acid, C₂₃H₄₇COOH). In further preferred embodiments, the hydrophobic moiety may be a C₁₄-C₂₀-saturated fatty acid selected from the group comprising or consisting of myristic acid (tetradecanoic acid, C₁₃H₂₇COOH), palmitic acid (hexadecanoic acid, C₁₅H₃₁COOH), stearic acid (octadecanoic acid, C₁₇H₃₅COOH), and arachidic acid (eicosanoic acid, C₁₉H₃₉COOH). ). In particularly preferred embodiments, the hydrophobic moiety may be a C₁₄-C₂₀-saturated fatty acid selected from the group comprising or consisting of palmitic acid (hexadecanoic acid, C₁₅H₃₁COOH), and stearic acid (octadecanoic acid, C₁₇H₃₅COOH). Herein particularly preferred is palmitic acid (hexadecanoic acid, C₁₅H₃₁COOH) as hydrophobic moiety.

In several preferred embodiments, the hydrophobic moiety may be monounsaturated C₁₆-C₂₄-cis-omega-n fatty acids selected from the group comprising or consisting cis-9-octadecenoic acid, cis-9-hexadecenoic acid, cis-11-octadecenoic acid, cis-13-eicosenoic acid, cis-13-eicosenoic acid, cis-11-eicosenoic acid, cis-13-docosenoic acid, and cis-15-tetracosenoic acid. In further preferred embodiments, the hydrophobic moiety may be monounsaturated C₁₈-C₂₂-cis-omega-n fatty acids selected from the group comprising or consisting cis-9-octadecenoic acid, cis-11-octadecenoic acid, cis-13-eicosenoic acid, cis-13-eicosenoic acid, cis-11-eicosenoic acid, and cis-13-docosenoic acid.

Most preferably, the hydrophobic moiety may be a polyunsaturated omega-n-fatty acid in *all-cis* configuration, preferably selected from the group comprising or consisting of a C₂₀-C₂₄-omega 3 fatty acid, a C₁₈-C₂₄-omega 6 fatty acid, and a C₁₈-C₂₄-omega 9 fatty acid, particularly preferably a C₂₀-C₂₄-omega 3 fatty acid, e.g. eicosapentaenoic acid, docosahexaenoic acid, or docosapentaenoic acid (n-3-DPA). The hydrophobic moiety may be covalently coupled to the N-terminus of the peptide moiety through an amide linkage. Thus, the amino acid at the N-terminus of the peptide moiety may be directly functionalized with a polyunsaturated all-cis-omega-n-fatty acid.

Thus, in particularly preferred embodiments, the hydrophobic moiety may be a polyunsaturated omega-n-fatty acid in *all-cis* configuration, preferably selected from the group comprising or consisting of a C₂₀-C₂₄-omega 3 fatty acid, a C₁₈-C₂₄-omega 6 fatty acid, and a C₁₈-C₂₄-omega 9 fatty acid, particularly preferably a C₂₀-C₂₄-omega 3 fatty acid, e.g. eicosapentaenoic acid, docosahexaenoic acid, or docosapentaenoic acid (n-3-DPA). The hydrophobic moiety may be covalently coupled to the the peptide moiety through an amide linkage. Thus, the peptide moiety may be directly functionalized with a polyunsaturated *all-cis*-omega-n-fatty acid.

In preferred embodiments, the amino acid at the second end of the β-sheet forming second peptide part may be selected from the group comprising or consisting of cysteine, serine, threonine, and lysine, wherein the hydrophobic moiety may be covalently coupled to the amino acid side chain of the respective amino acid, e.g. may be covalently coupled to serine or threonine through an ester linkage or to cysteine through an thioester linkage or to the amino group in the side chain of lysine through an amide linkage. In such preferred embodiments, the hydrophobic moiety may be a long-chain fatty acid of 14 to 22 or more carbons, wherein the fatty acid may be saturated, unsaturated, or polyunsaturated. Thus, in such embodiment, the hydrophobic moiety may be also selected from a C₁₄-C₂₂ saturated fatty acid, a C₁₄-C₂₄ monounsaturated fatty acid, or a C₁₈-C₂₄-polyunsaturated fatty acid. Preferably the amino acid linker is cysteine, and the fatty acid is covalently coupled to the cysteine linker via a thioester linkage

Thus, in particularly preferred embodiments, the hydrophobic moiety may be a C₁₈-C₂₄-all-cis-omega-n methylene-interrupted fatty acid selected from the group comprising or consisting of *all-cis-*5,8,11,14,17-eicosapentaenoic acid (EPA), *all-cis-*4,7,10,13,16,19-docosahexaenoic acid (DHA), *all-cis-*7,10,13,16,19-docosapentaenoic acid (n-3 DPA), *all-cis-*8,11,14,17-eicosatetraenoic acid (ETA), and *all-cis-*6,9,12,15,18-heneicosapentaenoic acid (HPA), *all-cis-*9,12,15-octadecatrienoic acid (ALA), *all-cis-*6,9,12,15,-octadecatetraenoic acid (SDA), *all-cis-*9,12,15,18,21-tetracosapentaenoic acid, *all-cis-*6,9,12,15,18,21-tetracosahexaenoic acid, *all-cis-*9,12-octadecadienoic acid (LA), *all-cis-*6,9,12-octadecatrienoic acid (GLA), *all-cis*-11,14-eicosadienoic acid, *all-cis-*8,11,14-eicosatrienoic acid, *all-cis-*5,8,11,14-eicosatetraenoic acid, *all-cis-*13,16-docosadienoic acid, *all-cis-*7,10,13,16-docosatetraenoic acid, *all-cis-*4,7,10,13,16-docosapentaenoic acid (n-6 DPA), *all-cis-*9,12,15,18-tetracosatetraenoic acid, *all-cis-*6,9,12,15,18-tetracosapentaenoic acid, and *all-cis*-5,8,11-eicosatrienoic acid.

In preferred embodiments, the hydrophobic moiety may be a C₁₈-C₂₄-all-cis-omega-3 methylene-interrupted fatty acids selected from the group comprising or consisting of *all-cis-*5,8,11,14,17-eicosapentaenoic acid (EPA), *all-cis-*4,7,10,13,16,19-docosahexaenoic acid (DHA), *all-cis-*7,10,13,16,19-docosapentaenoic acid (n-3 DPA), *all-cis-*8,11,14,17-eicosatetraenoic acid (ETA), and *all-cis-*6,9,12,15,18-heneicosapentaenoic acid (HPA), *all-cis-*9,12,15-octadecatrienoic acid (ALA), *all-cis-*6,9,12,15,-octadecatetraenoic acid (SDA), *all-cis-*9,12,15,18,21-tetracosapentaenoic acid, *all-cis-*6,9,12,15,18,21-tetracosahexaenoic acid.

In further preferred embodiments, the hydrophobic moiety may be a C₂₀-C₂₂-all-cis-omega-3 methylene-interrupted fatty acids selected from the group comprising or consisting of *all-cis-*5,8,11,14,17-eicosapentaenoic acid (EPA), *all-cis-*4,7,10,13,16,19-docosahexaenoic acid (DHA), *all-cis-*7,10,13,16,19-docosapentaenoic acid (n-3 DPA), *all-cis-*8,11,14,17-eicosatetraenoic acid (ETA), and *all-cis-*6,9,12,15,18-heneicosapentaenoic acid (HPA).

In preferred embodiments, the hydrophobic moiety may be a C₁₈-C₂₄-all-cis-omega-6 methylene-interrupted fatty acids include, *all-cis-*9,12-octadecadienoic acid (LA), *all-cis-*6,9,12-octadecatrienoic acid (GLA), *all-cis-*11,14-eicosadienoic acid, *all-cis-*8,11,14-eicosatrienoic acid, *all-cis-*5,8,11,14-eicosatetraenoic acid, *all-cis-*13,16-docosadienoic acid, *all-cis-*7,10,13,16-docosatetraenoic acid, *all-cis-*4,7,10,13,16-docosapentaenoic acid (n-6 DPA), *all-cis-*9,12,15,18-tetracosatetraenoic acid, *all-cis-*6,9,12,15,18-tetracosapentaenoic acid.

In further preferred embodiments, the hydrophobic moiety may be a C₂₀-C₂₂-all-cis-omega-6 methylene-interrupted fatty acids including, but not limited to *all-cis-*4,7,10,13,16-docosapentaenoic acid (n-6 DPA), *all-cis-*7,10,13,16-docosatetraenoic acid, *all-cis*-5,8,11,14-eicosatetraenoic acid, and *all-cis-*8,11,14-eicosatrienoic acid.

In particularly preferred embodiments, the hydrophobic moiety may be *all-cis-*5,8,11,14,17-eicosapentaenoic acid (EPA) and *all-cis-*4,7,10,13,16,19-docosahexaenoic acid (DHA), even more preferred is *all-cis-*5,8,11,14,17-eicosapentaenoic acid (EPA).

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,
wherein the hydrophobic moiety is selected from the group comprising or consisting of a polyunsaturated C₂₀-C₂₂-omega-3 fatty acid, a polyunsaturated C₂₀-C₂₂-omega-6 fatty acid, a monounsaturated C₁₈-C₂₄-omega 3 fatty acid, a monounsaturated C₁₈-C₂₄-omega 6 fatty acid, and a monounsaturated C₁₈-C₂₄-omega 9 fatty acid.

In preferred embodiments, the hydrophobic moiety may be a C₁₈-C₂₄-omega 3 fatty acid, more preferably a polyunsaturated C₂₀-C₂₂-omega-3 fatty acid, even more preferably a polyunsaturated C₂₀-C₂₂-omega-3 methylene-interrupted fatty acid, most preferably a polyunsaturated C₂₀-C₂₂--all-cis-omega-3 methylene-interrupted fatty acid. In other preferred embodiments, the hydrophobic moiety may be a C₁₈-C₂₄-omega 6 fatty acid more preferably a polyunsaturated C₂₀-C₂₂-omega-6 fatty acid, even more preferably more preferably a polyunsaturated C₂₀-C₂₂-omega-6 methylene-interrupted fatty acid, most preferably a polyunsaturated C₂₀-C₂₂--all-cis-omega-6 methylene-interrupted fatty acid.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

Preferably, the β-sheet forming second peptide part preferably consists of 2 - 12 amino acids, more preferably 3 - 10 amino acids, more preferably 4-9 amino acids, even more preferably 5 - 7 amino acids.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the hydrophobic moiety is a C₂₀-C₂₂-all-cis-omega-3 methylene-interrupted fatty acid or a C₂₀-C₂₂-all-cis-omega-6 methylene-interrupted fatty acid, preferably wherein the hydrophobic moiety is selected from the group comprising or consisting of *all-cis-*5,8,11,14,17-eicosapentaenoic acid (EPA), *all-cis-*4,7,10,13,16,19-docosahexaenoic acid (DHA), *all-cis-*7,10,13,16,19-docosapentaenoic acid (n-3 DPA), *all-cis-*8,11,14,17-eicosatetraenoic acid (ETA), and *all-cis-*6,9,12,15,18-heneicosapentaenoic acid (HPA), to *all-cis-*4,7,10,13,16-docosapentaenoic acid (n-6 DPA), *all-cis*-7,10,13,16-docosatetraenoic acid, *all-cis-*5,8,11,14-eicosatetraenoic acid, and *all-cis-*8,11,14-eicosatrienoic acid,
more preferably
wherein the hydrophobic moiety is selected from the group comprising or consisting of *all-cis-*5,8,11,14,17-eicosapentaenoic acid (EPA), *all-cis-*4,7,10,13,16,19-docosahexaenoic acid (DHA), and *all-cis-*7,10,13,16,19-docosapentaenoic acid (n-3 DPA),
particularly preferably
wherein the hydrophobic moiety is *all-cis-5,8,11,14,17-eicosapentaenoic* acid (EPA).

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

Preferably, the β-sheet forming second peptide part preferably consists of 2 - 12 amino acids, more preferably 3 - 10 amino acids, more preferably 4-9 amino acids, even more preferably 5 - 7 amino acids.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,
wherein the hydrophobic moiety is selected from the group comprising or consisting of *all-cis-*5,8,11,14,17-eicosapentaenoic acid (EPA), *all-cis-*4,7,10,13,16,19-docosahexaenoic acid (DHA), *all-cis-*7,10,13,16,19-docosapentaenoic acid (n-3 DPA), *all-cis-*8,11,14,17-eicosatetraenoic acid (ETA), and *all-cis-*6,9,12,15,18-heneicosapentaenoic acid (HPA), to *all-cis-*4,7,10,13,16-docosapentaenoic acid (n-6 DPA), *all-cis-*7,10,13,16-docosatetraenoic acid, *all-cis-*5,8,11,14-eicosatetraenoic acid, and *all-cis*-8,11,14-eicosatrienoic acid.

Preferably, the β-sheet forming second peptide part preferably consists of 2 - 12 amino acids, more preferably 3 - 10 amino acids, more preferably 4-9 amino acids, even more preferably 5 - 7 amino acids.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,
wherein the hydrophobic moiety is selected from the group comprising or consisting of *all-cis-*5,8,11,14,17-eicosapentaenoi*c* acid (EPA), *all-cis-*4,7,10,13,16,19-docosahexaenoic acid (DHA), and *all-cis-*7,10,13,16,19-docosapentaenoic acid (n-3 DPA).

Preferably, the β-sheet forming second peptide part preferably consists of 2 - 12 amino acids, more preferably 3 - 10 amino acids, more preferably 4-9 amino acids, even more preferably 5 - 7 amino acids.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the hydrophobic moiety is selected from the group comprising or consisting of a polyunsaturated C₂₀-C₂₂-omega-3 fatty acid, a polyunsaturated C₂₀-C₂₂-omega-6 fatty acid, a monounsaturated C₁₈-C₂₄-omega 3 fatty acid, a monounsaturated C₁₈-C₂₄-omega 6 fatty acid, and a monounsaturated C₁₈-C₂₄-omega 9 fatty acid.

Preferably, the second peptide part comprises an amino acid selected from asparagine, glutamine, arginine, histidine, and lysine, preferably selected from asparagine, glutamine, and lysine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

Preferably, the second peptide part comprises maximum one amino acid selected from the group comprising or consisting of cysteine, serine, threonine, and lysine.

Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, the hydrophobic moiety may be a C₁₈-C₂₄-omega 3 fatty acid, more preferably a polyunsaturated C₂₀-C₂₂-omega-3 fatty acid, even more preferably a polyunsaturated C₂₀-C₂₂-omega-3 methylene-interrupted fatty acid, most preferably a polyunsaturated C₂₀-C₂₂--all-cis-omega-3 methylene-interrupted fatty acid. In other preferred embodiments, the hydrophobic moiety may be a C₁₈-C₂₄-omega 6 fatty acid more preferably a polyunsaturated C₂₀-C₂₂-omega-6 fatty acid, even more preferably more preferably a polyunsaturated C₂₀-C₂₂-omega-6 methylene-interrupted fatty acid, most preferably a polyunsaturated C₂₀-C₂₂--all-cis-omega-6 methylene-interrupted fatty acid.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the hydrophobic moiety is a C₂₀-C₂₂-all-cis-omega-3 methylene-interrupted fatty acid or a C₂₀-C₂₂-all-cis-omega-3 methylene-interrupted fatty acid, preferably
wherein the hydrophobic moiety is selected from the group comprising or consisting of *all-cis-*5,8,11,14,17-eicosapentaenoic acid (EPA), *all-cis-*4,7,10,13,16,19-docosahexaenoic acid (DHA), *all-cis-*7,10,13,16,19-docosapentaenoic acid (n-3 DPA), *all-cis-*8,11,14,17-eicosatetraenoic acid (ETA), and *all-cis-*6,9,12,15,18-heneicosapentaenoic acid (HPA), to *all-cis-*4,7,10,13,16-docosapentaenoic acid (n-6 DPA), *all-cis-*7,10,13,16-docosatetraenoic acid, *all-cis-*5,8,11,14-eicosatetraenoic acid, and *all-cis-*8,11,14-eicosatrienoic acid,
more preferably
wherein the hydrophobic moiety is selected from the group comprising or consisting of *all-cis-*5,8,11,14,17-eicosapentaenoic acid (EPA), *all-cis-*4,7,10,13,16,19-docosahexaenoic acid (DHA), and *all-cis-*7,10,13,16,19-docosapentaenoic acid (n-3 DPA),
particularly preferably
wherein the hydrophobic moiety is *all-cis-*5,8,11,14,17-eicosapentaenoic acid (EPA).

Preferably, the second peptide part comprises an amino acid selected from asparagine, glutamine, arginine, histidine, and lysine, preferably selected from asparagine, glutamine, and lysine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

Preferably, the second peptide part comprises maximum one amino acid selected from the group comprising or consisting of cysteine, serine, threonine, and lysine. Preferably, the β-sheet forming second peptide part preferably consists of 2 - 12 amino acids, more preferably 3 - 10 amino acids, more preferably 4-9 amino acids, even more preferably 5 - 7 amino acids.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the hydrophobic moiety is selected from the group comprising or consisting of *all-cis-*5,8,11,14,17-eicosapentaenoic acid (EPA), *all-cis-*4,7,10,13,16,19-docosahexaenoic acid (DHA), *all-cis-*7,10,13,16,19-docosapentaenoic acid (n-3 DPA), *all-cis-*8,11,14,17-eicosatetraenoic acid (ETA), and *all-cis-*6,9,12,15,18-heneicosapentaenoic acid (HPA), to *all-cis-*4,7,10,13,16-docosapentaenoic acid (n-6 DPA), *all-cis-7,10,13,16-docosatetraenoic* acid, *all-cis-5,8,11,14-eicosatetraenoic* acid, and *all-cis-*8,11,14-eicosatrienoic acid.

Preferably, the second peptide part comprises an amino acid selected from asparagine, glutamine, arginine, histidine, and lysine, preferably selected from asparagine, glutamine, and lysine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

Preferably, the second peptide part comprises maximum one amino acid selected from the group comprising or consisting of cysteine, serine, threonine, and lysine.

Preferably, the β-sheet forming second peptide part preferably consists of 2 - 12 amino acids, more preferably 3 - 10 amino acids, more preferably 4-9 amino acids, even more preferably 5 - 7 amino acids.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the hydrophobic moiety is selected from the group comprising or consisting of *all-cis-5,8,11,14,17-eicosapentaenoic* acid (EPA), a//-c/s-4,7,10,13,16,19-docosahexaenoic acid (DHA), and *all-cis-*7,10,13,16,19-docosapentaenoic acid (n-3 DPA).

Preferably, the second peptide part comprises an amino acid selected from asparagine, glutamine, arginine, histidine, and lysine, preferably selected from asparagine, glutamine, and lysine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

Preferably, the second peptide part comprises maximum one amino acid selected from the group comprising or consisting of cysteine, serine, threonine, and lysine. Preferably, the β-sheet forming second peptide part preferably consists of 2 - 12 amino acids, more preferably 3 - 10 amino acids, more preferably 4-9 amino acids, even more preferably 5 - 7 amino acids.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part; and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the hydrophobic moiety is selected from the group comprising or consisting of a polyunsaturated C₂₀-C₂₂-omega-3 fatty acid, a polyunsaturated C₂₀-C₂₂-omega-6 fatty acid, a monounsaturated C₁₈-C₂₄-omega 3 fatty acid, a monounsaturated C₁₈-C₂₄-omega 6 fatty acid, a monounsaturated C₁₈-C₂₄-omega 9 fatty acid, and a saturated C₁₄-C₂₄- fatty acid,
wherein the amino acid at the second end of the second peptide part is selected from the group comprising or consisting of cysteine, serine, threonine, and lysine preferably cysteine or serine, more preferably cysteine, and
wherein the hydrophobic moiety is covalently coupled to the side chain of the amino acid at the second end of the second peptide part, and
wherein the hydrophobic moiety is covalently coupled to the side chain of the amino acid at the second end of the second peptide part through an ester linkage or thioester linkage or amide linkage.

Preferably, the second peptide part comprises an amino acid selected from asparagine, glutamine, arginine, histidine, and lysine, preferably selected from asparagine, glutamine, and lysine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part. In such embodiments, it is preferred that #the amino acid at the N-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the N-terminus of the second peptide part. Thus, the hydrophobic moiety is covalently coupled to the N-terminus of the second peptide part through an ester linkage, thioester linkage or amide linkage. In preferred embodiments, the amino acid at the N-terminus of the second peptide part is cysteine, wherein the hydrophobic moiety is covalently coupled to the N-terminus of the second peptide part through a thioester linkage. In further preferred embodiments the amino acid at the N-terminus of the second peptide part is serine, or threonine, preferably serine, wherein the hydrophobic moiety is covalently coupled to the N-terminus of the second peptide part through an ester linkage. In further preferred embodiments the amino acid at the N-terminus of the second peptide part is lysine, wherein the hydrophobic moiety is covalently coupled to N-terminus of the second peptide part through the amino group in the side chain of lysine through an amide linkage.

In further preferred embodiments, the first amino acid is the amino acid at the C-terminus of the first peptide part, and the terminal amino acid is the amino acid at the N-terminus of the first peptide part, and first end is the N-terminal end of the β-sheet forming second peptide part, and the second end is the C-terminal end of the β-sheet forming second peptide part. In such embodiments, it is preferred that the amino acid at the C-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the C-terminus of the second peptide part. Thus, the hydrophobic moiety is covalently coupled to the C-terminus of the second peptide part through an ester linkage, thioester linkage or amide linkage. In preferred embodiments, the amino acid at the C-terminus of the second peptide part is cysteine, wherein the hydrophobic moiety is covalently coupled to the C-terminus of the second peptide part through a thioester linkage. In further preferred embodiments the amino acid at the C-terminus of the second peptide part is serine, or threonine, preferably serine, wherein the hydrophobic moiety is covalently coupled to the C-terminus of the second peptide part through an ester linkage. In further preferred embodiments the amino acid at the C-terminus of the second peptide part is lysine, wherein the hydrophobic moiety is covalently coupled to C-terminus of the second peptide part through the amino group in the side chain of lysine through an amide linkage.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine, and
wherein the amino acid at the second end of the second peptide part is cysteine, and the hydrophobic moiety is covalently coupled to the side chain of the amino acid at the second end of the second peptide part through a thioester linkage; or
wherein the amino acid at the second end of the second peptide part is serine or threonine, preferably serine, and the hydrophobic moiety is covalently coupled to the side chain of the amino acid at the second end of the second peptide part through an ester linkage; or
wherein the amino acid at the second end of the second peptide part is lysine, and the hydrophobic moiety is covalently coupled to the side chain of the amino acid at the second end of the second peptide part through an amide linkage,
wherein the hydrophobic moiety is a C₂₀-C₂₂-all-cis-omega-3 methylene-interrupted fatty acid or a C₂₀-C₂₂-all-cis-omega-3 methylene-interrupted fatty acid, preferably wherein the hydrophobic moiety is selected from the group comprising or consisting of *all-cis-5,8,11,14,17-eicosapentaenoic* acid (EPA), a//-c/s-4,7,10,13,16,19-docosahexaenoic acid (DHA), *all-cis-*7,10,13,16,19-docosapentaenoic acid (n-3 DPA), *all-cis-*8,11,14,17-eicosatetraenoic acid (ETA), and *all-cis-*6,9,12,15,18-heneicosapentaenoic acid (HPA), to *all-cis-*4,7,10,13,16-docosapentaenoic acid (n-6 DPA), *all-cis-*7,10,13,16-docosatetraenoic acid, *all-cis-*5,8,11,14-eicosatetraenoic acid, and *all-cis-*8,11,14-eicosatrienoic acid,
more preferably
wherein the hydrophobic moiety is selected from the group comprising or consisting of *all-cis-*5,8,11,14,17-eicosapentaenoic acid (EPA), *all-cis-*4,7,10,13,16,19-docosahexaenoic acid (DHA), and *all-cis-*7,10,13,16,19-docosapentaenoic acid (n-3 DPA),
particularly preferably
wherein the hydrophobic moiety is *all-cis-*5,8,11,14,17-eicosapentaenoic acid (EPA).

Preferably, the second peptide part further comprises an amino acid selected from asparagine, glutamine, arginine, histidine, and lysine, preferably selected from asparagine, glutamine, and lysine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

Preferably, the second peptide part comprises maximum one amino acid selected from the group comprising or consisting of cysteine, serine, threonine, and lysine. Preferably, the β-sheet forming second peptide part preferably consists of 2 - 12 amino acids, more preferably 3 - 10 amino acids, more preferably 4-9 amino acids, even more preferably 5 - 7 amino acids.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part. In such embodiments, it is preferred that #the amino acid at the N-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the N-terminus of the second peptide part. Thus, the hydrophobic moiety is covalently coupled to the N-terminus of the second peptide part through an ester linkage, thioester linkage or amide linkage. In preferred embodiments, the amino acid at the N-terminus of the second peptide part is cysteine, wherein the hydrophobic moiety is covalently coupled to the N-terminus of the second peptide part through a thioester linkage. In further preferred embodiments the amino acid at the N-terminus of the second peptide part is serine, or threonine, preferably serine, wherein the hydrophobic moiety is covalently coupled to the N-terminus of the second peptide part through an ester linkage. In further preferred embodiments the amino acid at the N-terminus of the second peptide part is lysine, wherein the hydrophobic moiety is covalently coupled to N-terminus of the second peptide part through the amino group in the side chain of lysine through an amide linkage.

In further preferred embodiments, the first amino acid is the amino acid at the C-terminus of the first peptide part, and the terminal amino acid is the amino acid at the N-terminus of the first peptide part, and first end is the N-terminal end of the β-sheet forming second peptide part, and the second end is the C-terminal end of the β-sheet forming second peptide part. In such embodiments, it is preferred that the amino acid at the C-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the C-terminus of the second peptide part. Thus, the hydrophobic moiety is covalently coupled to the C-terminus of the second peptide part through an ester linkage, thioester linkage or amide linkage. In preferred embodiments, the amino acid at the C-terminus of the second peptide part is cysteine, wherein the hydrophobic moiety is covalently coupled to the C-terminus of the second peptide part through a thioester linkage. In further preferred embodiments the amino acid at the C-terminus of the second peptide part is serine, or threonine, preferably serine, wherein the hydrophobic moiety is covalently coupled to the C-terminus of the second peptide part through an ester linkage. In further preferred embodiments the amino acid at the C-terminus of the second peptide part is lysine, wherein the hydrophobic moiety is covalently coupled to C-terminus of the second peptide part through the amino group in the side chain of lysine through an amide linkage.

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine;
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid, and

wherein the β-sheet forming second peptide part comprises at least 55 % amino acids selected from alanine, valine, leucine, isoleucine, and glycine, and further comprises maximum 45 % glycine; and
wherein the hydrophobic moiety is a C₂₀-C₂₂-all-cis-omega-3 methylene-interrupted fatty acid or a C₂₀-C₂₂-all-cis-omega-6 methylene-interrupted fatty acid, and
wherein the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm² in said aqueous medium.

In preferred embodiments, the second peptide part has the peptide sequence LNGGV (SEQ ID NO 13), AKAVG (SEQ ID NO 65), ALAAG (SEQ ID NO 64), or VWAAA (SEQ ID NO 74).

In preferred embodiments, the amino acid at the second end of the second peptide part is selected from the group comprising or consisting of cysteine, serine, threonine, and lysine, and wherein the hydrophobic moiety is covalently coupled to the side chain of said amino acid through a thioester linkage, an ester linkage or amide linkage.In such preferred embodiments, the second peptide part has preferably the peptide sequence CLNGGV (SEQ ID NO 68), CAKAVG (SEQ ID NO 72), CALAAG (SEQ ID NO 70), CVWAAA (SEQ ID NO 66), SLNGGV (SEQ ID NO 69), SAKAVG (SEQ ID NO 73), SALAAG (SEQ ID NO 71), or SVWAAA (SEQ ID NO 67).

Thus, the present invention preferably relates to a self-assembling peptide amphiphile consisting of:
(i) a non-self-assembling peptide moiety having a positive net charge under physiological pH, wherein the peptide moiety consist of at least 5 amino acids, wherein less than 50 % of the at least 5 amino acids are lysine, arginine, and histidine;
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid, and
wherein the β-sheet forming second peptide part comprises at least 55 % amino acids selected from alanine, valine, leucine, isoleucine, and glycine, and further comprises maximum 45 % glycine; and
(a) wherein the β-sheet forming second peptide part comprises at least three different amino acids selected from the group comprising or consisting of alanine, glycine, valine, leucine, and isoleucine, or
(b) wherein the β-sheet forming second peptide part comprises an amino acid selected from asparagine, glutamine, lysine, and wherein the amino acid at second end of the second peptide part is alanine, valine, leucine, or isoleucine; or
(c) wherein the amino acid at the second end of the second peptide part is selected from the group comprising or consisting of cysteine, serine, and threonine, and wherein the hydrophobic moiety is covalently coupled to the side chain of said cysteine, serine, or threonine, through a thioester linkage, or an ester linkage;
and
wherein the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm² in said aqueous medium.

In preferred embodiments, the hydrophobic moiety is a C₂₀-C₂₂-all-cis-omega-3 methylene-interrupted fatty acid or a C₂₀-C₂₂-all-cis-omega-6 methylene-interrupted fatty acid, preferably wherein the hydrophobic moiety is eicosapentaenoic acid.

In preferred embodiments, the second peptide part has preferably the peptide sequence LNGGV (SEQ ID NO 13), AKAVG (SEQ ID NO 65), or ALAAG (SEQ ID NO 64).

Preferred is a self-assembling peptide amphiphile selected from

| | |
|---|---|
| C₁₆-*ester*-SVVVAAAKKK-NH₂ | (SEQ ID NO 10), |
| C₁₆-*thioester*-CVVVAAAKKK-NH₂ | (SEQ ID NO 12), |
| eicosapentaene acid-VVVAAAKKK-NH₂ | (SEQ ID NO 14), |
| C₁₆-LNGGVKK-NH₂ | (SEQ ID NO 20), |
| C₁₆-ALAAGKK-NH₂ | (SEQ ID NO 22), |
| C₁₆-AKAVGK-NH₂ | (SEQ ID NO 24), |
| C₁₆-IIQKIK-NH₂ | (SEQ ID NO 26), |
| C₁₆-AGGRVK-NH₂ | (SEQ ID NO 28), and |
| C₁₆-LLLKK-NH₂ | (SEQ ID NO 30). |

The present invention further relates to a composition comprising a peptide amphiphile as described herein in aqueous medium, optionally comprising a non-pathogenic viral particle, wherein the peptide amphiphile self-assembles into fibrils having a diameter between 5 - 20 nm and a length greater than 100 nm in aqueous medium, and wherein said fibrils form µm-sized fibrillar aggregates in the area range between 10 µm² to 1000 µm² in said aqueous medium.

### Use

The present invention further relates to the use of a peptide amphiphile as retroviral transduction enhancer for retroviral gene delivery into cells, wherein the peptide amphiphile consisting of:
(i) a non-self-assembling peptide moiety having a positive net charge under physiological pH and having an amino acid sequence that gives rise to ordered β-sheet structures, and
(ii) a hydrophobic moiety covalently coupled to the peptide moiety,
wherein the peptide amphiphile self-assembles into fibrils having a diameter between 5 - 20 nm and a length greater than 100 nm in aqueous medium, and wherein said fibrils form µm-sized fibrillar aggregates in the area range between 10 µm² to 1000 µm² in said aqueous medium.

Thus, the present invention further relates to the use of a peptide amphiphile as retroviral transduction enhancer for retroviral gene delivery into cells, wherein the peptide amphiphile comprises:
(i) a non-self-assembling peptide moiety having a positive net charge under physiological pH, wherein the peptide moiety consist of at least 5 amino acids, wherein less than 50 % of the at least 5 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid.

According to the invention the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm², preferably in the area range between 10 µm² to 200 µm² in said aqueous medium , more preferably in the area range between 10 µm² to 100 µm² in said aqueous medium. Moreover, the fibrils of the self-assembled peptide forming the µm-sized fibrillar aggregates amphiphile have a zeta-potential of > 5 mV at pH 7.4, and at 298K, preferably a zeta-potential of at least > 10 mV at pH 7.4, 298K, and particularly preferably a zeta-potential of at least > 15 mV at pH 7.4, 298K in said aqueous medium.

The present invention further relates to the use of a peptide amphiphile as retroviral transduction enhancer for retroviral gene delivery into cells, wherein the peptide amphiphile comprises:
(i) a non-self-assembling peptide moiety having a positive net charge under physiological pH, wherein the peptide moiety consist of at least 5 amino acids, wherein less than 50 % of the at least 4 amino acids are lysine, arginine, and histidine; and
   wherein the non-self-assembling peptide moiety further comprises:
   (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
   (ib) a β-sheet forming second peptide part consisting of at least 3 amino acids, wherein a first end of the β-sheet forming second peptide part is covalently coupled to the first amino acid of the first peptide part;
   and
(ii) a hydrophobic moiety covalently coupled to the second end of the β-sheet forming second peptide part, wherein the hydrophobic moiety is selected from the group comprising or consisting of a C₁₄-C₂₄-fatty acid,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

In preferred embodiments hydrophobic moiety is selected from the group comprising or consisting of a polyunsaturated C₂₀-C₂₂-omega-3 fatty acid, a polyunsaturated C₂₀-C₂₂-omega-6 fatty acid, a monounsaturated C₁₈-C₂₄-omega 3 fatty acid, a monounsaturated C₁₈-C₂₄-omega 6 fatty acid, a monounsaturated C₁₈-C₂₄-omega 9 fatty acid, and a saturated C₁₄-C₂₄- fatty acid.

In preferred embodiments, the hydrophobic moiety may selected from the group comprising or consisting of a C₂₀-C₂₂-all-cis-omega-3 methylene-interrupted fatty acid or a C₂₀-C₂₂-all-cis-omega-3 methylene-interrupted fatty acid, preferably 3wherein the hydrophobic moiety is selected from the group comprising or consisting of *all-cis-*5,8,11,14,17-eicosapentaenoic acid (EPA), *all-cis-*4,7,10,13,16,19-docosahexaenoic acid (DHA), *all-cis-*7,10,13,16,19-docosapentaenoic acid (n-3 DPA), *all-cis-*8,11,14,17-eicosatetraenoic acid (ETA), and *all-cis-*6,9,12,15,18-heneicosapentaenoic acid (HPA), to *all-cis-*4,7,10,13,16-docosapentaenoic acid (n-6 DPA), *all-cis-*7,10,13,16-docosatetraenoic acid, *all-cis-*5,8,11,14-eicosatetraenoic acid, and *all-cis-*8,11,14-eicosatrienoic acid,
more preferably
4wherein the hydrophobic moiety is selected from the group comprising or consisting of *all-cis-*5,8,11,14,17-eicosapentaenoic acid (EPA), *all-cis-*4,7,10,13,16,19-docosahexaenoic acid (DHA), and *all-cis-*7,10,13,16,19-docosapentaenoic acid (n-3 DPA),
particularly preferably
5 wherein the hydrophobic moiety is *all-cis-*5,8,11,14,17-eicosapentaenoic acid (EPA).

In preferred embodiments, the amino acid at the second end of the second peptide part is alanine, leucine, or isoleucine.

In further preferred embodiments, the amino acid wherein the amino acid at the second end of the second peptide part is selected from the group comprising or consisting of cysteine, serine, threonine, and lysine preferably cysteine or serine, more preferably cysteine, wherein the hydrophobic moiety is covalently coupled to the side chain of the amino acid at the second end of the second peptide part, and wherein the hydrophobic moiety is covalently coupled to the side chain of the amino acid at the second end of the second peptide part through an ester linkage or thioester linkage or amide linkage. In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part. In such preferred embodiments, it may be preferred that the amino acid at the N-terminus of the second peptide part is alanine, leucine, or isoleucine.

In further preferred embodiments, it may be preferred that the amino acid at the N-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the N-terminus of the second peptide part. Thus, the hydrophobic moiety is covalently coupled to the N-terminus of the second peptide part through an ester linkage, thioester linkage or amide linkage. In preferred embodiments, the amino acid at the N-terminus of the second peptide part is cysteine, wherein the hydrophobic moiety is covalently coupled to the N-terminus of the second peptide part through a thioester linkage. In further preferred embodiments the amino acid at the N-terminus of the second peptide part is serine, or threonine, preferably serine, wherein the hydrophobic moiety is covalently coupled to the N-terminus of the second peptide part through an ester linkage. In further preferred embodiments the amino acid at the N-terminus of the second peptide part is lysine, wherein the hydrophobic moiety is covalently coupled to N-terminus of the second peptide part through the amino group in the side chain of lysine through an amide linkage.

In further preferred embodiments, the first amino acid is the amino acid at the C-terminus of the first peptide part, and the terminal amino acid is the amino acid at the N-terminus of the first peptide part, and first end is the N-terminal end of the β-sheet forming second peptide part, and the second end is the C-terminal end of the β-sheet forming second peptide part. In such embodiments, it is preferred that the amino acid at the C-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the C-terminus of the second peptide part. Thus, the hydrophobic moiety is covalently coupled to the C-terminus of the second peptide part through an ester linkage, thioester linkage or amide linkage. In preferred embodiments, the amino acid at the C-terminus of the second peptide part is cysteine, wherein the hydrophobic moiety is covalently coupled to the C-terminus of the second peptide part through a thioester linkage. In further preferred embodiments the amino acid at the C-terminus of the second peptide part is serine, or threonine, preferably serine, wherein the hydrophobic moiety is covalently coupled to the C-terminus of the second peptide part through an ester linkage. In further preferred embodiments the amino acid at the C-terminus of the second peptide part is lysine, wherein the hydrophobic moiety is covalently coupled to C-terminus of the second peptide part through the amino group in the side chain of lysine through an amide linkage.

### Method

The present invention further relates to an *in vitro* method for retroviral gene delivery into cells comprising the following steps:
a) providing fibrils of a self-assembled peptide amphiphile dissolved in an aqueous medium, wherein the fibrils have a diameter between 5 - 20 nm and a length greater than 100 nm, and wherein the fibrils form µm-sized fibrillar aggregates in the area range between 10 µm² to 200 µm² in said aqueous medium, wherein the fibrils of the self-assembled peptide forming the µm-sized fibrillar aggregates amphiphile have a zeta-potential of > 5 mV at pH 7.4, and at 298 K; and
   the self-assembling peptide amphiphile consists of:
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
   and
b) incubating the cells with the fibrils of the self-assembled peptide amphiphile in the presence of a non-pathogenic viral particle for gene delivery,
wherein said zeta-potential is measurable by dynamic light scattering.

Preferably, the aqueous medium is phosphate buffered saline (PBS) or water. Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured ex *vivo,* e.g. primary cells. Preferably, the non-pathogenic viral particle is a non-pathogenic retroviral particle, i.e. a retroviral vector.

Preferably the genetic material delivered by the inventive method encodes a chimeric antigen receptor (CAR) and the cells are T cells, and preferably CD4+ T cells.

The present invention further relates to an *in vitro* method for retroviral gene delivery into cells comprising the following steps:
a) providing an aqueous medium containing fibrils of a self-assembled peptide amphiphile, wherein the fibrils have a diameter between 5 - 20 nm and a length greater than 100 nm and wherein the fibrils form µm-sized fibrillar aggregates in the area range between 10 µm² to 1000 µm² in said aqueous medium, wherein the fibrils of the self-assembled peptide amphiphile forming the µm-sized fibrillar aggregates have a positive zeta-potential of > 5 mV at pH 7.4, and at 298K; and wherein the peptide amphiphile consisting of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
   and
b) incubating the cells with the aqueous medium containing the fibrils of the self-assembled peptide amphiphile in the presence of a non-pathogenic viral particle for gene delivery.

Preferably, the aqueous medium is phosphate buffered saline (PBS) or water. Preferably, the aqueous medium is water or phosphate buffered saline (PBS).

Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured ex *vivo,* e.g. primary cells. Preferably, the non-pathogenic viral particle is a non-pathogenic retroviral particle, i.e. a retroviral vector.

Preferably the genetic material delivered by the inventive method encodes a chimeric antigen receptor (CAR) and the cells are T cells, and preferably CD4+ T cells.

The present invention further relates to an *in vitro* method for retroviral gene delivery into cells comprising the following steps:
a) providing fibrils of a self-assembled peptide amphiphile in an aqueous medium, wherein the fibrils have a diameter between 5 - 20 nm and a length greater than 100 nm and wherein the fibrils form µm-sized fibrillar aggregates in the area range between 10 µm² to 1000 µm² in said aqueous medium, wherein the fibrils of the self-assembled peptide amphiphile forming the µm-sized fibrillar aggregates have a positive zeta-potential of > 5 mV at pH 7.4, and at 298K; and wherein the peptide amphiphile consisting of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
   and
b) incubating the cells with the fibrils of the self-assembled peptide amphiphile in the presence of a non-pathogenic viral particle for gene delivery.

Preferably, the aqueous medium is phosphate buffered saline (PBS) or water. Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured ex *vivo,* e.g. primary cells. Preferably, the non-pathogenic viral particle is a non-pathogenic retroviral particle, i.e. a retroviral vector.

Preferably the genetic material delivered by the inventive method encodes a chimeric antigen receptor (CAR) and the cells are T cells, and preferably CD4+ T cells.

The present invention further relates to an *in vitro* method for retroviral gene delivery into cells comprising the following steps:
a) providing an aqueous medium containing a self-assembling peptide amphiphile consisting of
   (i) a non-self-assembling peptide moiety having a positive net charge under physiological pH, wherein the non-self-assembling peptide moiety further comprises:
      (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
      (ib) a β-sheet forming second peptide part consisting of at least 3 amino acids, wherein a first end of the β-sheet forming second peptide part is covalently coupled to the first amino acid of the first peptide part;
      and
   (ii) a hydrophobic moiety covalently coupled to the second end of the β-sheet forming second peptide part, wherein the hydrophobic moiety is a C₁₄-C₂₄-fatty acid;
   wherein the peptide amphiphile self-assembles into fibrils having a diameter between 5 - 20 nm and a length greater than 100 nm in said aqueous medium, and wherein said fibrils form µm-sized fibrillar aggregates in the area range between 10 µm² to 1000 µm² in said aqueous medium; and
b) incubating the cells with the fibrils of the self-assembled peptide amphiphile in the presence of a non-pathogenic viral particle for gene delivery.

According to the invention the fibrils and/or µm-sized fibrillar aggregates of the peptide amphiphiles have a positiv zeta-potential of at least > 5 mV at pH 7.4, and at 298K. Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured ex *vivo,* e.g. primary cells. Preferably, the non-pathogenic viral particle is a non-pathogenic retroviral particle, i.e. a retroviral vector.

Preferably the genetic material delivered by the inventive method encodes a chimeric antigen receptor (CAR) and the cells are T cells, and preferably CD4+ T cells.

The present invention further relates to an *in vitro* method for retroviral gene delivery into cells comprising the following steps:
a) providing fibrils of self-assembled peptide amphiphiles having a diameter between 5 - 20 nm and a length greater than 100 nm and forming µm-sized fibrillar aggregates in the area range between 10 µm² to 1000 µm² in aqueous medium,
   wherein the peptide amphiphiles consist of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
b) incubating the cells with the fibrils of the self-assembled peptide amphiphile in the presence of a non-pathogenic viral particle for gene delivery.

According to the invention the fibrils and/or µm-sized fibrillar aggregates of the peptide amphiphiles have a zeta-potential of at least > 5 mV at pH 7.4, and at 298K. Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured ex *vivo,* e.g. primary cells. Preferably, the non-pathogenic viral particle is a non-pathogenic retroviral particle, i.e. a retroviral vector.

Preferably the genetic material delivered by the inventive method encodes a chimeric antigen receptor (CAR) and the cells are T cells, and preferably CD4+ T cells.

The present invention further relates to an *in vitro* method for retroviral gene delivery into cells comprising the following steps:
a') providing a peptide amphiphile consisting of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
a") incubating the peptide amphiphile in an aqueous medium to induce fibril formation of the peptide amphiphile.

Preferably, the peptide amphiphile is incubated in an aqueous medium to induce fibril formation of the peptide amphiphile at step a") for at least 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, and most preferred for at least 24 hours.

According to the invention the fibrils and/or µm-sized fibrillar aggregates of the peptide amphiphiles have a zeta-potential of at least > 5 mV at pH 7.4, and at 298K. Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured ex *vivo,* e.g. primary cells. Preferably, the non-pathogenic viral particle is a non-pathogenic retroviral particle, i.e. a retroviral vector.

Preferably the genetic material delivered by the inventive method encodes a chimeric antigen receptor (CAR) and the cells are T cells, and preferably CD4+ T cells.

The present invention further relates to an *in vitro* method for retroviral gene delivery into cells comprising the following steps:
a') providing a peptide amphiphile consisting of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
a") incubating the peptide amphiphile at 1 mg/mL concentration into an aqueous medium, pH 7.4 to induce fibril formation of the peptide amphiphile.

The present invention further relates to an *in vitro* method for retroviral gene delivery into cells further comprising the following steps:
a') providing a peptide amphiphile consisting of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
a") predissolving the peptide amphiphile at 10 mg/mL concentration in dimethylsulfoxid (DMSO) and subsequently diluting to 1 mg/mL in aqueous medium, pH 7.4 to induce fibril formation of the peptide amphiphile.

The present invention further relates to an *in vitro* method for retroviral gene delivery into cells further comprising the following steps:
a') providing a peptide amphiphile consisting of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
a") incubating the peptide amphiphile in an aqueous medium to induce fibril formation of the peptide amphiphile,
   wherein the fibrils have a diameter between 5 - 20 nm and a length greater than 100 nm and form µm-sized fibrillar aggregates in the area range between 10 µm² to 1000 µm² in aqueous medium.

According to the invention the fibrils and/or µm-sized fibrillar aggregates of the peptide amphiphiles have a zeta-potential of at least > 5 mV at pH 7.4, and at 298K. Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured ex *vivo,* e.g. primary cells. Preferably, the non-pathogenic viral particle is a non-pathogenic retroviral particle, i.e. a retroviral vector.

Preferably the genetic material delivered by the inventive method encodes a chimeric antigen receptor (CAR) and the cells are T cells, and preferably CD4+ T cells.

The present invention further relates to an *in vitro* method for retroviral gene delivery into cells further comprising the following steps:
a') providing a peptide amphiphile consisting of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
a") incubating the peptide amphiphile at 1 mg/mL concentration into an aqueous medium, pH 7.4 to induce fibril formation of the peptide amphiphile wherein the fibrils have a diameter between 5 - 20 nm and a length greater than 100 nm and form µm-sized fibrillar aggregates in the area range between 10 µm² to 1000 µm² in aqueous medium.

Preferably, the present invention relates to an *in vitro* method for retroviral gene delivery into cells further comprising the following steps:
a') providing a peptide amphiphile consisting of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
a") predissolving the peptide amphiphile at 10 mg/mL concentration in dimethylsulfoxid (DMSO) and subsequently diluting to 1 mg/mL in aqueous medium, pH 7.4 to induce fibril formation of the peptide amphiphile,
   wherein the fibrils have a diameter between 5 - 20 nm and a length greater than 100 nm and form µm-sized fibrillar aggregates in the area range between 10 µm² to 1000 µm² in aqueous medium.

According to the invention the fibrils and/or µm-sized fibrillar aggregates of the peptide amphiphiles have a zeta-potential of at least > 5 mV at pH 7.4, and at 298K. Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured ex *vivo,* e.g. primary cells. Preferably, the non-pathogenic viral particle is a non-pathogenic retroviral particle, i.e. a retroviral vector.

Preferably the genetic material delivered by the inventive method encodes a chimeric antigen receptor (CAR) and the cells are T cells, and preferably CD4+ T cells.

The present invention further relates to an *in vitro* method for retroviral gene delivery into cells comprising the following steps:
a') providing a peptide amphiphile consisting of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
a") incubating the peptide amphiphile in an aqueous medium to induce fibril formation of the peptide amphiphile,
a) providing fibrils of the peptide amphiphiles have a diameter between 5 - 20 nm and a length greater than 100 nm and form µm-sized fibrillar aggregates in the area range between 10 µm² to 1000 µm² in aqueous medium,
b) incubating the cells with the fibrils of the self-assembled peptide amphiphile in the presence of a non-pathogenic viral particle for gene delivery.

According to the invention the fibrils and/or µm-sized fibrillar aggregates of the peptide amphiphiles have a zeta-potential of at least > 5 mV at pH 7.4, and at 298K. Preferably, the aqueous medium is water or phosphate buffered saline (PBS). Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured ex *vivo,* e.g. primary cells. Preferably, the non-pathogenic viral particle is a non-pathogenic retroviral particle, i.e. a retroviral vector.

Preferably the genetic material delivered by the inventive method encodes a chimeric antigen receptor (CAR) and the cells are T cells, and preferably CD4+ T cells.

With other words, the present invention further relates to an *in vitro* method for retroviral gene delivery into cells comprising the following steps:
a') providing a peptide amphiphile consisting of
   (i) a non-self-assembling peptide moiety having a positive net charge under physiological pH and having an amino acid sequence that gives rise to ordered β-sheet structures, and
   (ii) a hydrophobic moiety covalently coupled to the peptide moiety,
a") incubating the peptide amphiphile in an aqueous medium to induce fibril formation of the peptide amphiphile,
a) providing fibrils of the peptide amphiphiles have a diameter between 5 - 20 nm and a length greater than 100 nm and form µm-sized fibrillar aggregates in the area range between 10 µm² to 1000 µm² in aqueous medium,
b) incubating the cells with the fibrils of the self-assembled peptide amphiphile in the presence of a non-pathogenic viral particle for gene delivery.

According to the invention the fibrils and/or µm-sized fibrillar aggregates of the peptide amphiphiles have a zeta-potential of at least > 5 mV at pH 7.4, and at 298K. Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured ex *vivo,* e.g. primary cells. Preferably, the non-pathogenic viral particle is a non-pathogenic retroviral particle, i.e. a retroviral vector.

Preferably the genetic material delivered by the inventive method encodes a chimeric antigen receptor (CAR) and the cells are T cells, and preferably CD4+ T cells.

The present invention preferably relates to an *in vitro* method for retroviral gene delivery into cells comprising the following steps:
a) providing fibrils of a self-assemblied peptide amphiphile having a diameter between 5 - 20 nm and a length greater than 100 nm and forming µm-sized fibrillar aggregates in the area range between 10 µm² to 1000 µm² in an aqueous medium,
   wherein the peptide amphiphile consisting of
   (i) a non-self-assembling peptide moiety having a positive net charge under physiological pH, wherein the peptide moiety consist of at least 5 amino acids, wherein less than 50 % of the at least 5 amino acids are lysine, arginine, and histidine; and
      wherein the non-self-assembling peptide moiety further comprises:
      (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
      (ib) a β-sheet forming second peptide part consisting of at least 3 amino acids, wherein a first end of the β-sheet forming second peptide part is covalently coupled to the first amino acid of the first peptide part;
      and
   (ii) a hydrophobic moiety covalently coupled to the second end of the β-sheet forming second peptide part, wherein the hydrophobic moiety is selected from the group comprising or consisting of a C₁₄-C₂₄-fatty acid.
b) incubating the cells with the fibrils of the self-assembled peptide amphiphile in the presence of a non-pathogenic viral particle for gene delivery.

According to the invention the fibrils and/or µm-sized fibrillar aggregates of the peptide amphiphiles have a zeta-potential of at least > 5 mV at pH 7.4, and at 298K. Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured *ex vivo,* e.g. primary cells. Preferably, the non-pathogenic viral particle is a non-pathogenic retroviral particle, i.e. a retroviral vector. Preferably the genetic material delivered by the inventive method encodes a chimeric antigen receptor (CAR) and the cells are T cells, and preferably CD4+ T cells.

The present invention preferably relates to an *in vitro* method for retroviral gene delivery into cells comprising the following steps:
a) providing fibrils of self-assemblied peptide amphiphiles having a diameter between 5 - 20 nm and a length greater than 100 nm and forming µm-sized fibrillar aggregates in the area range between 10 µm² to 1000 µm² in aqueous medium,
   the peptide amphiphiles consisting of
   (i) a non-self-assembling peptide moiety having a positive net charge under physiological pH, wherein the peptide moiety consist of at least 5 amino acids, wherein less than 50 % of the at least 4 amino acids are lysine, arginine, and histidine; and
      wherein the non-self-assembling peptide moiety further comprises:
      (ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
      (ib) a β-sheet forming second peptide part consisting of at least 3 amino acids, wherein a first end of the β-sheet forming second peptide part is covalently coupled to the first amino acid of the first peptide part;
      and
   (ii) a hydrophobic moiety covalently coupled to the second end of the β-sheet forming second peptide part, wherein the hydrophobic moiety is selected from the group comprising or consisting of a C₁₄-C₂₄-fatty acid.
b) incubating the cells with the fibrils of the self-assembled peptide amphiphile in the presence of a non-pathogenic viral particle for gene delivery,

wherein the second peptide part comprises at least 40 % amino acids selected from alanine, valine, leucine, and isoleucine, and/or
wherein the second peptide part preferably comprises at least 55 % amino acids selected from alanine, valine, leucine, isoleucine, and glycine, and preferably further comprises maximum 45 % glycine.

According to the invention the fibrils and/or µm-sized fibrillar aggregates of the peptide amphiphiles have a zeta-potential of at least > 5 mV at pH 7.4, and at 298K. Preferably, the aqueous medium is water or phosphate buffered saline (PBS). Preferably, the cells are mammalian cells, and more preferably human cells. Also more preferably the cells are selected from the group comprising or consisting of T cells, CD4+ T cells, CD8+ T cells, TZM-bl cells, HeLa cells, and Jurkat cells. Said cells can be *in vitro* cultured cells, e.g. stabilized cell lines, cancer lines or similar, or human cells isolated from a subject specimen, for example from a subject blood or bone marrow or tissue, and cultured *ex vivo,* e.g. primary cells. Preferably, the non-pathogenic viral particle is a non-pathogenic retroviral particle, i.e. a retroviral vector. Preferably the genetic material delivered by the inventive method encodes a chimeric antigen receptor (CAR) and the cells are T cells, and preferably CD4+ T cells.

Preferably, the second peptide part further comprises at least three different amino acids selected from the group comprising or consisting of alanine, glycine, valine, leucine, and isoleucine.

Preferably, the second peptide part further comprises at least two amino acids selected from leucine and/or isoleucine, and preferably wherein the second peptide part comprises maximum three amino acids selected from leucine and/or isoleucine. Preferably, the second peptide part does not comprise a negatively charged amino acid, i.e. does not comprise aspartic acid or glutamic acid. Preferably, the second peptide part does not comprise proline.

In preferred embodiments hydrophobic moiety is selected from the group comprising or consisting of a polyunsaturated C₂₀-C₂₂-omega-3 fatty acid, a polyunsaturated C₂₀-C₂₂-omega-6 fatty acid, a monounsaturated C₁₈-C₂₄-omega 3 fatty acid, a monounsaturated C₁₈-C₂₄-omega 6 fatty acid, a monounsaturated C₁₈-C₂₄-omega 9 fatty acid, and a saturated C₁₄-C₂₄- fatty acid.

In preferred embodiments, the hydrophobic moiety may selected from the group comprising or consisting of a C₂₀-C₂₂-all-cis-omega-3 methylene-interrupted fatty acid or a C₂₀-C₂₂-all-cis-omega-3 methylene-interrupted fatty acid, preferably wherein the hydrophobic moiety is selected from the group comprising or consisting of *all-cis-*5,8,11,14,17*-eicosapentaenoic* acid (EPA), *all-cis-*4,7,10,13,16,19-docosahexaenoic acid (DHA), a//-c/s-7,10,13,16,19-docosapentaenoic acid (n-3 DPA), *all-cis-*8,11,14,17-eicosatetraenoic acid (ETA), and *all-cis*-6,9,12,15,18-heneicosapentaenoic acid (HPA), to a//-c/s-4,7,10,13,16-docosapentaenoic acid (n-6 DPA), *all-cis-*7,10,13,16-docosatetraenoic acid, *all-cis-*5,8,11,14-eicosatetraenoic acid, and *all-cis*-8,11,14-eicosatrienoic acid,
more preferably
wherein the hydrophobic moiety is selected from the group comprising or consisting of *all-cis*-5,8,11,14,17-eicosapentaenoic acid (EPA), *all-cis*-4,7,10,13,16,19-docosahexaenoic acid (DHA), and *all-cis*-7,10,13,16,19-docosapentaenoic acid (n-3 DPA),
particularly preferably
wherein the hydrophobic moiety is *all*-*cis*-5,8,11,14,17-eicosapentaenoic acid (EPA). In preferred embodiments, the amino acid at the second end of the second peptide part is alanine, leucine, or isoleucine.

In further preferred embodiments, the amino acid wherein the amino acid at the second end of the second peptide part is selected from the group comprising or consisting of cysteine, serine, threonine, and lysine preferably cysteine or serine, more preferably cysteine, wherein the hydrophobic moiety is covalently coupled to the side chain of the amino acid at the second end of the second peptide part, and wherein the hydrophobic moiety is covalently coupled to the side chain of the amino acid at the second end of the second peptide part through an ester linkage or thioester linkage or amide linkage. Preferably, the β-sheet forming second peptide part preferably consists of 2 - 12 amino acids, more preferably 3-10 amino acids, more preferably 4-9 amino acids, even more preferably 5 - 7 amino acids.

In preferred embodiments, the first amino acid is the amino acid at the N-terminus of the first peptide part, and the terminal amino acid is the amino acid at the C-terminus of the first peptide part, and first end is the C-terminal end of the β-sheet forming second peptide part, and the second end is the N-terminal end of the β-sheet forming second peptide part. In such preferred embodiments, it may be preferred that the amino acid at the N-terminus of the second peptide part is alanine, leucine, or isoleucine.

In further preferred embodiments, it may be preferred that the amino acid at the N-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the N-terminus of the second peptide part. Thus, the hydrophobic moiety is covalently coupled to the N-terminus of the second peptide part through an ester linkage, thioester linkage or amide linkage. In preferred embodiments, the amino acid at the N-terminus of the second peptide part is cysteine, wherein the hydrophobic moiety is covalently coupled to the N-terminus of the second peptide part through a thioester linkage. In further preferred embodiments the amino acid at the N-terminus of the second peptide part is serine, or threonine, preferably serine, wherein the hydrophobic moiety is covalently coupled to the N-terminus of the second peptide part through an ester linkage. In further preferred embodiments the amino acid at the N-terminus of the second peptide part is lysine, wherein the hydrophobic moiety is covalently coupled to N-terminus of the second peptide part through the amino group in the side chain of lysine through an amide linkage.

In further preferred embodiments, the first amino acid is the amino acid at the C-terminus of the first peptide part, and the terminal amino acid is the amino acid at the N-terminus of the first peptide part, and first end is the N-terminal end of the β-sheet forming second peptide part, and the second end is the C-terminal end of the β-sheet forming second peptide part. In such embodiments, it is preferred that the amino acid at the C-terminus of the second peptide part is selected from the group comprising or consisting of cysteine, serine, threonine, and lysine preferably cysteine or serine, more preferably cysteine, and wherein the hydrophobic moiety is covalently coupled to the side chain of the amino acid at the C-terminus of the second peptide part. Thus, the hydrophobic moiety is covalently coupled to the C-terminus of the second peptide part through an ester linkage, thioester linkage or amide linkage. In preferred embodiments, the amino acid at the C-terminus of the second peptide part is cysteine, wherein the hydrophobic moiety is covalently coupled to the C-terminus of the second peptide part through a thioester linkage. In further preferred embodiments the amino acid at the C-terminus of the second peptide part is serine, or threonine, preferably serine, wherein the hydrophobic moiety is covalently coupled to the C-terminus of the second peptide part through an ester linkage. In further preferred embodiments the amino acid at the C-terminus of the second peptide part is lysine, wherein the hydrophobic moiety is covalently coupled to C-terminus of the second peptide part through the amino group in the side chain of lysine through an amide linkage.

### Description of the figures

**Figure 1** shows the structures of PA1 (SEQ ID NO. 4), PA2 (SEQ ID NO. 8), PA3 (SEQ ID NO. 5) and the respective peptides without palmitic acid conjugation P1 (SEQ ID NO. 1), P2 (SEQ ID NO. 3), and P3 (SEQ ID NO. 2).
**Figure 2** shows representative TEM micrographs of a) P1 (SEQ ID NO. 1), b) P2 (SEQ ID NO. 3), and c) P3 (SEQ ID NO. 2) that reveal the morphologies of peptide assemblies (1 mg/mL in PBS). Formation of fibrils are observed for P2 (SEQ ID NO. 3), whereas P1 (SEQ ID NO. 1), and P3 (SEQ ID NO. 3) assemble in amorphous aggregates (scale bars 1 µm, inset: 100 nm).
**Figure 3** shows representative TEM micrographs of a) PA1 (SEQ ID NO. 4), b) PA2 (SEQ ID NO. 8), and c) PA3 (SEQ ID NO. 5) that reveal the morphologies of peptide assemblies (1 mg/mL in PBS). Formation of fibrils are observed for all three peptide amphiphiles (scale bars 1 µm, inset: 100 nm).
**Figure 4** shows the formation of µm-sized aggregates of PA1 (SEQ ID NO. 4) and PA2 (SEQ ID NO. 8) in PBS, whereas PA3 (SEQ ID NO. 5), P1 (SEQ ID NO. 1), P2 (SEQ ID NO. 3), and P3 (SEQ ID NO. 2) does not form µm-sized aggregates in PBS.
**Figure 5** shows an ATR-IR spectrum of the amide I and II regions of PA1 (SEQ ID NO. 4) and P1 (SEQ ID NO. 1) (1 mg/mL in PBS lyophilized).
**Figure 6** shows CD spectra displaying β-sheet secondary order for PA1-PA3 (SEQ ID NO. 4, 5, 8) and random coil conformation for P1 - P3 (SEQ ID NO. 1 -3) (all peptides 0.1 mg/mL in pure water).
**Figure 7** shows N-fold Thioflavin-T fluorescence intensity at 488 nm excitation for palmitic acid, PA1-PA3 (SEQ ID NO. 4, 5, 8) and P1 - P3 (SEQ ID NO. 1 -3) relative to PBS (control).
**Figure 8** shows peptide amphiphiles PA1 SEQ ID NO. 4) and PA2 (SEQ ID NO. 8) are potent enhancers of HIV-1 infection and γ-retroviral transduction. **A** Infectivity assay showing HIV-1 infection rates of TZM-bl cells observed in the presence of increasing concentrations of peptides PA1-PA3 (SEQ ID NO. 4, 5, 8) and P1-P3 (SEQ ID NO. 1-3) (6.5 µM, 1.3 µM, 0.26 µM). The baseline (0 µM) shows infection rate without the addition of peptides. Mean values and standard deviations are derived from three repetitions of triplicate infections. Two repetitions of triplicate infections were used for samples marked with an asterisk (*). RLU/s are relative light units per second. **B** Percentage of green fluorescent protein (GFP) positive Jurkat cells at indicated concentrations of peptides three days after transduction with GALV-pseudotyped γ-retroviral vector (GALV-RV). **C** Percentage of GFP positive CD4+ T cells after transduction with GALV-RV. Comparing the transduction efficiency enhancement of EF-C, PA1 (SEQ ID NO. 4) and PA2 (SEQ ID NO. 8) (6.5 µM, 1.3 µM, 0.26 µM) with benchmark retroviral transduction enhancers RetroNectin (20 µg/mL, 0.3 µM) and Vectofusin-1 (10 µg/mL, 3.3 µM). **D** CellTiter-Glo cell viability assay of Jurkat cells treated with increasing concentrations of the peptides of biological triplicate with standard deviation.
**Figure 9** **A** shows a representative laser scanning microscopy images of Proteostat stained PAs red) and HeLa cells (nucleus in blue) reveal formation of µm-sized PA clusters for PA1 (SEQ ID NO. 4) and PA2 (SEQ ID NO. 8). The peptides (PA1-PA3 (SEQ ID NO. 4, 5, 8), P1-P3 (SEQ ID NO. 1-3)) were added to cells for 30 min, washed and samples were analyzed. PA1 (SEQ ID NO. 4) and PA2 (SEQ ID NO. 8) revealed association with cellular membranes while P1-P3 (SEQ ID NO. 1-3) and PA3 (SEQ ID NO. 5) did not associate with cells, scalebar 50 µm. **B** shows the representative laser scanning microscopy images of Figure 9A in black and white color with Proteostat stained PAs (black) and HeLa cells (nucleus in grey).
**Figure 10** shows a schematic illustration of the assembly process of PA1 (SEQ ID NO. 4) and its proposed interaction with viral particles and cells. I: The PA assembles into core-shell like fibrils with a hydrophobic palmitic acid core (orange) and charged lysine residues facing outwards (blue). II Valine and alanine (red) determine secondary structure order and result in fibrillar assembly. III The subsequent addition of virions and cells to the fibrils result in charge-driven colocalization of virions with cells, which enhances the infectivity.
**Figure 11** shows degradability of µm-sized fibrillar aggregates formed by EF-C, PA1 (SEQ ID NO. 4) and PA2 (SEQ ID NO. 8) after 30 min and 3 d in HeLa and CD4+ T cell culture. **A** Representative laser scanning microscopy images of Proteostat-labeled EF-C, PA1 (SEQ ID NO. 4) and PA2 (SEQ ID NO. 8) (red) incubated with HeLa cells (nucleus in blue). In general, PA1 (SEQ ID NO. 4) reveal less and smaller µm-sized aggregates than PA2 (SEQ ID NO. 8) and EF-C. After 3 days in cell-culture, almost all small aggregates of PA1 (SEQ ID NO. 4) are degraded, whereas aggregates formed by PA2 (SEQ ID NO. 8) stay almost unchanged, scale bar 200 µm. **B** Box plot showing size distribution and bar plot showing number of aggregates > 10 µm² in an area of approximately 581.25 µm × 581.25 µm in cell culture after 30 min and 3 d incubation (4 µg peptide added to 40,000 cells). **C** Representative laser scanning microscopy images of Proteostat-labeled EF-C (red) incubated with CD4+ T cells (nucleus in blue). **D** Representative laser scanning microscopy images of Proteostat-labeled PA1 (SEQ ID NO. 4) (red) incubated with CD4+ T cells (nucleus in blue). **E** Representative laser scanning microscopy images of Proteostat-labeled PA2 (SEQ ID NO. 8) (red) incubated with CD4+ T cells (nucleus in blue). PA1 (SEQ ID NO. 4) reveal less and smaller µm-sized aggregates that attach as thin layers to cell-membrane compared than PA2 (SEQ ID NO. 8) and EF-C (indicated by red arrows). After 3 days in cell-culture, almost all small aggregates of PA1 (SEQ ID NO. 4) are degraded by CD4+ T cells (1 µg peptide added to 250,000 cells), scalebar 20 µm.
**Figure 12** shows experimental characterization, infectivity enhancement and degradability of the PA C₁₆-*ester*-SVVVAAAKKK-NH₂ (Pat1) (SEQ. ID NO. 10). The data show that Pat1 forms β-sheet rich fibrils (43% β-sheet) that form µm-sized aggregates that can interact with HeLa cells, thus are potent enhancers of HIV-1 infection. They can be degraded in presence of HeLa cells within 3 days (30 %). **A** Infectivity assay showing HIV-1 infection rates of TZM-bl cells observed in the presence of increasing concentrations of peptides PA1 (left data, SEQ ID NO. 4) and Pat1 (right data, (SEQ. ID NO. 10), 6.5 µM, 1.3 µM, 0.26 µM). The baseline (0 µM) shows infection rate without the addition of peptides. Mean values and standard deviations are derived from triplicate infections. RLU/s are relative light units per second. Pat1 enhances infectivity at 1.3 µM concentration 17 times more efficient compared to virus only infection. **B** ATR-IR spectrum of the amide I and II regions of Pat1 (1 mg/mL in PBS lyophilized). The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 43%. **C** Representative TEM micrographs of Pat1 that reveal the fibrillar morphologies of PA assembly (1 mg/mL in PBS), scale bars left 1 µm, right 100 nm. **D, E** Quantification of degradability by analyzing Proteostat stained aggregates via the 3D objects counter function of ImageJ 1.53f51 for 3 individual measurements (area 1008 µm × 1008 µm). **D** Box plot showing size distribution and **E** bar plot showing number of aggregates > 10 µm² in an area of approximately 1008 µm × 1008 µm in cell culture after 30 min and 3 d incubation. The percentual degradability was determined by number of aggregates after 3 days (d3, white boxes and bars) relative to the initial number of aggregates after 30 min incubation (d0 black boxes and bars). Box plots show aggregate size distribution with 10-90% shown as whiskers, 25-75% as boxes and the median line.Aggregate size decreases from 43 to 22 µm² and aggregate number decreases 30%, characterizing Pat1 (SEQ. ID NO. 10), as a degradable PA. Degradability of PAs investigated via the aggregate size and number of aggregates larger than 10 µm² in an area of 1008 µm × 1008 µm. Error bars in aggregate number depict technical triplicate measurements.
**Figure 13** shows experimental characterization, infectivity enhancement and degradability of the PA C₁₆-*thioester*-CVVVAAAKKK-NH₂ (SEQ. ID NO. 12) (Pat3). The data show that Pat3 forms β-sheet rich fibrils (50% β-sheet) that form µm-sized aggregates that can interact with HeLa cells, thus are potent enhancers of HIV-1 infection. They can be degraded in presence of HeLa cells within 3 days (87 %). **A** Infectivity assay showing HIV-1 infection rates of TZM-bl cells observed in the presence of increasing concentrations of peptides PA1 (left data, SEQ ID NO. 4) and Pat3 (right data, (SEQ. ID NO. 12) (6.5 µM, 1.3 µM, 0.26 µM). The baseline (0 µM) shows infection rate without the addition of peptides. Mean values and standard deviations are derived from triplicate infections. RLU/s are relative light units per second. Pat3 enhances infectivity at 1.3 µM concentration 11 times more efficient compared to virus only infection. **B** ATR-IR spectrum of the amide I and II regions of Pat3 (SEQ. ID NO. 12) (1 mg/mL in PBS lyophilized). The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 50%. **C** Representative TEM micrograph of Pat3 (SEQ. ID NO. 12) that reveal the fibrillar morphologies of PA assembly (1 mg/mL in PBS), scale bar 100 nm. **D, E** Quantification of degradability by analyzing Proteostat stained aggregates via the 3D objects counter function of ImageJ 1.53f51 for 3 individual measurements (area 1008 µm × 1008 µm). **D** Box plot showing size distribution and E bar plot showing number of aggregates > 10 µm² in an area of approximately 1008 µm × 1008 µm in cell culture after 30 min and 3 d incubation. The percentual degradability was determined by number of aggregates after 3 days (d3, white boxes and bars) relative to the initial number of aggregates after 30 min incubation (d0 black boxes and bars). Box plots show aggregate size distribution with 10-90% shown as whiskers, 25-75% as boxes and the median line. Aggregate size decreases from 22 to 10 µm² and aggregate number decreases 87%, characterizing Pat3 (SEQ. ID NO. 12) as a degradable PA.
**Figure 14** shows experimental characterization, infectivity enhancement and degradability of the PA eicosapentaenoic acid-VVVAAAKKK-NH₂ (SEQ. ID NO. 14) (Pat10). The data show that Pat10 (SEQ. ID NO. 14) forms β-sheet rich fibrils (51% β-sheet) that form µm-sized aggregates that can interact with HeLa cells, thus are potent enhancers of HIV-1 infection. They can be degraded in presence of HeLa cells within 3 days (99 %). **A** Infectivity assay showing HIV-1 infection rates of TZM-bl cells observed in the presence of increasing concentrations of peptides PA1 (left data, SEQ ID NO. 4) and Pat10 (right data, SEQ. ID NO. 14) (6.5 µM, 1.3 µM, 0.26 µM). The baseline (0 µM) shows infection rate without the addition of peptides. Mean values and standard deviations are derived from triplicate infections. RLU/s are relative light units per second. Pat10 (SEQ. ID NO. 14) enhances infectivity at 1.3 µM concentration 12 times more efficient compared to virus only infection. **B** ATR-IR spectrum of the amide I and II regions of Pat10 (SEQ. ID NO. 14) (1 mg/mL in PBS lyophilized). The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 51%. **C** Representative TEM micrograph of Pat10 (SEQ. ID NO. 14) that reveal morphology of PA assembly to be condensed spherical structures composed of fibrils (1 mg/mL in PBS), scale bars 100 nm. **D, E** Quantification of degradability by analyzing Proteostat stained aggregates via the 3D objects counter function of ImageJ 1.53f51 for 3 individual measurements (area 1008 µm × 1008 µm). **D** Box plot showing size distribution and **E** bar plot showing number of aggregates > 10 µm² in an area of approximately 1008 µm × 1008 µm in cell culture after 30 min and 3 d incubation. Aggregate size decreases from 21 to 17 µm² and aggregate number decreases 99%, characterizing Pat10 (SEQ. ID NO. 14) as a degradable PA.

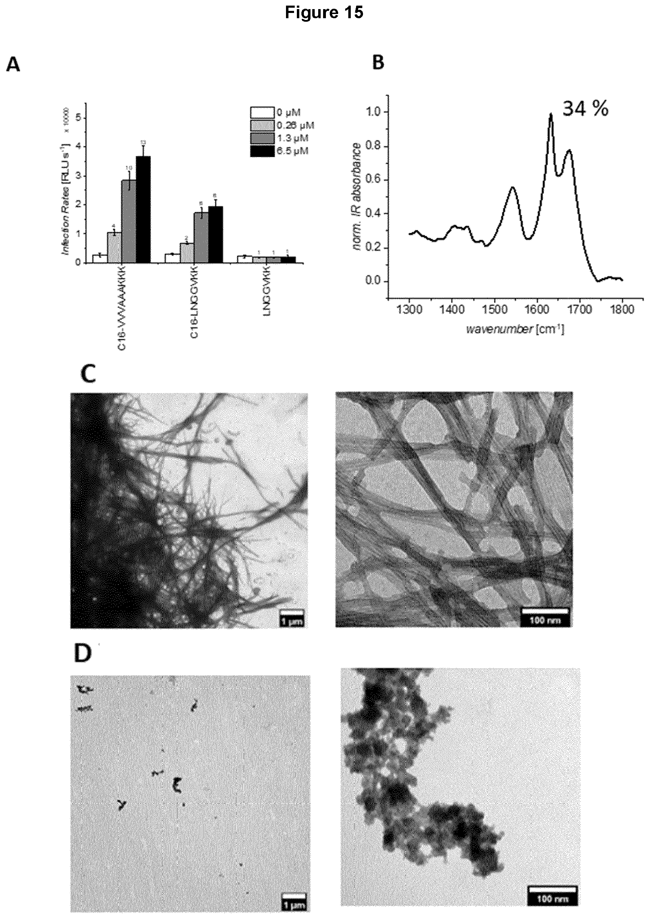

**Figure 15** shows experimental characterization, infectivity enhancement and degradability of the PA C₁₆-LNGGVKK-NH₂ (SEQ. ID NO. 20) (Pat19). The data show that Pat19 (SEQ. ID NO. 20) forms β-sheet containing fibrils (34% β-sheet) that form µm-sized aggregates that can interact with HeLa cells, thus are potent enhancers of HIV-1 infection. They can be degraded in presence of HeLa cells within 3 days (48 %). **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat19 (SEQ. ID NO. 20) (middle data) and Pat38 (SEQ. ID NO. 21) (LNGGVKK-NH₂, right data) (6.5 µM, 1.3 µM, 0.26 µM). The data show that Pat38 (SEQ. ID NO. 21) form no β-sheet rich fibrils that cannot form µm-sized aggregates that cannot interact with HeLa cells, thus are not potent enhancers of HIV-1 infection.The baseline (0 µM) shows infection rate without the addition of peptides. Mean values and standard deviations are derived from triplicate infections. RLU/s are relative light units per second. Pat19 (SEQ. ID NO. 20) enhances infectivity at 1.3 µM concentration 6 times more efficient compared to virus only infection. **B** ATR-IR spectrum of the amide I and II regions of Pat19 (SEQ. ID NO. 20) (1 mg/mL in PBS lyophilized). The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 34%. **C** Representative TEM micrographs of Pat19 (SEQ. ID NO. 20) that reveal the fibrillar morphologies of PA assembly (1 mg/mL in PBS), scale bars left 1 µm, right 100 nm. **D** Representative TEM micrographs of Pat38 that reveal no fibrillar morphologies of the peptide (1 mg/mL in PBS), scale bars left 1 µm, right 100 nm

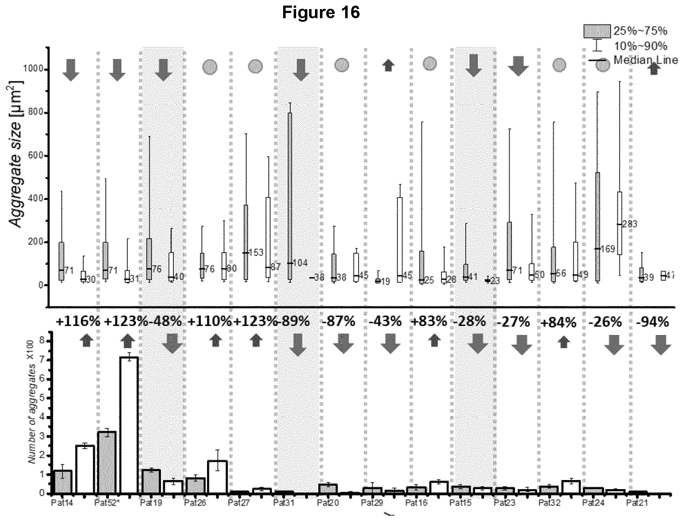

**Figure 16** shows Quantification of degradability by analyzing Proteostat stained aggregates via the 3D objects counter function of ImageJ 1.53f51 for 3 individual measurements (area 1008 µm × 1008 µm). Box plot showing size distribution and bar plot showing number of aggregates > 10 µm² in an area of approximately 1008 µm × 1008 µm in cell culture after 30 min and 3 d incubation. Aggregate size of C₁₆-LNGGVKK-NH₂ (SEQ. ID NO. 20) (Pat19) decreases from 76 to 40 µm² and aggregate number decreases 48%, characterizing Pat19 as a degradable PA. Aggregate size of C₁₆-ALAAGKK-NH₂ (SEQ. ID NO. 22) (Pat20) slightly increases from 38 to 45 µm² and aggregate number decreases 87%, characterizing Pat20 (SEQ. ID NO. 22) as a degradable PA. Aggregate size of C₁₆-AKAVGK-NH₂ (SEQ. ID NO. 24) (Pat31) decreases from 104 to 38 µm² and aggregate number decreases 89%, characterizing Pat31 NH₂ (SEQ. ID NO. 24) as a degradable PA. Aggregate size of C₁₆-IIQKIK-NH₂ (SEQ. ID NO. 26) (Pat15) decreases from 41 to 23 µm² and aggregate number decreases 28%, characterizing Pat15 (SEQ. ID NO. 26) as a degradable PA. Aggregate size of PA C₁₆-LLLKK-NH₂ (Pat23) (SEQ. ID NO. 30) decreases from 71 to 60 µm² and aggregate number decreases 27%, characterizing Pat23 (SEQ. ID NO. 30) as a moderately degradable PA. Aggregate size decreases of Pat14 (SEQ. ID NO. 32) from 71 to 30 µm² but aggregate number increases 116%, characterizing Pat14 as a non-degradable PA. Median aggregate size of Pat16 (SEQ. ID NO. 34) stays similar from 25 to 28 µm² with less very large aggregates (>100 µm²) and aggregate number increases 83%, characterizing Pat16 (SEQ. ID NO. 34) as a non-degradable PA. Median aggregate size measurements of Pat21 (SEQ. ID NO. 38) stays similar from 39 to 47 µm² and aggregate number decreases 84%, characterizing Pat21 (SEQ. ID NO. 38) as a degradable PA that is not an efficient enhancer of viral infectivity. Median aggregate size of Pat24 (SEQ. ID NO. 42) increases in size from 169 to 283 µm² and aggregate number decreases slightly 26%, characterizing Pat24 (SEQ. ID NO. 42) as a non-degradable PA that is also not infectivity enhancing. Median aggregate size of Pat26 (SEQ. ID NO. 46) stays similar from 76 to 80 µm² with less very large aggregates (>100 µm²) and aggregate number increases 110%, characterizing Pat26 (SEQ. ID NO. 46) as a non-degradable PA but infectivity enhancing PA. Median aggregate size of Pat27 (SEQ. ID NO. 48) stays similar from 153 to 87 µm² with less very large aggregates (>100 µm²) and aggregate number increases 123%, characterizing Pat27 (SEQ. ID NO. 48) as a non-degradable PA but infectivity enhancing PA. Median aggregate size of Pat29 (SEQ. ID NO. 52) increases from 19 to 45 µm² and aggregate number decreases 43%, indicating further aggregation during in vitro incubation and characterizing Pat29 (SEQ. ID NO. 52) as a non-degradable PA infectivity enhancing PA. Median aggregate size of Pat32 (SEQ. ID NO. 56) stays similar from 56 to 49 µm² with very large aggregates (>100 µm²) and aggregate number increases 84%, characterizing Pat32 (SEQ. ID NO. 56) as a non-degradable PA. Median aggregate size of Pat52* (SEQ. ID NO. 58) stays similar from 71 to 31 µm² with less very large aggregates (>100 µm²) and aggregate number increases 123%, characterizing Pat52* (SEQ. ID NO. 58) as a non-degradable PA but infectivity enhancing PA.

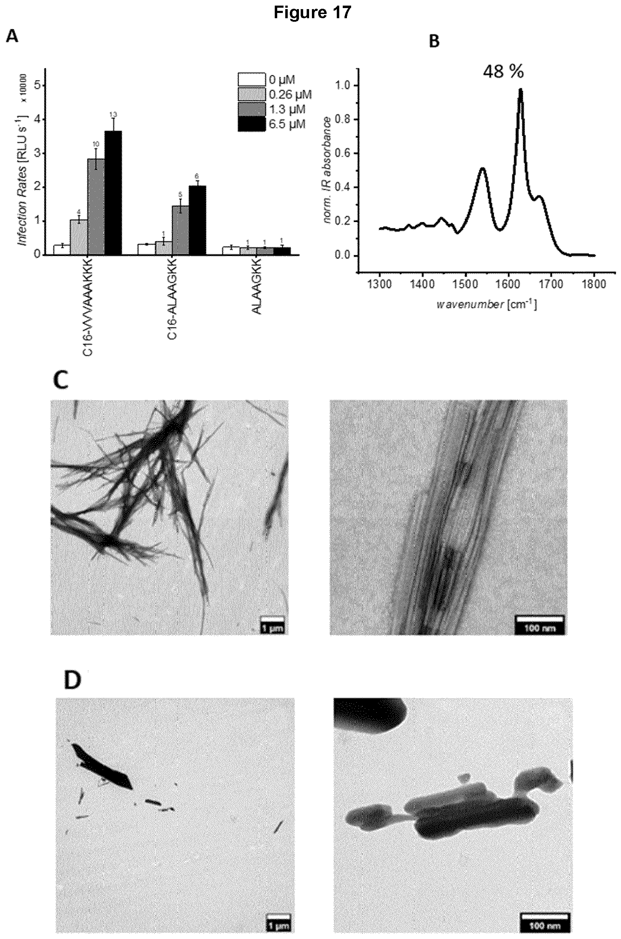

**Figure 17** shows experimental characterization, infectivity enhancement and degradability of the PA C₁₆-ALAAGKK-NH₂ (SEQ. ID NO. 22) (Pat20) and of the peptide moiety ALAAGKK-NH₂ (SEQ. ID NO. 23) (Pat39). The data show that Pat20 (SEQ. ID NO. 22) forms β-sheet containing fibrils (48% β-sheet) that form µm-sized aggregates that can interact with HeLa cells, thus are potent enhancers of HIV-1 infection. They can be degraded in presence of HeLa cells within 3 days (87 %). The data show that Pat39 (SEQ. ID NO. 23) form no β-sheet rich fibrils that cannot form µm-sized aggregates that cannot interact with HeLa cells, thus are not potent enhancers of HIV-1 infection. **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat20 (middle data, SEQ. ID NO. 22) and Pat39 (ALAAGKK-NH₂ (SEQ. ID NO. 23) right data) (6.5 µM, 1.3 µM, 0.26 µM). The baseline (0 µM) shows infection rate without the addition of peptides. Mean values and standard deviations are derived from triplicate infections. RLU/s are relative light units per second. Pat20 (SEQ. ID NO. 22) enhances infectivity at 1.3 µM concentration 5 times more efficient compared to virus only infection. Pat39 (SEQ. ID NO. 23) cannot enhance infectivity at 1.3 µM concentration (same as virus only infection). **B** ATR-IR spectrum of the amide I and II regions of Pat20 (SEQ. ID NO. 22) (1 mg/mL in PBS lyophilized); The β-sheet amount of Pat20 (SEQ. ID NO. 22) was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 48%. **C** Representative TEM micrographs of Pat20 (SEQ. ID NO. 22) that reveal the fibrillar morphologies of PA assembly (1 mg/mL in PBS), scale bars left 1 µm, right 100 nm. **D** Representative TEM micrographs of Pat39 (SEQ. ID NO. 23) that reveal no fibrillar morphologies of the peptide (1 mg/mL in PBS), scale bars left 1 µm, right 100 nm.

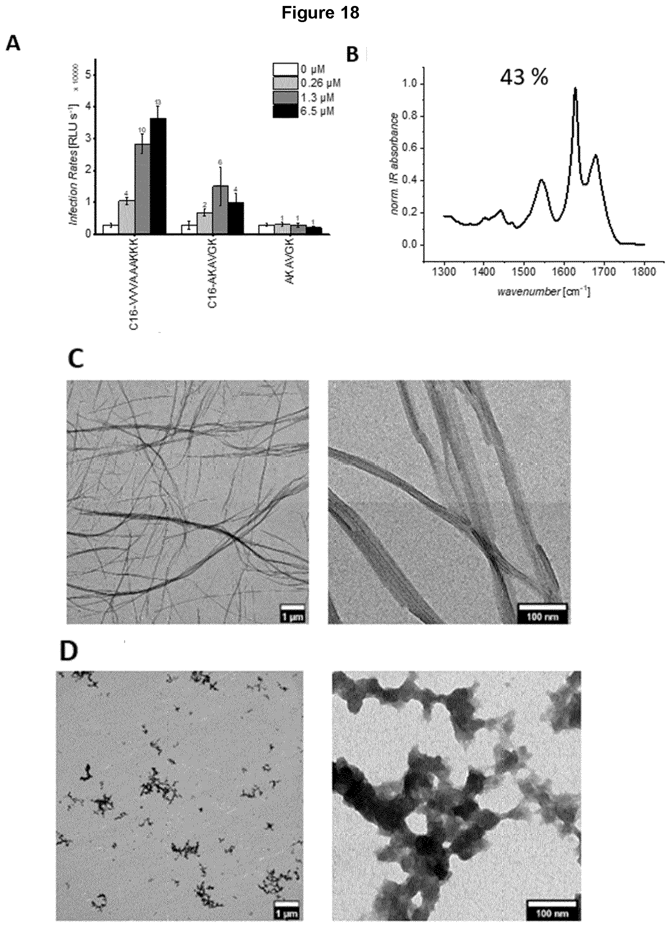

**Figure 18** shows experimental characterization, infectivity enhancement and degradability of the PA C₁₆-AKAVGK-NH₂ (SEQ. ID NO. 24) (Pat31) and of the peptide moiety AKAVGK-NH₂ (SEQ. ID NO. 25) (Pat50). The data show that Pat31 (SEQ. ID NO. 24) forms β-sheet containing fibrils (43% β-sheet) that form µm-sized aggregates that can interact with HeLa cells, thus are potent enhancers of HIV-1 infection. They can be degraded in presence of HeLa cells within 3 days (89 %). The data show that Pat50 (SEQ. ID NO. 25) β-sheet containing amorpheous aggregates that cannot form µm-sized aggregates that cannot interact with HeLa cells, thus are not potent enhancers of HIV-1 infection. **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat31 (SEQ. ID NO. 24) (middle data) and Pat50 (AKAVGK-NH₂ (SEQ. ID NO. 25), right data) (6.5 µM, 1.3 µM, 0.26 µM). The baseline (0 µM) shows infection rate without the addition of peptides. Mean values and standard deviations are derived from triplicate infections. RLU/s are relative light units per second. Pat31 (SEQ. ID NO. 24) enhances infectivity at 1.3 µM concentration 6 times more efficient compared to virus only infection. Pat50 (SEQ. ID NO. 25) cannot enhance infectivity at 1.3 µM concentration (same as virus only infection). **B** ATR-IR spectrum of the amide I and II regions of Pat31 (SEQ. ID NO. 24) (1 mg/mL in PBS lyophilized); The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 43%. **C** Representative TEM micrographs of Pat31 (SEQ. ID NO. 24) that reveal the fibrillar morphologies of PA assembly (1 mg/mL in PBS), scale bars left 1 µm, right 100 nm. **D** Representative TEM micrographs of Pat50 (SEQ. ID NO. 25) that reveal no fibrillar morphologies but amorphous aggregates for the peptide (1 mg/mL in PBS), scale bars left 1 µm, right 100 nm.

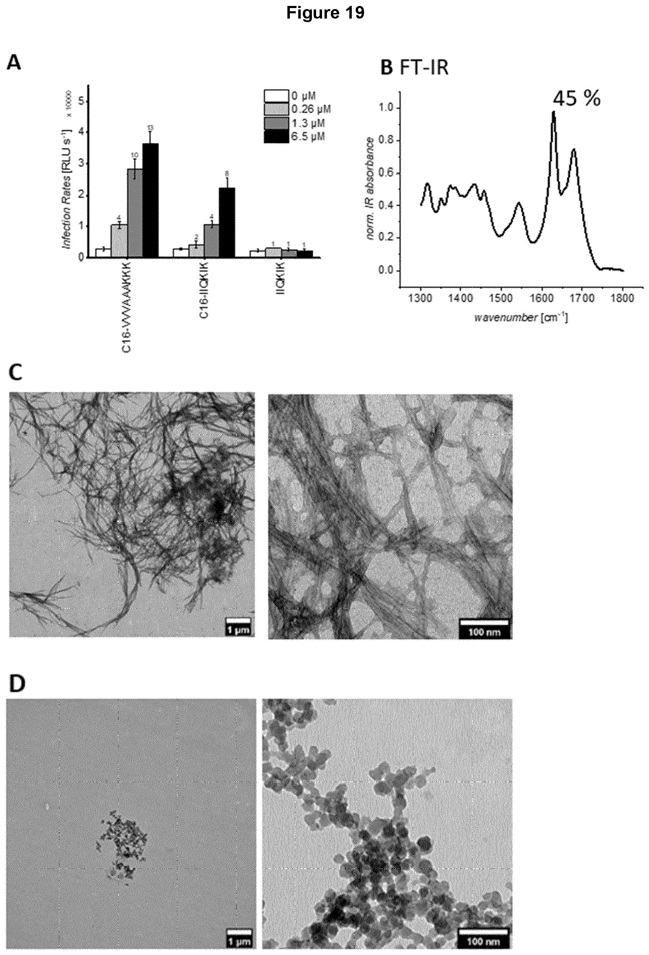

**Figure 19** shows experimental characterization, infectivity enhancement and degradability of the PA C₁₆-IIQKIK-NH₂ (SEQ. ID NO. 26) (Pat15) and of the peptide moiety IIQKIK-NH₂ (SEQ. ID NO. 27) (Pat34). The data show that Pat15 (SEQ. ID NO. 26) forms β-sheet containing fibrils (45% β-sheet) that form µm-sized aggregates that can interact with HeLa cells, thus are potent enhancers of HIV-1 infection. They can be degraded in presence of HeLa cells within 3 days (28 %). The data show that Pat34 (SEQ. ID NO. 27) form no β-sheet rich fibrils that cannot form µm-sized aggregates that cannot interact with HeLa cells, thus are not potent enhancers of HIV-1 infection. **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat15 (SEQ. ID NO. 26) (middle data) and Pat34 (IIQKIK-NH₂ (SEQ. ID NO. 27), right data) (6.5 µM, 1.3 µM, 0.26 µM). The baseline (0 µM) shows infection rate without the addition of peptides. Mean values and standard deviations are derived from triplicate infections. RLU/s are relative light units per second. Pat15 (SEQ. ID NO. 26) enhances infectivity at 1.3 µM concentration 4 times more efficient compared to virus only infection. B ATR-IR spectrum of the amide I and II regions of Pat15 (SEQ. ID NO. 26) (1 mg/mL in PBS lyophilized); The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 45%. C Representative TEM micrographs of Pat15 (SEQ. ID NO. 26) that reveal the fibrillar morphologies of PA assembly (1 mg/mL in PBS), scale bars left 1 µm, right 100 nm. D Representative TEM micrographs of Pat34 (SEQ. ID NO. 27) that reveal no fibrillar morphologies of the peptide (1 mg/mL in PBS), scale bars left 1 µm, right 100 nm.

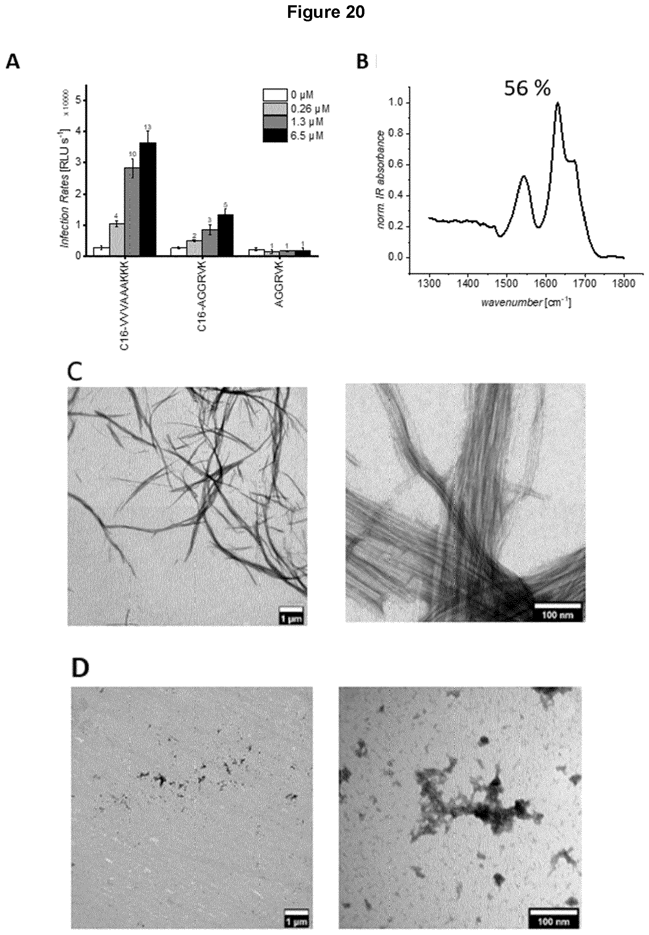

**Figure 20** shows experimental characterization, infectivity enhancement and degradability of the PA C₁₆-AGGRVK-NH₂ (SEQ. ID NO. 28) (Pat18) and of the peptide moiety AGGRVK-NH₂ (SEQ. ID NO. 29) (Pat37). The data show that Pat18 (SEQ. ID NO. 28) forms β-sheet containing fibrils (56% β-sheet) that do not form µm-aggregates thus they weakly interact with HeLa cells and are weak enhancers of HIV-1 infection. ). The data show that Pat37 (SEQ. ID NO. 29) form no β-sheet rich fibrils that cannot form µm-sized aggregates that cannot interact with HeLa cells, thus are not potent enhancers of HIV-1 infection. A Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat18 (SEQ. ID NO. 28) (middle data) and Pat37 (AGGRVK-NH₂ (SEQ. ID NO. 29), right data) (6.5 µM, 1.3 µM, 0.26 µM). The baseline (0 µM) shows infection rate without the addition of peptides. Mean values and standard deviations are derived from triplicate infections. RLU/s are relative light units per second. Pat18 (SEQ. ID NO. 28) enhances infectivity at 1.3 µM concentration 3 times more efficient compared to virus only infection. Pat37 (SEQ. ID NO. 29) cannot enhance infectivity at 1.3 µM concentration (same as virus only infection). B ATR-IR spectrum of the amide I and II regions of Pat18 (SEQ. ID NO. 28) (1 mg/mL in PBS lyophilized); The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 56%. C Representative TEM micrographs of Pat18 (SEQ. ID NO. 28) that reveal the fibrillar morphologies of PA assembly (1 mg/mL in PBS), scale bars left 1 µm, right 100 nm. D_Representative TEM micrographs of Pat37 (SEQ. ID NO. 29) that reveal no fibrillar morphologies of the peptide (1 mg/mL in PBS), scale bars left 1 µm, right 100 nm.

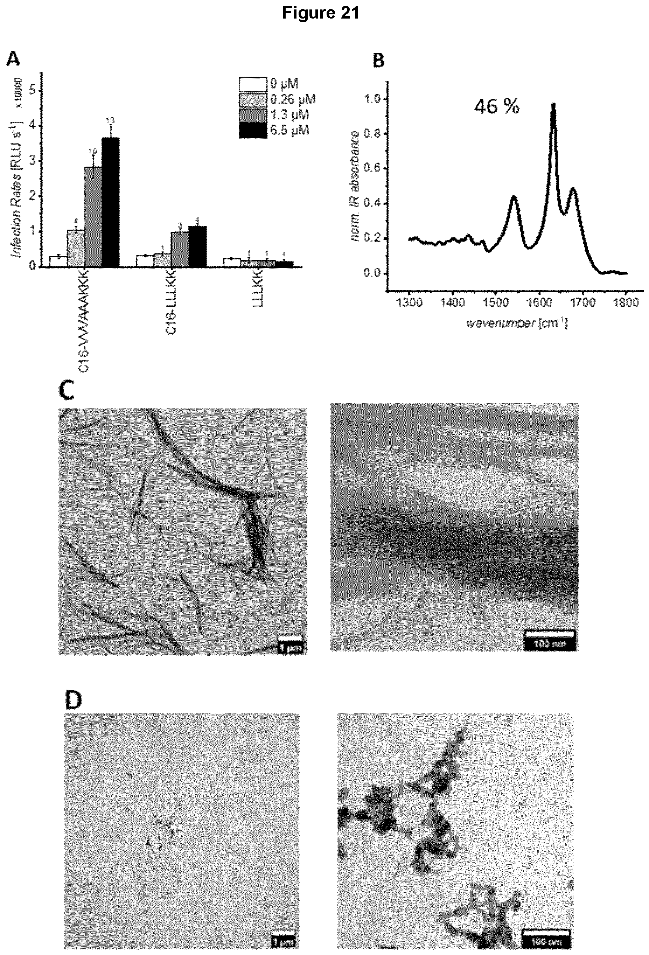

**Figure 21** shows experimental characterization, infectivity enhancement and degradability of the PA C₁₆-LLLKK-NH₂ (Pat23) (SEQ. ID NO. 30) and of the peptide moiety LLLKK-NH₂ (Pat42) (SEQ. ID NO. 31). The data show that Pat23 (SEQ. ID NO. 30) forms β-sheet containing fibrils (46% β-sheet) that form µm-sized aggregates that can interact with HeLa cells, thus are potent enhancers of HIV-1 infection. They can be degraded in presence of HeLa cells within 3 days (27 %). The data show that Pat42 (SEQ. ID NO. 31) form no β-sheet rich fibrils that cannot form µm-sized aggregates that cannot interact with HeLa cells, thus are not potent enhancers of HIV-1 infection. **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat23 (SEQ. ID NO. 30) (middle data) and Pat42 (LLLKK-NH₂ (SEQ. ID NO. 31), right data) (6.5 µM, 1.3 µM, 0.26 µM). The baseline (0 µM) shows infection rate without the addition of peptides. Mean values and standard deviations are derived from triplicate infections. RLU/s are relative light units per second. Pat23 (SEQ. ID NO. 30) enhances infectivity at 1.3 µM concentration 3 times more efficient compared to virus only infection. Pat42 (SEQ. ID NO. 31) cannot enhance infectivity at 1.3 µM concentration (same as virus only infection). **B** ATR-IR spectrum of the amide I and II regions of Pat23 (SEQ. ID NO. 30) (1 mg/mL in PBS lyophilized); The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 46%. **C** Representative TEM micrographs of Pat23 (SEQ. ID NO. 30) that reveal the fibrillar morphologies of PA assembly (1 mg/mL in PBS), scale bars left 1 µm, right 100 nm. **D** Representative TEM micrographs of Pat42 (SEQ. ID NO. 31) that reveal no fibrillar morphologies of the peptide (1 mg/mL in PBS), scale bars left 1 µm, right 100 nm.

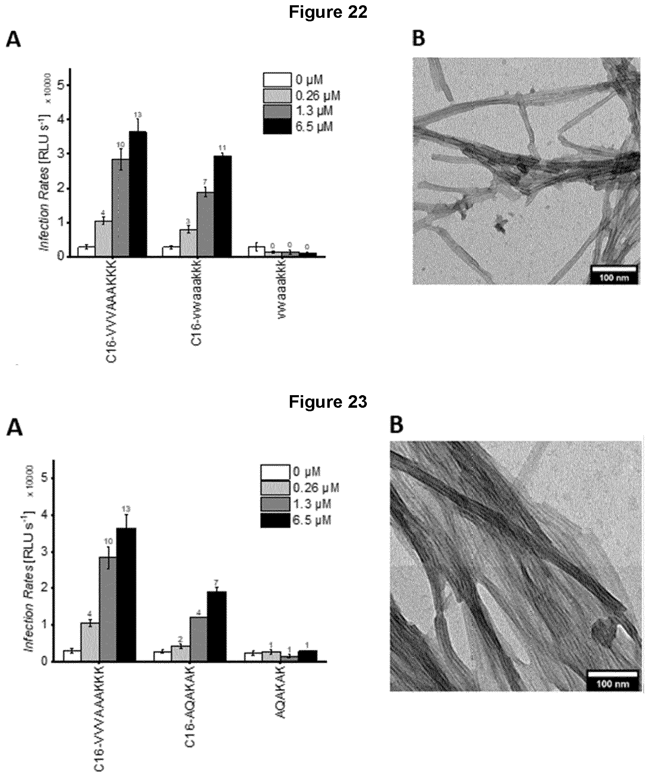

**Figure 22** shows experimental characterization, infectivity enhancement and degradability of the PA C₁₆-vvvaaakkk-NH₂ (SEQ. ID NO. 32) (Pat14) and of the peptide moiety vvvaaakkk-NH₂ (SEQ. ID NO. 33) (Pat33). **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat14 (SEQ. ID NO. 32) (middle data) and Pat33 (vvvaaakkk-NH₂ (SEQ. ID NO. 33), right data) (6.5 µM, 1.3 µM, 0.26 µM). The baseline (0 µM) shows infection rate without the addition of peptides. Mean values and standard deviations are derived from triplicate infections. RLU/s are relative light units per second. Pat14 (SEQ. ID NO. 32) enhances infectivity at 1.3 µM concentration 7 times more efficient compared to virus only infection. Pat33 (SEQ. ID NO. 33) cannot enhance infectivity at 1.3 µM concentration (same as virus only infection). **B** Representative TEM micrographs of Pat14 (SEQ. ID NO. 32) that reveal the fibrillar morphologies of PA assembly (1 mg/mL in PBS), scale bar 100 nm.
**Figure 23** shows experimental characterization, infectivity enhancement and degradability of the PA C₁₆-AQAKAK-NH₂ (Pat16) (SEQ. ID NO. 34) of the peptide moiety AQAKAK-NH₂ (SEQ. ID NO. 35) (Pat35). **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat16 (SEQ. ID NO. 34) (middle data) and Pat35 (SEQ. ID NO. 35) (AQAKAK-NH₂, right data) (6.5 µM, 1.3 µM, 0.26 µM). The baseline (0 µM) shows infection rate without the addition of peptides. Mean values and standard deviations are derived from triplicate infections. RLU/s are relative light units per second. Pat16 (SEQ. ID NO. 34) enhances infectivity at 1.3 µM concentration 4 times more efficient compared to virus only infection. Pat35 (SEQ. ID NO. 35) cannot enhance infectivity at 1.3 µM concentration (same as virus only infection). **B** Representative TEM micrographs of Pat16 (SEQ. ID NO. 34) that reveal the fibrillar morphologies of PA assembly (1 mg/mL in PBS), scale bar 100 nm.

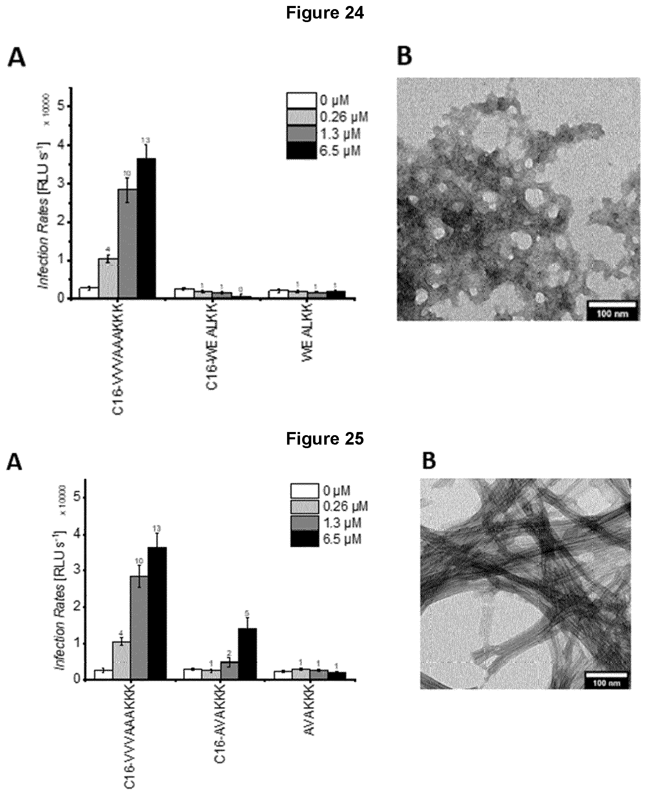

**Figure 24** shows experimental characterization, infectivity enhancement and degradability of the PA C₁₆-WEALKK-NH₂ (SEQ. ID NO. 36) (Pat17) and of the peptide moiety WEALKK-NH₂ (SEQ. ID NO. 37) (Pat36). **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat17 (SEQ. ID NO. 36) (middle data) and Pat36 (WEALKK-NH₂ (SEQ. ID NO. 37), right data) (6.5 µM, 1.3 µM, 0.26 µM). The baseline (0 µM) shows infection rate without the addition of peptides. Mean values and standard deviations are derived from triplicate infections. RLU/s are relative light units per second. Pat17 (SEQ. ID NO. 36) does not enhance infectivity at 1.3 µM concentration compared to virus only infection. Pat36 (SEQ. ID NO. 37) cannot enhance infectivity at 1.3 µM concentration (same as virus only infection). **B** Representative TEM micrograph of Pat17 (SEQ. ID NO. 36) that reveal the amorphous aggregate morphology of PA assembly (1 mg/mL in PBS), scale bar 100 nm.
**Figure 25** shows experimental characterization, infectivity enhancement and degradability of the PA C₁₆-AVAKKK-NH₂ (SEQ. ID NO. 38) (Pat21) and of the peptide moiety AVAKKK-NH₂ (SEQ. ID NO. 39) (Pat40). **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat21 (SEQ. ID NO. 38) (middle data) and Pat40 (AVAKKK-NH₂ (SEQ. ID NO. 39) right data) (6.5 µM, 1.3 µM, 0.26 µM). The baseline (0 µM) shows infection rate without the addition of peptides. Mean values and standard deviations are derived from triplicate infections. RLU/s are relative light units per second. Pat21 (SEQ. ID NO. 38) enhances infectivity at 1.3 µM concentration 2 times more efficient compared to virus only infection. Pat40 (SEQ. ID NO. 39) cannot enhance infectivity at 1.3 µM concentration (same as virus only infection). **B** Representative TEM micrograph of Pat21 (SEQ. ID NO. 38) that reveal the fibrillar morphologies of PA assembly (1 mg/mL in PBS), scale bar 100 nm.

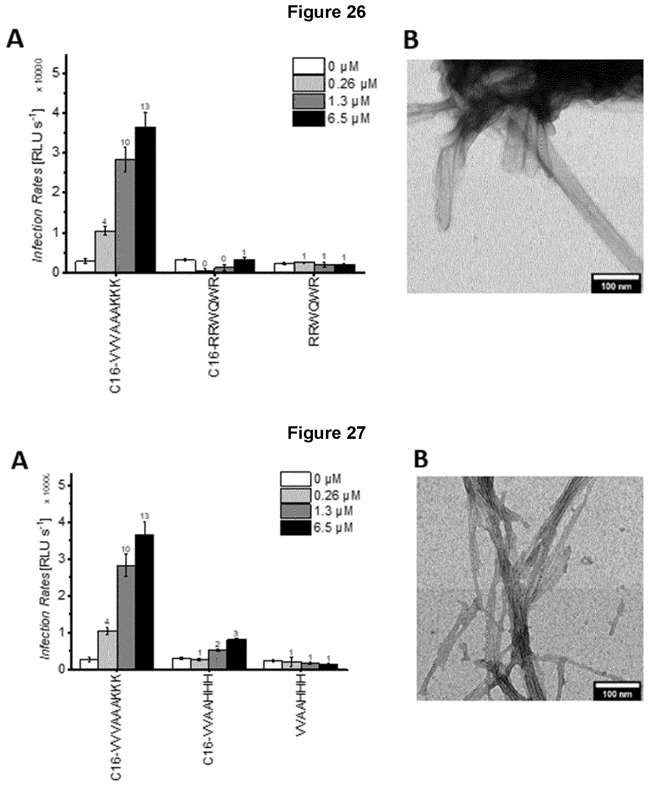

**Figure 26** shows experimental characterization, infectivity enhancement and degradability of the PA C₁₆-RRWQWR-NH₂ (SEQ. ID NO. 40) (Pat22) and of the peptide moiety RRWQWR-NH₂ (SEQ. ID NO. 41) (Pat41). **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat22 (SEQ. ID NO. 40) (middle data) and Pat41 (RRWQWR-NH₂ (SEQ. ID NO. 41) right data) (6.5 µM, 1.3 µM, 0.26 µM). The baseline (0 µM) shows infection rate without the addition of peptides. Mean values and standard deviations are derived from triplicate infections. RLU/s are relative light units per second. Pat22 (SEQ. ID NO. 40) does not enhance infectivity at 1.3 µM concentration compared to virus only infection. Pat41 (SEQ. ID NO. 41) cannot enhance infectivity at 1.3 µM concentration (same as virus only infection). **B** Representative TEM micrograph of Pat22 (SEQ. ID NO. 40) that reveal the fibrillar morphologies of PA assembly (1 mg/mL in PBS), scale bar 100 nm.
**Figure 27** shows experimental characterization, infectivity enhancement and degradability of the PA C₁₆-VVAAHHH-NH₂ (SEQ. ID NO. 42) (Pat24) and of the peptide moiety VVAAHHH-NH₂ (SEQ. ID NO. 43) (Pat43). **A** Infectivity assay showing HIV-1 infection rates of TZM-bl cells observed in the presence of increasing concentrations of peptides PA1 (left data, C₁₆-VVVAAAKKK-NH₂, SEQ ID NO. 4), Pat24 (SEQ. ID NO. 42) (middle data) and Pat43 (SEQ. ID NO. 43) (VVAAHHH-NH₂, right data) (6.5 µM, 1.3 µM, 0.26 µM). The baseline (0 µM) shows infection rate without the addition of peptides. Mean values and standard deviations are derived from triplicate infections. RLU/s are relative light units per second. Pat24 (SEQ. ID NO. 42) does not enhance infectivity at 1.3 µM concentration compared to virus only infection. **B** Representative TEM micrographs of Pat24 (SEQ. ID NO. 42) that reveal the fibrillar morphologies of PA assembly (1 mg/mL in PBS), scale bar 100 nm.

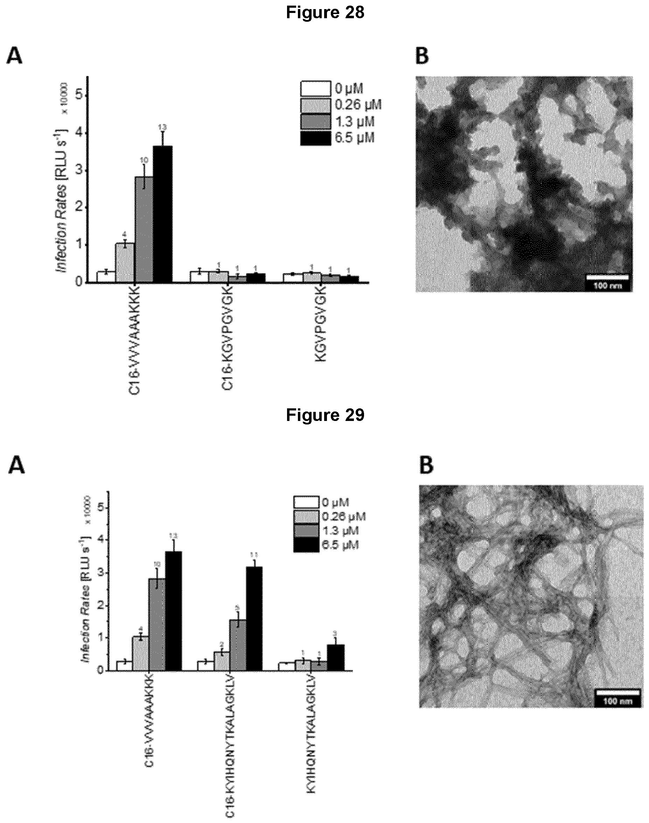

**Figure 28** shows experimental characterization, infectivity enhancement and degradability of the PA C₁₆-KGVPGVGK-NH₂ (Pat25) (SEQ. ID NO. 44) and of the peptide moiety KGVPGVGK-NH₂ (Pat44) (SEQ. ID NO. 45). **A** Infectivity assay showing HIV-1 infection rates of TZM-bl cells observed in the presence of increasing concentrations of peptides PA1 (left data, SEQ ID NO. 4), Pat25 (SEQ. ID NO. 44) (middle data) and Pat44 (SEQ. ID NO. 45) (KGVPGVGK-NH₂, right data) (6.5 µM, 1.3 µM, 0.26 µM). The baseline (0 µM) shows infection rate without the addition of peptides. Mean values and standard deviations are derived from triplicate infections. RLU/s are relative light units per second. Pat25 (SEQ. ID NO. 44) and Pat44 (SEQ. ID NO. 45) do not enhance infectivity at 1.3 µM compared to virus only infection. **B** Representative TEM micrographs of Pat25 (SEQ. ID NO. 44) that reveal no fibrillar morphologies of PA assembly (1 mg/mL in PBS), scale bars 100 nm.
**Figure 29** shows experimental characterization, infectivity enhancement and degradability of the PA C₁₆-KYIHQNYTKALAGKLV-NH₂ (Pat26) (SEQ. ID NO. 46) and of the peptide moiety KYIHQNYTKALAGKLV-NH₂ (Pat45) (SEQ. ID NO. 47). **A** Infectivity assay showing HIV-1 infection rates of TZM-bl cells observed in the presence of increasing concentrations of peptides PA1 (left data, SEQ ID NO. 4), Pat26 (SEQ. ID NO. 46) (middle data) and Pat45 (SEQ. ID NO. 47) (KYIHQNYTKALAGKLV-NH₂, right data) (6.5 µM, 1.3 µM, 0.26 µM). The baseline (0 µM) shows infection rate without the addition of peptides. Mean values and standard deviations are derived from triplicate infections. RLU/s are relative light units per second. Pat26 enhances infectivity at 1.3 µM concentration 5 times more efficient compared to virus only infection. **B** Representative TEM micrographs of Pat26 (SEQ. ID NO. 46) that reveal the fibrillar morphologies of PA assembly (1 mg/mL in PBS), scale bar 100 nm.

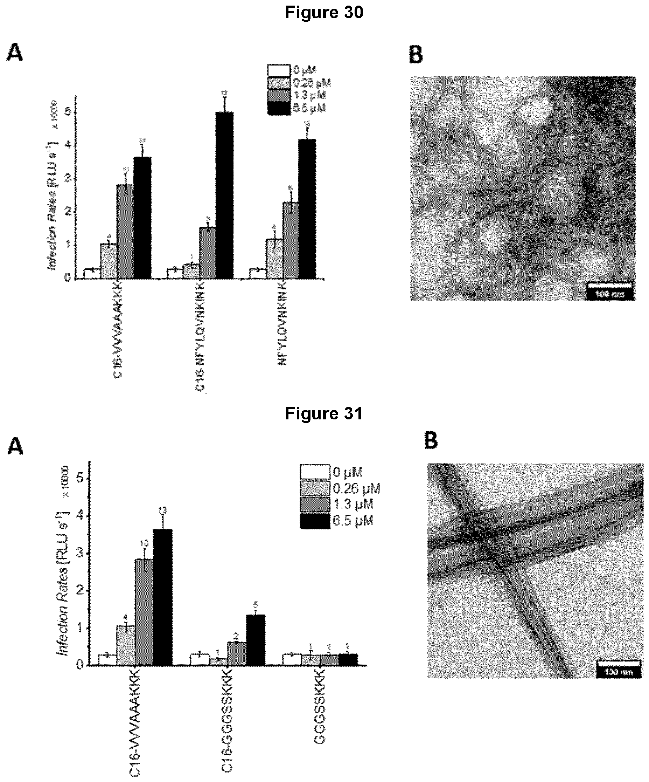

**Figure 30** shows experimental characterization, infectivity enhancement and degradability of the PA C₁₆-NFYLQVNKINK-NH₂ (Pat27) (SEQ. ID NO. 48) and of the peptide moiety NFYLQVNKINK-NH₂ (Pat46) (SEQ. ID NO. 49). **A** Infectivity assay showing HIV-1 infection rates of TZM-bl cells observed in the presence of increasing concentrations of peptides PA1 (left data, SEQ ID NO. 4), Pat27 (SEQ. ID NO. 48) (middle data) and Pat46 (SEQ. ID NO. 49) (right data) (6.5 µM, 1.3 µM, 0.26 µM). The baseline (0 µM) shows infection rate without the addition of peptides. Mean values and standard deviations are derived from triplicate infections. RLU/s are relative light units per second. Pat27 enhances infectivity at 1.3 µM concentration 5 times more efficient compared to virus only infection. **B** Representative TEM micrographs of Pat27 (SEQ. ID NO. 48) that reveal the fibrillar morphologies of PA assembly (1 mg/mL in PBS), scale bar 100 nm.
**Figure 31** shows experimental characterization, infectivity enhancement and degradability of the PA C₁₆-GGGSSKKK-NH₂ (Pat28) (SEQ. ID NO. 50) of the peptide moiety GGGSSKKK-NH₂ (Pat47) (SEQ. ID NO. 51 **A** Infectivity assay showing HIV-1 infection rates of TZM-bl cells observed in the presence of increasing concentrations of peptides PA1 (left data, SEQ ID NO. 4), Pat26 (SEQ. ID NO. 50) (middle data) and Pat47 (SEQ. ID NO. 51) (right data) (6.5 µM, 1.3 µM, 0.26 µM). The baseline (0 µM) shows infection rate without the addition of peptides. Mean values and standard deviations are derived from triplicate infections. RLU/s are relative light units per second. Pat26 does not enhance infectivity at 1.3 µM concentration compared to virus only infection. **B** Representative TEM micrographs of Pat28 (SEQ. ID NO. 50) that reveal the fibrillar morphologies of PA assembly (1 mg/mL in PBS), scale bars 100 nm.

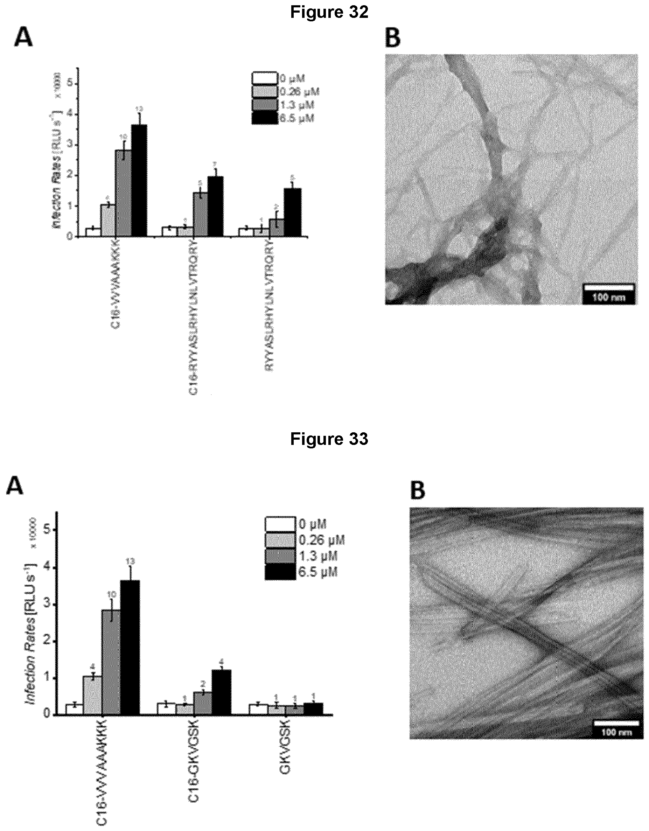

**Figure 32** shows experimental characterization, infectivity enhancement and degradability of the PA C₁₆-RYYASLRHYLNLVTRQRY-NH₂ (Pat29) (SEQ. ID NO. 51) and of the peptide moiety RYYASLRHYLNLVTRQRY-NH₂ (Pat48) (SEQ. ID NO. 52). **A** Infectivity assay showing HIV-1 infection rates of TZM-bl cells observed in the presence of increasing concentrations of peptides PA1 (left data, SEQ ID NO. 4), Pat29 (SEQ. ID NO. 51) (middle data) and Pat48 (SEQ. ID NO. 52) (right data) (6.5 µM, 1.3 µM, 0.26 µM). The baseline (0 µM) shows infection rate without the addition of peptides. Mean values and standard deviations are derived from triplicate infections. RLU/s are relative light units per second. Pat29 enhances infectivity at 1.3 µM concentration 5 times more efficient compared to virus only infection. **B** Representative TEM micrographs of Pat29 (SEQ. ID NO. 51) that reveal the fibrillar morphologies of PA assembly (1 mg/mL in PBS), scale bars 100 nm.
**Figure 33** shows experimental characterization, infectivity enhancement and degradability of the PA C₁₆-GKVGSK-NH₂ (Pat30) (SEQ. ID NO. 54) and of the peptide moiety GKVGSK-NH₂ (Pat49) (SEQ. ID NO. 55). **A** Infectivity assay showing HIV-1 infection rates of TZM-bl cells observed in the presence of increasing concentrations of peptides PA1 (left data, SEQ ID NO. 4), Pat30 (SEQ. ID NO. 54) (middle data) and Pat49 (SEQ. ID NO. 55) (right data) (6.5 µM, 1.3 µM, 0.26 µM). The baseline (0 µM) shows infection rate without the addition of peptides. Mean values and standard deviations are derived from triplicate infections. RLU/s are relative light units per second. Pat30 (SEQ. ID NO. 54) does not enhance infectivity at 1.3 µM concentration compared to virus only infection. **B** Representative TEM micrographs of Pat30 (SEQ. ID NO. 54) that reveal the fibrillar morphologies of PA assembly (1 mg/mL in PBS), scale bar 100 nm.

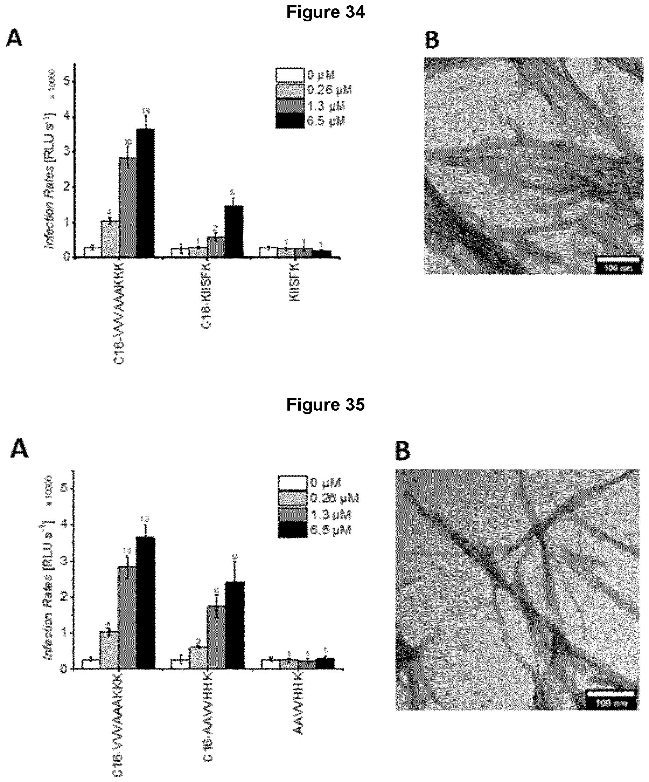

**Figure 34** shows experimental characterization, infectivity enhancement and degradability of the PA C₁₆-KIISFK-NH₂ (Pat32) (SEQ. ID NO. 56) and of the peptide moiety KIISFK-NH₂ (Pat51) (SEQ. ID NO. 57). **A** Infectivity assay showing HIV-1 infection rates of TZM-bl cells observed in the presence of increasing concentrations of peptides PA1 (left data, SEQ ID NO. 4), Pat32 (SEQ. ID NO. 56) (middle data) and Pat51 (SEQ. ID NO. 57) (right data) (6.5 µM, 1.3 µM, 0.26 µM). The baseline (0 µM) shows infection rate without the addition of peptides. Mean values and standard deviations are derived from triplicate infections. RLU/s are relative light units per second. Pat32 (SEQ. ID NO. 56) does not enhances infectivity at 1.3 µM concentration more efficient compared to virus only infection. **B** Representative TEM micrographs of Pat32 (SEQ. ID NO. 56) that reveal the fibrillar morphologies of PA assembly (1 mg/mL in PBS), scale bar 100 nm.
**Figure 35** shows experimental characterization, infectivity enhancement and degradability of the PA C₁₆-AAVVHHK-NH₂ (Pat52*) (SEQ. ID NO. 58) and of the peptide moiety AAVVHHK-NH₂ (Pat52) (SEQ. ID NO. 59). **A** Infectivity assay showing HIV-1 infection rates of TZM-bl cells observed in the presence of increasing concentrations of peptides PA1 (left data, SEQ ID NO. 4), Pat52* (SEQ. ID NO. 58) (middle data) and Pat52 ((SEQ. ID NO. 59) (right data) (6.5 µM, 1.3 µM, 0.26 µM). The baseline (0 µM) shows infection rate without the addition of peptides. Mean values and standard deviations are derived from triplicate infections. RLU/s are relative light units per second. Pat52* (SEQ. ID NO. 58) enhances infectivity at 1.3 µM concentration 6 times more efficient compared to virus only infection. **B** Representative TEM micrographs of Pat52* (SEQ. ID NO. 58) that reveal the fibrillar morphologies of PA assembly (1 mg/mL in PBS), scale bar 100 nm.

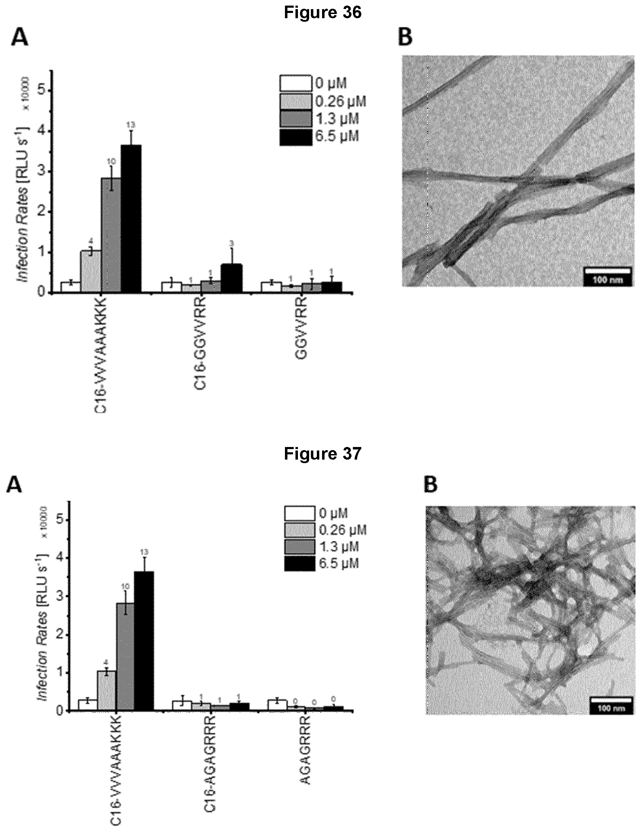

**Figure 36** shows experimental characterization, infectivity enhancement and degradability of the PA C₁₆-GGVVRR-NH₂ (Pati*) (SEQ. ID NO. 60) and of the peptide moiety GGVVRR-NH₂ (Pati) (SEQ. ID NO. 61). **A** Infectivity assay showing HIV-1 infection rates of TZM-bl cells observed in the presence of increasing concentrations of peptides PA1 (left data, SEQ ID NO. 4), Pati* (SEQ. ID NO. 60) (middle data) and Pati (SEQ. ID NO. 61) (right data) (6.5 µM, 1.3 µM, 0.26 µM). The baseline (0 µM) shows infection rate without the addition of peptides. Mean values and standard deviations are derived from triplicate infections. RLU/s are relative light units per second. Pati* (SEQ. ID NO. 60) does not enhance infectivity at 1.3 µM concentration more efficient compared to virus only infection. **B** Representative TEM micrographs of Pati* (SEQ. ID NO. 60) that reveal the fibrillar morphologies of PA assembly (1 mg/mL in PBS), scale bar 100 nm.
**Figure 37** shows experimental characterization, infectivity enhancement and degradability of the PA C₁₆-AGAGRRR-NH₂ (Patj*) (SEQ. ID NO. 62) and of the peptide moiety AGAGRRR-NH₂ (PatJ) (SEQ. ID NO. 63). **A** Infectivity assay showing HIV-1 infection rates of TZM-bl cells observed in the presence of increasing concentrations of peptides PA1 (left data, SEQ ID NO. 4), Patj* (SEQ. ID NO. 62) (middle data) and Patj (SEQ. ID NO. 63) (right data) (6.5 µM, 1.3 µM, 0.26 µM). The baseline (0 µM) shows infection rate without the addition of peptides. Mean values and standard deviations are derived from triplicate infections. RLU/s are relative light units per second. PatJ* does not enhance infectivity at 1.3 µM concentration more efficient compared to virus only infection. **B** Representative TEM micrographs of PatJ* (SEQ. ID NO. 62) that reveal the fibrillar morphologies of PA assembly (1 mg/mL in PBS), scale bar 100 nm.

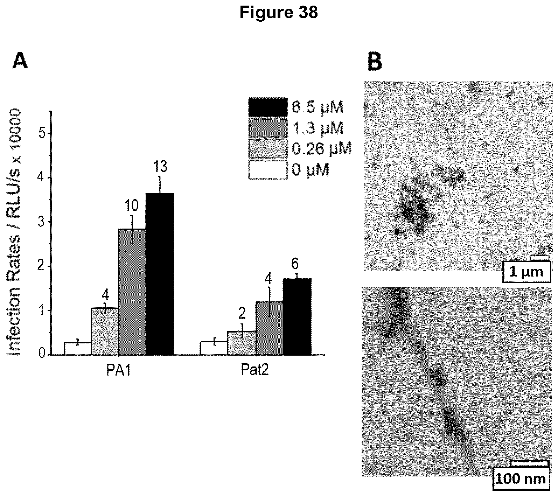

**Figure 38** shows experimental characterization, infectivity enhancement and degradability of the PA C₁₆-*disulfide*-CVVVAAAKKK-NH₂ (SEQ ID NO. 11) (Pat2). **A** Infectivity assay showing HIV-1 infection rates of TZM-bl cells observed in the presence of increasing concentrations of peptides PA1 (left data, SEQ ID NO. 4) and Pat2 (right data, SEQ ID NO. 11) 6.5 µM, 1.3 µM, 0.26 µM). The baseline (0 µM) shows infection rate without the addition of peptides. Mean values and standard deviations are derived from triplicate infections. RLU/s are relative light units per second. Pat2 enhances infectivity at 1.3 µM concentration 4 times more efficient compared to virus only infection. **B** Representative TEM micrographs of Pat2 (SEQ ID NO. 11) that reveal the fibrillar morphologies of PA assembly (1 mg/mL in PBS), scale bars top 1 µm, bottom 100 nm.

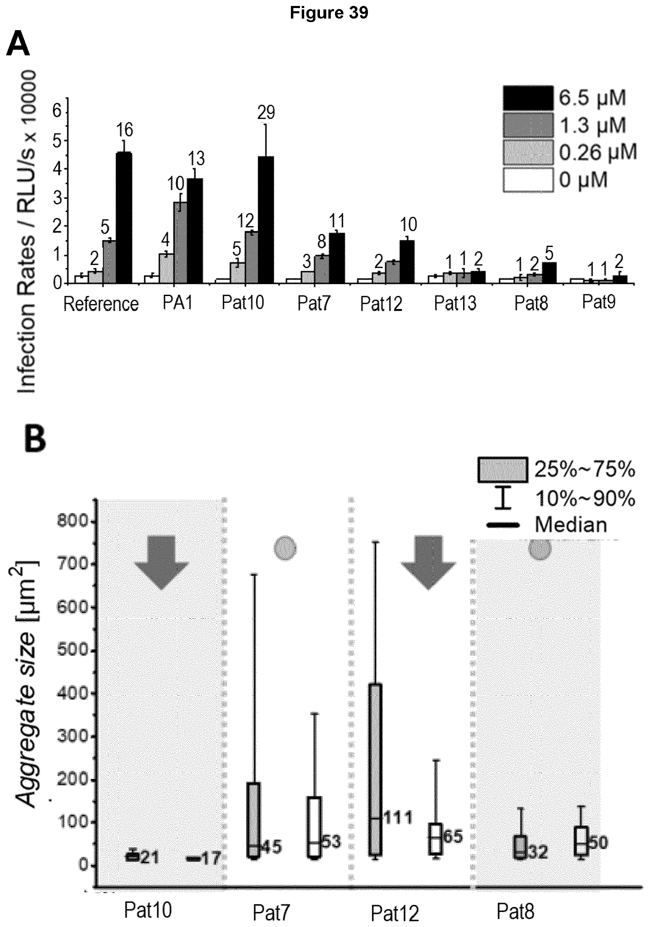

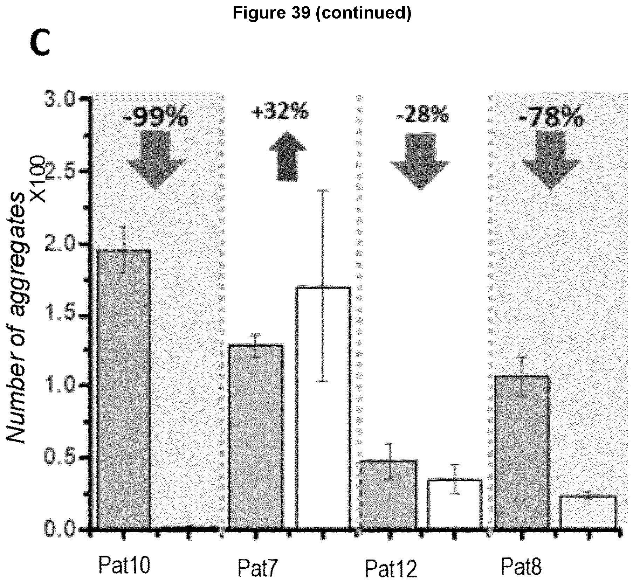

**Figure 39** shows experimental characterization, infectivity enhancement and degradability of the PA C₁₂-VVVAAAKKK-NH₂ (Pat7), PA C₉-VVVAAAKKK-NH₂ (Pat8). PA Fmoc-VVVAAAKKK-NH₂ (Pat12), PA Nap-VVVAAAKKK-NH₂ (Pat13), PA Eicosapentaenic acids-VVVAAAKKK-NH₂ (Pat10). α-Linolenic acd acids-VVVAAAKKK-NH₂ (Pat19) and the Reference peptide (EF-C). **A** Infectivity assay showing HIV-1 infection rates of TZM-bl cells observed in the presence of increasing concentrations of PAs (6.5 µM, 1.3 µM, 0.26 µM). The baseline (0 µM) shows infection rate without the addition of peptides. Mean values and standard deviations are derived from triplicate infections. RLU/s are relative light units per second. **B** Quantification of degradability by analyzing Proteostat stained aggregates via the 3D objects counter function of ImageJ 1.53f51 for 3 individual measurements (area 1008 µm × 1008 µm). Box plot showing size distribution and **C** bar plot showing number of aggregates > 10 µm² in an area of approximately 1008 × 1008 µm in cell culture after 30 min and 3 d incubation.
**Figure 40** shows CellTiter-Glo cell viability assay of TZM-bl cells treated with increasing concentrations (6.5 µM, 1.3 µM, 0.26 µM) of the PAs of biological triplicate with standard deviation. All PAs show viability above 80% and thus are biocompatible with the cells at tested concentrations: **A** Pati*: C₁₆-GGVVRR-NH₂ (SEQ. ID NO. 60); Patj*: C₁₆-AGAGRRR-NH₂ (SEQ. ID NO. 62); Pat33: vvvaaakkk-NH₂ (SEQ. ID NO. 33); Pat34: IIQKIK-NH₂ (SEQ. ID NO. 27); Pat35: AQAKAK-NH₂ (SEQ. ID NO. 35); Pat36: WEALKK-NH₂ (SEQ. ID NO. 37); Pat37: AGGRVK-NH₂ (SEQ. ID NO. 29); Pat38: LNGGVKK-NH₂ (SEQ. ID NO. 21); Pat39: ALAAGKK-NH₂ (SEQ. ID NO. 23); Pat40: AVAKKK-NH₂ (SEQ. ID NO. 39); Pat41: RRWQWR-NH₂ (SEQ. ID NO. 41); Pat42: LLLKK-NH₂ (SEQ. ID NO. 31); Pat43: VVAAHHH-NH₂ (SEQ. ID NO. 43); Pat44: KGVPGVGK-NH₂ (SEQ. ID NO. 45); Pat45: KYIHQNYTKALAGKLV-NH₂ (SEQ. ID NO. 47); Pat46: NFYLQVNKINK-NH₂ (SEQ. ID NO. 49); Pat47: GGGSSKKK-NH₂ (SEQ. ID NO. 51); Pat48: RYYASLRHYLNLVTRQRY-NH₂ (SEQ. ID NO. 53); Pat49: GKVGSK-NH₂ (SEQ. ID NO. 55); Pat50: AKAVGK-NH₂ (SEQ. ID NO. 25); Pat51: KIISFK-NH₂ (SEQ. ID NO. 57); Pat52: AAVVHHK-NH₂ (SEQ. ID NO. 59); Pati: GGVVRR-NH₂ (SEQ. ID NO. 61); Patj: AGAGRRR-NH₂ (SEQ. ID NO. 63); **B** EF-C: QCKIKQIINMWQ (SEQ. ID NO. 9); PA1: C₁₆-VVVAAAKKK-NH₂ (SEQ. ID NO. 4); Pat1: C₁₆-*ester-*SVVVAAAKKK-NH₂ (SEQ. ID NO. 10);Pat2: C₁₆-*disulfide*-CVVVAAAKKK-NH₂ (SEQ. ID NO. 11); Pat3: C₁₆-*thioester*-CVVVAAAKKK-NH₂ (SEQ. ID NO. 12); Pat7: C₁₂-VVVAAAKKK-NH₂ (SEQ. ID NO. 15); Pat8: C₉-VVVAAAKKK-NH₂ (SEQ. ID NO. 16); Pat10: eicosapentaene acid-VVVAAAKKK-NH₂ (SEQ. ID NO. 14); Pat12: Fmoc-VVVAAAKKK-NH₂ (SEQ. ID NO. 17); Pat13: Nap-VWAAAKKK- NH₂ (SEQ. ID NO. 18); Pat14: C₁₆-vvvaaakkk-NH₂ (SEQ. ID NO. 32); Pat15: C₁₆-IIQKIK-NH₂ (SEQ. ID NO. 26); Pat16: C₁₆-AQAKAK-NH₂ (SEQ. ID NO. 34); Pat17: C₁₆-WEALKK-NH₂ (SEQ. ID NO. 36); Pat18: C₁₆-AGGRVK-NH₂ (SEQ. ID NO. 28); Pat19: C₁₆-LNGGVKK-NH₂ (SEQ. ID NO. 20); Pat20: C₁₆-ALAAGKK-NH₂ (SEQ. ID NO. 22); Pat21: C₁₆-AVAKKK-NH₂ (SEQ. ID NO. 38); Pat22: C₁₆-RRWQWR-NH₂ (SEQ. ID NO. 40); Pat23: C₁₆-LLLKK-NH₂ (SEQ. ID NO. 30); Pat24: C₁₆-VVAAHHH-NH₂ (SEQ. ID NO. 42); Pat25: C₁₆-KGVPGVGK-NH₂ (SEQ. ID NO. 44); Pat26: C₁₆-KYIHQNYTKALAGKLV-NH₂ (SEQ. ID NO. 46); Pat27: C₁₆-NFYLQVNKINK-NH₂ (SEQ. ID NO. 47); Pat28: C₁₆-GGGSSKKK-NH₂ (SEQ. ID NO. 50); Pat29: C₁₆-RYYASLRHYLNLVTRQRY-NH₂ (SEQ. ID NO. 52); Pat30: C₁₆-GKVGSK-NH₂ (SEQ. ID NO. 54); Pat31: C₁₆-AKAVGK-NH₂ (SEQ. ID NO. 24); Pat32: C₁₆-KIISFK-NH₂ (SEQ. ID NO. 56); Pat52*: C₁₆-AAVVHHK-NH₂ (SEQ. ID NO. 58); Pati*: C₁₆-GGVVRR-NH₂ (SEQ. ID NO. 60).
**Figure 41** shows Proteostat Assay on PAs and peptides. A Linker exchange includes original sequence PA1 and variants with different linkers. Pat10: eicosapentaene acid-VVVAAAKKK-NH₂ (SEQ. ID NO. 14); Pat7: C₁₂-VVVAAAKKK-NH₂ (SEQ. ID NO. 15); Pat12: Fmoc-VVVAAAKKK-NH₂ (SEQ. ID NO. 17); ); Pat8: C₉-VVVAAAKKK-NH₂ (SEQ. ID NO. 16); Pat13: Nap-VVVAAAKKK-NH₂ (SEQ. ID NO. 18); B Exchange of hydrophobic moiety For reference also EF-C and Control (PBS with 10% DMSO) are shown. EF-C: QCKIKQIINMWQ (SEQ. ID NO. 9); PA1: C₁₆-VVVAAAKKK-NH₂ (SEQ. ID NO. 4); Pat1: C₁₆-*ester*-SVVVAAAKKK-NH₂ (SEQ. ID NO. 10); Pat3: C₁₆-*thioester*-CVVVAAAKKK-NH₂ (SEQ. ID NO. 12); Pat2: C₁₆*-disulfide-*CVVVAAAKKK-NH₂ (SEQ. ID NO. 11);**C** Exchange of peptide sequence. Pat14: C₁₆-vvvaaakkk-NH₂ (SEQ. ID NO. 32); Pat52*: C₁₆-AAVVHHK-NH₂ (SEQ. ID NO. 58); Pat19: C₁₆-LNGGVKK-NH₂ (SEQ. ID NO. 20); Pat26: C₁₆-KYIHQNYTKALAGKLV-NH₂ (SEQ. ID NO. 46); Pat27: C₁₆-NFYLQVNKINK-NH₂ (SEQ. ID NO. 48); Pat31: C₁₆-AKAVGK-NH₂ (SEQ. ID NO. 24); Pat20: C₁₆-ALAAGKK-NH₂ (SEQ. ID NO. 22); Pat29: C₁₆-RYYASLRHYLNLVTRQRY-NH₂ (SEQ. ID NO. 52); Pat16: C₁₆-AQAKAK-NH₂ (SEQ. ID NO. 34); Pat15: C₁₆-IIQKIK-NH₂ (SEQ. ID NO. 26); Pat23: C₁₆-LLLKK-NH₂ (SEQ. ID NO. 30); Pat18: C₁₆-AGGRVK-NH₂ (SEQ. ID NO. 28); Pat30: C₁₆-GKVGSK-NH₂ (SEQ. ID NO. 54); Pat28: C₁₆-GGGSSKKK-NH₂ (SEQ. ID NO. 50); Pat32: C₁₆-KIISFK-NH₂ (SEQ. ID NO. 56); Pat24: C₁₆-VVAAHHH-NH₂ (SEQ. ID NO. 42); Pat21: C₁₆-AVAKKK-NH₂ (SEQ. ID NO. 38); Pati*: C₁₆-GGVVRR-NH₂ (SEQ. ID NO. 60); Pat25: C₁₆-KGVPGVGK-NH₂ (SEQ. ID NO. 44); Pat17: C₁₆-WEALKK-NH₂ (SEQ. ID NO. 36); Patj*. C₁₆-AGAGRRR-NH₂ (SEQ. ID NO. 62); Pat22: C₁₆-RRWQWR-NH₂ (SEQ. ID NO. 40); **D** Controls showing peptide sequences without fatty acid residues. Pat46: NFYLQVNKINK-NH₂ (SEQ. ID NO. 49); Pat48: RYYASLRHYLNLVTRQRY-NH₂ (SEQ. ID NO. 53); Pat40: AVAKKK-NH₂ (SEQ. ID NO. 39); Pat45: KYIHQNYTKALAGKLV-NH₂ (SEQ. ID NO. 47); Pat47: GGGSSKKK-NH₂ (SEQ. ID NO. 51); Pat50: AKAVGK-NH₂ (SEQ. ID NO. 25); Pat34: IIQKIK-NH₂ (SEQ. ID NO. 27); Pat51: KIISFK-NH₂ (SEQ. ID NO. 57); Pati: GGVVRR-NH₂ (SEQ. ID NO. 61); Pat49: GKVGSK-NH₂ (SEQ. ID NO. 55); Pat52: AAVVHHK-NH₂ (SEQ. ID NO. 59); Pat39: ALAAGKK-NH₂ (SEQ. ID NO. 23); Pat44: KGVPGVGK-NH₂ (SEQ. ID NO. 45); Pat41: RRWQWR-NH₂ (SEQ. ID NO. 41); Pat38: LNGGVKK-NH₂ (SEQ. ID NO. 21); Pat42: LLLKK-NH₂ (SEQ. ID NO. 31); Pat36: WEALKK-NH₂ (SEQ. ID NO. 37); Pat37: AGGRVK-NH₂ (SEQ. ID NO. 29); Pat43: VVAAHHH-NH₂ (SEQ. ID NO. 43); Pat35: AQAKAK-NH₂ (SEQ. ID NO. 35); Pat33: vvvaaakkk-NH₂ (SEQ. ID NO. 33); Patj: AGAGRRR-NH₂ (SEQ. ID NO. 63). All PAs except of C₁₆-WEALKK-NH₂ (SEQ. ID NO. 36); C₁₆-AGAGRRR-NH₂ (SEQ. ID NO. 62), C₁₆-KGVPGVGK-NH₂ (SEQ. ID NO. 44); show Proteostat fluorescence above 1000 au and thus are well-visible via fluorescence microscopy.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### EXAMPLES

### Materials and Methods

### Materials

For peptide synthesis, Rink Amide AM resin LL (100-200 mesh), OxymaPure^{®}, (2-(1H-benzotriazol-1yl)-1,1,3,3-tetramethyluronium hexafluorophosphate (HBTU), Fmoc-Cys(Trt)-OH, Fmoc-Gln(Trt)-OH, Fmoc-Val-OH, Fmoc-Ala-OH and Fmoc-Glu(OtBu)-OH were purchased from Novabiochem^{®}, Merck. Fmoc-Lys(Boc)-OH, Fmoc-Phe-OH, Piperidine, Trifluoroacetic acid, N,N-Diisopropylethylamine and N,N'-Diisopropylcarbodiimide were purchased from Carl Roth GmbH & Co. KG. Palmitic acid and Triisopropylsilane were purchased from TCI Tokyo Chemical Industry. Peptide-synthesis-grade solvents (Dichloromethane, Dimethylformamide) were acquired from Acros Organics. Solvents were purchased in HPLC grade purity or higher from Sigma-Aldrich (Diethyl ether, Dimethylsulfoxide (DMSO)), Fisher Scientific (Dichloromethane, Dimethylformamide, Acetonitrile). Peptides EF-C and PNF18 (>95% purity) were purchased from Zhengzhou Phtd Peptides industrial Co. Vectofusin-1 was purchased from Miltenyi Biotech and RetroNectin was purchased from Takara Bio. Proteostat^{®} was purchased from Enzo Life Sciences. Thioflavin-T, Uranyl acetate and Dulbecco's Phosphate Buffered Saline without Magnesium and Calcium (DPBS) were purchased from Sigma-Aldrich. Dulbecco's Modified Eagle Medium (DMEM), Roswell Park Memorial Institute (RPMI) 1640 Medium, fetal calf serum (FCS), Dynabeads^{™} Human T-Activator CD3/CD28 were purchased from Gibco (Germany) and 1% aq. MEM non-essential amino acid solution was purchased from Sigma-Aldrich. Penicillin-Streptomycin and L-Glutamine (200 mM) were purchased from PAN-Biotech. Human interleukin-2 (IL-2), interleukin-7 (IL-7) and interleukin-15 (IL-15) were purchased from Miltenyi Biotech. RosetteSep^{™} Human CD4+ Enrichment Cocktail was purchased from STEMCELL Technologies.

### Instruments

Peptides were synthesized with an automated microwave peptide synthesizer (CEM, Liberty BlueTM). High pressure liquid chromatography (HPLC) on preparative scale was conducted with a Shimadzu system with following modules DGU-20A5R, LC-20AP, CBM-20A, SPD-M20A, SIL-10AP, FRC-18A with a reverse phase column Phenomenex Gemini^{®} (5 µm NX-C18, 110 Å, 150 × 30 mm). Analytical scale HPLC was performed using a Shimadzu system with modules DGU-20A5R, LC-20AT, CBM-20A, SPD-M20A, SIL-10ACHT, CTO-20AC, column Zorbax XDB-C18, 9.4 × 250 mm, 5 µm pore size. LC-MS was conducted on a Shimadzu LC-2020 Single Quadrupole MS instrument with the modules LC-20AD, SIL-20ACHT, SPD-20A, CTO-using a Kinetex EVO and C18 100 Å LC 50 × 2.1 mm column with 2.6 µm pore size. MALDI-TOF was accomplished on a Bruker rapifleX MALDI-TOF/TOF and a Waters MALDI Synapt G2-SI instrument. TEM measurements were performed on a JEOL 1400 instrument with 120 kV acceleration voltage equipped with a CCD camera. Fluorescence intensity was measured using a Spark 20 M microplate reader by the company Tecan Group, Ltd. Zeta-potential measurements were conducted with a Zeta Nanosizer ZS (Malvern Instruments) with 1 mL disposable folded capillary cells. FT-IR spectra of solid samples using a Bruker Tensor II spectrometer equipped with a diamond crystal as ATR element with a spectral resolution of 2 cm-1, each spectrum was an average of 64 scans. Circular dichroism measurements were conducted on a JASCO 1500 instrument. Fluorescence microscopy measurements were recorded on a Leica DMi8 microscope with 10x air objective and equipped with a Leica MC170 HD camera. Confocal laser scanning microscopy was performed on a Stellaris 8 confocal laser scanning microscope (Leica) equipped with a 20x air objective or on a Zeiss LSM 710 confocal microscope equipped with a 20x and 63x objective. Luminescence measurement for the evaluation of infection efficiency was conducted on Orion microplate luminometer (Berthold). Flow cytometry was conducted on CytoFLEX LX Flow Cytometer (Beckman Coulter) and flow cytometry data were analyzed using FlowJo (BD Bioscience).

### Synthesis and Purification of Peptides

Peptides were synthesized using an automated microwave peptide synthesizer (CEM, Liberty BlueTM) from C to N-terminus according to fluorenylmethyloxycarbonyl (Fmoc) solid phase peptide synthesis (SPPS) strategy by Merrifield using Rink Amide AM Resin LL (100-200 mesh, Novabiochem). The beads were swollen in DMF while shaking the reaction vessel for 1 h. The Fmoc-protecting group was cleaved off by a piperidine solution (20 % in DMF) at 155 W, 75 °C for 15 s and at 30 W, 90 °C for 50 s. The beads were washed three times with DMF before the addition of the Fmoc-protected amino-acid (2M, 5 equiv. relative to the resin loading capacity) in DMF, DIC (5 equiv.) in DMF and Oxyma Pure^{®} (10 equiv) in DMF. The coupling was conducted by microwaving at 170 W, 75 °C for 15 s and at 30 W, 90 °C for 110 s, the solution was removed, and the beads were washed with DMF. This procedure was repeated for each amino acid. Coupling of the fatty acid chain was conducted according to known procedure by adding palmitic acid (4 molar equiv), DIPEA (6 molar equiv), HBTU (4 molar equiv) in DMF/ DCM (5/1, v/v) at rt and shaking for 2 h. Cleavage from the resin was carried out in TFA/water/triisopropylsilane (95/2.5/2.5, v/v/v) for 2 h at room temperature. This solution was precipitated in cold diethyl ether (40 mL) and centrifuged 3 times at 4 °C, 4000 rpm for 15 min. The precipitate was dissolved in water with 0.1 % TFA for peptides with lysine residue and with 0.1 % NH4OH for peptides with glutamic acid residues yielding PAs as colourless powders (PA1 34%, PA2 25%, PA3 46% yield). The purification was conducted via HPLC (reverse phase column Phenomenex Gemini^{®} 5 µm NX-C18, 110 Å, 150 × 30 mm) using a gradient of water and acetonitrile containing 0.1 % TFA for peptides with lysine residues or 0.1 % NH₄OH for peptides with glutamic acid residues as the mobile phase at a flow rate of 25 mL/min. Chromatography was monitored with an UV absorption detector at 190 and 254 nm. The peptides were identified via MALDI-TF and purity was confirmed via LC-MS or analytical HPLC. The data was processed with Shimadzu LabSolutions and OriginLab.

### Fibril Formation

The peptides were predissolved in DMSO to a concentration of 10 mg/mL. The fibril formation was induced by further diluting the solution to a final incubation concentration of 650 µM or 1 mg/mL in PBS as indicated in the text and incubated for 1 d (1 day) at rt (room temperature, 20 °C - 25 °C). Other concentrations as described herein were achieved by further dilution of preformed fibrils.

### Characterization of Fibrils

Transmission electron microscopy (TEM) measurements, Thioflavin (ThT), zeta-potential measurements for fibril characterization were prepared and conducted according to prior art procedures. The morphology of the peptide assemblies was evaluated via TEM to determine the self-assembly at the nanoscale into fibrillar structures or amorphous aggregates. Amorphous aggregates are lacking fibrillar structures and have less internal order. Fluorescence microscopy of dye-stained peptides was applied to distinguish between microscopically aggregating or distinct fibrils. Fibrils were defined as distinct, if no visible structures could be identified in fluorescence and brightfield microscopy. A peptide fibril was considered as ThT-active if the fluorescence intensity was at least twice as strong compared to the control (PBS containing 10% DMSO).

Zeta-potential: 60 µL of the peptides (1 mg mL-1) were diluted in 600 µL, 1 mM KCI solution and placed in a disposable folded capillary cuvette. All zeta-potential measurements were conducted by dynamic light scattering technology (Zetasizer, Malvern Instruments) and in triplicates for each sample. The derived count rate of the zeta potential measurement was used as information on the light scattering intensity and turbidity of the sample as a further indicator for microscopic aggregation.

ATR-FT-IR: ATR-IR spectra acquisition and the calculation of β-sheet content were conducted according prior art procedures. Briefly, FT-IR spectra of solid samples were recorded after lyophilization of fibril solutions using a Bruker Tensor II spectrometer equipped with a diamond crystal as ATR element with a spectral resolution of 2 cm⁻¹, each spectrum was an average of 64 scans. The data was processed with Origin software. β-sheet amount was determined by calculating the integral of the peak at 1630 cm-1 (β-sheet) relative to the integral between 1650-1700 cm-1 (native structure) from the 2^{nd} derivative of the FT-IR spectra.

CD: Circular dichroism measurements were performed on a JASCO J-1500 instrument in a 1 mm path length quartz cuvette (HellmaAnalytics) from 190 - 250 nm with peptides directly dissolved in MilliQ water to 1 mg mL⁻¹ and subsequently pH adjusted to pH 7.4 with 0.1 M NaOH (aq.) or HCl (aq.) and incubated for 1 d to induce assembly. CD measurements were conducted in pure water to avoid aggregate formation which occurs in presence of phosphate ions from PB buffer and disturbs the measurement. It was previously shown that CD measurements in PB or MilliQ yield comparable results for other PA. The samples were incubated for 1 d and diluted to 0.1 mg mL⁻¹ before measurement. Three accumulations were averaged for each measurement with a wavelength step size of 2 nm and scanning speed of 20 nm min⁻¹. For each measurement, the corresponding background measurements were conducted and subtracted from the original spectrum.

Fluorescence microscopy: 1 µL Fibrils (1 mg mL⁻¹) were stained with 9 µL, 50 µM ThT. 10 µL of the stained fibrils (0.1 mg mL⁻¹) were placed on a microscopy glass. Fluorescence microscopy measurements were performed on a Leica DMi8 microscope with 10x air objective and equipped with a Leica MC170 HD camera with a filter setting λₑₘ = 527/30 nm upon excitation at λₑₓ = 480/40 nm) and processed with the Leica LASX software.

### Cell culture

TZM-bl and HeLa cells were cultured in Dulbecco's Modified Eagle Medium (DMEM) supplemented with 10 % (v/v) inactivated fetal calf serum (FCS), L-glutamine (2 mM), penicillin (100 units/ml) and streptomycin (100 µg/ml). Jurkat cells were cultured in Roswell Park Memorial Institute (RPMI) 1640 Medium supplemented with 10 % (v/v) fetal calf serum (FCS), L-glutamine (2 mM), penicillin (100 units/ml) and streptomycin (100 µg/ml). CD4+ T cells were cultured in supplemented RPMI medium in presence of interleukin-2 (IL-2), interleukin-7 (IL-7) and interleukin-15 (IL-15) as described below.

### Virus-Peptide Fibril-Cell Interaction

Infectious HIV-1 stocks, GALV-pseudotyped γ-retroviral vector (GALV-RV) were prepared according prior art procedures.

**HIV-1 Infection Assay with TZM-bl cells:** The effect of peptide fibrils on HIV-1 infection was studied via a luminescence assay for β-galactosidase, which was reported by TZM-bl cells upon infection. The HIV-1 infection assay was conducted in three technical replicates and repeated in three independent experiments unless stated otherwise. The assay was conducted according prior art procedures.

γ-**Retroviral transduction Assay with Jurkat and CD4+ T cells:** The effect of peptide fibrils on retroviral transduction of Jurkat cells was analyzed 3 days post transduction by determining the percentage of GFP + cells by flow cytometry. The assay was conducted according prior art procedures.

Human CD4+ T cells were isolated from a Buffy coat using RosetteSep^{™} Human CD4+ Enrichment Cocktail (STEMCELL Technologies) according to manufacturer's protocol. Isolated T cells were activated using Dynabeads^{™} Human T-Activator CD3/CD28 (GibcoTM) according to manufacturer's protocol in presence of 10 ng mL⁻¹ IL-2 (Miltenyi Biotec) for three days. After 3 days the magnetic Dynabeads were removed using a magnet and T cells were cultured in presence of 9 ng mL⁻¹ IL-7 (Miltenyi Biotec) and 10 ng mL⁻¹ IL-15 (Miltenyi Biotec). After 1 day 5×10⁵ T cells were transduced with GALV-γ-RV in absence and presence of PA1, PA2, EF-C (0.26, 1.3 and 6.5 µM) and Vectofusin^{®}-1 (10 µg mL⁻¹; Miltenyi Biotec), or RetroNectin (20 µg mL⁻¹; Takara Bio). The concentration of the transduction enhancers corresponds to the concentration on cells and in the case for RetroNectin it corresponds to the concentration that was used to precoat the 24-well plate. The 24-well plate was coated with RetroNectin and incubated overnight at 4 °C. Wells were blocked with PBS containing 2 % BSA for 30 min and washed twice with PBS. Virus was centrifuged onto coated wells at 2,000 g at 32 °C for 2 h. Viral supernatant was removed and CD4+ T cells were added and subsequently spin-infected (300 g for 10 min) with virus again. 1 day after transduction the medium was exchanged. After 3 days GFP+ T cells were analyzed by flow cytometry. Viability was determined using LIVE/DEAD^{™} Fixable Aqua Dead Cell Stain Kit (ThermoFisher Scientific) according to manufacturer's protocol.

**Cell Viability with TZM-bl and Jurkat cells:** The cell viability after addition of peptides to TZM-bl cells and Jurkat was studied via the CellTiter-Glo assay. To this end, 20,000 cells TZM-bl or 50,000 Jurkat cells were incubated with serial diluted peptides. After 3 days the supernatant was removed and 100 µL CellTiter-Glo Reagent 1:1 diluted in PBS was added. After 10 min 50 µL was transferred to white microplate and luminescence was recorded by Orion microplate luminometer.

**Confocal laser scanning microscopy and degradation study with PA1-3 and HeLa cells:** HeLa cells were seeded one day prior to conducting the assay (40,000/well) in an 8-well IBIDI slide. 4 µL of the preformed peptide fibrils (1 mg mL⁻¹) were diluted with 4 µL Proteostat (Enzo Life Science, 1 µL stock in 999 µL PBS) and incubated for 10 min and further diluted with medium to receive a final peptide concentration of 20 µg mL⁻¹. The nucleus of the HeLa cells was stained with Hoechst 33342 (NucBlue^{™}, Thermo Fisher Scientific). The peptide solution was transferred to the HeLa cells and incubated for 30 min at 37°C before washing three times with PBS. The interaction of fibril clusters with cells was monitored after 30 min incubation time on a Stellaris 8 confocal laser scanning microscope (Leica) equipped with a 20x air objective and laser excitation wavelengths of 405 nm (Hoechst) and 561 nm (Proteostat). For the evaluation of aggregate degradation, the samples were further incubated at 37°C for 3 d and analyzed without any additional washing step. The evaluation of aggregate size and number was conducted by analyzing Proteostat stained aggregates via the 3D objects counter function of ImageJ 1.53f51 for 3 individual measurements (area 581.25 × 581.25 µm). The size distribution shows aggregates sizes larger than the threshold 10 µm² in an area of 581.25 × 581.25 µm and repeated in three technical triplicates. The number of aggregates were determined by accumulating the number of aggregates larger than 10 µm² in an area of 581.25 × 581.25 µm, repeated in three technical triplicates.

**Confocal laser scanning microscopy and degradation study with PA1-3 and CD4+ T cells:** The degradation of the peptide assemblies on CD4+ T cells was determined by analyzing fluorescence microscopy images. To this end, 4 µL of the preassembled peptide fibrils EF-C, PA1 and PA2 (1 mg mL⁻¹, 1 d in PBS) were mixed with 4 µL of Proteostat (Enzo Life Science, 1 µL stock in 99 µL PBS) and incubated for 15 min. 2 µL of the stained fibrils were added to 198 µL preactivated and nucleus stained (Hoechst 33342) CD4+ T cells (200 µL, 0.25×10⁶ resulting in a final 5 µg mL⁻¹concentration of the peptide. After incubation for 30 min at 37°C the cells were washed with 3x with PBS and incubated in medium (supplemented RPMI containing IL-15 and 7). Microscopy was conducted on a Zeiss LSM 710 confocal microscope with 20x and 63x objective and laser excitation wavelengths of 405 nm (Hoechst) and 561 nm (Proteostat). The measurements were performed after 30 min and 3 d of incubation at 37°C without any washing step in between. Control experiments to analyze changes of fibril aggregation were conducted analogously without the presence of cells. The evaluation of aggregate size and number was conducted by analyzing Proteostat stained aggregates via the 3D objects counter function of ImageJ 1.53f51 for 5 individual measurements (area 708.5 × 708.5 µm) of three individually repeated experiments.

**Confocal laser scanning microscopy and degradation study with PA library and HeLa cells:** Confocal laser scanning microscopy studies were performed for the visualization of the cell-PA interaction. HeLa cells were seeded one day prior to conducting the assay (20,000/well) in an 8-well IBIDI slide. 2 µL of the preformed peptide fibrils (1 mg mL⁻¹) were diluted with 2 µL Proteostat (Enzo Life Science, 1 µL stock in 99 µL PBS) and incubated for 10 min and further diluted with medium to receive a final peptide concentration of 10 µg mL⁻¹. The nucleus of the HeLa cells was stained with Hoechst 33342 (NucBlue^{™}, Thermo Fisher Scientific). The peptide solution mixture was transferred to the HeLa cells and incubated for 30 min at 37°C before washing three times with PBS. The interaction of fibril clusters with cells was monitored after 30 min incubation time on a Stellaris 8 confocal laser scanning microscope (Leica) equipped with a 20x air objective and laser excitation wavelengths of 405 nm (Hoechst), and 561 nm (Proteostat). For the evaluation of aggregate degradation, the samples were further incubated at 37°C for 3 d and analyzed without any additional washing step. The evaluation of aggregate size and number was conducted by analyzing Proteostat stained aggregates via the 3D objects counter function of ImageJ 1.53f51 for 3 individual measurements (area 1008 × 1008 µm). The size distribution shows aggregates sizes larger than the threshold 10 µm² in an area of 1008 × 1008 µm. The number of aggregates were determined by counting aggregates larger than the threshold 10 µm² in an area of 1008 × 1008 µm and repeated in three technical triplicates.

Tested and investigated Compounds are shown in table 5.

**Table 5: Tested and investigated Compounds**

| SEQ ID NO | Peptide/ Peptid Amphiphile | Code |
|---|---|---|
| 1 | VVVAAAKKK-NH₂ | P1 |
| 2 | VVVAAAEEE-NH₂ | P3 |
| 3 | FQFKFKC-NH₂ | P2 |
| 4 | C₁₆-VVVAAAKKK-NH₂ | PA1 |
| 5 | C₁₆-VVVAAAEEE-NH₂ | PA3 |
| 6 | CKFKFQF-NH₂ | Reverse P2 |
| 7 | C₁₆-CKFKFQF-NH₂ | PA2 |
| 8 | C₁₆-FQFKFKC-NH₂ | Reverse PA2 |
| 9 | QCKIKQIINMWQ | EF-C (Reference) |
| 10 | C₁₆-*ester*-SVVVAAAKKK-NH₂ | Pat1 |
| 11 | C₁₆-*disulfide*-CVVVAAAKKK-NH₂ | Pat2 |
| 12 | C₁₆-*thioester*-CVVVAAAKKK-NH₂ | Pat3 |
| 13 | LNGGV | Beta Pat19 |
| 14 | eicosapentaene acid-VWAAAKKK- NH₂ | Pat10 |
| 15 | C₁₂-VVVAAAKKK-NH₂ | Pat7 |
| 16 | C₉-VVVAAAKKK-NH₂ | Pat8 |
| 17 | Fmoc-VVVAAAKKK-NH₂ | Pat12 |
| 18 | Nap-VVVAAAKKK- NH₂ | Pat13 |
| 19 | α-linolenic acid- VWAAAKKK- NH₂ | Pat9 |
| 20 | C₁₆-LNGGVKK-NH₂ | Pat19 |
| 21 | LNGGVKK-NH₂ | Pat38 |
| 22 | C₁₆-ALAAGKK-NH₂ | Pat20 |
| 23 | ALAAGKK-NH₂ | Pat39 |
| 24 | C₁₆-AKAVGK-NH₂ | Pat31 |
| 25 | AKAVGK-NH₂ | Pat50 |
| 26 | C₁₆-IIQKIK-NH₂ | Pat15 |
| 27 | IIQKIK-NH₂ | Pat34 |
| 28 | C₁₆-AGGRVK-NH₂ | Pat18 |
| 29 | AGGRVK-NH₂ | Pat37 |
| 30 | C₁₆-LLLKK-NH₂ | Pat23 |
| 31 | LLLKK-NH₂ | Pat42 |
| 32 | C₁₆-vvvaaakkk-NH₂ | Pat14 |
| 33 | vvvaaakkk-N H₂ | Pat33 |
| 34 | C₁₆-AQAKAK-NH₂ | Pat16 |
| 35 | AQAKAK-NH₂ | Pat35 |
| 36 | C₁₆-WEALKK-NH₂ | Pat17 |
| 37 | WEALKK-NH₂ | Pat36 |
| 38 | C₁₆-AVAKKK-NH₂ | Pat21 |
| 39 | AVAKKK-NH₂ | Pat40 |
| 40 | C₁₆-RRWQWR-NH₂ | Pat22 |
| 41 | RRWQWR-NH₂ | Pat41 |
| 42 | C₁₆-VVAAHHH-NH₂ | Pat24 |
| 43 | VVAAHHH-NH₂ | Pat43 |
| 44 | C₁₆-KGVPGVGK-NH₂ | Pat25 |
| 45 | KGVPGVGK-NH₂ | Pat44 |
| 46 | C₁₆-KYIHQNYTKALAGKLV-NH₂ | Pat26 |
| 47 | KYIHQNYTKALAGKLV-NH₂ | Pat45 |
| 48 | C₁₆-NFYLQVNKINK-NH₂ | Pat27 |
| 49 | NFYLQVNKINK-NH₂ | Pat46 |
| 50 | C₁₆-GGGSSKKK-NH₂ | Pat28 |
| 51 | GGGSSKKK-NH₂ | Pat47 |
| 52 | C₁₆-RYYASLRHYLNLVTRQRY-NH₂ | Pat29 |
| 53 | RYYASLRHYLNLVTRQRY-NH₂ | Pat48 |
| 54 | C₁₆-GKVGSK-NH₂ | Pat30 |
| 55 | GKVGSK-NH₂ | Pat49 |
| 56 | C₁₆-KIISFK-NH₂ | Pat32 |
| 57 | KIISFK-NH₂ | Pat51 |
| 58 | C₁₆-AAVVHHK-NH₂ | Pat52* |
| 59 | AAVVHHK-NH₂ | Pat52 |
| 60 | C₁₆-GGVVRR-NH₂ | Pati* |
| 61 | GGVVRR-NH₂ | Pati |
| 62 | C₁₆-AGAGRRR-NH₂ | Patj* |
| 63 | AGAGRRR-NH₂ | Patj |
| 64 | ALAAG | Beta Pat20 |
| 65 | AKAVG | Beta pat31 |
| 66 | CVWAAA | |
| 67 | SVWAAA | |
| 68 | CLNGGV | |
| 69 | SLNGGV | |
| 70 | CALAAG | |
| 71 | SALAAG | |
| 72 | CAKAVG | |
| 73 | SAKAVG | |
| 74 | VWAAA | |
| 75 | VVVAAAKKK | |
| 76 | CVWAAAKKK | |
| 77 | SVWAAAKKK | |
| 78 | LNGGVKK | |
| 79 | ALAAGKK | |
| 80 | AKAVGK | |
| 81 | IIQKIK | |
| 82 | AGGRVK | |
| 83 | LLLKK | |
| 84 | AQAKAK | |
| 85 | WEALKK | |
| 86 | AVAKKK | |
| 87 | CKFKFQF | |
| 88 | RRWQWR | |
| 89 | WAAHHH | |
| 90 | KGVPGVGK | |
| 91 | KYIHQNYTKALAGKLV | |
| 92 | NFYLQVNKINK | |
| 93 | GGGSSKKK | |
| 94 | RYYASLRHYLNLVTRQRY | |
| 95 | GKVGSK | |
| 96 | KIISFK | |
| 97 | AAWHHK | |
| 98 | GGWRR | |
| 99 | AGAGRRR | |
| 100 | VWAAAEEE | |
| 101 | FQFKFKC | |

### Example 1 - Peptide Amphiphiles PA1-PA3 and Peptides P1-P3

The peptides P1-3 and PA1-3 were synthesized via standard solid-phase chemistry using the Fmoc protection strategy (see methods). PAs were accomplished by linking a palmitic acid (C₁₆) to the N-terminus. The purity of the conjugates (>90%) was determined by LC-MS or HPLC and the expected m/z values were determined by MALDI-TOF for each peptide. After confirming the successful synthesis of the peptides, the peptides were characterized by various methods to assess aggregation and fibril formation as discussed in the Examples (zeta-potential, Thioflavin T (ThT) fluorescence, circular dichroism (CD), attenuated total reflection infrared (ATR-IR) spectroscopy, and transmission electron microscopy (TEM) (Figure 2-7). The peptides P1 and P3 without the palmitic tail, formed non-fibrillar amorphous aggregates and P2 assembled into twisted fibrils (Figure 2) in PBS according to TEM images. Substituting lysine residues with glutamic acid in PA3 results in distinct fibrils (Figure 3), which did not bundle or adopt µm-sized aggregates in PBS.

The evaluation of aggregate size and number was conducted by analyzing Proteostat-stained aggregates via the 3D objects counter function of ImageJ 1.53f51 for 6 individual measurements (area 581.25 µm × 581.25 µm) of three individually repeated experiments. Control experiments to analyze changes of fibril aggregation were conducted analogously without cells and analyzed with fluorescence microscopy measurements (Leica DMi8 microscope, 10x air objective, Leica MC170 HD camera, λₑₘ = 527/30 nm, λₑₓ = 480/40 nm). The evaluation of aggregate size and number was conducted by analyzing Proteostat stained aggregates via the 3D objects counter function of ImageJ 1.53f51 for 3 individual measurements (area 1331.2 µm × 1331.2 µm). The degradation of the peptide assemblies on CD4+ T cells was determined by analyzing fluorescence microscopy images. To this end, 4 µL of the preassembled peptide fibrils EF-C, PA1 and PA2 (1 mg/mL, 1 d in PBS) were mixed with 4 µL of Proteostat (Enzo Life Science, 1 µL stock in 99 µL PBS) and incubated for 15 min. 2 µL of the stained fibrils were added to 198 µL preactivated and nucleus stained (Hoechst 33342) CD4+ T cells (200 µL, 0.25x106 resulting in a final 5 µg/mL concentration of the peptide. After incubation for 30 min at 37°C the cells were washed with 3x with PBS and incubated in medium (supplemented RPMI containing IL-15 and 7). Microscopy was conducted on a Zeiss LSM 710 confocal microscope with 20x and 63x objective and laser excitation wavelengths of 405 nm (Hoechst) and 561 nm (Proteostat). The measurements were performed after 30 min and 3 d of incubation at 37°C without any washing step in between. Control experiments to analyze changes of fibril aggregation were conducted analogously without the presence of cells. The evaluation of aggregate size and number was conducted by analyzing Proteostat stained aggregates via the 3D objects counter function of ImageJ 1.53f51 for 5 individual measurements (area 708.5 µm × 708.5 µm) of three individually repeated experiments.

### Example 2 - Linker exchange

To cover a wide variety of different PA designs and study its influence on infectivity enhancement, aggregate formation and degradation, a PA library inspired by PA1 and PA2 consisting distinct peptide amphiphiles by varying the linkage chemistry (Figures 12, 13, 38). Peptide synthesis was conducted via standard solid-phase chemistry using the Fmoc protection strategy (see methods). PAs were accomplished by linking a palmitic acid (C₁₆) to the side chain of the N-terminus via known methods. The purity of the conjugates (> 90%) was determined by LC-MS or HPLC and the expected *m*/*z* values were determined by MALDI-TOF for each peptide. After confirming the successful synthesis of the peptides, they were characterized by various methods to assess aggregation and fibril formation as discussed below (zeta-potential, Thioflavin T (ThT) fluorescence, attenuated total reflection infrared (ATR-IR) spectroscopy, and transmission electron microscopy (TEM)). The peptides were tested for cell-viability and infectivity enhancement by a HIV-1 infection assay on TZM-bl cells (see methods). The peptides were characterized for fibril formation, β-sheet content, µm-sized aggregation, surface charge and amyloid character via TEM, FT-IR, widefield and confocal microscopy, zeta-potential measurements, and ThT-assay, respectively (see methods). The number and degradability of µm-sized aggregates were evaluated via confocal microscopy in presence of Hela cells after 30 min and 3 d of incubation at 37°C (see methods). The initial result are summarized in tables 6 and 7.

**Table 6: Summary of results of linkage chemistry exchange with palmitic acid as hydrophobic moiety. Summary of the infectivity enhancing N fold at 1.3 µM, Assembly, and degradability (ND = not detected**

| Code | Sequence | SEQ ID NO | Infectivity enhancing N fold at 1.3 µM | Assembly | Assembly without hydrophob residue | Degradability / % (rel. to initial particles > 10 µm²) |
|---|---|---|---|---|---|---|
| PA1 | C₁₆-Amide-VVVAAAKKK-NH₂ | 4 | 10±3 | Yes | No | 94% |
| Pat1 | C₁₆-Ester-SVVVAAAKKK-NH₂ | 10 | 17±5 | Yes | No | 31% |
| Pat2 | C₁₆-Disulfide-CVVVAAAKKK-NH₂ | 11 | 4±2 | Yes | No | ND |
| Pat3 | C₁₆-Thioester-CVVVAAAKKK-NH₂ | 12 | 11±4 | Yes | No | 87% |

**Table 7: Summary of the physicochemical properties of linkage chemistry exchange with palmitic acid as hydrophobic moiety: zeta-potential, derived count rate of scattered light during zeta-potential measurements, N-fold ThT fluorescence relative to PBS control and fibril formation (via TEM, "1" means fibril formation observed, "0" means no fibrils observed). (Reference = QCKIKQIINMWQ (SEQ ID NO 9**

| Peptide Amphiphile | Reference (SEQ ID NO 9) | PA1 (SEQ ID NO 4) | Pat1 (SEQ ID NO 10) | Pat3 (SEQ ID NO 12) |
|---|---|---|---|---|
| Zeta-Potential / mV | 6.6 | 22.2 | 20.5 | 18.3 |
| Count Rate / kcps | 2602.3 | 3138.4 | 3217.3 | 2058.5 |
| N-fold ThT-fluorescence | 16.2 | 278.7 | 277.1 | 166.1 |
| Fibril Formation | 1 | 1 | 1 | 1 |

In each case the palmitic acid functionalized and non-functionalized peptides were tested to investigate the influence of the fatty acid. An increase of the infectivity enhancement is observed upon exchange of the linker from amide to ester or thioester. Exchange of the linker from amide to ester linkage enhances infectivity at 1.3 µM concentration 17 times more efficient compared to virus only infection. However, changing from amide to disulfide is reducing infectivity enhancement significantly (n-fold at 1.3 µM 10 → 4) but the peptide is still increasing infectivity.

All of these peptides with exchanged linker (N-terminal cysteine or N-terminal serine) show comparable physicochemical properties, but the disulfide exchange results in less aggregates, which is likely the reason why less infectivity enhancement is observed.

While no decrease of the infectivity enhancement is observed upon exchange of the linker, a great variability in aggregation behavior and degradation of these aggregates is observed. The peptides can all be stained with the imaging agent Proteostat, but C16-disulfide-CVWAAAKKK (SEQ. ID NO. 11) cannot be observed via microscopy, because it is not forming large (>10 µm²) aggregates.

Aggregate size of PA1 (SEQ. ID NO. 4) used as reference decreases from 28 to 23 µm² and aggregate number decreases 94%.

Aggregate size of Pat1 (SEQ. ID NO. 10) decreases from about 43 to 22 µm² and aggregate number decreases by 30% over 3 days (Figure 12D,E). Thus, Pat1 (SEQ. ID NO. 10) forms larger µm-sized fibrillar aggregates in the preferred area range of < 50 µm² than PA1 (SEQ. ID NO. 4). The formation of µm-sized fibrillar aggregates has been found to be an important aspect for the infectivity enhancement. Therefore, the formation of µm-sized fibrillar aggregates of about 43 µm² is likely the reason why more efficient infectivity enhancement is observed in comparison to PA1 (SEQ. ID NO. 4). Although the aggregate number decreases by 30%, it should be noted that the average size of the aggregates is reduced to about 22 µm² in case of Pat1 (SEQ. ID NO. 10) which is comparable with the average size of aggregates of PA1 (SEQ. ID NO. 4) of about 23 µm² after 3 days. (Figure 12D,E)

Aggregate size of Pat3 (SEQ. ID NO. 12) decreases from 22 to 10 µm² and aggregate number decreases by 87%, characterizing Pat3 (SEQ. ID NO. 12) as a degradable PA. Thus, Pat3 (SEQ. ID NO. 12) forms µm-sized fibrillar aggregates comparable to the larger µm-sized fibrillar aggregates of PA1 (SEQ. ID NO. 4). The aggregate number of Pat3 (SEQ. ID NO. 12) aggregates decreases by 87%, a little less reduction in comparison to PA1 (SEQ. ID NO. 4). However, the average size of aggregates of Pat3 (SEQ. ID NO. 12) strongly decreases which is especially beneficial for clinical applications. (Figure 13D,E).

Thus, all investigated linker-modified PAs are degradable. The best performing linker exchange is observed for the thioester modification (Pat3), which shows a degradation of 87% and small, well distributed aggregates. The ester modified PA (Pat1) is also forming small aggregates and degraded well, but not as efficient as PA1 or Pat3. The disulfide-modified linker is reducing aggregates size drastically. No aggregates above 10 µm² microscope can be observed. However, reduced aggregation also means that infectivity enhancement is significantly (n-fold at 1.3 µM 10 → 4) reduced.

### C₁₆-ester-SVVVAAAKKK-NH₂ (SEQ. ID NO. 10) (Pat1)

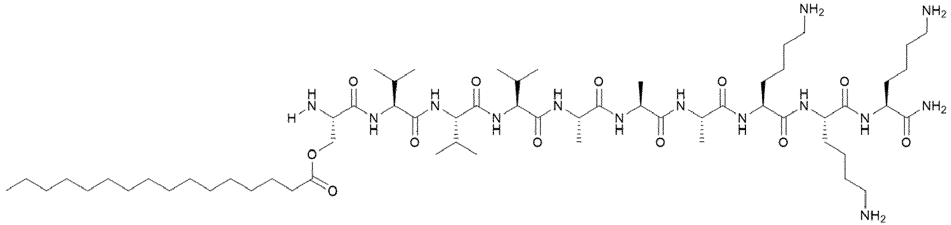

Infectivity enhancement and degradability of the PA C₁₆-*ester*-SVVVAAAKKK-NH₂ (Pat1) (SEQ. ID NO. 10) was investigated. The data show that Pat1 (SEQ. ID NO. 10) forms β-sheet rich fibrils (43% β-sheet) that form µm-sized aggregates that can interact with HeLa cells, thus are potent enhancers of HIV-1 infection. They can be degraded in presence of HeLa cells within 3 days (30 %). Infectivity assay (Figure 12A) showing HIV-1 infection rates of TZM-bl cells observed in the presence of increasing concentrations of peptides PA1 (SEQ. ID NO. 4) and Pat1 (SEQ. ID NO. 10) (6.5 µM, 1.3 µM, 0.26 µM). Pat1 enhances infectivity at 1.3 µM concentration 17 times more efficient compared to virus only infection. The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 43% (Figure 12B). The TEM micrographs of Pat1 that reveal the fibrillar morphologies of PA assembly (1 mg/mL in PBS) (Figure 12C). Quantification of degradability by analyzing Proteostat stained aggregates via the 3D objects counter function of ImageJ 1.53f51 for 3 individual measurements (area 1008 × 1008 µm) (Figure 12D,E). Aggregate size decreases from 43 to 22 µm² and aggregate number decreases 30%, characterizing Pat1 (SEQ. ID NO. 10), as a degradable PA. Fluorescence microscopy measurements of 1 µL Pat1 (SEQ. ID NO. 10), (1 mg/mL) stained with 9 µL, 50 µM ThT with a filter setting λₑₘ = 527/30 nm and λₑₓ = 480/40 nm showed fluorescent, µm-sized aggregates for Pat1 (not shown). Laser scanning microscopy images of Proteostat stained PAs and HeLa cells (not shown) revealed formation of µm-sized PA clusters for Pat1 (SEQ. ID NO. 10). Pat1 (SEQ. ID NO. 10) (2 µg) was added to cells (20.000 cells per well) for 30 min, washed and samples were analyzed (d0). Pat1 (SEQ. ID NO. 10) revealed association with cellular membranes. After 3 days of incubation (d3) less aggregates are observed, which reveals the degradability of Pat1 (SEQ. ID NO. 10).

### C₁₆-thioester-CVVVAAAKKK-NH₂ (SEQ. ID NO. 12) (Pat3)

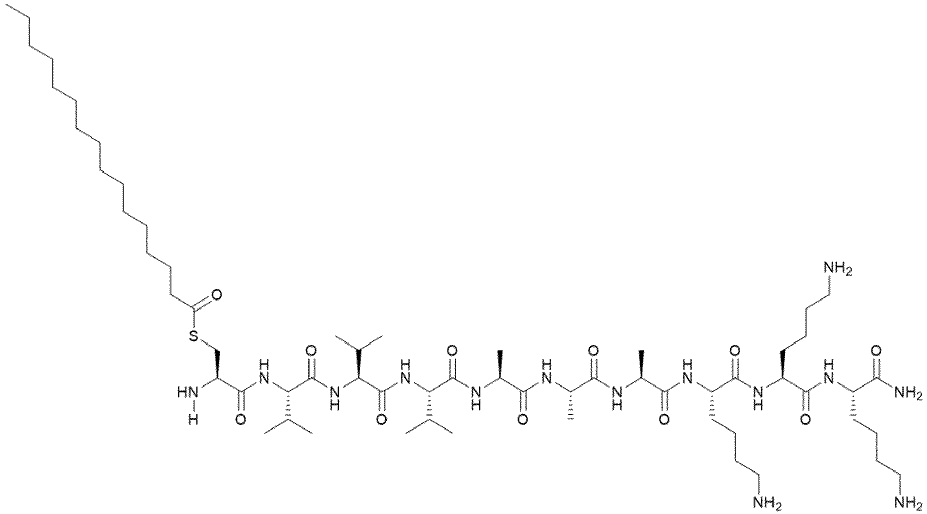

Infectivity enhancement and degradability of the PA C₁₆-*thioester*-CVVVAAAKKK-NH₂ (SEQ. ID NO. 12) (Pat3). The data showed that Pat3 forms β-sheet rich fibrils (50% β-sheet) that form µm-sized aggregates that can interact with HeLa cells, thus are potent enhancers of HIV-1 infection. They can be degraded in presence of HeLa cells within 3 days (87 %). Infectivity assay (Figure 13A) showed HIV-1 infection rates of TZM-bl cells observed in the presence of increasing concentrations of peptides PA1 (SEQ ID NO. 4) and Pat3 (SEQ. ID NO. 12) (6.5 µM, 1.3 µM, 0.26 µM). The β-sheet amount of Pat3 (SEQ. ID NO. 12) (1 mg/mL in PBS lyophilized) was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 50% (Figure 12B). TEM micrographs of Pat3 (SEQ. ID NO. 12) revealed the fibrillar morphologies of PA assembly (1 mg/mL in PBS) (Figure 12C). Quantification of degradability was conducted by analyzing Proteostat stained aggregates via the 3D objects counter function of ImageJ 1.53f51 for 3 individual measurements (area 1008 × 1008 µm) (Figure 13D,E). Aggregate size decreases from 22 to 10 µm² and aggregate number decreases 87%, characterizing Pat3 (SEQ. ID NO. 12) as a degradable PA. Fluorescence microscopy measurements of 1 µL Pat3 (1 mg/mL) stained with 9 µL, 50 µM ThT with a filter setting λₑₘ = 527/30 nm and λₑₓ = 480/40 nm showing fluorescent, µm-sized aggregates for Pat3 (SEQ. ID NO. 12) (not shown). Laser scanning microscopy images (not shown) of Proteostat stained PAs and HeLa cells revealed formation of µm-sized PA clusters for Pat3 (SEQ. ID NO. 12). Pat3 (SEQ. ID NO. 12) (2 µg) was added to cells (20.000 cells per well) for 30 min, washed and samples were analyzed (d0). Pat3 (SEQ. ID NO. 12) revealed association with cellular membranes. After 3 days of incubation (d3) less aggregates are observed, which reveals the degradability of Pat3 (SEQ. ID NO. 12).

### C₁₆-disulfid-SVVVAAAKKK-NH₂ (SEQ. ID NO. 11) (Pat2)

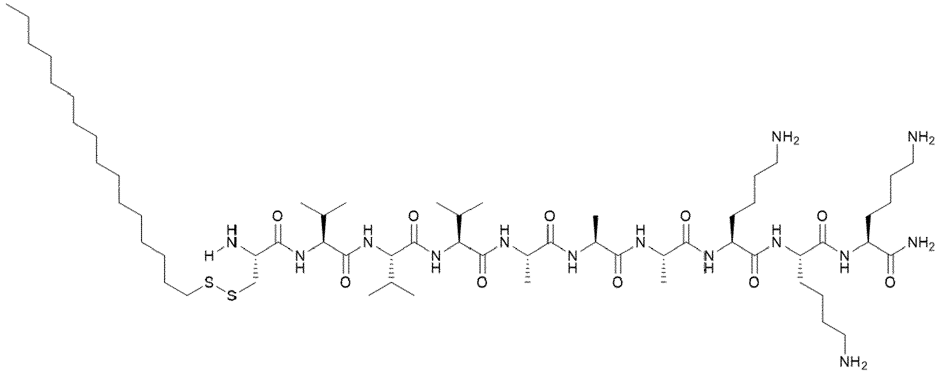

Infectivity enhancement and degradability of the PA C₁₆-*disulfide*-CVVVAAAKKK-NH₂ (Pat2) was investigated. The data show that Pat2 (SEQ. ID NO. 11) forms β-sheet rich fibrils (57% β-sheet) (not shown) that can form very small µm-sized aggregates that can interact with HeLa cells, thus are weak enhancers of HIV-1 infection. Infectivity assay showing HIV-1 infection rates of TZM-bl cells observed in the presence of increasing concentrations of peptides PA1 (SEQ ID NO. 4) and Pat2 (SEQ. ID NO. 11) at 6.5 µM, 1.3 µM, 0.26 µM (Figure 38A). The baseline (0 µM) shows infection rate without the addition of peptides. Pat2 (SEQ. ID NO. 11) enhances infectivity at 1.3 µM concentration 4 times more efficient compared to virus only infection. The β-sheet amount of Pat2 (SEQ. ID NO. 11) (1 mg/mL in PBS lyophilized) was determined by calculating the integral of the peak at 1630 cm⁻¹ (β-sheet) relative to the integral between 1650-1700 cm⁻¹ (native structure) from the 2^{nd} derivative of the FT-IR spectra (not shown) and is determined to be 57%. **C** Fluorescence microscopy measurements of 1 µL Pat2 (SEQ. ID NO. 11) (1 mg/mL) stained with 9 µL, 50 µM ThT with a filter setting λₑₘ = 527/30 nm and λₑₓ = 480/40 nm showing fluorescent, µm-sized aggregates for Pat2 (not shown). Representative TEM micrographs of Pat2 (Figure 38B) reveal the fibrillar morphologies of PA assembly (1 mg/mL in PBS). Representative laser scanning microscopy images of Proteostat stained PAs and HeLa cells reveal no formation of µm-sized PA clusters for Pat2 (SEQ. ID NO. 11) (not shown). Pat2 (SEQ. ID NO. 11) (2 µg) was added to cells (20.000 cells per well) for 30 min, washed and samples were analyzed (d0). Pat2 revealed no visible association with cellular membranes.

### Example 3 - Hydrophobic moiety exchange

To cover a wide variety of different PA designs and study its influence on infectivity enhancement, aggregate formation and degradation, a PA library inspired by PA1 and PA2 consisting distinct peptide amphiphiles by varying the hydrophobic moiety (Figures 14, 39). The peptides were tested for cell-viability and infectivity enhancement by a HIV-1 infection assay on TZM-bl cells (see methods). The peptides were characterized for fibril formation, β-sheet content, µm-sized aggregation, surface charge and amyloid character via TEM, FT-IR, widefield and confocal microscopy, zeta-potential measurements, and ThT-assay, respectively (see methods). The number and degradability of µm-sized aggregates were evaluated via confocal microscopy in presence of Hela cells after 30 min and 3 d of incubation at 37°C (see methods).

The following hydrophobic moieties have been tested: the two aromatic hydrophobic moieties fluorenylmethyloxycarbonyl (Fmoc) or 2-naphthylmethyl (NAP), the saturated fatty acids C₉ (C₈H₁₇COOH), and C₁₂ (lauric acid; C₁₁H₂₃COOH), the polyunsaturated fatty acids α-linolenic acid (ALA) and eicosapentaenoic acid (EPA).

First it was tested if the peptide amphiphiles with different hydrophobic moieties are also able to form fibrils, aggregate into µm-sized particles and show a positive zeta-potential. A peptide amphiphile is forming fibrils if fibril morphology is observed in TEM. An infectivity enhancing PA preferably shows no other morphologies such as amorphous aggregates. A peptide amphiphile is forming β-sheet secondary structure if a peak around 1625-1635 cm⁻¹ in FT-IR spectroscopy is observed. An active peptide amphiphile must show β-sheet secondary structure, preferably with a high amount ( >40%) of β-sheets. A peptide amphiphile is forming µm-sized aggregates if visible particles (> 10 µm²) were observed in widefield microscopy (brightfield or ThT-staining filter, objective 10 x) or confocal microscopy (stained with proteostat, objective 20 x) at concentrations tested for viral infectivity measurements (0.26 µM, 1.3 µM, 6.5 µM). An infectivity enhancing PA particularly preferably forms high-aspect ratio nanostructures and particularly preferably forms µm-sized aggregates. Summary of results and of the physicochemical properties are shown in tables 8 and 9.

**Table 8 Summary of results of hydrophobic moiety exchange with peptide moiety P1 (SEQ ID NO. 1). Summary of the infectivity enhancement N fold at 1.3 µM, assembly of PA and assembly of corresponding peptide without hydrophobic moiety, and degradability (ND = not detected).**

| **code** | **Sequence** | **SEQ ID NO.** | **Infectivity enhancing N fold at 1.3 µM** | **Assembly** | **Assembly without hydrophob moiety** | **Degradability / % (rel. to initial particles > 10 µm²)** |
|---|---|---|---|---|---|---|
| PA1 | C16-VVVAAAKKK-NH₂ | 4 | 10±3 | Yes | No | 94% |
| Pat10 | eicosapentaene acid Amid VVVAAAKKK-NH₂ | 14 | 12±1 | Yes | No | 99% |
| Pat7 | C12 Amid VVVAAAKKK-NH₂ | 15 | 6±1 | Yes | No | -32% |
| Pat8 | C9 Amid VVVAAAKKK-NH₂ | 16 | 2±0 | Yes | No | 78% |
| Pat12 | Fmoc Amid VVVAAAKKK-NH2 | 17 | 5±1 | Yes | No | 28% |
| Pat13 | Nap Amid VVVAAAKKK-NH₂ | 18 | 1±1 | Yes | No | ND |
| Pat9 | α-linolenic acid-amide VVVAAAKKK-NH₂ | 19 | 1±0 | No | No | ND |

**Table 9: Summary of the physicochemical properties hydrophobic moiety exchange with peptide moiety P1 (SEQ ID NO. 1): zeta-potential, derived count rate of scattered light during zeta-potential measurements, N-fold ThT fluorescence relative to PBS control and fibril formation (via TEM, "1" means fibril formation observed, "0" means no fibrils observed). (Reference = QCKIKQIINMWQ (SEQ ID NO 9)**

| **Peptide amphiphile** | **Reference** (SEQ. ID NO. 9) | **PA1** (SEQ. ID NO. 4) | **Pat10** (SEQ. ID NO. 14) | **Pat7** (SEQ. ID NO. 15) | **Pat12** (SEQ. ID NO. 17) | **Pat13** (SEQ. ID NO. 17) | **Pat8** (SEQ. ID NO. 16) |
|---|---|---|---|---|---|---|---|
| Zeta-Potential / mV | 6.6 | 22.2 | 16.9 | 19.4 | 15.2 | 1.2 | 13.5 |
| Count Rate / kcps | 2602.3 | 3138.4 | 6069.8 | 3558.1 | 509.5 | 9.3 | 77.2 |
| N-fold ThT-fluorescence | 16.2 | 278.7 | 61.9 | 185.7 | 269.9 | 1.3 | 37.1 |
| Fibril Formation | 1 | 1 | 1 | 1 | 1 | 1 | 1 |

The peptide amphiphiles with naphthalene (Nap) or α-linolenic acid (ALA) as hydrophobic moiety show almost neutral zeta-potential and the peptide amphiphile with α-linolenic acid (α-LA) failed to form fibrils in aqueous medium. The peptide amphiphile with naphthalene (Nap) or perlargonic acid (C₉, C₈H₁₇COOH) as hydrophobic moiety further failed to form µm-sized aggregates in aqueous medium. These three peptide amphiphiles showed a reduced activity in viral transduction. Thus, although the peptide amphiphile with perlargonic acid (C₉, C₈H₁₇COOH) as hydrophobic moiety forms fibrils it is not forming µm-sized aggregates in aqueous medium, which if an important factor for efficient virus-cell interaction. Thus, a minimum alkyl chain length of lauric acid (C₁₂) is therefore necessary to observe infectivity enhancement as the peptide amphiphile with lauric acid (C₁₂) as hydrophobic moiety showed infectivity enhancement. Also the peptide amphiphiles with fluorenylmethyloxycarbonyl (Fmoc), and eicosapentaenoic acid (EPA) showed infectivity enhancement.

It was surprising that if the length and amount of double bonds ("unsaturatedness") of the fatty acid residue is increased (α-linolenic acid (ALA) → Eicosapentaenoic acid (EPA)) infectivity enhancement is increased strongly. The peptide amphiphile with eicosapentaenoic acid (EPA) as hydrophobic moiety showed even stronger infectivity enhancement than the reference compounds EF-C or PA1 (SEQ ID NO. 4). This peptide amphiphile with eicosapentaenoic acid (EPA) as hydrophobic moiety is forming spherical µm-sized aggregates with composed of β-sheet fibrillar structures, resembling of phase separated peptides, and show a high number of equally distributed small aggregates. The peptide amphiphile with Fmoc as hydrophobic moiety is forming strongly aggregated fibrils, which cluster to very large µm-sized aggregates, which might be challenging to degrade by cells.

Exchange of the hydrophobic moiety results in almost all of the investigated cases to a reduced activity and degradability. Thus, the peptide amphiphile with eicosapentaenoic acid (EPA) as hydrophobic moiety is performing extraordinary good in terms of activity (outperforming EF-C and PA1) and also has a surprising good degradability (99%). The peptide amphiphile with eicosapentaenoic acid (EPA) forms very small (average size of 6 µm²) and equally distributed aggregates (Figure 14). In that means, it is in the sweet spot of forming aggregates, which is required to transport viral particles to cells but not too large aggregates to avoid degradability problems.

The peptide amphiphile with lauric acid (C₁₂) results in large aggregate formation, which even increases the number of aggregates after 3 days, because the aggregates cannot be degraded fully - most likely due to their initial size. The peptide amphiphile with Fmoc as hydrophobic moiety also form very large initial aggregates which degradable, but not efficiently as the reference peptide amphiphile PA1. The peptide amphiphiles with perlargonic acid (C₉, C₈H₁₇COOH) as hydrophobic moiety is reduced in number of aggregates, but increases in size after 3 days, which indicates that beside degradation also aggregation into larger clusters takes place (increase in size, decrease in number).

### EPA-VVVAAAKKK-NH₂ (SEQ. ID NO. 14) (Pat10)

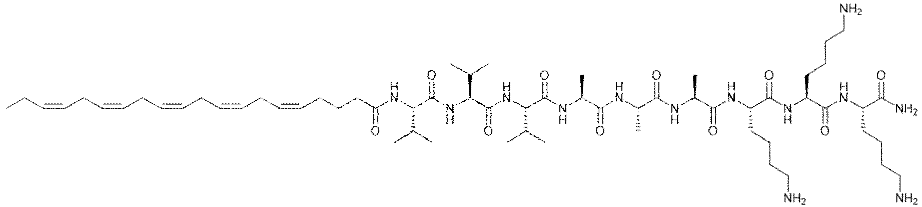

Infectivity enhancement and degradability of the PA eicosapentaenoic acid-VVVAAAKKK-NH₂ (SEQ. ID NO. 14) (Pat10) was investigated (Figure 14). The data show that Pat10 (SEQ. ID NO. 14) forms β-sheet rich fibrils (51% β-sheet) that form µm-sized aggregates that can interact with HeLa cells, thus are potent enhancers of HIV-1 infection. They can be degraded in presence of HeLa cells within 3 days (99 %). Infectivity assay showing HIV-1 infection rates of TZM-bl cells observed in the presence of increasing concentrations of peptides PA1 (SEQ ID NO. 4) and Pat10 (SEQ. ID NO. 14) (6.5 µM, 1.3 µM, 0.26 µM). Pat10 (SEQ. ID NO. 14) enhances infectivity at 1.3 µM concentration 12 times more efficient compared to virus only infection. The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 51%. Fluorescence microscopy measurements of 1 µL Pat1 (1 mg/mL) stained with 9 µL, 50 µM ThT with a filter setting λₑₘ = 527/30 nm and λₑₓ = 480/40 nm show fluorescent, µm-sized aggregates for Pat10 (SEQ. ID NO. 14). TEM micrographs of Pat10 (SEQ. ID NO. 14) reevaled morphology of PA assembly to be condensed spherical structures composed of fibrils (1 mg/mL in PBS). Laser scanning microscopy images of Proteostat stained PAs and HeLa cells revealed formation of µm-sized PA clusters for Pat10 (SEQ. ID NO. 14). Pat10 (SEQ. ID NO. 14) (2 µg) was added to cells (20.000 cells per well) for 30 min, washed and samples were analyzed (d0). Pat10 (SEQ. ID NO. 14) revealed association with cellular membranes. After 3 days of incubation (d3) less aggregates are observed, which reveals the degradability of Pat10(SEQ. ID NO. 14). Quantification of degradability by analyzing Proteostat stained aggregates via the 3D objects counter function of ImageJ 1.53f51 for 3 individual measurements (area 1008 µm × 1008 µm). Aggregate size decreases from 21 to 17 µm² and aggregate number decreases 99%, characterizing Pat10 (SEQ. ID NO. 14) as a degradable PA.

### C₁₂-VVVAAAKKK-NH₂ (SEQ. ID NO. 15) (Pat7)

Infectivity assay showing HIV-1 infection rates of TZM-bl cells observed in the presence of increasing concentrations of peptides PA1 (left data, SEQ ID NO. 4) and Pat7 (SEQ. ID NO. 15) (6.5 µM, 1.3 µM, 0.26 µM) (Figure 39). The data showed that Pat7 (SEQ. ID NO. 15) forms β-sheet rich fibrils (49% β-sheet) that form µm-sized aggregates that can interact with HeLa cells, thus are potent enhancers of HIV-1 infection. They cannot be degraded in presence of HeLa cells within 3 days. Pat7 (SEQ. ID NO. 15) enhances infectivity at 1.3 µM concentration 6 times more efficient compared to virus only infection. The β-sheet amount was determined to be 49%. Fluorescence microscopy measurements showed fluorescent, µm-sized aggregates for Pat7(SEQ. ID NO. 15). TEM micrographs of Pat7 (SEQ. ID NO. 15) revealed the fibrillar morphologies of PA assembly (1 mg/mL in PBS). Laser scanning microscopy images of Proteostat stained PAs and HeLa cells revealed formation of µm-sized PA clusters for Pat7 (SEQ. ID NO. 15). Pat7 (SEQ. ID NO. 15) revealed association with cellular membranes. After 3 days of incubation (d3) size and number of aggregates are not changed, which that Pat7 (SEQ. ID NO. 15) is not degradable. Aggregate size increases from 45 to 63 µm² and aggregate number decreases 32%, characterizing Pat7 as a non-degradable PA that can enhance infectivity.

### C₉-VVVAAAKKK-NH₂ (SEQ. ID NO. 16) (Pat8)

Infectivity enhancement and degradability of the PA C₉-VVVAAAKKK-NH₂ (SEQ. ID NO. 16) (Pat8) was investigated (Figure 39). The data show that Pat8 (SEQ. ID NO. 16) forms β-sheet rich fibrils (49% β-sheet) that form µm-sized aggregates that can interact with HeLa cells, but are not enhancers of HIV-1 infection. They cannot be degraded in presence of HeLa cells within 3 days. Pat8 (SEQ. ID NO. 16) does not enhance infectivity at 1.3 µM concentration more efficient compared to virus only infection. The β-sheet amount was determined to be 49%. Fluorescence microscopy measurements of 1 µL Pat8 (SEQ. ID NO. 16) showed fluorescent, µm-sized aggregates for Pat8 (SEQ. ID NO. 16). TEM micrographs of Pat8 (SEQ. ID NO. 16) revealed t fibrillar morphologies of PA assembly (1 mg/mL in PBS). Laser scanning microscopy images of Proteostat stained PAs and HeLa cells revealed formation of µm-sized PA clusters for Pat8 (SEQ. ID NO. 16). Pat8 (SEQ. ID NO. 16) revealed association with cellular membranes. After 3 days of incubation (d3) size and number of aggregates are not changed, which that Pat8 (SEQ. ID NO. 16) is not degradable, scale bar 100 µm. Aggregate size increases from 32 to 50 µm² and aggregate number decreases 78%, characterizing Pat8 as a non-degradable PA that cannot enhance infectivity.

### Fmoc-VVVAAAKKK-NH₂(SEQ. ID NO. 17) (Pat12)

Infectivity enhancement and degradability of the PA Fmoc-VVVAAAKKK-NH₂ (SEQ. ID NO. 17) (Pat12) was investigated (Figure 39). The data show that Pat12 (SEQ. ID NO. 16) forms β-sheet rich fibrils (52% β-sheet) that form µm-sized aggregates that can interact with HeLa cells, thus are potent enhancers of HIV-1 infection. They cannot be degraded in presence of HeLa cells within 3 days. Pat12 (SEQ. ID NO. 16) enhances infectivity at 1.3 µM concentration 5 times more efficient compared to virus only infection. The β-sheet amount was determined to be 52%. Fluorescence microscopy measurements shoed fluorescent, µm-sized aggregates for Pat12 (SEQ. ID NO. 16). TEM micrographs of Pat12 (SEQ. ID NO. 16) revealed the fibrillar morphologies of PA assembly. Laser scanning microscopy images of Proteostat stained PAs and HeLa cells revealed formation of µm-sized PA clusters for Pat12(SEQ. ID NO. 16). Pat12 (SEQ. ID NO. 16) revealed association with cellular membranes. After 3 days of incubation (d3) aggregates are observed, which reveals that Pat12 (SEQ. ID NO. 16) is not degradable. Aggregate size decreases from 111 to 65 µm² and aggregate number decreases slightly (28%,) characterizing Pat12 (SEQ. ID NO. 16) as a non-degradable PA.

### Nap-VVVAAAKKK-NH₂ (SEQ. ID NO. 18) (Pat13).

Infectivity enhancement and degradability of the PA Nap-VVVAAAKKK-NH₂ (SEQ. ID NO. 18) (Pat13) was investigated (Figure 39). The data show that Pat13 (SEQ. ID NO. 18) forms β-sheet rich fibrils (46% β-sheet) that cannot form µm-sized aggregates that cannot interact with HeLa cells, thus are not potent enhancers of HIV-1 infection. Pat13 (SEQ. ID NO. 18) does not enhance infectivity at 1.3 µM concentration more efficient compared to virus only infection. Laser scanning microscopy images of Proteostat stained PAs and HeLa revealed no formation of µm-sized PA clusters for Pat13 (SEQ. ID NO. 18).

### Example 4 - Peptide moiety exchange

First it was tested if the peptide amphiphiles with different peptide moieties are also able to form fibrils, aggregate into µm-sized particles and show a positive zeta-potential. All of the tested peptides were biocompatible and not cytotoxic at the tested concentrations on TZMbl cells (Figure 40). Proteostat Assay on PAs and peptides is shown in Figure 41. A peptide amphiphile is forming fibrils if fibril morphology is observed in TEM. An infectivity enhancing PA preferably shows no other morphologies such as amorphous aggregates. A peptide amphiphile is forming β-sheet secondary structure if a peak around 1625-1635 cm⁻¹ in FT-IR spectroscopy is observed. An active peptide amphiphile must show β-sheet secondary structure, preferably with a high amount ( >40%) of β-sheets. A peptide amphiphile is forming µm-sized aggregates if visible particles (> 10 µm²) were observed in widefield microscopy (brightfield or ThT-staining filter, objective 10 x) or confocal microscopy (stained with proteostat, objective 20 x) at concentrations tested for viral infectivity measurements (0.26 µM, 1.3 µM, 6.5 µM). An infectivity enhancing PA particularly preferably forms high-aspect ratio nanostructures and particularly preferably forms µm-sized aggregates. The first results are summarized in table 10 and 11.

**Table 10: Summary of effects of peptide moiety exchange with palmitic acid as hydrophobic moiety.**

| **PA** | **Seq. ID No.** | **Assembly** | **Assembly withut hydrophobic moiety** | **Zeta-Potential / mV** |
|---|---|---|---|---|
| C₁₆-VVVAAAKKK-NH₂ | 4 | Yes | No | 22.2 |
| C₁₆-IIQKIK-NH₂ | 26 | Yes | No | 12.6 |
| C₁₆-AGGRVK-NH₂ | 29 | Yes | No | 15.9 |
| C₁₆-LNGGVKK-NH₂ | 20 | Yes | No | 19.4 |
| C₁₆-ALAAGKK-NH₂ | 22 | Yes | No | 20.2 |
| C₁₆-LLLKK-NH₂ | 30 | Yes | No | 20.9 |
| C₁₆-AKAVGK-NH₂ | 24 | Yes | No | 19.7 |

**Table 11 Summary of infectivity enhancement and degradability of peptide moiety exchange with palmitic acid as hydrophobic moiety.**

| **Code** | **Sequence** | **Seq. ID No.** | **Infectivity enhancing N fold at 1.3 µM** | **Degradability / % (rel. to initial particles > 10 µm²)** |
|---|---|---|---|---|
| PA1 | C₁₆-VVVAAAKKK-NH₂ | 4 | 10±3 | 94% |
| Pat15 | C₁₆-IIQKIK-NH₂ | 26 | 4±1 | -28% |
| Pat18 | C₁₆-AGGRVK-NH₂ | 29 | 3±1 | ND |
| Pat19 | C₁₆-LNGGVKK-NH₂ | 20 | 6±1 | 48% |
| Pat20 | C₁₆-ALAAGKK-NH₂ | 22 | 5±1 | 87% |
| Pat23 | C₁₆-LLLKK-NH₂ | 30 | 3±0 | 27% |
| Pat31 | C₁₆-AKAVGK-NH₂ | 24 | 6±3 | 89% |

Attaching palmitic acid (C₁₆-Amid) to N-terminus is a reliable way to create peptide amphiphiles which form fibrils. The peptide amphiphiles with different sequences of table X and X how infectivity enhancement (n fold > 2 at 1.3 µM) with comparable or less activity compared to PA1 (C₁₆-VVVAAAKKK-NH₂ (SEQ. ID NO. 4)). Attaching palmitic acid to net positive charged peptide sequences yields positive zeta-potential likely because the sequences form fibrils with C-terminal positive charged residues facing outwards from the fiber. The best performing candidates (Figure 8) in terms of infectivity and degradability is Pat31 (infect n fold = 6, degradability 89%), then Pat19 (infect n fold = 6, degradability 48%) and finally Pat 15 (infect n fold = 4, degradability 28%).

### C₁₆- LNGGVKK-NH₂(SEQ. ID NO. 20) (Pat19)

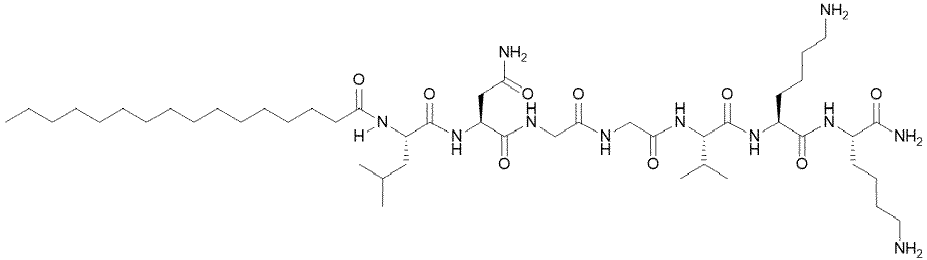

Infectivity enhancement and degradability of the PA C₁₆-LNGGVKK-NH₂ (SEQ. ID NO. 20) (Pat19) and LNGGVKK-NH₂ (SEQ. ID NO. 21) (Pat38). The data show that Pat19 (SEQ. ID NO. 20) forms β-sheet containing fibrils (34% β-sheet) that form µm-sized aggregates that can interact with HeLa cells, thus are potent enhancers of HIV-1 infection. They can be degraded in presence of HeLa cells within 3 days (48 %). The data show that Pat38 (SEQ. ID NO. 21) forms no β-sheet rich fibrils that cannot form µm-sized aggregates that cannot interact with HeLa cells, thus are not potent enhancers of HIV-1 infection. Infectivity assay showed HIV-1 infection rates of TZM-bl cells observed in the presence of increasing concentrations of peptides PA1 (SEQ ID NO. 4), Pat19 (SEQ. ID NO. 20) and Pat38 (SEQ. ID NO. 21) (LNGGVKK-NH₂) (6.5 µM, 1.3 µM, 0.26 µM) (Figure 15A). The data show that Pat38 (SEQ. ID NO. 21) form no β-sheet rich fibrils that cannot form µm-sized aggregates that cannot interact with HeLa cells, thus are not potent enhancers of HIV-1 infectionPat19 (SEQ. ID NO. 20) enhances infectivity at 1.3 µM concentration 6 times more efficient compared to virus only infection. ATR-IR spectrum of the amide I and II regions of Pat19 (SEQ. ID NO. 20) (1 mg/mL in PBS lyophilized) is shown in Figure 15B. The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 34%. The β-sheet amount of Pat38 (SEQ. ID NO. 21) was determined to be 0%. Fluorescence microscopy measurements of 1 µL Pat19 (SEQ. ID NO. 20) (1 mg/mL) stained with 9 µL, 50 µM ThT with a filter setting λₑₘ = 527/30 nm and λₑₓ = 480/40 nm showed fluorescent, µm-sized aggregates for Pat19 (SEQ. ID NO. 20) (not shown). In contrast, fluorescence microscopy measurements of 1 µL Pat38 (SEQ. ID NO. 21) (1 mg/mL) stained with 9 µL, 50 µM ThT with a filter setting λₑₘ = 527/30 nm and λₑₓ = 480/40 nm showed no fluorescent, µm-sized aggregates for Pat38 (SEQ. ID NO. 21) (now shown). TEM micrographs of Pat19 (SEQ. ID NO. 20) revealed the fibrillar morphologies of PA assembly (1 mg/mL in PBS) (Figure 15C). TEM micrographs of Pat38 (SEQ. ID NO. 21) revealed no fibrillar morphologies of the peptide (1 mg/mL in PBS) (Figure 15D). Laser scanning microscopy images of Proteostat stained PAs and HeLa cells revealed formation of µm-sized PA clusters for Pat19 (SEQ. ID NO. 20) (not shown). Pat19 (SEQ. ID NO. 20) (2 µg) was added to cells (20.000 cells per well) for 30 min, washed and samples were analyzed (d0). Pat19 (SEQ. ID NO. 20) revealed association with cellular membranes. After 3 days of incubation (d3) less aggregates are observed, which reveals the degradability of Pat19 (SEQ. ID NO. 20). Quantification of degradability was performed by analyzing Proteostat stained aggregates via the 3D objects counter function of ImageJ 1.53f51 for 3 individual measurements (area 1008 µm × 1008 µm). Box plot showing size distribution and bar plot showing number of aggregates > 10 µm² in an area of approximately 1008 µm × 1008 µm in cell culture after 30 min and 3 d incubation (Figure 16). Aggregate size of Pat19 (SEQ. ID NO. 20) decreases from 76 to 40 µm² and aggregate number decreases 48%, characterizing Pat19 as a degradable PA.

### C₁₆-ALAAGKK-NH₂(SEQ. ID NO. 22) (Pat20)

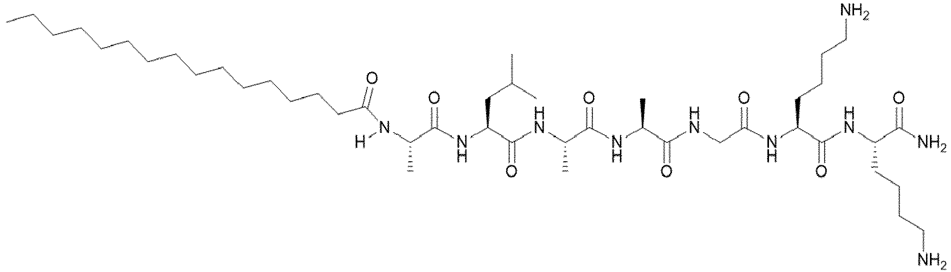

Infectivity enhancement and degradability of the PA C₁₆-ALAAGKK-NH₂ (SEQ. ID NO. 22) (Pat20) and of the peptide moiety ALAAGKK-NH₂ (SEQ. ID NO. 23) (Pat39) was investigated. The data show that Pat20 (SEQ. ID NO. 22) forms β-sheet containing fibrils (48% β-sheet) that form µm-sized aggregates that can interact with HeLa cells, thus are potent enhancers of HIV-1 infection. They can be degraded in presence of HeLa cells within 3 days (87 %). The data show that Pat39 (SEQ. ID NO. 23) form no β-sheet rich fibrils that cannot form µm-sized aggregates that cannot interact with HeLa cells, thus are not potent enhancers of HIV-1 infection. Infectivity assay (Figure17A) showed HIV-1 infection rates of TZM-bl cells observed in the presence of increasing concentrations of peptides PA1 (SEQ ID NO. 4), Pat20 (SEQ. ID NO. 22) and Pat39 (ALAAGKK-NH₂ (SEQ. ID NO. 23)) (6.5 µM, 1.3 µM, 0.26 µM). Pat20 (SEQ. ID NO. 22) enhances infectivity at 1.3 µM concentration 5 times more efficient compared to virus only infection. Pat39 (SEQ. ID NO. 23) cannot enhance infectivity at 1.3 µM concentration (same as virus only infection). ATR-IR spectrum of the amide I and II regions of Pat20 (SEQ. ID NO. 22) (1 mg/mL in PBS lyophilized) is shown in Figure 17B. The β-sheet amount of Pat20 (SEQ. ID NO. 22) was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 48%. The β-sheet amount of Pat39 (SEQ. ID NO. 23) was determined to be 0% (not shown). TEM micrographs of Pat20 (SEQ. ID NO. 22) revealed the fibrillar morphologies of PA assembly (1 mg/mL in PBS) (Figure17C). TEM micrographs of Pat39 (SEQ. ID NO. 23) revealed no fibrillar morphologies of the peptide (1 mg/mL in PBS) (Figure17D). Laser scanning microscopy images of Proteostat stained PAs and HeLa cells revealed formation of µm-sized PA clusters for Pat20(SEQ. ID NO. 22). Pat20 (SEQ. ID NO. 22) (2 µg) was added to cells (20.000 cells per well) for 30 min, washed and samples were analyzed (d0). Pat20 (SEQ. ID NO. 22) revealed association with cellular membranes. After 3 days of incubation (d3) less aggregates are observed, which reveals the degradability of Pat20(SEQ. ID NO. 22). Quantification of degradability was performed by analyzing Proteostat stained aggregates via the 3D objects counter function of ImageJ 1.53f51 for 3 individual measurements (area 1008 µm × 1008 µm). Aggregate size of Pat20 (SEQ. ID NO. 22) slightly increases from 38 to 45 µm² and aggregate number decreases 87%, characterizingPat20 (SEQ. ID NO. 22) as a degradable PA (Figure16). Fluorescence microscopy measurements of 1 µL Pat20 (SEQ. ID NO. 22) (1 mg/mL) stained with 9 µL, 50 µM ThT with a filter setting λₑₘ = 527/30 nm and λₑₓ = 480/40 nm showed fluorescent, µm-sized aggregates for Pat20(SEQ. ID NO. 22). Representative fluorescence microscopy measurements of 1 µL Pat39 (SEQ. ID NO. 23) (1 mg/mL) stained with 9 µL, 50 µM ThT with a filter setting λₑₘ = 527/30 nm and λₑₓ = 480/40 nm showed no fluorescent, µm-sized aggregates for Pat39 (SEQ. ID NO. 23).

### C₁₆-AKAVGK-NH₂(SEQ. ID NO. 24) (Pat31)

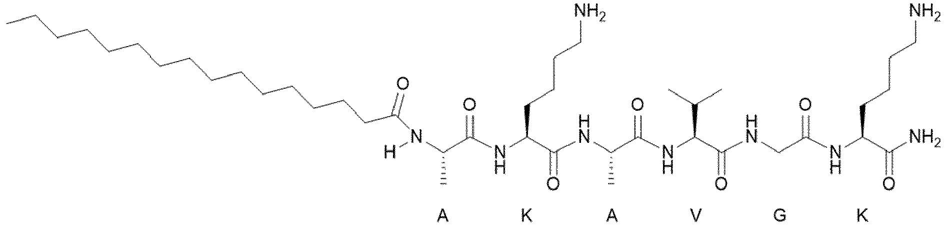

Infectivity enhancement and degradability of the PA C₁₆-AKAVGK-NH₂ (SEQ. ID NO. 24) (Pat31) and of the peptide moiety AKAVGK-NH₂ (SEQ. ID NO. 25) (Pat50) was investigated. The data show that Pat31 (SEQ. ID NO. 24) forms β-sheet containing fibrils (43% β-sheet) that form µm-sized aggregates that can interact with HeLa cells, thus are potent enhancers of HIV-1 infection. They can be degraded in presence of HeLa cells within 3 days (89 %). The data show that Pat50 (SEQ. ID NO. 25) forms β-sheet containing amorphous aggregates that cannot form µm-sized aggregates that cannot interact with HeLa cells, thus are not potent enhancers of HIV-1 infection. Infectivity assay (Figure18A) showed HIV-1 infection rates of TZM-bl cells observed in the presence of increasing concentrations of peptides PA1 (C₁₆-VVVAAAKKK-NH₂, SEQ ID NO. 4), Pat31 (SEQ. ID NO. 24) and Pat50 (AKAVGK-NH₂ (SEQ. ID NO. 25)) (6.5 µM, 1.3 µM, 0.26 µM). Pat31 (SEQ. ID NO. 24) enhances infectivity at 1.3 µM concentration 6 times more efficient compared to virus only infection. Pat50 (SEQ. ID NO. 25) cannot enhance infectivity at 1.3 µM concentration (same as virus only infection). ATR-IR spectrum of the amide I and II regions of Pat31 (SEQ. ID NO. 24) (1 mg/mL in PBS lyophilized) is shown in Figure18B. The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 43%. The β-sheet amount of Pat50 (SEQ. ID NO. 25) was determined to be 33% (not shown). TEM micrographs of Pat31 (SEQ. ID NO. 24) revealed the fibrillar morphologies of PA assembly (1 mg/mL in PBS) (Figure18C). TEM micrographs of Pat50 (SEQ. ID NO. 25) revealed no fibrillar morphologies but amorphous aggregates for the peptide (1 mg/mL in PBS) (Figure18D). Laser scanning microscopy images of Proteostat stained PAs and HeLa cells revealed formation of µm-sized PA clusters for Pat31 (SEQ. ID NO. 24). Pat31 (SEQ. ID NO. 24) (2 µg) was added to cells (20.000 cells per well) for 30 min, washed and samples were analyzed (d0). Pat31 (SEQ. ID NO. 24) revealed association with cellular membranes. After 3 days of incubation (d3) less aggregates are observed, which reveals the degradability of Pat31 (SEQ. ID NO. 24). Quantification of degradability was performed by analyzing Proteostat stained aggregates via the 3D objects counter function of ImageJ 1.53f51 for 3 individual measurements (area 1008 µm × 1008 µm). Box plot showing size distribution and bar plot showing number of aggregates > 10 µm² in an area of approximately 1008 µm × 1008 µm in cell culture after 30 min and 3 d incubation (Figure16). Aggregate size of Pat31 (SEQ. ID NO. 24) decreases from 104 to 38 µm² and aggregate number decreases 89%, characterizing Pat31 (SEQ. ID NO. 24) as a degradable PA. Fluorescence microscopy measurements of 1 µL Pat31 (SEQ. ID NO. 24) (1 mg/mL) stained with 9 µL, 50 µM ThT with a filter setting λₑₘ = 527/30 nm and λₑₓ = 480/40 nm showed fluorescent, µm-sized aggregates for Pat31 (SEQ. ID NO. 24) (not shown). Fluorescence microscopy measurements of 1 µL Pat50 (SEQ. ID NO. 25) (1 mg/mL) stained with 9 µL 50 µM ThT with a filter setting λₑₘ = 527/30 nm and λₑₓ = 480/40 nm showed no fluorescent, µm-sized aggregates for Pat50 (SEQ. ID NO. 25).

### C₁₆-IIQKIK-NH₂ (SEQ. ID NO. 26) (Pat15)

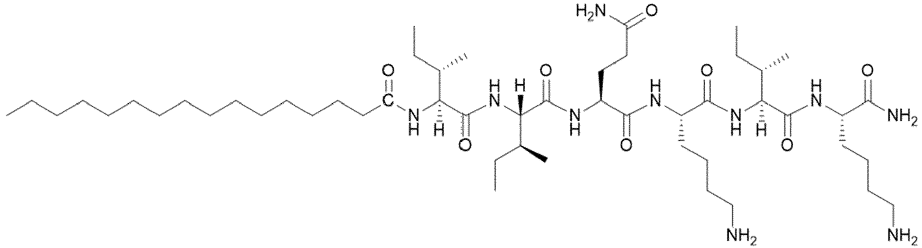

Infectivity enhancement and degradability of the PA C₁₆-IIQKIK-NH₂ (SEQ. ID NO. 26) (Pat15) and of the peptide moiety IIQKIK-NH₂ (SEQ. ID NO. 27) (Pat34) was investigated. The data show that Pat15 (SEQ. ID NO. 26) forms β-sheet containing fibrils (45% β-sheet) that form µm-sized aggregates that can interact with HeLa cells, thus are potent enhancers of HIV-1 infection. They can be degraded in presence of HeLa cells within 3 days (28 %). The data show that Pat34 (SEQ. ID NO. 27) form no β-sheet rich fibrils that cannot form µm-sized aggregates that cannot interact with HeLa cells, thus are not potent enhancers of HIV-1 infection. Infectivity assay (Figure19A) showed HIV-1 infection rates of TZM-bl cells observed in the presence of increasing concentrations of peptides PA1 (C₁₆-VVVAAAKKK-NH₂, SEQ ID NO. 4), Pat15 (SEQ. ID NO. 26) and Pat34 (IIQKIK-NH₂ (SEQ. ID NO. 27)) (6.5 µM, 1.3 µM, 0.26 µM). Pat15 (SEQ. ID NO. 26) enhances infectivity at 1.3 µM concentration 4 times more efficient compared to virus only infection. ATR-IR spectrum of the amide I and II regions of Pat15 (SEQ. ID NO. 26) (1 mg/mL in PBS lyophilized) is shown in Figure19B. The β-sheet amount of Pat15 (SEQ. ID NO. 26) was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 45%. The β-sheet amount Pat34 (SEQ. ID NO. 27) was determined to be 31%. TEM micrographs of Pat15 (SEQ. ID NO. 26) revealed the fibrillar morphologies of PA assembly (1 mg/mL in PBS) (Figure19C). TEM micrographs of Pat34 (SEQ. ID NO. 27) revealed no fibrillar morphologies of the peptide (1 mg/mL in PBS) (Figure19D). Laser scanning microscopy images of Proteostat stained PAs and HeLa cells revealed formation of µm-sized PA clusters for Pat15 (SEQ. ID NO. 26) (not shown). Pat15 (SEQ. ID NO. 26) (2 µg) was added to cells (20.000 cells per well) for 30 min, washed and samples were analyzed (d0). Pat15 (SEQ. ID NO. 26) revealed association with cellular membranes. After 3 days of incubation (d3) less aggregates are observed, which reveals the degradability of Pat15 (SEQ. ID NO. 26). Quantification of degradability was performed by analyzing Proteostat stained aggregates via the 3D objects counter function of ImageJ 1.53f51 for 3 individual measurements (area 1008 µm × 1008 µm). Box plot showing size distribution and bar plot showing number of aggregates > 10 µm² in an area of approximately 1008 µm × 1008 µm in cell culture after 30 min and 3 d incubation (Figure 16). Aggregate size of Pat15 (SEQ. ID NO. 26) decreases from 41 to 23 µm² and aggregate number decreases 28%, characterizing Pat15 (SEQ. ID NO. 26) as a degradable PA. Fluorescence microscopy measurements of 1 µL Pat15 (SEQ. ID NO. 26) (1 mg/mL) stained with 9 µL, 50 µM ThT with a filter setting λₑₘ = 527/30 nm and λₑₓ = 480/40 nm showed fluorescent, µm-sized aggregates for Pat15 (SEQ. ID NO. 26) (not shown). Fluorescence microscopy measurements of 1 µL Pat34 (SEQ. ID NO. 27) (1 mg/mL) stained with 9 µL, 50 µM ThT with a filter setting λₑₘ = 527/30 nm and λₑₓ = 480/40 nm showed no fluorescent, µm-sized aggregates for Pat34 (SEQ. ID NO. 27) (not shown).

### C₁₆-AGGRVK-NH₂ (SEQ. ID NO. 28) (Pat18)

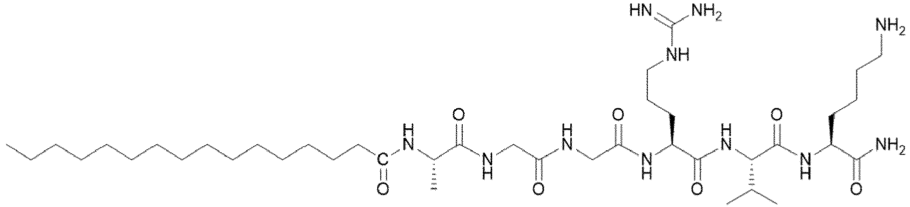

Infectivity enhancement and degradability of the PA C₁₆-AGGRVK-NH₂ (SEQ. ID NO. 28) (Pat18) and of the peptide moiety AGGRVK-NH₂ (SEQ. ID NO. 29) (Pat37) was investigated. The data show that Pat18 (SEQ. ID NO. 28) forms β-sheet containing fibrils (56% β-sheet) that do not form µm-aggregates thus they weakly interact with HeLa cells and are weak enhancers of HIV-1 infection. ). The data show that Pat37 (SEQ. ID NO. 29) form no β-sheet rich fibrils that cannot form µm-sized aggregates that cannot interact with HeLa cells, thus are not potent enhancers of HIV-1 infection. Infectivity assay (Figure20A) showed HIV-1 infection rates of TZM-bl cells observed in the presence of increasing concentrations of peptides PA1 (left data, C₁₆-VVVAAAKKK-NH₂, SEQ ID NO. 4), Pat18 (SEQ. ID NO. 28) (middle data) and Pat37 (AGGRVK-NH₂ (SEQ. ID NO. 29), right data) (6.5 µM, 1.3 µM, 0.26 µM). Pat18 (SEQ. ID NO. 28) enhances infectivity at 1.3 µM concentration 3 times more efficient compared to virus only infection. Pat37 (SEQ. ID NO. 29) cannot enhance infectivity at 1.3 µM concentration (same as virus only infection). ATR-IR spectrum of the amide I and II regions of Pat18 (SEQ. ID NO. 28) (1 mg/mL in PBS lyophilized) is shown in Figure20B. The β-sheet of Pat18 (SEQ. ID NO. 28) amount was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 56%. The β-sheet amount of Pat37 (SEQ. ID NO. 29) was determined to be 0% (not shown). TEM micrographs of Pat18 (SEQ. ID NO. 28) revealed the fibrillar morphologies of PA assembly (1 mg/mL in PBS) (Figure20C). TEM micrographs of Pat37 (SEQ. ID NO. 29) revealed no fibrillar morphologies of the peptide (1 mg/mL in PBS) (Figure20D). Laser scanning microscopy images of Proteostat stained PAs and HeLa cells revealed no formation of µm-sized PA clusters for Pat18 (SEQ. ID NO. 28). Pat18 (SEQ. ID NO. 28) (2 µg) was added to cells (20.000 cells per well) for 30 min, washed and samples were analyzed (d0). Fluorescence microscopy measurements of 1 µL Pat18 (1 mg/mL) stained with 9 µL, 50 µM ThT with a filter setting λₑₘ = 527/30 nm and λₑₓ = 480/40 nm showed no fluorescent, µm-sized aggregates for Pat18 (SEQ. ID NO. 28) (not shown). Fluorescence microscopy measurements of 1 µL Pat37 (SEQ. ID NO. 29) (1 mg/mL) stained with 9 µL, 50 µM ThT with a filter setting λₑₘ = 527/30 nm and λₑₓ = 480/40 nm showed no fluorescent, µm-sized aggregates for Pat37 (SEQ. ID NO. 29) (not shown).

### C₁₆-LLLKK-NH₂ (SEQ. ID NO. 30) (Pat23)

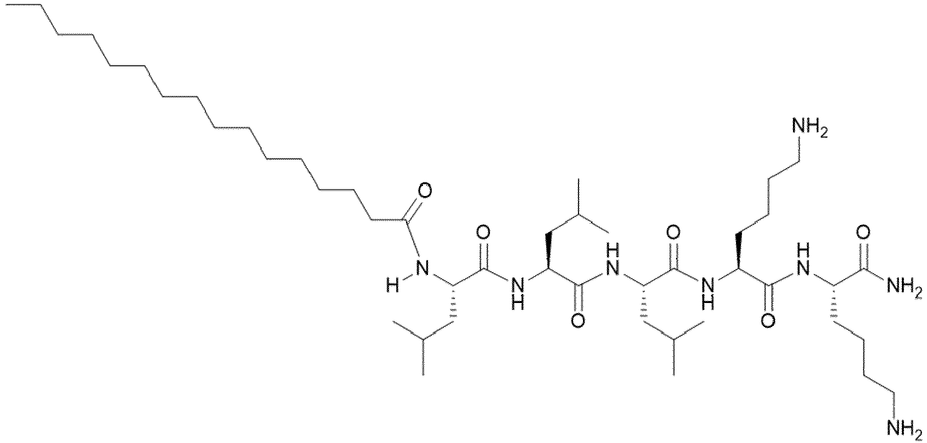

Infectivity enhancement and degradability of the PA C₁₆-LLLKK-NH₂ (Pat23) (SEQ. ID NO. 30) and of the peptide moiety LLLKK-NH₂ (Pat42) (SEQ. ID NO. 31) was investigated. The data show that Pat23 (SEQ. ID NO. 30) forms β-sheet containing fibrils (46% β-sheet) that form µm-sized aggregates that can interact with HeLa cells, thus are potent enhancers of HIV-1 infection. They can be degraded in presence of HeLa cells within 3 days (27 %). The data show that Pat42 (SEQ. ID NO. 31) form no β-sheet rich fibrils that cannot form µm-sized aggregates that cannot interact with HeLa cells, thus are not potent enhancers of HIV-1 infection. Infectivity assay (Figure21A) showed HIV-1 infection rates of TZM-bl cells observed in the presence of increasing concentrations of peptides PA1 (C₁₆-VVVAAAKKK-NH₂, SEQ ID NO. 4), Pat23 (SEQ. ID NO. 30) and Pat42 (LLLKK-NH₂ (SEQ. ID NO. 31)) (6.5 µM, 1.3 µM, 0.26 µM). Pat23 (SEQ. ID NO. 30) enhances infectivity at 1.3 µM concentration 3 times more efficient compared to virus only infection. Pat42 (SEQ. ID NO. 31) cannot enhance infectivity at 1.3 µM concentration (same as virus only infection). ATR-IR spectrum of the amide I and II regions of Pat23 (SEQ. ID NO. 30) (1 mg/mL in PBS lyophilized) is shown in Figure20B. The β-sheet amount of Pat23 (SEQ. ID NO. 30) was determined by calculating the integral of the peak at 1630 cm⁻¹ (β-sheet) relative to the integral between 1650-1700 cm⁻¹ (native structure) from the 2^{nd} derivative of the FT-IR spectra and is determined to be 46%. The β-sheet amount of Pat42 (SEQ. ID NO. 31) was determined to be 33% (not shown). TEM micrographs of Pat23 (SEQ. ID NO. 30) revealed the fibrillar morphologies of PA assembly (1 mg/mL in PBS) (Figure21C). TEM micrographs of Pat42 (SEQ. ID NO. 31) revealed no fibrillar morphologies of the peptide (1 mg/mL in PBS) (Figure21D). Laser scanning microscopy images of Proteostat stained PAs and HeLa cells revealed formation of µm-sized PA clusters for Pat23 (SEQ. ID NO. 30) (not shown). Pat23 (SEQ. ID NO. 30) (2 µg) was added to cells (20.000 cells per well) for 30 min, washed and samples were analyzed (d0). Pat23 (SEQ. ID NO. 30) revealed association with cellular membranes. After 3 days of incubation (d3) less aggregates are observed, which reveals the degradability of Pat23 (SEQ. ID NO. 30). Fluorescence microscopy measurements of 1 µL Pat23 (SEQ. ID NO. 30) (1 mg/mL) stained with 9 µL, 50 µM ThT with a filter setting λₑₘ = 527/30 nm and λₑₓ = 480/40 nm showing fluorescent, µm-sized aggregates for Pat23 (SEQ. ID NO. 30) (not shown). Fluorescence microscopy measurements of 1 µL Pat42 (SEQ. ID NO. 31) (1 mg/mL) stained with 9 µL, 50 µM ThT with a filter setting λₑₘ = 527/30 nm and λₑₓ = 480/40 nm showed no fluorescent, µm-sized aggregates for Pat42 (SEQ. ID NO. 31) (not shown). Quantification of degradability was performed by analyzing Proteostat stained aggregates via the 3D objects counter function of ImageJ 1.53f51 for 3 individual measurements (area 1008 µm × 1008 µm). Box plot showing size distribution and bar plot showing number of aggregates > 10 µm² in an area of approximately 1008 µm × 1008 µm in cell culture after 30 min and 3 d incubation (Figure 16). Aggregate size of Pat23 (SEQ. ID NO. 30) decreases from 71 to 60 µm² and aggregate number decreases 27%, characterizing Pat23 (SEQ. ID NO. 30) as a moderately degradable PA.

Further peptide sequences have been tested and the results are summarized in table 12.

**Table 12 Summary of resultsof further peptide moiety exchange with palmitic acid as hydrophobic moiety. Summary of the infectivity enhancement N fold at 1.3 µM, assembly of PA and assembly of corresponding peptide without hydrophobic moiety, and degradability ND = not detected.**

| Code | Sequence | SEQ ID NO. | Infectivity enhancing N fold at 1.3 µM | Asse mbly | Assembly without hydrophob residue | Degradability / % (rel. to initial particles > 10 µm²) |
|---|---|---|---|---|---|---|
| Pat14 | C₁₆-vvvaaakkk-NH₂ | 32 | 7±1 | Yes | No | -116% |
| Pat16 | C₁₆-AQAKAK-NH₂ | 34 | 4±1 | Yes | No | -83% |
| Pat17 | C₁₆-WEALKK-NH₂ | 36 | 1±0 | No | No | ND |
| Pat21 | C₁₆-AVAKKK-NH₂ | 38 | 2±0 | Yes | No | 68% |
| Pat22 | C₁₆-RRWQWR-NH₂ | 40 | 0±0 | Yes | No | ND |
| Pat24 | C₁₆-VVAAHHH-NH₂ | 42 | 2±0 | Yes | No | 26% |
| Pat25 | C₁₆-KGVPGVGK-NH₂ | 44 | 1±0 | No | Yes | ND |
| Pat26 | C16-KYIHQNYTKALAG KLV-NH₂ | 46 | 5±2 | Yes | No | -110% |
| Pat27 | C16-NFYLQVNKINK-NH₂ | 48 | 5±1 | Yes | Yes | -123% |
| Pat28 | C₁₆-GGGSSKKK-NH₂ | 50 | 2±1 | Yes | No | ND |
| Pat29 | C16-RYYASLRHYLNLV TRQRY-NH₂ | 52 | 5±1 | Yes | Yes | 43% |
| Pat30 | C₁₆-GKVGSK-NH₂ | 54 | 2±1 | Yes | No | ND |
| Pat32 | C₁₆-KIISFK-NH₂ | 56 | 2±1 | Yes | No | -84% |
| Pat52* | C₁₆-AAVVHHK-NH₂ | 58 | 6±3 | Yes | No | -123% |
| Pati* | C₁₆-GGVVRR-NH₂ | 60 | 1±1 | Yes | No | ND |
| Patj* | C₁₆-AGAGRRR-NH₂ | 62 | 1±0 | Yes | No | ND |

The only peptide sequence which are not forming fibrils after functionalization with palmitic acid are C₁₆-KGVPGVGK (Pat25) (SEQ. ID NO. 44) and C₁₆-WEALKK (Pat17) (SEQ. ID NO. 36). These both peptides are also not preferred because they include a negatively charged amino acid E or a proline P. Moreover, the peptides NFYLQVNKINK (SEQ. ID NO. 49), RYYASLRHYLNLVTRQRY (SEQ. ID NO. 53) and KGVPGVGK (SEQ. ID NO. 45) are unexpectedly forming fibrils by themselves without palmitic acid and the respective palmitic acid modified PAs are not degradable. Strikingly, all of the PAs (except C₁₆-KGVPGVGK (Pat25) (SEQ. ID NO. 44)) showed a positive Zeta-potential, while their corresponding peptide-only sequences showed negative or almost neutral (<5mV) zeta-potential. The peptides which are not enhancing infectivity are either not forming µm-sized aggregates or too large aggregates. If fibrils do not form µm-sized aggregation or too few µm-sized aggregates, no infectivity enhancement can be observed. Exemplary sequences are: Pat17: C₁₆-WEALKK-NH₂ (SEQ. ID NO. 36); Pat18: C₁₆-AGGRVK-NH₂ (SEQ. ID NO. 28), Pat21: C₁₆-AVAKKK-NH₂ (SEQ. ID NO. 38), Pat22: C₁₆-RRWQWR-NH₂ (SEQ. ID NO. 40), Pat25: C₁₆-KGVPGVGK-NH₂ (SEQ. ID NO. 44), Pat28: C₁₆-GGGSSKKK-NH₂ (SEQ. ID NO. 50), Pati*: C₁₆-GGVVRR-NH₂ (SEQ. ID NO. 60), and Patj*. C₁₆-AGAGRRR-NH₂ (SEQ. ID NO. 62). In contrast very large aggregates also reduce infectivity enhancement. For example, the sequences Pat16: C₁₆-AQAKAK-NH₂ (SEQ. ID NO. 34), Pat30: C₁₆-GKVGSK-NH₂ (SEQ. ID NO. 54), and Pat24: C₁₆-VVAAHHH-NH₂ (SEQ. ID NO. 42), form very large aggregates and do not efficiently enhance infectivity. One consideration might be that residues with high flexibility close to the hydrophobic residue (G, A) can increase dynamicity of PAs and at the same time a high number of charged residues at the C-terminus increase solvability and result in less aggregating fibers - as shown for ), Pat28: C₁₆-GGGSSKKK-NH₂ (SEQ. ID NO. 50), Patj*. C₁₆-AGAGRRR-NH₂ (SEQ. ID NO. 62), Pati*: C₁₆-GGVVRR-NH₂ (SEQ. ID NO. 60), which all show too few aggregates. Another consideration might be that charged residues, bulky hydrophobic residues, or kinked residues (proline) close to hydrophobic residue hinder efficient assembly, therefore increase dynamicity of the fibers and therefore less aggregation occurs, as shown for: Pat25: C₁₆-KGVPGVGK-NH₂ (SEQ. ID NO. 44), Pat22: C₁₆-RRWQWR-NH₂ (SEQ. ID NO. 40), Pat17: C₁₆-WEALKK-NH₂ (SEQ. ID NO. 36). Pat14 (SEQ. ID NO. 32) is analogous to PA1 (SEQ. ID NO. 4) but contains all d-amino acids, which cannot be degraded from cells. As expected Pat14 (SEQ. ID NO. 32) is not degraded and forms much larger aggregates (71 µm² for Pat14 (SEQ. ID NO. 32) instead of 36 µm² for PA1) which are further decreasing in size (71 µm² at d0 → 30 µm² at d3) but not in number (increase in 116%), which indicates that aggregates are not degraded but rather breaking up into smaller parts.

Infectivity enhancement and degradability of the PA C₁₆-vvvaaakkk-NH₂ (SEQ. ID NO. 32) (Pat14) and of the peptide moiety vvvaaakkk-NH₂ (SEQ. ID NO. 33) (Pat33) was investigated. The data show that Pat14 (SEQ. ID NO. 32) forms β-sheet containing fibrils (49% β-sheet) that form µm-sized aggregates that can interact with HeLa cells, thus are potent enhancers of HIV-1 infection. However, they cannot be degraded in presence of HeLa cells within 3 days, which can be traced back to the non-degradable peptide sequences composed of D-amino acids. The data show that Pat33 (SEQ. ID NO. 33) forms no β-sheet rich fibrils that cannot form µm-sized aggregates that cannot interact with HeLa cells, thus are not potent enhancers of HIV-1 infection. Infectivity assay (Figure22A) showed HIV-1 infection rates of TZM-bl cells observed in the presence of increasing concentrations of peptides PA1 (C₁₆-VVVAAAKKK-NH₂, SEQ ID NO. 4), Pat14 (SEQ. ID NO. 32) and Pat33 (SEQ. ID NO. 33) (6.5 µM, 1.3 µM, 0.26 µM). Pat14 (SEQ. ID NO. 32) enhances infectivity at 1.3 µM concentration 7 times more efficient compared to virus only infection. Pat33 (SEQ. ID NO. 33) cannot enhance infectivity at 1.3 µM concentration (same as virus only infection).

Infectivity enhancement and degradability of the PA C₁₆-AQAKAK-NH₂ (Pat16) (SEQ. ID NO. 34) of the peptide moiety AQAKAK-NH₂ (SEQ. ID NO. 35) (Pat35) was investigated. The data show that Pat16 (SEQ. ID NO. 34) forms β-sheet containing fibrils (50% β-sheet) that form µm-sized aggregates that can interact with HeLa cells, thus are potent enhancers of HIV-1 infection. They cannot be degraded in presence of HeLa cells within 3 days. The data show that Pat35 (SEQ. ID NO. 35) forms no β-sheet rich fibrils that cannot form µm-sized aggregates that cannot interact with HeLa cells, thus are not potent enhancers of HIV-1 infection. Infectivity assay (Figure23A) showed HIV-1 infection rates of TZM-bl cells observed in the presence of increasing concentrations of peptides PA1 (C₁₆-VVVAAAKKK-NH₂, SEQ ID NO. 4), Pat16 (SEQ. ID NO. 34) and Pat35 (SEQ. ID NO. 35) (6.5 µM, 1.3 µM, 0.26 µM). Pat16 (SEQ. ID NO. 34) enhances infectivity at 1.3 µM concentration 4 times more efficient compared to virus only infection. Pat35 (SEQ. ID NO. 35) cannot enhance infectivity at 1.3 µM concentration (same as virus only infection).

Infectivity enhancement and degradability of the PA C₁₆-WEALKK-NH₂ (SEQ. ID NO. 36) (Pat17) and of the peptide moiety WEALKK-NH₂ (SEQ. ID NO. 37) (Pat36) was investigated. The data show that Pat17 (SEQ. ID NO. 36) forms no β-sheet containing fibrils that cannot form µm-sized aggregates and cannot interact with HeLa cells, thus are not potent enhancers of HIV-1 infection. The data show that Pat36 (SEQ. ID NO. 37) forms no β-sheet rich fibrils that cannot form µm-sized aggregates that cannot interact with HeLa cells, thus are not potent enhancers of HIV-1 infection. Infectivity assay (Figure24A) showed HIV-1 infection rates of TZM-bl cells observed in the presence of increasing concentrations of peptides PA1 (C₁₆-VVVAAAKKK-NH₂, SEQ ID NO. 4), Pat17 (SEQ. ID NO. 36) and Pat36 (SEQ. ID NO. 37) (6.5 µM, 1.3 µM, 0.26 µM). Pat17 (SEQ. ID NO. 36) does not enhance infectivity at 1.3 µM concentration compared to virus only infection. Pat36 (SEQ. ID NO. 37) cannot enhance infectivity at 1.3 µM concentration (same as virus only infection).

Infectivity enhancement and degradability of the PA C₁₆-AVAKKK-NH₂ (SEQ. ID NO. 38) (Pat21) and of the peptide moiety AVAKKK-NH₂ (SEQ. ID NO. 39) (Pat40) was investigated. The data show that Pat21 (SEQ. ID NO. 38) forms β-sheet containing fibrils (45% β-sheet) that form µm-sized aggregates that can interact with HeLa cells, but are not potent enhancers of HIV-1 infection. They can be degraded in presence of HeLa cells within 3 days. The data show that Pat40 (SEQ. ID NO. 39) form no β-sheet rich fibrils that cannot form µm-sized aggregates that cannot interact with HeLa cells, thus are not potent enhancers of HIV-1 infection. Infectivity assay (Figure25A) showed HIV-1 infection rates of TZM-bl cells observed in the presence of increasing concentrations of peptides PA1 (C₁₆-VVVAAAKKK-NH₂, SEQ ID NO. 4), Pat21 (SEQ. ID NO. 38) and Pat40 (SEQ. ID NO. 39) (6.5 µM, 1.3 µM, 0.26 µM). Pat21 (SEQ. ID NO. 38) enhances infectivity at 1.3 µM concentration 2 times more efficient compared to virus only infection. Pat40 (SEQ. ID NO. 39) cannot enhance infectivity at 1.3 µM concentration (same as virus only infection).

Infectivity enhancement and degradability of the PA C₁₆-RRWQWR-NH₂ (SEQ. ID NO. 40) (Pat22) and of the peptide moiety RRWQWR-NH₂ (SEQ. ID NO. 41) (Pat41) was investigated. The data show that Pat22 (SEQ. ID NO. 40) forms fibrils without β-sheet that form µm-sized aggregates that cannot interact with HeLa cells, thus are not potent enhancers of HIV-1 infection. The data show that Pat41 (SEQ. ID NO. 41) form no β-sheet rich fibrils that cannot form µm-sized aggregates that cannot interact with HeLa cells, thus are not potent enhancers of HIV-1 infection. Infectivity assay (Figure26A) showed HIV-1 infection rates of TZM-bl cells observed in the presence of increasing concentrations of peptides PA1 (C₁₆-VVVAAAKKK-NH₂, SEQ ID NO. 4), Pat22 (SEQ. ID NO. 40) and Pat41 (SEQ. ID NO. 41) (6.5 µM, 1.3 µM, 0.26 µM). Pat22 (SEQ. ID NO. 40) does not enhance infectivity at 1.3 µM concentration compared to virus only infection. Pat41 (SEQ. ID NO. 41) cannot enhance infectivity at 1.3 µM concentration (same as virus only infection).

Infectivity enhancement and degradability of the PA C₁₆-VVAAHHH-NH₂ (SEQ. ID NO. 42) (Pat24) and of the peptide moiety VVAAHHH-NH₂ (SEQ. ID NO. 43) (Pat43) was investigated. Pat24 (SEQ. ID NO. 42) forms β-sheet containing fibrils (70% β-sheet) that form µm-sized aggregates that can interact with HeLa cells, thus are potent enhancers of HIV-1 infection. They cannot efficiently be degraded in presence of HeLa cells within 3 days. Pat43 (SEQ. ID NO. 43) form no β-sheet rich fibrils that cannot form µm-sized aggregates that cannot interact with HeLa cells, thus are not potent enhancers of HIV-1 infection. Infectivity assay (Figure27A) showed HIV-1 infection rates of TZM-bl cells observed in the presence of increasing concentrations of peptides PA1 (C₁₆-VVVAAAKKK-NH₂, SEQ ID NO. 4), Pat24 (SEQ. ID NO. 42) and Pat43 (SEQ. ID NO. 43) (6.5 µM, 1.3 µM, 0.26 µM). Pat24 (SEQ. ID NO. 42) does not enhance infectivity at 1.3 µM concentration compared to virus only infection.

Infectivity enhancement and degradability of the PA C₁₆-KGVPGVGK-NH₂ (Pat25) (SEQ. ID NO. 44) and of the peptide moiety KGVPGVGK-NH₂ (Pat44) (SEQ. ID NO. 45) was investigated. Pat25 (SEQ. ID NO. 44) forms no β-sheet containing fibrils that cannot form µm-sized aggregates that cannot interact with HeLa cells, thus are not potent enhancers of HIV-1 infection. Pat44 (SEQ. ID NO. 45) form β-sheet containing fibrils that cannot form µm-sized aggregates that cannot interact with HeLa cells, thus are not potent enhancers of HIV-1 infection. Infectivity assay (Figure28A) showed HIV-1 infection rates of TZM-bl cells observed in the presence of increasing concentrations of peptides PA1 (C₁₆-VVVAAAKKK-NH₂, SEQ ID NO. 4), Pat25 (SEQ. ID NO. 44) and Pat44 (SEQ. ID NO. 45) (6.5 µM, 1.3 µM, 0.26 µM). Pat25 (SEQ. ID NO. 44) and Pat44 (SEQ. ID NO. 45) do not enhance infectivity at 1.3 µM compared to virus only infection.

Infectivity enhancement and degradability of the PA C₁₆-KYIHQNYTKALAGKLV-NH₂ (Pat26) (SEQ. ID NO. 46) and of the peptide moiety KYIHQNYTKALAGKLV-NH₂ (Pat45) (SEQ. ID NO. 47) was investigated. Pat26 (SEQ. ID NO. 46) forms β-sheet containing fibrils (51% β-sheet) that form µm-sized aggregates that can interact with HeLa cells, thus are potent enhancers of HIV-1 infection. They cannot be degraded in presence of HeLa cells within 3 days. Pat45 (SEQ. ID NO. 47) form no β-sheet rich fibrils that cannot form µm-sized aggregates that cannot interact with HeLa cells, thus are not potent enhancers of HIV-1 infection. Infectivity assay (Figure29A) showed HIV-1 infection rates of TZM-bl cells observed in the presence of increasing concentrations of peptides PA1 (C₁₆-VVVAAAKKK-NH₂, SEQ ID NO. 4), Pat26 (SEQ. ID NO. 46) and Pat45 (SEQ. ID NO. 47) (6.5 µM, 1.3 µM, 0.26 µM). Pat26 (SEQ. ID NO. 46) enhances infectivity at 1.3 µM concentration 5 times more efficient compared to virus only infection.

Infectivity enhancement and degradability of the PA C₁₆-NFYLQVNKINK-NH₂ (Pat27) (SEQ. ID NO. 48) and of the peptide moiety NFYLQVNKINK-NH₂ (Pat46) (SEQ. ID NO. 49) was investigated. Pat27 (SEQ. ID NO. 48) forms β-sheet containing fibrils (60% β-sheet) that form µm-sized aggregates that can interact with HeLa cells, thus are potent enhancers of HIV-1 infection. They cannot be degraded in presence of HeLa cells within 3 days. Pat46 (SEQ. ID NO. 49) form β-sheet rich fibrils that can form µm-sized aggregates that can interact with HeLa cells, thus are potent enhancers of HIV-1 infection. Infectivity assay (Figure30A) showed HIV-1 infection rates of TZM-bl cells observed in the presence of increasing concentrations of peptides PA1 (C₁₆-VVVAAAKKK-NH₂, SEQ ID NO. 4), Pat27 (SEQ. ID NO. 48) and Pat46 (SEQ. ID NO. 49) (6.5 µM, 1.3 µM, 0.26 µM). Pat27 (SEQ. ID NO. 48) enhances infectivity at 1.3 µM concentration 5 times more efficient compared to virus only infection.

Infectivity enhancement and degradability of the PA C₁₆-GGGSSKKK-NH₂ (Pat28) (SEQ. ID NO. 50) of the peptide moiety GGGSSKKK-NH₂ (Pat47) (SEQ. ID NO. 51) was investigated. Pat28 (SEQ. ID NO. 50) forms β-sheet containing fibrils (26% β-sheet) that cannot form µm-sized aggregates and cannot interact with HeLa cells, thus are not potent enhancers of HIV-1 infection. Pat47 (SEQ. ID NO. 51) form no β-sheet rich fibrils that cannot form µm-sized aggregates that cannot interact with HeLa cells, thus are not potent enhancers of HIV-1 infection. Infectivity assay (Figure31A) showed HIV-1 infection rates of TZM-bl cells observed in the presence of increasing concentrations of peptides PA1 (C₁₆-VVVAAAKKK-NH₂, SEQ ID NO. 4), Pat26 (SEQ. ID NO. 50) and Pat47 (SEQ. ID NO. 51) (6.5 µM, 1.3 µM, 0.26 µM). Pat26 (SEQ. ID NO. 50) does not enhance infectivity at 1.3 µM concentration compared to virus only infection.

Infectivity enhancement and degradability of the PA C₁₆-RYYASLRHYLNLVTRQRY-NH₂ (Pat29) (SEQ. ID NO. 51) and of the peptide moiety RYYASLRHYLNLVTRQRY-NH₂ (Pat48) (SEQ. ID NO. 52) was investigated. Pat29 (SEQ. ID NO. 51) forms no β-sheet containing fibrils (4% β-sheet) that form µm-sized aggregates that can interact with HeLa cells, thus are potent enhancers of HIV-1 infection. They cannot be degraded in presence of HeLa cells within 3 days. The data show that Pat48 (SEQ. ID NO. 52) form no β-sheet rich fibrils that cannot form µm-sized aggregates that cannot interact with HeLa cells, thus are not potent enhancers of HIV-1 infection. Infectivity assay (Figure32A) showed HIV-1 infection rates of TZM-bl cells observed in the presence of increasing concentrations of peptides PA1 (C₁₆-VVVAAAKKK-NH₂, SEQ ID NO. 4), Pat29 (SEQ. ID NO. 51) and Pat48 (SEQ. ID NO. 52) (6.5 µM, 1.3 µM, 0.26 µM). Pat29 (SEQ. ID NO. 51) enhances infectivity at 1.3 µM concentration 5 times more efficient compared to virus only infection

Infectivity enhancement and degradability of the PA C₁₆-GKVGSK-NH₂ (Pat30) (SEQ. ID NO. 54) and of the peptide moiety GKVGSK-NH₂ (Pat49) (SEQ. ID NO. 55) was investigated. Pat30 (SEQ. ID NO. 54) forms β-sheet containing fibrils (41% β-sheet) that cannot form a large number of µm-sized aggregates that can interact with HeLa cells, thus are not potent enhancers of HIV-1 infection. Pat49 (SEQ. ID NO. 55) forms no β-sheet rich fibrils that cannot form µm-sized aggregates that cannot interact with HeLa cells, thus are not potent enhancers of HIV-1 infection. Infectivity assay (Figure33A) showed HIV-1 infection rates of TZM-bl cells observed in the presence of increasing concentrations of peptides PA1 (C₁₆-VVVAAAKKK-NH₂, SEQ ID NO. 4), Pat30 (SEQ. ID NO. 54) and Pat49 (SEQ. ID NO. 55) (6.5 µM, 1.3 µM, 0.26 µM). Pat30 (SEQ. ID NO. 54) does not enhance infectivity at 1.3 µM concentration compared to virus only infection

Infectivity enhancement and degradability of the PA C₁₆-KIISFK-NH₂ (Pat32) (SEQ. ID NO. 56) and of the peptide moiety KIISFK-NH₂ (Pat51) (SEQ. ID NO. 57) was investigated. Pat32 (SEQ. ID NO. 56) forms β-sheet containing fibrils (46% β-sheet) that form µm-sized aggregates that can interact with HeLa cells, but are not potent enhancers of HIV-1 infection. They cannot be degraded in presence of HeLa cells within 3 days. Pat51 (SEQ. ID NO. 57) forms no β-sheet rich fibrils that cannot form µm-sized aggregates that cannot interact with HeLa cells, thus are not potent enhancers of HIV-1 infection. Infectivity assay (Figure34A) showed HIV-1 infection rates of TZM-bl cells observed in the presence of increasing concentrations of peptides PA1 (C₁₆-VVVAAAKKK-NH₂, SEQ ID NO. 4), Pat32 (SEQ. ID NO. 56) and Pat51 (SEQ. ID NO. 57) (6.5 µM, 1.3 µM, 0.26 µM). Pat32 (SEQ. ID NO. 56) does not enhances infectivity at 1.3 µM concentration more efficient compared to virus only infection.

Infectivity enhancement and degradability of the PA C₁₆-AAVVHHK-NH₂ (Pat52*) (SEQ. ID NO. 58) and of the peptide moiety AAVVHHK-NH₂ (Pat52) (SEQ. ID NO. 59) was investigated. Pat52* (SEQ. ID NO. 58) forms β-sheet containing fibrils (41% β-sheet) that form µm-sized aggregates that can interact with HeLa cells, thus are potent enhancers of HIV-1 infection. They cannot be degraded in presence of HeLa cells within 3 days. Pat52 (SEQ. ID NO. 59) forms no β-sheet rich fibrils that cannot form µm-sized aggregates that cannot interact with HeLa cells, thus are not potent enhancers of HIV-1 infection. Infectivity assay (Figure35A) showed HIV-1 infection rates of TZM-bl cells observed in the presence of increasing concentrations of peptides PA1 (C₁₆-VVVAAAKKK-NH₂, SEQ ID NO. 4), Pat52* (SEQ. ID NO. 58) and Pat52 (SEQ. ID NO. 59) (6.5 µM, 1.3 µM, 0.26 µM). Pat52* (SEQ. ID NO. 58) enhances infectivity at 1.3 µM concentration 6 times more efficient compared to virus only infection.

Infectivity enhancement and degradability of the PA C₁₆-GGVVRR-NH₂ (Pati*) (SEQ. ID NO. 60) and of the peptide moiety GGVVRR-NH₂ (Pati) (SEQ. ID NO. 61) was investigated. Pati* (SEQ. ID NO. 60) forms β-sheet containing fibrils (34% β-sheet) that form few µm-sized aggregates that cannot interact with HeLa cells, thus are not enhancers of HIV-1 infection. Pati (SEQ. ID NO. 61) forms no β-sheet rich fibrils that cannot form µm-sized aggregates that cannot interact with HeLa cells, thus are not potent enhancers of HIV-1 infection. Infectivity assay (Figure36A) showed HIV-1 infection rates of TZM-bl cells observed in the presence of increasing concentrations of peptides PA1 (C₁₆-VVVAAAKKK-NH₂, SEQ ID NO. 4), Pati* (SEQ. ID NO. 60) and Pati (SEQ. ID NO. 61) (right data) (6.5 µM, 1.3 µM, 0.26 µM). Pati* (SEQ. ID NO. 60) does not enhance infectivity at 1.3 µM concentration more efficient compared to virus only infection.

Infectivity enhancement and degradability of the PA C₁₆-AGAGRRR-NH₂ (Patj*) (SEQ. ID NO. 62) and of the peptide moiety AGAGRRR-NH₂ (PatJ) (SEQ. ID NO. 63) was investigated. Patj* (SEQ. ID NO. 62) forms β-sheet containing fibrils (35% β-sheet) that cannot form µm-sized aggregates that cannot interact with HeLa cells, thus are not potent enhancers of HIV-1 infection. Patj (SEQ. ID NO. 63) form no β-sheet rich fibrils that cannot form µm-sized aggregates that cannot interact with HeLa cells, thus are not potent enhancers of HIV-1 infection. Infectivity assay (Figure37A) showed HIV-1 infection rates of TZM-bl cells observed in the presence of increasing concentrations of peptides PA1 (C₁₆-VVVAAAKKK-NH₂, SEQ ID NO. 4), Patj* (SEQ. ID NO. 62) and Patj (SEQ. ID NO. 63) (6.5 µM, 1.3 µM, 0.26 µM). Patj* (SEQ. ID NO. 62) does not enhance infectivity at 1.3 µM concentration more efficient compared to virus only infection.

ATR-IR spectrum of the amide I and II regions of each peptide amphiphile (1 mg/mL in PBS lyophilized) was measured. The β-sheet amount was determined by calculating the integral of the peak at 1630 cm⁻¹ (β-sheet) relative to the integral between 1650-1700 cm⁻¹ (native structure) from the 2^{nd} derivative of the FT-IR spectra. The β-sheet amount of Pat14 (SEQ. ID NO. 32) was determined to be 49%. The β-sheet amount of Pat33 (SEQ. ID NO. 33) was determined to be 45%. The β-sheet amount of Pat16 (SEQ. ID NO. 34) was determined to be 50%. The β-sheet amount of Pat35 (SEQ. ID NO. 35) was determined to be 29%. The β-sheet amount of Pat17 (SEQ. ID NO. 36) was determined to be 38%. The β-sheet amount of Pat36 (SEQ. ID NO. 37) was determined to be 31%. The β-sheet amount of Pat21 (SEQ. ID NO. 38) was determined to be 45%. The β-sheet amount of Pat40 (SEQ. ID NO. 39) was determined to be 29%. The β-sheet amount of Pat22 (SEQ. ID NO. 40) was determined to be 0%. The β-sheet amount of Pat41 (SEQ. ID NO. 41) was determined to be 0%. The β-sheet amount of Pat24 (SEQ. ID NO. 42) was determined to be 70%. The β-sheet amount of Pat43 (SEQ. ID NO. 43) was determined to be 0%. The β-sheet amount of Pat25 (SEQ. ID NO. 44) was determined to be 0%. The β-sheet amount of Pat44 (SEQ. ID NO. 45) was determined to be 22%. The β-sheet amount of Pat26 (SEQ. ID NO. 46) was determined to be 51%. The β-sheet amount of Pat45 (SEQ. ID NO. 47) was determined to be 34%. The β-sheet amount of Pat27 (SEQ. ID NO. 48) was determined to be 60%. The β-sheet amount of Pat46 (SEQ. ID NO. 49) was determined to be 49%. The β-sheet amount of Pat28 (SEQ. ID NO. 50) was determined to be 26%. The β-sheet amount of Pat47 (SEQ. ID NO. 51) was determined to be 10%. The β-sheet amount of Pat29 (SEQ. ID NO. 52) was determined to be 4%. The β-sheet amount of Pat48 (SEQ. ID NO. 53) was determined to be 1%. The β-sheet amount of Pat30 (SEQ. ID NO. 54) was determined to be 41%. The β-sheet amount of Pat49 (SEQ. ID NO. 55) was determined to be 4%. The β-sheet amount of Pat32 (SEQ. ID NO. 56) was determined to be 46%. The β-sheet amount of Pat51 (SEQ. ID NO. 57) was determined to be 38%. The β-sheet amount of Pat52* (SEQ. ID NO. 58) was determined to be 41%. The β-sheet amount of Pat52 (SEQ. ID NO. 59) was determined to be 40%. The β-sheet amount of Pati* (SEQ. ID NO. 60) was determined to be 34%. The β-sheet amount of Pati (SEQ. ID NO. 61) was determined to be 31%. The β-sheet amount of Patj* (SEQ. ID NO. 62) was determined to be 35%. The β-sheet amount of Patj (SEQ. ID NO. 63) was determined to be 0%.

Fluorescence microscopy measurements of each peptide amphiphile (1 mg/mL) stained with 9 µL, 50 µM ThT with a filter setting λₑₘ = 527/30 nm and λₑₓ = 480/40 nm were performed. Fluorescence microscopy measurements showed fluorescent, µm-sized aggregates for Pat14 (SEQ. ID NO. 32), Pat16 (SEQ. ID NO. 34), Pat17 (SEQ. ID NO. 36), Pat21 (SEQ. ID NO. 38), Pat24 (SEQ. ID NO. 42), Pat25 (SEQ. ID NO. 44), Pat26 (SEQ. ID NO. 46), Pat27 (SEQ. ID NO. 48), Pat29 (SEQ. ID NO. 52), Pat32 (SEQ. ID NO. 56), Pat52* (SEQ. ID NO. 58), and Patj* (SEQ. ID NO. 62, but also for peptide Pat46 (SEQ. ID NO. 49). Fluorescence microscopy measurements showed less fluorescent, µm-sized aggregates for Pat30 (SEQ. ID NO. 54) and some few fluorescent, µm-sized aggregates for Pati* (SEQ. ID NO. 60). Fluorescence microscopy measurements showed no fluorescent, µm-sized aggregates for Pat33 (SEQ. ID NO. 33), Pat35 (SEQ. ID NO. 35), Pat36 (SEQ. ID NO. 37), Pat40 (SEQ. ID NO. 39), Pat43 (SEQ. ID NO. 43), Pat45 (SEQ. ID NO. 47), Pat28 (SEQ. ID NO. 50), Pat47 (SEQ. ID NO. 51), Pat48 (SEQ. ID NO. 53), Pat49 (SEQ. ID NO. 55), Pat51 (SEQ. ID NO. 57), Pat52 (SEQ. ID NO. 59), Pati (SEQ. ID NO. 61) and Patj (SEQ. ID NO. 63).

TEM micrographs of Pat14 (SEQ. ID NO. 32) revealed the fibrillar morphologies of PA assembly (1 mg/mL in PBS) (Figure 22B). TEM micrographs of Pat33 SEQ. ID NO. 33) revealed no fibrillar morphologies but amorphous aggregates of the peptide (1 mg/mL in PBS) (not shown). TEM micrographs of Pat16 (SEQ. ID NO. 34) revealed the fibrillar morphologies of PA assembly (1 mg/mL in PBS) (Figure 23B). TEM micrographs of Pat35 (SEQ. ID NO. 35) revealed no fibrillar morphologies but amorphous aggregates of the peptide (1 mg/mL in PBS) (not shown). TEM micrographs of Pat17 (SEQ. ID NO. 36) revealed amorphous aggregate morphology of PA assembly (1 mg/mL in PBS) (Figure 24B). TEM micrographs of Pat36 (SEQ. ID NO. 37) revealed no fibrillar morphologies but amorphous aggregates of the peptide (1 mg/mL in PBS) (not shown). TEM micrographs of Pat21 (SEQ. ID NO. 38) revealed fibrillar morphologies of PA assembly (1 mg/mL in PBS) (Figure 25B). TEM micrographs of Pat40 (SEQ. ID NO. 39) revealed no fibrillar morphologies but amorphous aggregates of the peptide (1 mg/mL in PBS) (not shown). TEM micrographs of Pat22 (SEQ. ID NO. 40) (Figure 26B) revealed fibrillar morphologies but amorphous aggregates of the corresponding peptide Pat41 (SEQ. ID NO. 41). Representative TEM micrographs of Pat24 (SEQ. ID NO. 42) revealed the fibrillar morphologies of PA assembly (1 mg/mL in PBS) (Figure 27B). TEM micrographs of Pat43 (SEQ. ID NO. 43) revealed no fibrillar morphologies of the peptide (1 mg/mL in PBS) (not shown). TEM micrographs of Pat25 (SEQ. ID NO. 44) revealed no fibrillar morphologies of PA assembly (1 mg/mL in PBS) (Figure 28B). TEM micrographs of Pat44 (SEQ. ID NO. 45) revealed fibrillar morphologies of the peptide (1 mg/mL in PBS) (not shown). TEM micrographs Pat26 (SEQ. ID NO. 46) revealed the fibrillar morphologies of PA assembly (1 mg/mL in PBS) (Figure 29B). TEM micrographs of Pat45 (SEQ. ID NO. 47) revealed no fibrillar morphologies of the peptide (1 mg/mL in PBS) (not shown). TEM micrographs of Pat27 (SEQ. ID NO. 48) revealed the fibrillar morphologies of PA assembly (1 mg/mL in PBS) (Figure 30B). TEM micrographs of Pat46 (SEQ. ID NO. 49) revealed fibrillar morphologies of the peptide (1 mg/mL in PBS) (not shown). TEM micrographs of Pat28 (SEQ. ID NO. 50) revealed the fibrillar morphologies of PA assembly (1 mg/mL in PBS) (Figure 31B). TEM micrographs of Pat47 (SEQ. ID NO. 51) revealed no fibrillar morphologies of the peptide (1 mg/mL in PBS) (not shown). TEM micrographs of Pat29 (SEQ. ID NO. 52) revealed the fibrillar morphologies of PA assembly (1 mg/mL in PBS) (Figure 32B). TEM micrographs of Pat48 (SEQ. ID NO. 53) revealed fibrillar morphologies of the peptide (1 mg/mL in PBS) (not shown). TEM micrographs of Pat30 (SEQ. ID NO. 54) revealed the fibrillar morphologies of PA assembly (1 mg/mL in PBS) (Figure 33B). TEM micrographs of Pat49 (SEQ. ID NO. 55) revealed no fibrillar morphologies of the peptide (1 mg/mL in PBS) (not shown). TEM micrographs of Pat32 (SEQ. ID NO. 56) revealed the fibrillar morphologies of PA assembly (1 mg/mL in PBS) (Figure 34B). TEM micrographs of Pat51 (SEQ. ID NO. 57) revealed no fibrillar morphologies of the peptide (1 mg/mL in PBS) (not shown). TEM micrographs of Pat52* (SEQ. ID NO. 58) revealed the fibrillar morphologies of PA assembly (1 mg/mL in PBS) (Figure 35B). TEM micrographs of Pat52 (SEQ. ID NO. 59) revealed no fibrillar morphologies of the peptide (1 mg/mL in PBS) (not shown). TEM micrographs of Pati* (SEQ. ID NO. 60) revealed the fibrillar morphologies of PA assembly (1 mg/mL in PBS) (Figure 36B). TEM micrographs of Pati (SEQ. ID NO. 61) revealed no fibrillar morphologies of the peptide (1 mg/mL in PBS) (not shown). TEM micrographs of Patj* (SEQ. ID NO. 62) revealed the fibrillar morphologies of PA assembly (1 mg/mL in PBS) (Figure 37B). TEM micrographs of Patj (SEQ. ID NO. 63) revealed no fibrillar morphologies of the peptide (1 mg/mL in PBS) (not shown).

Laser scanning microscopy images of Proteostat stained PAs and HeLa cells revealed formation of µm-sized PA clusters for Pat14 (SEQ. ID NO. 32), for Pat16 (SEQ. ID NO. 34) and no formation of µm-sized PA clusters for Pat17 (SEQ. ID NO. 36). The peptide amphihile (2 µg) was added to cells (20.000 cells per well) for 30 min, washed and samples were analyzed (d0). Pat14 (SEQ. ID NO. 32) revealed association with cellular membranes After 3 days of incubation (d3) less aggregates are observed, which reveals the degradability of each peptide amphiphile. Pat17 (SEQ. ID NO. 36) revealed no association with cellular membranes. Pat21 (SEQ. ID NO. 38) revealed association with cellular membranes. After 3 days of incubation (d3) more but smaller aggregates are observed, which reveals that Pat21 (SEQ. ID NO. 38) is not efficiently degradable. Pat24 (SEQ. ID NO. 42) revealed association with cellular membranes. After 3 days of incubation (d3) larger aggregates are observed, which reveals that Pat24 (SEQ. ID NO. 42) is not efficiently degradable. Pat25 (SEQ. ID NO. 44) revealed no association with cellular membranes. Pat26 (SEQ. ID NO. 46) revealed association with cellular membranes. After 3 days of incubation (d3) more aggregates are observed, which reveals that Pat26 (SEQ. ID NO. 46) is not efficiently degradable. Pat27 (SEQ. ID NO. 48) revealed association with cellular membranes. After 3 days of incubation (d3) more but slightly smaller aggregates are observed, which reveals that Pat27 (SEQ. ID NO. 48) is not efficiently degradable. Pat46 (SEQ. ID NO. 49) revealed association with cellular membranes. After 3 days of incubation (d3) no significant change in the aggregates are observed, which reveals that Pat46 (SEQ. ID NO. 49) is not efficiently degradable. Pat28 (SEQ. ID NO. 50) revealed no association with cellular membranes. Pat29 (SEQ. ID NO. 52) revealed association with cellular membranes. After 3 days of incubation (d3) less but larger aggregates are observed, which reveals that Pat29 (SEQ. ID NO. 52) is not efficiently degradable. Pat30 (SEQ. ID NO. 54) revealed no association with cellular membranes. Pat32 (SEQ. ID NO. 56) revealed association with cellular membranes. After 3 days of incubation (d3) more but slightly smaller aggregates are observed, which reveals that Pat32 (SEQ. ID NO. 56) is not efficiently degradable. Pat52* (SEQ. ID NO. 58) revealed association with cellular membranes. After 3 days of incubation (d3) more but smaller aggregates are observed, which reveals that Pat52* (SEQ. ID NO. 58) is not efficiently degradable. Pati* (SEQ. ID NO. 60) revealed no association with cellular membranes. Patj* (SEQ. ID NO. 62) revealed no association with cellular membranes.

Quantification of degradability was performed by analyzing Proteostat stained aggregates via the 3D objects counter function of ImageJ 1.53f51 for 3 individual measurements (area 1008 µm × 1008 µm). Box plot showing size distribution and bar plot showing number of aggregates > 10 µm² in an area of approximately 1008 µm × 1008 µm in cell culture after 30 min and 3 d incubation (Figure 16). Aggregate size decreases of Pat14 (SEQ. ID NO. 32) from 71 to 30 µm² but aggregate number increases 116%, characterizing Pat14 (SEQ. ID NO. 32) as a non-degradable PA. Median aggregate size of Pat16 (SEQ. ID NO. 34) stays similar from 25 to 28 µm² with less very large aggregates (>100 µm²) and aggregate number increases 83%, characterizing Pat16 (SEQ. ID NO. 34) as a non-degradable PA. Median aggregate size of Pat21 (SEQ. ID NO. 38) stays similar from 39 to 47 µm² and aggregate number decreases 84%, characterizing Pat21 (SEQ. ID NO. 38) as a degradable PA that is not an efficient enhancer of viral infectivity. Pat22 (SEQ. ID NO. 40) and Pat41 (SEQ. ID NO. 41) revealed no association with cellular membranes. Median aggregate size of Pat24 (SEQ. ID NO. 42) increases in size from 169 to 283 µm² and aggregate number decreases slightly 26%, characterizing Pat24 (SEQ. ID NO. 42) as a non-degradable PA that is also not infectivity enhancing. Median aggregate size of Pat26 (SEQ. ID NO. 46) stays similar from 76 to 80 µm² with less very large aggregates (>100 µm²) and aggregate number increases 110%, characterizing Pat26 (SEQ. ID NO. 46) as a non-degradable PA but infectivity enhancing PA. Median aggregate size of Pat27 (SEQ. ID NO. 48) stays similar from 153 to 87 µm² with less very large aggregates (>100 µm²) and aggregate number increases 123%, characterizing Pat27 (SEQ. ID NO. 48) as a non-degradable PA but infectivity enhancing PA. Median aggregate size of Pat46 (SEQ. ID NO. 49) stays similar from 61 to 44 µm² and aggregate number stays also similar, characterizing Pat46 (SEQ. ID NO. 49) as a non-degradable PA but infectivity enhancing PA. Median aggregate size of Pat29 (SEQ. ID NO. 52) increases from 19 to 45 µm² and aggregate number decreases 43%, indicating further aggregation during in vitro incubation and characterizing Pat29 (SEQ. ID NO. 52) as a non-degradable PA infectivity enhancing PA. Median aggregate size of Pat32 (SEQ. ID NO. 56) stays similar from 56 to 49 µm² with very large aggregates (>100 µm²) and aggregate number increases 84%, characterizing Pat32 (SEQ. ID NO. 56) as a non-degradable PA. Median aggregate size of Pat52* (SEQ. ID NO. 58) stays similar from 71 to 31 µm² with less very large aggregates (>100 µm²) and aggregate number increases 123%, characterizing Pat52* (SEQ. ID NO. 58) as a non-degradable PA but infectivity enhancing PA.

## Claims

1. An *in vitro* method for retroviral gene delivery into cells comprising the following steps:
a) providing fibrils of a self-assembled peptide amphiphile dissolved in an aqueous medium, wherein the fibrils have a diameter between 5 - 20 nm and a length greater than 100 nm, and wherein the fibrils form µm-sized fibrillar aggregates in the area range between 10 µm² to 200 µm² in said aqueous medium, wherein the fibrils of the self-assembled peptide forming the µm-sized fibrillar aggregates amphiphile have a zeta-potential of > 5 mV at pH 7.4, and at 298 K; and
the self-assembling peptide amphiphile consists of:
(i) a non-self-assembling peptide moiety having a positive net charge under physiological pH and having an amino acid sequence that gives rise to ordered β-sheet structures, and
(ii) a hydrophobic moiety covalently coupled to the peptide moiety,
and
b) incubating the cells with the fibrils of the self-assembled peptide amphiphile in the presence of a non-pathogenic viral particle for gene delivery,
wherein said zeta-potential is measurable by dynamic light scattering.

2. The method according to claim 1, wherein the method further comprises the following steps:
a') providing the self-assembling peptide amphiphile; and
a") incubating the self-assembling peptide amphiphile in the aqueous medium to induce fibril formation of the self-assembled peptide amphiphile.

3. The method according to claim 1 or 2, wherein the aqueous medium is water or phosphate buffered saline (PBS).

4. The method according to any one of claims 1 to 3, wherein the cells are selected from the group comprising or consisting of T cells, CD4+ T cells, CD8+ T cells, TZM-bl cells, HeLa cells, and Jurkat cells.

5. The method according to any one of claims 1 to 4, wherein the self-assembling peptide amphiphile consists of:
(i) a non-self-assembling peptide moiety having a positive net charge under physiological pH, wherein the peptide moiety consists of at least 5 amino acids, wherein less than 50 % of the at least 5 amino acids are lysine, arginine, and histidine; and
wherein the non-self-assembling peptide moiety further comprises:
(ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
(ib) a β-sheet forming second peptide part consisting of at least 3 amino acids, wherein a first end of the β-sheet forming second peptide part is covalently coupled to the first amino acid of the first peptide part;
and
(ii) a hydrophobic moiety covalently coupled to the second end of the β-sheet forming second peptide part, wherein the hydrophobic moiety is selected from the group comprising or consisting of a C₁₄-C₂₄-fatty acid.

6. A self-assembling peptide amphiphile consisting of:
(i) a non-self-assembling peptide moiety having a positive net charge under physiological pH, wherein the peptide moiety consists of at least 5 amino acids, wherein less than 50 % of the at least 5 amino acids are lysine, arginine, and histidine;
wherein the non-self-assembling peptide moiety further comprises:
(ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
(ib) a β-sheet forming second peptide part consisting of at least 3 amino acids, wherein a first end of the β-sheet forming second peptide part is covalently coupled to the first amino acid of the first peptide part;
and
(ii) a hydrophobic moiety covalently coupled to the second end of the β-sheet forming second peptide part, wherein the hydrophobic moiety is selected from the group comprising or consisting of a C₁₄-C₂₄-fatty acid, and
wherein the β-sheet forming second peptide part comprises at least 55 % amino acids selected from alanine, valine, leucine, isoleucine, and glycine, and further comprises maximum 45 % glycine; and
wherein the hydrophobic moiety is a C₂₀-C₂₂-all-cis-omega-3 methylene-interrupted fatty acid or a C₂₀-C₂₂-all-cis-omega-6 methylene-interrupted fatty acid, and
wherein the peptide amphiphile self-assembles into fibrils having a diameter between 5 - 20 nm and a length greater than 100 nm in aqueous medium, and wherein the fibrils form µm-sized fibrillar aggregates in the area range between 10 µm² to 200 µm² in said aqueous medium, wherein the fibrils of the self-assembled peptide forming the µm-sized fibrillar aggregates amphiphile have a zeta-potential of > 5 mV at pH 7.4, and at 298K, wherein said zeta-potential is measurable by dynamic light scattering.

7. The self-assembling peptide amphiphile according to claim 6, wherein the second peptide part has the peptide sequence LNGGV (SEQ ID NO 13), AKAVG (SEQ ID NO 65), ALAAG (SEQ ID NO 64), or VWAAA (SEQ ID NO 74).

8. The self-assembling peptide amphiphile of claim 6, wherein the amino acid at the second end of the second peptide part is selected from the group comprising or consisting of cysteine, serine, threonine, and lysine, and wherein the hydrophobic moiety is covalently coupled to the side chain of said amino acid through a thioester linkage, an ester linkage or amide linkage.

9. The self-assembling peptide amphiphile according to claim 8, wherein the second peptide part has the peptide sequence CLNGGV (SEQ ID NO 68), CAKAVG (SEQ ID NO 72), CALAAG (SEQ ID NO 70), CVWAAA (SEQ ID NO 66), SLNGGV (SEQ ID NO 69), SAKAVG (SEQ ID NO 73), SALAAG (SEQ ID NO 71), or SVWAAA (SEQ ID NO 67).

10. A self-assembling peptide amphiphile consisting of:
(i) a non-self-assembling peptide moiety having a positive net charge under physiological pH, wherein the peptide moiety consists of at least 5 amino acids, wherein less than 50 % of the at least 5 amino acids are lysine, arginine, and histidine;
wherein the non-self-assembling peptide moiety further comprises:
(ia) a first peptide part consisting of 1 to 3 amino acids having a first amino acid and a terminal amino acid, wherein the first amino acid is selected from lysine, arginine, and histidine, and wherein at least one of the 1 to 3 amino acids is selected from lysine or lysine amide; and
(ib) a β-sheet forming second peptide part consisting of at least 3 amino acids, wherein a first end of the β-sheet forming second peptide part is covalently coupled to the first amino acid of the first peptide part;
and
(ii) a hydrophobic moiety covalently coupled to the second end of the β-sheet forming second peptide part, wherein the hydrophobic moiety is selected from the group comprising or consisting of a C₁₄-C₂₄-fatty acid, and
wherein the β-sheet forming second peptide part comprises at least 55 % amino acids selected from alanine, valine, leucine, isoleucine, and glycine, and further comprises maximum 45 % glycine; and
(a) wherein the β-sheet forming second peptide part comprises at least three different amino acids selected from the group comprising or consisting of alanine, glycine, valine, leucine, and isoleucine, or
(b) wherein the β-sheet forming second peptide part comprises an amino acid selected from asparagine, glutamine, lysine, and wherein the amino acid at second end of the second peptide part is alanine, valine, leucine, or isoleucine; or
(c) wherein the amino acid at the second end of the second peptide part is selected from the group comprising or consisting of cysteine, serine, and threonine, and wherein the hydrophobic moiety is covalently coupled to the side chain of said cysteine, serine, or threonine, through a thioester linkage, or an ester linkage;
and
wherein the peptide amphiphile self-assembles into fibrils having a diameter between 5 - 20 nm and a length greater than 100 nm in aqueous medium, and wherein the fibrils form µm-sized fibrillar aggregates in the area range between 10 µm² to 200 µm² in said aqueous medium, wherein the fibrils of the self-assembled peptide forming the µm-sized fibrillar aggregates amphiphile have a zeta-potential of > 5 mV at pH 7.4, and at 298K, wherein said zeta-potential is measurable by dynamic light scattering.

11. The self-assembling peptide amphiphile of claim 10, wherein the hydrophobic moiety is a C₂₀-C₂₂-all-cis-omega-3 methylene-interrupted fatty acid or a C₂₀-C₂₂-all-cis-omega-6 methylene-interrupted fatty acid, preferably wherein the hydrophobic moiety is eicosapentaenoic acid.

12. The self-assembling peptide amphiphile of any one of claims 10 or 11, wherein the second peptide part has the peptide sequence LNGGV (SEQ ID NO 13), AKAVG (SEQ ID NO 65), or ALAAG (SEQ ID NO 64).

13. A self-assembling peptide amphiphile selected from
| | |
|---|---|
| C₁₆-*ester*-SVVVAAAKKK-NH₂ | (SEQ ID NO 10), |
| C₁₆-*thioester*-CVVVAAAKKK-NH₂ | (SEQ ID NO 12), |
| eicosapentaene acid-VVVAAAKKK-NH₂ | (SEQ ID NO 14), |
| C₁₆-LNGGVKK-NH₂ | (SEQ ID NO 20), |
| C₁₆-ALAAGKK-NH₂ | (SEQ ID NO 22), |
| C₁₆-AKAVGK-NH₂ | (SEQ ID NO 24), |
| C₁₆-IIQKIK-NH₂ | (SEQ ID NO 26), |
| C₁₆-AGGRVK-NH₂ | (SEQ ID NO 28), and |
| C₁₆-LLLKK-NH₂ | (SEQ ID NO 30). |

14. A composition comprising a peptide amphiphile according to any one of claims 6 to 13 in aqueous medium, optionally comprising a non-pathogenic viral particle, wherein the peptide amphiphile self-assembles into fibrils having a diameter between 5 - 20 nm and a length greater than 100 nm in aqueous medium, and wherein the fibrils form µm-sized fibrillar aggregates in the area range between 10 µm² to 200 µm² in said aqueous medium,wherein the fibrils of the self-assembled peptide forming the µm-sized fibrillar aggregates amphiphile have a zeta-potential of > 5 mV at pH 7.4, and at 298K, wherein said zeta-potential is measurable by dynamic light scattering..

15. Use of a peptide amphiphile as retroviral transduction enhancer for retroviral gene delivery into cells, the self-assembling peptide amphiphile consisting of:
(i) a non-self-assembling peptide moiety having a positive net charge under physiological pH and having an amino acid sequence that gives rise to ordered β-sheet structures, and
(ii) a hydrophobic moiety covalently coupled to the peptide moiety,
wherein the peptide amphiphile self-assembles into fibrils having a diameter between 5 - 20 nm and a length greater than 100 nm in aqueous medium, and wherein the fibrils form µm-sized fibrillar aggregates in the area range between 10 µm² to 200 µm² in said aqueous medium, wherein the fibrils of the self-assembled peptide forming the µm-sized fibrillar aggregates amphiphile have a zeta-potential of > 5 mV at pH 7.4, and at 298K, wherein said zeta-potential is measurable by dynamic light scattering.
